# FASCICULE DE BREVET EUROPEEN

(11) **EP 2 542 239 B1**
(45) Date de publication et mention de la délivrance du brevet: **11.11.2015**
(21) Numéro de dépôt: 11712950.2
(22) Date de dépôt: 01.03.2011
(51) Int. Cl.: A61K 31/4375, A61K 31/4704, A61K 31/4709, C07D 217/22, C07D 217/24, C07D 215/233, A61P 35/00

(54) **UTILISATION D'ISOQUINOLONES POUR LA PREPARATION DE MEDICAMENTS, NOUVELLES ISOQUINOLONES ET LEUR PROCEDE DE SYNTHESE**
VERWENDUNG VON ISOCHINOLONEN ZUR HERSTELLUNG VON ARZNEIMITTELN, NEUE ISOCHINOLONE UND VERFAHREN ZU IHRER SYNTHETISIERUNG
USE OF ISOQUINOLONES FOR PREPARING DRUGS, NOVEL ISOQUINOLONES AND METHOD FOR SYNTHESISING SAME

(30) Priorité: 01.03.2010 FR 1000829
(43) Date de publication de la demande: 09.01.2013
(73) Titulaire: Université Joseph Fourier, 38041 Grenoble Cedex 09 (FR); Centre National de la Recherche Scientifique, 75794 Paris Cedex 16 (FR); Institut Curie, 75248 Paris Cedex 05 (FR)
(72) Inventeur: POPOV, Andrei, F-38340 Voreppe (FR); JUHEM, Aurélie, F-38000 Grenoble (FR); FLORENT, Jean-Claude, F-91190 Gif Sur Yvette (FR); N'GUYEN, Chi-Hung, F-92160 Antony (FR)
(74) Mandataire: Regimbeau
(86) Numéro de dépôt international: PCT/FR2011/050431
(87) Numéro de publication internationale: WO 2011/107709

(56) Documents cités:
- EP-A1- 1 724 262
- EP-A1- 1 854 792
- WO-A1-92/03419
- WO-A1-2010/133647
- WO-A2-2006/058201
- WO-A2-2008/063548
- CHO W-J ET AL: "Molecular Modeling of 3-Arylisoquinoline Antitumor Agents Active Against A-549. A Comparative Molecular Field Analysis Study", BIOORGANIC & MEDICINAL CHEMISTRY, PERGAMON, GB, vol. 10, no. 9, 1 janvier 2002 (2002-01-01), pages 2953-2961, XP002485790, ISSN: 0968-0896, DOI: DOI:10.1016/S0968-0896(02)00137-2
- WON-JEA CHO ET AL: "Synthesis and comparative molecular field analysis (CoMFA) of antitumor 3-arylisoquinoline derivatives", BIOORGANIC & MEDICINAL CHEMISTRY, PERGAMON, GB, vol. 6, 1 janvier 1998 (1998-01-01), pages 2449-2458, XP009143318, ISSN: 0968-0896
- CHO W-J ET AL: "Synthesis and biological evaluation of 3-arylisoquinolines as antitumor agents", BIOORGANIC & MEDICINAL CHEMISTRY LETTERS, PERGAMON, ELSEVIER SCIENCE, GB, vol. 8, no. 1-6, 6 janvier 1998 (1998-01-06), pages 41-46, XP004136619, ISSN: 0960-894X, DOI: DOI:10.1016/S0960-894X(97)10190-1
- BISAGNI E ET AL: "A convenient way to dibenzo[c,h]-1,5-naphthyridines (11-aza-benzo[c] phenanthridines)", TETRAHEDRON, ELSEVIER SCIENCE PUBLISHERS, AMSTERDAM, NL, vol. 52, no. 31, 29 juillet 1996 (1996-07-29), pages 10427-10440, XP004104029, ISSN: 0040-4020, DOI: DOI:10.1016/0040-4020(96)00579-0
- CHO W-J ET AL: "Synthesis of New 3-Arylisoquinolinamines: Effect on Topoisomerase I Inhibition and Cytotoxicity", BIOORGANIC & MEDICINAL CHEMISTRY LETTERS, PERGAMON, ELSEVIER SCIENCE, GB, vol. 13, no. 24, 1 janvier 2003 (2003-01-01), pages 4451-4454, XP002485791, ISSN: 0960-894X, DOI: DOI:10.1016/J.BMCL.2003.09.001
- CHO ET AL: "Design, docking, and synthesis of novel indeno[1,2-c]isoquinolines for the development of antitumor agents as topoisomerase I inhibitors", BIOORGANIC & MEDICINAL CHEMISTRY LETTERS, PERGAMON, vol. 17, no. 13, 1 July 2007 (2007-07-01), pages 3531-3534, XP022114533, ISSN: 0960-894X, DOI: 10.1016/J.BMCL.2007.04.064
- LEE S H ET AL: "Molecular design, synthesis and docking study of benz[b]oxepines and 12-oxobenzo[c]phenanthridinones as topoisomerase 1 inhibitors", BIOORGANIC & MEDICINAL CHEMISTRY LETTERS, PERGAMON, vol. 19, no. 9, 1 May 2009 (2009-05-01), pages 2444-2447, XP026041288, ISSN: 0960-894X, DOI: 10.1016/J.BMCL.2009.03.058 [retrieved on 2009-03-18]

## Description

L'invention concerne l'utilisation d'isoquinolones pour la préparation de médicaments, parmi lesquelles de nouvelles isoquinolones.

L'invention concerne également ces nouvelles isoquinolones ainsi que leur procédé de synthèse.

En particulier, l'invention concerne des dérivés isoquinolones utilisés dans le traitement d'angiogénèses pathologiques, et plus particulièrement du cancer.

La chimiothérapie, avec la chirurgie et la radiothérapie, reste une des approches les plus utilisées pour le traitement du cancer. Bien que des dizaines de composés anticancéreux aient été approuvés pour une utilisation clinique, il demeure un besoin constant de nouvelles thérapeutiques plus sélectives, plus efficaces et moins toxiques.

Les médicaments de chimiothérapie sont nombreux et variés tant au niveau de leur mécanisme d'action qu'au niveau de leur cible cellulaire. La majorité de ces médicaments entrent dans les catégories suivantes: agents alkylants, anti-métabolites, alcaloïdes végétaux, inhibiteurs de topoisomérases, antibiotiques antitumoraux et anti-mitotiques. La plupart des composés anti-mitotiques interagissent avec le cytosquelette microtubulaire. Ces composés agissent directement sur la tubuline et/ou les microtubules, soit en dépolymérisant les microtubules, comme le font la vinblastine et la vincristine, soit en les stabilisant comme le font le Taxol et ses dérivés (Jordan MA and Wilson L, Nat Rev Cancer. 2004, v4(4): pp 253-65). Ces molécules, dépolymérisantes ou stabilisantes, agissent principalement en mitose, et leur activité anti-proliférative est due à la suppression de la dynamique « physiologique » des microtubules (Jordan MA et al., Cancer Res. 1991, v51(8): pp 2212-22; Derry WB, Wilson L and Jordan MA, Biochemistry. 1995 ; 34(7): pp 2203-11). L'inhibition de la dynamique microtubulaire, entraîne la formation d'un fuseau aberrant, ce qui maintient le point de contrôle mitotique activé, et induit l'arrêt des cellules en métaphase. L'arrêt mitotique prolongé déclenche la catastrophe mitotique et donc la mort de la cellule (Jordan MA and Wilson L, Nat Rev Cancer. 2004 Apr; v4(4): pp 253-65). Ce mécanisme d'action constitue la base de l'utilisation thérapeutique de ces composés, car les cellules cancéreuses en division rapide sont particulièrement sensibles aux perturbations de la dynamique microtubulaire, et seront donc préférentiellement ciblées par rapport à la plupart des cellules somatiques de l'organisme.

Les composés stabilisants les plus connus sont le Taxol et ses dérivés, et sont largement utilisés pour le traitement des cancers du sein ou de l'ovaire. Parmi les composés déstabilisant les microtubules, on retrouve les vinca-alcaloïdes comme la vinblastine et la vincristine, utilisés préférentiellement pour les tumeurs hématologiques mais aussi pour certaines tumeurs solides comme les cancers du poumon et des testicules.

Aussi, des produits de faibles poids moléculaires, tels que des 2-phényle-4-quinolones (Lee et al., J. Med. Chem., 1994, v37 : pp1126-35 et pp3400-07; 2009, 52, 4883-4891) ou des 1,2,3,4-tetrahydro-2-phényle-4-quinolones (Lee et al., J. Med. Chem., 1998, v41, pp.1155-62) ont également montré des propriétés d'inhibition de la polymérisation de la tubuline (composés anti-tubuline).

Cependant, bien que les molécules anti-tubuline soient efficaces sur un grand nombre de cancers, et largement utilisées en clinique, la neurotoxicité périphérique et les phénomènes de résistance développés par les mammifères en particulier l'homme, démontrent la nécessité de rechercher de nouveaux agents anti-mitotiques agissant sur de nouvelles cibles.

Par « mammifère », il faut comprendre notammment un animal domestique.

Une autre grande classe de médicaments anticancéreux est représentée par les inhibiteurs d'enzymes, tels que le méthotrexate (inhibiteur de la dihydrofolate réductase : DHFR), le fluorouracile (inhibiteur de la thymidylate synthase) le Gleevec et le BMS-354825 (inhibiteurs de l'activité tyrosine kinase de Bcr-Abl), l'erlonitib (inhibiteur du récepteur du facteur de croissance épidermique: l'EGFR), le PALACI-1040 et le PD0325901 (inhibiteurs de la protéine kinase activée par des mitogènes: la MAPKK), le PALA (inhibiteur de l'aspartate transcarbamoylase), le flavopiridol (Kaur G et al., J Natl Cancer Inst. 1992, v84(22): pp 1736-40) et la R-roskovitine (Meijer L et al., Eur J Biochem. 1997, v243(1-2): pp 527-36), inhibiteurs de kinases dépendantes des cyclines.

Parmi les inhibiteurs d'enzymes, le prétraitement *in vitro* par des dérivés benzopyrone inhibiteurs de la poly-ADP ribose polymérase (pADPRT) et en particulier par le composé 5-iodo-6-amino-1,2-benzopyrone (INH₂BP) des cellules endothéliales de vache transformées par E-*ras* et des cellules d'adénocarcinome humain de prostate, DU145, a démontré une activité d'inhibition de la tumorigénicité (de ces cellules prétraitées) dans le modèle de xénogreffe sur les souris nude.

D'autres composés inhibiteurs de la pADPRT, de type isoquinolinone, sont décrits dans une demande internationale WO 98/51307 (Octamer), pour le traitement de l'inflammation et des maladies inflammatoires, de l'arthrite et de l'attaque cardiaque.

La phosphorylation des protéines se définit par le transfert d'un phosphate de haute énergie à partir de molécules d'ATP (adénosine triphosphate) sur une protéine par le biais d'une protéine kinase. Son enlèvement par une protéine phosphatase est appelé déphosphorylation. Plus de 98 % du phosphate attaché aux protéines est lié par le biais d'une sérine ou d'une thréonine (Berndt, Progress in Cell Cycle Res. 2003, (5): pp 497-510).

La phosphorylation réversible des protéines représente le phénomène le plus important pour la régulation rapide des activités des protéines dans les cellules eucaryotes. Elle est impliquée dans la régulation (activation/inactivation) de diverses activités cellulaires, comme par exemple l'expression des gènes, le cycle cellulaire et la différentiation cellulaire.

Les cycles de phosphorylation/déphosphorylation sont assurés par un grand nombre de kinases (518) et de phosphatases (plus de 130) (Alonso et al., Cell. 2004 (117): pp 699-711). Parmi ces dernières, on compte la famille PPP (Phospho-Protéine Phosphatase) comprenant : PP1, PP2A, PP2B, PP4, PP5, PP6 et PP7. Et, au sein de cette famille, les protéines phosphatases PP1 et PP2A sont responsables à elles deux, de plus de 90% de l'activité sérine/thréonine phosphatase intracellulaire (Stewart et al., Bioorg. Med. Chem., 2007 (15) : pp7301-10). PP1 et PP2A sont des enzymes formées d'une sous-unité catalytique associée à une ou plusieurs sous-unités régulatrices. Ces parties régulatrices régulent la partie catalytique et déterminent la localisation intracellulaire de l'holoenzyme et sa spécificité. De cette façon, les sous-unités catalytiques peuvent agir sur un large panel de substrats.

Le domaine catalytique de la protéine phosphatase 1 (PP1C) existe sous trois isoformes : alpha, bêta (aussi nommé delta) et gamma. En mitose, on les retrouve sur le fuseau mitotique, précisément au niveau du centrosome, des kinétochores, des microtubules et des chromosomes (Andreassen PR et al., J Cell Biol. 1998, 141(5):pp 1207-15; Trinkle-Mulcahy L and Lamond AI, Curr Opin Cell Biol. 2006, (6): pp 623-31).

La PP1 participe, par le biais des sous-unités régulatrices contrôlant sa localisation subcellulaire, à de nombreux processus physiologiques tels que la synthèse protéique, la division cellulaire, le métabolisme du glycogène, l'apoptose, le transport du calcium ou encore la contraction musculaire.

De nombreux articles ont montré que la PP1 est impliquée dans la régulation du cycle cellulaire:
(i) PP1 bloque l'entrée en phase S arrêtant les cellules en G1 (Bemdt N et al., Curr Biol 1997, v7: pp 375-86).
(ii) L'activité de PP1 est nécessaire pour l'entrée en phase M (Margolis SS et al., EMBO J. 2003, 22(21): pp 5734-45).
(iii) PP1 est nécessaire à la déphosphorylation de l'histone H3 et s'oppose à l'activité de la kinase Aurora-B (Goto H et al., Genes Cells. 2002, 7(1): pp 11-7).
(iv) PP1 est impliquée dans la cohésion des centrioles formant le centrosome (Meraldi P et Nigg EA, J Cell Sci. 2001, 114 (Pt 20): pp 3749-57).
(v) L'activité PP1 participe au point de contrôle associé au dommage à l'ADN (Tang X et al., 2008, Mol Cell Biol., 28(8): pp 2559-66).
(vi) PP1 est indispensable pour l'inhibition du point de contrôle du fuseau et le désassemblage des kinétochores à la sortie de la phase M (Emanuele MJ et al., J Cell Biol. 2008, 181(2): pp241-54).
(vii) La déphosphorylation des phosphoprotéines par PP1 est indispensable pour la sortie de la phase M (Wu JQ et al. Nature Cell Biol, 2009, v11(5): pp 644-51).
(viii) PP1 joue un rôle à la transition M/G1 en contribuant au réassemblage de l'enveloppe nucléaire à la fin de mitose en déphosphorylant les lamines B (Thompson LJ et al., 1997, J Biol Chem. 1997,272(47): pp 29693-7).

Tous les éléments (i) à (viii) ci-dessus suggèrent qu'une inhibition sélective de PP1 aurait un effet profond sur la régulation du cycle cellulaire et particulièrement au niveau de la phase M. Et, l'utilisation en chimiothérapie de médicaments anti-prolifératifs ayant un effet sur la progression du cycle cellulaire, fait de PP1 une cible de choix pour le développement de nouvelles molécules anti-cancéreuses.

De plus, PP1 est impliquée dans la progression du cancer. En effet, l'analyse de transcrits de 60 lignées cellulaires cancéreuses (NCI-60: http://genome-www.stanford.edu/nci60/) a montré que les différents isoformes de PP1 étaient surexprimés dans les cancers du colon, du sein, du poumon et du système nerveux central (Ross DT et al., 2000; Nat Genet., v24(3): pp 227-35).

De plus, une amplification génique de la bande chromosomique 11q13 est fréquemment observée dans les carcinomes squameux de l'oropharynx (OSCC), et cette amplification génique serait sous contrôle du gène PP1CA (codant pour la sous unité catalytique de PP1, isoforme alpha). D'autres études suggèrent que PP1CA est impliquée directement dans la tumorigénicité et/ou la progression tumorale dans ce type de cancers (Hsu LC et al., Oncogene, 2006, v25 (40): pp 5517-26).
Il existe de nombreuses molécules inhibant l'activité de PP1, la plupart n'étant pas sélectives et ciblant préférentiellement PP2A.

Par exemple, PP1 et PP2A sont inhibées par un grand nombre de toxines naturelles, comme l'acide okadaïque, la calyculine A, la tautomycine, la tautomycétine, ou la fostriécine (McConnell JL and Wadzinski BE, Mol Pharmacol, 2009, v75: pp 1249-61). Parmi ces toxines, la fostriécine présente une forte sélectivité pour PP2A (40 000 fois), alors que la tautomycine et la tautomycétine (qui sont plus spécifiques de PP1) montrent une faible sélectivité pour PP1 par rapport à PP2A (4 fois et 40 fois respectivement). L'acide phosphatidique présente une sélectivité pour PP1 100 fois supérieure par rapport à PP2A, mais cette fenêtre reste serrée pour une étude spécifique de PP1 avec des inhibiteurs (Stewart SG et al., Bioorg. Med. Chem. 2007 (15): pp 7301-10).

La découverte d'un inhibiteur sélectif de PP1 permettrait de séparer les effets de l'inhibition de PP1 de ceux de l'inactivation de PP2A.

Bien que le potentiel des inhibiteurs de protéines phosphatases en tant que médicaments anticancéreux soit reconnu depuis longtemps, seules la fostriécine et la cantharidine ont été évaluées en clinique. La fostriécine, un inhibiteur sélectif de PP2A, a été testée dans un essai clinique de phase I, dans lequel elle s'est avérée inactive en raison de son instabilité structurale (Lê LH et al., Invest New Drugs. 2004, v22(2):pp 159-67). De la même façon, les essais avec la cantharidine (inhibiteur non-sélectif de PP2A et PP1) ont aussi été arrêtés en raison d'une trop forte néphrotoxicité (Honkanen; « Serine/Threonine Protein Phosphatase Inhibitors with Antitumor Activity », in Inhibitors of Protein Kinases and Protein Phosphatases, Berlin : Springer-Verlag, 2005, v167, ISBN 3-540-21242-6: pp 295-317).

L'idée de détruire les néovaisseaux tumoraux pour traiter le cancer, soulevée par J. Denekamp en 1982, a donné naissance à une nouvelle classe de molécules anticancéreuses, les «VDA» pour Vascular Disrupting Agents (Denekamp J, Br J Cancer. 1982, 45(1): pp 136-9; Tozer G et al., Nature Rev. 2005, v5: pp 423-35). Ces composés sont clairement différents des agents anti-angiogéniques qui interfèrent avec l'apparition et la croissance des nouveaux vaisseaux.

Les molécules VDA agissent sur les cellules endothéliales constituant les néovaisseaux tumoraux et provoquent un collapsus du système vasculaire qui produit un arrêt du flux sanguin conduisant rapidement à une nécrose de la masse tumorale.

La plupart des agents de destruction vasculaire (VDA) sont capables de dépolymériser les microtubules des cellules endothéliales constituant les néovaisseaux. Cette dépolymérisation provoque un arrondissement de la cellule et un véritable affaissement du vaisseau, ce qui empêche ainsi le sang de s'écouler.

Parmi les VDA anti-tubuline, on peut citer: ZD6126 (ANG453; AZD6126), CA4 (Combretastatine A-4), CA4P (Zybrestat, OXi2021, Fosbretabulin, Combretastatine A-4 disodium phosphate), BNC105, MN-029 (Denibulin), CYT997, AVE8062 (AC7700, Ombrabulin), NPI-2358 (Diketopiperazine), EPC2407 (MX-116407, MX-2407), TZT-1027 (Auristatin PE, Soblidotin, NSC-654663), MPC-6827 (Azixa), ABT751, Trisenox (Arsenic trioxide), OXi4503 (CA1P), 2-Methoxyestradiol (Panzem, NSC-659853; 2-ME).

Il existe aussi une autre catégorie de VDA qui n'agit pas sur la tubuline, parmi lesquels on retrouve : SU6668 (inhibiteur de VEGFR, PDGFR, FGFR), PTK787 (ZK222584 ; inhibiteur des VEGFR1, 2, 3), ZD6474 (inhibiteur des VEGFR2 et EGFR), et l'Exherin (ADH-1, Adherex; inhibiteur de la N-cadhérine), ASA404 (DMXAA, AS 1404 ; inducteur de cytokines tel que TNF et INF) et FAA (Flavone Acetic Acid, NSC 347512, LM975).

La sélectivité associée à ces agents est principalement due à une sensibilité plus élevée des cellules endothéliales immatures (plus fragiles et plus perméables) formant les parois des nouveaux vaisseaux (Tozer G et al., Nature Reviews. 2006, v5: pp 423-35).

D'une façon importante, souvent les antitumoraux de la classe des VDA sont plus actifs sur les grosses tumeurs à croissance agressive, plutôt que sur les petites tumeurs, probablement parce ces dernières sont moins vascularisées que les grosses néoplasies (Landuyt W., Intl J of Rad Oncol Biol Phys. 2001, v49(2): pp 443-50 ; Sieman DW and Shi W, Sem in Rad Oncol. 2003, v13(1): pp 53-61).

Les VDA semblent donc particulièrement adaptés pour le traitement de l'angiogénèse pathologique. Cette dernière, caractérisée par la formation excessive ou/et anormale de vaisseaux sanguins, est impliquée dans un grand nombre de maladies, surtout les maladies prolifératives, particulièrement dépendantes de la croissance des vaisseaux. Une angiogénèse excessive et/ou anormale est observée dans le cancer, le psoriasis, les maladies infectieuses (les pathogènes peuvent exprimer eux-mêmes des facteurs angiogènes, induire l'expression de tels facteurs par l'hôte, ou peuvent transformer les cellules endothéliales), les maladies autoimmunes (avec une activation des leucocytes et/ou des mastocytes).

De plus, dans le tissu adipeux, l'angiogénèse peut être induite par une alimentation trop grasse (l'utilisation d'inhibiteurs de l'angiogénèse induit une perte de poids).

La néovascularisation rétinienne, cause majeure de cécité, est une complication d'un grand nombre de maladies, incluant la rétinopathie diabétique, la dégénérescence maculaire liée à l'âge, et les occlusions vasculaires.

Un des buts de l'invention est de fournir des inhibiteurs: sélectifs de PP1, possédant de plus une activité VDA, et/ou de dépolymérisation des microtubules, pour la préparation d'un médicament destiné à la prévention ou au traitement d'angiogénèses pathologiques et/ou de tumeurs bégnines ou malignes (cancéreuses), quelle que soit leur origine ou leur taille, dans le cadre d'un traitement de première ligne ou chez des mammifères en particulier l'homme, résistants aux traitements conventionnels.

Un autre but de l'invention est de fournir des compositions pharmaceutiques contenant des inhibiteurs sélectifs de PP1, possédant de plus une activité VDA, et/ou une activité dépolymérisante sur les microtubules, pour la prévention ou le traitement d'angiogénèses pathologiques et/ou de tumeurs bénignes ou malignes.

La présente invention concerne l'utilisation d'au moins un composé de formule générale (I) ci-dessous en tant qu'inhibiteur de protéine phosphatase 1 et/ou de polymérisation de la tubuline, et/ou agent de destruction vasculaire (VDA), pour la préparation d'un médicament destiné à la prévention et/ou au traitement de patients atteints d'angiogénèses pathologiques, en particulier de rétinopathies, de tumeurs bégnines, ou de tumeurs malignes (cancéreuses).

La présente invention revendique les composés, objets de la revendication 1 pour les utilisations revendiquées.

La présente invention décrit l'utilisation d'au moins un composé de formule générale (I) suivante : 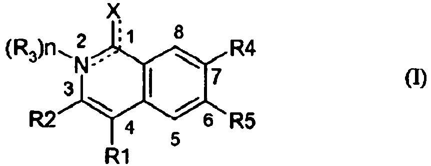 dans laquelle :
1) la liaison entre le carbone 1 et le groupement X est simple ou double, la liaison entre l'azote 2 et le carbone 1 pourra être simple ou double
   étant entendu que :
   a) lorsque la liaison entre X et ledit carbone 1 est double, alors alors la liaison entre l'azote 2 et le carbone 1 est simple: et,
   b) lorsque la liaison entre X et ledit carbone 1 est simple, alors la liaison entre l'azote 2 et le carbone 1 est double et la formule 1 correspond à un système aromatique de formule I-A suivante : 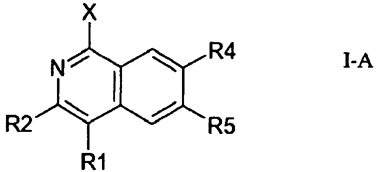
2) n représente 0 ou 1,
3) X représente :
   a) lorsque n =1:
      O, S, NH, N-(C1-C6)alkyle,
   b) lorsque n = 0 :
      OH, OPO₃H₂, OPO-(O-(C₁-C₂)alkyle)₂, l'alkyle étant éventuellement substitué par un ou plusieurs fluors, O-(C₁-C₆)alkyle, en particulier OCH₃, l'alkyle étant éventuellement substitué par un ou plusieurs fluors, O(C₃-C₆)cycloalkyle, NH₂, NH-(C₁-C₆)alkyle, N-[(C₁-C₆)alkyle]₂, NH-(C₃-C₆)cycloalkyle, N-[(C₃-C₆)cycloalkyle]₂, SH, S-(C₁-C₆)alkyle, l'alkyle étant éventuellement substitué par un ou plusieurs fluors, en particulier SCH₃, S(C₃-C₆)cycloalkyle, SO₃H, NH-CH₂-aryl ou heteroaryl, OPh, SPh, OCH₂Ph, SCH₂Ph, le phényle de ces quatre groupes pouvant être substitué ou non par un ou plusieurs (C₁-C₆)alkyle, l'alkyle étant éventuellement substitué par un ou plusieurs fluors, 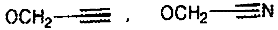
4) R1 représente :
   H, un halogène, OH, OPO₃H₂, OPO-(O-(C₁-C₂)alkyle)₂, l'alkyle étant éventuellement substitué par un ou plusieurs fluors,
   CF₃, CH₂OR', R' représentant H ou (C₁-C₆)alkyle, l'alkyle étant éventuellement substitué par un ou plusieurs fluors, CHO,
   CO₂R", R" représentant H, (C₁-C₆)alkyle, l'alkyle étant éventuellement substitué par un ou plusieurs fluors,
   CONR_{c}R_{d}, CH2SO₂NRR_{d}, CH2NHSO₂R_{c}, R_{c} et R_{d} représentant indépendamment l'un de l'autre : H, un alkyle en C₁-C₆, et NR_{c}R_{d} représente un acide aminé lié par sa fonction amine, en particulier une sérine ou une thréonine, un cycloalkyle en C₃-C₆, ou R_{c} et R_{d} représentent ensemble un alkyle en C₂-C₆,
   NO₂, N₃, ≡, CN, C(=N)NH₂, SO₃H, SO₂NH₂, SO₂NHCH₃, NHSO₂CH₃, CH₂SO₂NR_{c}R_{d}, CH₂NHSO₂Rc, SO₂-NRaRb, SO₂-imidazole,
   NRₐR_{b}, où :
      Rₐ et R_{b} représentent indépendamment l'un de l'autre : H, un alkyle en C₁-C₆, un cycloallcyle en C₃-C₆, ou
   Ra est H, un alkyle en C₁-C₆, un cycloalkyle en C₃-C₆, et Rb = COR_{f}, COOR_{f} ou CONR_{f}R_{f} R_{f} et R_{f} représentant H, un alkyle en C₁-C₆, un cycloalkyle en C₃-C₆ ou une chaîne d'un amino acide (telle que CH(CH₂OH)NH pour la sérine) Rₐ et R_{b} représentent ensemble un alkyle en C₂-C₆,
   Rₐ et R_{b} peuvent former un cycle en C₅ à C₇, notamment une pyrrolidine, une pipérazine, une morpholine, une thiomorpholine,
      un hétéroaryle substitué ou non, en particulier une pyridine, un imidazole, un oxazole, un triazole, un pyrrole, un tétrazole.
5) R2 représente :
   un phényle substitué ou non par un à trois substituants choisis parmi :
      un halogène, un OH, NHRa,
      ORe, Re représentant un benzyle, un triazole méthylène substitué ou non, notamment par un alkyle en C₁-C₆,
      OPO₃H₂, OPO-(O-(C₁-C₂)alkyle)₂, l'alkyle étant éventuellement substitué par un ou plusieurs fluors, un NH₂,
      un groupe NH-CORc dans lequel Rc représente H, un alkyle en C₁-C₆, un cycloalkyle en C₃-C₆ ou une chaîne d'un amino acide (telle que CH(CH₂OH)NH₂ pour la sérine),
      un O-alkyle en C₁-C₆, en particulier OCH₃, l'alkyle étant éventuellement substitué par un ou plusieurs fluors,
      un O-COR1 où R1 représente O-alkyle en C₁-C₆, ou NRiRii, Ri et Rii pouvant être alkyle en C₁-C₆,
      un groupe alkyle en C₂-C₄, le groupe alkyle étant éventuellement substitué par un ou plusieurs fluors, un groupement alcényle en C2-C4 substitué ou non,
      un groupe alcynyle en C₂-C₄ substitué ou non, en particulier par un triméthylsilyle, un tert-butyle, un hydroxy-2-propyle ou un isopropyle
      un hétéroaryle substitué ou non, en particulier une pyridine, un imidazole, un oxazole, un triazole, un pyrrole, un benzofurane, un thiophène, un benzothiophène, un indole,
      un cyclohéxyle, une pipérazine, une morpholine, une thiomorpholine, une pipéridine,
      un groupe 4-(NH₂-(CH₂-CH₂O)p)Ph dans lequel p est un entier de 1 à 6
6) R3 représente :
   H, (C₁-C₆)alkyle, l'alkyle étant éventuellement substitué par un ou plusieurs fluors, (C₃-C₆)cycloalkyle, OH, OPO₃H₂, OPO-(O-(C₁-C₂)alkyle)₂,OSO₃H, SO₃H, O-alkyle en C₁ à C₆, NH₂, NH-(C₁-C₆)alkyle, N-[(C₁-C₆)alkyle]₂, NH-(C₃-C₆)cycloalkyle, N-[(C₃-C₆)cycloalkyle]₂, un groupe propargyle, CH₂CN, (CH₂)ₚOH, p variant de 1 à 6, CH₂OPO₃H₂, CH₂OPO-(O-(C₁-C₂)alkyle)₂, CH₂CH₂F, CH₂CHF₂, CH₂CF₃,
7) R4 et R5 représentent indépendamment l'un de l'autre :
   H, OH, OPO₃H₂, OPO-(O-(C₁-C₂)alkyle)₂, l'alkyle étant éventuellement substitué par un ou plusieurs fluors, un O-alkyle en C₁-C₆, en particulier OCH₃, l'alkyle étant éventuellement substitué par un ou plusieurs fluors, un alkyle en C₁-C₆, l'alkyle étant éventuellement substitué par un ou plusieurs fluors, un cycloalkyle en C₃-C₆, un halogène,
   R4 et R5 représentent ensemble un alcényle (en C₁-C₂) dioxy éventuellement substitué par un ou plusieurs fluors,
   et les sels pharmaceutiquement acceptables,
   en tant qu'agent de destruction vasculaire, pour la préparation d'un médicament destiné à la prévention et au traitement des mammifères, en particulier l'homme, atteints d'angiogénèses pathologiques, en particulier de rétinopathies, ou de tumeurs bénignes ou malignes (cancéreuses).

Dans ce mode de réalisation, la formule générale (I) et les pointillés peuvent donc correspondre à trois structures a), b) et c) respectivement):

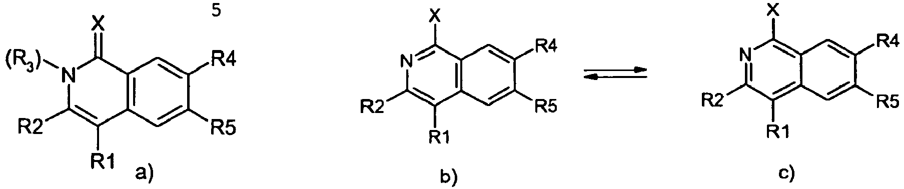

Les différents termes ci-dessous définis ont la même signification dans toute la description :
n = 0 signifie qu'il n'existe pas de substituant R3 et par conséquent, la formule correspond à b) ou c) ci-dessus.
n = 1 signifie qu'il existe un substituant R3 qui peut être soit H soit les autres substituants ci-dessus définis et la formule correspond donc à a) ci-dessus.

Le terme halogène désigne : le brome (Br), le chlore (Cl), le fluor (F) et l'iode (I),

Le terme alkyle désigne des radicaux hydrocarbonés linéaires ou ramifiées qui dérivent des alkanes par perte d'un hydrogène. Sauf précision contraire, ils comportent de 1 à 10 atomes de carbone et comprennent tous les isomères de position possibles.

Le terme cycloalkyle correspond à un alkane cyclisé et comporte de 3 à 10 atomes de carbones.

Un acide aminé lié par sa fonction acide désigne n'importe quel acide aminé, naturel ou non naturel qui est lié par une liaison amidique (NH-CO) entre une amine de la molécule et la fonction acide principale ou de la chaîne latérale pour les acides aminés acides tels que l'acide aspartique ou glutamique de l'acide aminé.

Le terme phényle substitué désigne un groupement phényle qui peut comprendre de 1 à 5 substituants, lesdits substituants pouvant être indépendamment les uns des autres, un halogène, un hydroxy, un thiol, un cyano, un azido, un nitro, un amino éventuellement substitué par un mono-ou di(C₁₋₄)alkyle, un (C₁₋₄)alkyle, un (C₃₋₆)cycloalkyle, un (C₂₋₄)alkényle, un (C₂₋₄)alkynyle, (C₁-₄)alkyloxy ou un phényle.

Le terme hétéroaryle substitué désigne un noyau aromatique comportant au moins un hétéroatome tel que O, N ou S, et qui peut comprendre un ou plusieurs substituants, indépendamment les uns des autres, tel(s) que défini(s) ci-dessus.

Les groupes R₄ et R₅ ci-dessus définis peuvent également représenter un hétéroaryle ou un phényle, substitués éventuellement par les substituants ci-dessus définis, mais également par un groupement phosphate.

De la même manière, le groupe R₁ ci-dessus défini lorsqu'il correspond à un phényle substitué ou non ou un hétéroaryle substitué ou non, peut également être substitué par un groupement phosphate.

L'expression « et les sels pharmaceutiquement acceptables » désigne les sels des molécules de l'invention qui possèdent une fonction salifiable telle que:
- une fonction acide carboxylique, ou phosphorique (OPO₃H) ou sulfonique (SO₃H) ou sulfurique (OSO₃H), ou une fonction phénol, ledit sel étant obtenu avec des bases organiques ou minérales, pour conduire par exemple à des sels des métaux alcalins, comme les sels de lithium, de sodium ou de potassium.
- une fonction amine substituée ou non, ledit sel étant obtenu par réaction d'un acide inorganique, d'un acide organique ou d'un halogénure d'alkyle, sur l'amine pour conduire à un ammonium quaternaire.

Des exemples d'acides inorganiques permettant l'obtention de sels pharmaceutiquement acceptables incluent, sans être limités à ceux-ci, l'acide chlorhydrique, l'acide bromhydrique, l'acide nitrique, l'acide carbonique, l'acide formique, l'acide monohydrogénocarbonique, l'acide phosphorique, l'acide monohydrogénophosphorique, l'acide dihydrogénophosphorique, l'acide perchlorique, l'acide sulfurique, l'acide monohydrogénosulfurique, l'acide iodhydrique.

Des exemples d'acides organiques permettant l'obtention de sels pharmaceutiquement acceptables incluent, sans être limités à ceux-ci, l'acide acétique, l'acide lactique, l'acide propionique, l'acide butyrique, l'acide isobutyrique, l'acide palmique, l'acide maléique, l'acide glutamique, l'acide hydroxymaléique, l'acide malonique, l'acide benzoïque, l'acide succinique, l'acide glycolique, l'acide subérique, l'acide fumarique, l'acide mandélique, l'acide phthalique, l'acide salicylique, l'acide benzènesulfonique, l'acide p-toluènesulfonique, l'acide citrique, l'acide tartrique, l'acide méthanesulfonique, l'acide hydroxynaphthoïque.

Les sels d'acides aminés, tels que les arginates et leurs équivalents sont également inclus ainsi que les sels d'acides organiques tels que l'acide glucuronique ou l'acide galacturonique et leurs équivalents (voir, par exemple, Berge et al, "Pharmaceutical Salts", Journal of Pharmaceutical Science, 1977, 66, 1-19).

Les halogénures d'alkyle permettant l'obtention de sels pharmaceutiquement acceptables incluent, sans être limités à ceux-ci, les bromures, iodure, fluorure ou chlorure d'alkyle dans lesquels ledit résidu alkyle est saturé ou non saturé, linéaire ou ramifié de 1 à 20 atomes de carbone, ou un groupe O-cycloalkyle de 3 à 8 atomes de carbone.

Le terme angiogénèse pathologique (anormale) désigne un processus pathologique consistant en une prolifération, et la persistance ensuite de ladite prolifération, des vaisseaux sanguins dans les tissus "anormaux", par exemple et sans être limité à ceux-ci, dans les tissus tumoraux, ou dans les tissus sains mais "en positions anormales" ou présentant une « morphologie anormale ».

L'expression "traitement de l'angiogénèse pathologique" signifie que les mammifères, en particulier l'homme, souffre d'une angiogénèse/vascularisation pathologique diagnostiquée et va être traité par un médicament pour conduire à la disparition de ladite angiogénèse/vascularisation.

L'expression "prévention de l'angiogénèse pathologique" signifie que le mammifère, en particulier l'homme, n'a pas été diagnostiqué, c'est-à-dire qu'il ne souffre pas d'angiogénèse pathologique, mais est susceptible d'en développer une, et que la prise ou l'administration d'un médicament de l'invention empêche le développement ou l'apparition d'une angiogénèse pathologique.

La prévention de l'angiogénèse pathologique peut être envisagée pour un mammifère, en particulier l'homme, qui souffre déjà d'une angiogénèse pathologique, dans le cadre d'une thérapie qui vise à limiter l'expansion ou la prolongation du processus de l'angiogénèse pathologique.

Ce processus d'angiogénèse pathologique est observé dans de nombreuses pathologies telles que la rétinopathie qui désigne toutes les affections de la rétine, notamment les rétinopathies diabétiques, hypertensives ou pigmentaires, la dégénérescence maculaire, la rubéose, le glaucome néovasculaire, ou la fibroplasie rétrocristallinienne.

L'angiogénèse pathologique est également caractéristique, mais sans être limitée à celles-ci, des lésions de la peau dans le psoriasis ou l'angiogénèse du myocarde, de l'athérosclérose, de l'angiogénèse coronarienne collatérale, de l'angiogénèse cérébrale collatérale, des malformations artérioveineuses, de l'angiogénèse ischémique des membres, de l'angioplastie ou le shunt artérioveineux, du syndrome de Di George, des malformations vasculaires, de la telangiectasie hémorragique héréditaire, de l'hémangiome caverneux ou cutané, des malformations lymphatiques, de l'artériopathie, de la néovascularisation des plaques athéroscléreuses, de l'hémangiome, de l'hémangiopéricytome, de l'hémangio-endothéliome kaposiforme, de l'angiokératome, de l'hémangiome capillaire, du lymphangiome, du neurinome de l'acoustique, des neurofibromes, de l'angiofibrome, du granulome pyogène, des phacomatoses, de la maladie de Rendu-Osler-Weber.

Le terme « tumeur » désigne une néoformation de tissus corporels (« néoplasie »), liée à un dérèglement de la croissance cellulaire de type bénin ou malin.

L'expression «les tumeurs bénignes » désigne les tumeurs, quelque soit leur origine ou leur taille, à croissance lente, bien délimitées, formées d'un tissu homogène avec de cellules bien différenciées, à faible activité mitotique, sans métastases.

L'expression « tumeurs cancéreuses » ou « tumeurs malignes » désigne les tumeurs, quelle que soit leur origine ou leur taille, à croissance rapide et invasive, mal délimitées, formées d'un tissu hétérogène avec des cellules non différenciées qualifiées d'immatures, à division cellulaire importante, souvent avec des métastases.

Les expressions « tumeurs cancéreuses » ou « tumeurs malignes » désignent également sans être limitées à ceux-ci : les tumeurs primaires, les métastases tumorales, les tumeurs solides, les cancers hématopoïétiques, les leucémies, les lymphomes, les carcinomes, les adénocarcinomes, les sarcomes, le mélanome, le carcinome de la tête et du cou, le cancer de l'oesophage, le cancer buccal et du pharynx, le cancer du larynx, le cancer de la vessie, le cancer colorectal, le cancer ovarien, le cancer de l'utérus, le cancer du pénis, le cancer de la vulve et du vagin, le cancer cervical, le cancer de la prostate, le cancer rénal, le cancer de la peau, le cancer de l'os, le cancer des articulations et des cartilages articulaires, le cancer des testicules, le cancer de l'estomac, le cancer gastrointestinal, le cancer genito-urinaire, le cancer du poumon, le thymome, le mésothéliome, le tératome, le cancer du cerveau, le cancer du foie, le cancer du pancréas, le gliome, le glioblastome, l'oligoastrocytome, le méningiome, l'adénome de l'hypophyse, le glioblastome multiforme, le médulloblastome, l'épendymome, l'astrocytome anaplasique, l'oligodendrogliome, le cancer de la thyroide, le cancer anaplasique de la thyroïde, l'hémangiosarcome, le sarcome de Kaposi, le lymphangiosarcome, les lymphomes malins ganglionnaires et extra-ganglionnaires, le lymphome de Hodgkin, les lymphomes non hodgkiniens indolents, le rétinoblastome.

L'un des avantages des molécules de l'invention est de posséder des propriétés de destruction vasculaire provoquant rapidement la nécrose de la tumeur par rupture mécanique du réseau sanguin dans la tumeur. De plus, en provoquant une rupture du flux sanguin, les molécules de l'invention (ainsi que d'autres produits anticancéreux administrés préalablement ou simultanément avec les molécules de l'invention) sont retenues dans la tumeur, plutôt que d'être éliminées par la circulation sanguine ce qui aboutit à une efficacité accrue.

Selon un autre aspect, la présente invention décrit l'utilisation d'au moins un composé de formule générale (I) ci-dessus défini en tant qu'inhibiteur de protéine phosphatase 1.
L'expression « protéine phosphatase 1 » désigne aussi bien le domaine catalytique de la protéine phosphatase 1C (PP1C) sous l'une de ses trois isoformes que la sous unité catalytique de la phosphatase de la chaîne légère de la myosine (MLCP).
L'expression « protéine phosphatase 1 » désigne aussi la protéine phosphatase 1 holoenzyme tant qu'elle contient l'une de ses trois isoformes (alpha, beta/delta et gamma) de la partie catalytique de la protéine phosphatase 1.

Il est connu que les différentes isoformes de PP1 sont surexprimées dans les lignées cellulaires venant de plusieurs types de cancers, tels que le cancer du colon, du sein, du poumon et du SNC (Ross et al., Nature Genet. 2000; v24(3): pp 208-9). De plus, l'amplification génique de la bande chromosomale 11q13 est souvent observée dans le cancer squameux de la bouche. Le gène PPP1CA (sous-unité catalytique de PP1, isoforme alpha) est associé avec cette amplification de 11q13, qui est à son tour est corrélée avec la surexpression de PP1 alpha. La diminution du niveau squameux de la bouche (Hsu et al., Oncongene. 2006, v25(40): pp5517-26). Par conséquent, un inhibiteur sélectif de PP1 permettrait le traitement de ce type de cancer.

Un deuxième avantage des molécules de l'invention est de posséder une inhibition sélective de la protéine phosphatase 1 versus la protéine phosphatase 2A ce qui leur confère un avantage par rapport aux inhibiteurs non-spécifiques en limitant les effets toxiques.

Dans un mode de réalisation avantageux, la présente invention décrit l'utilisation d'au moins un composé de formule générale (I) définie dans la revendication 1, en tant agent de destruction vasculaire, inhibiteur de protéine phosphatase 1, et agent antiprolifératif, pour la préparation d'un médicament destiné à la prévention et au traitement de mammifères, en particulier l'homme, atteints d'angiogénèses pathologiques, choisies parmi les rétinopathies, et les tumeurs bénignes ou malignes (cancéreuses).
Par agent « antiprolifératif » on entend un médicament qui interfère avec la prolifération de cellules quelquesoit la phase du cycle cellulaire affectée par ledit agent.
L'interférence d'un inhibiteur de PPI avec le cycle le cycle cellulaire (comme détaillé ci-dessus) est équivalent à l'effet antiprolifératif.

Un autre avantage des molécules de l'invention est de posséder à la fois des propriétés de destruction vasculaire et une inhibition sélective de la protéine phosphatase 1.

Dans un mode de réalisation avantageux, la présente invention décrit l'utilisation d'au moins un composé de formule générale (I) définie ci-dessus, en tant qu'agent de destruction vasculaire et inhibiteur de protéine phosphatase 1 et agent antiprolifératif, mais dépourvu d'activité inhibitrice de polymérisation de la tubuline. L'activité inhibitrice de polymérisation de la tubuline peut être néfaste dans le cas où les mammifères, en particulier l'homme, sont susceptibles de développer une neuropathie périphérique (Clinical Lung Cancer, Volume 12, Number 1 / January 2011 , pages 18-25).

L'activité inhibitrice de polymérisation de la tubuline peut également provoquer une inhibition de l'hématopoïèse.

Par conséquent, un des avantages de ce genre de composés est de pouvoir cibler une population de mammifères, en particulier l'homme, susceptibles de développer une neuropathie périphérique et sans provoquer l'inhibition de l'hématopoïèse et notamment de pouvoir traiter des mammifères, en particulier l'homme, agés.

Les composés anti-PP1 sont susceptibles de présenter une toxicité moindre ou au moins très différente de celle des agents anti-tubuline, qui provoquent des effets de neurotoxicité périphérique, surtout chez des mammifères, en particulier l'homme, âgés (Phister JE et al., Drugs, 1989, 37[4]pp.551-65 ; Repetto L., J Support Oncol., 2003,1 [4 Suppl2] : ppl8-24).

Dans un mode de réalisation avantageux, la présente invention décrit utilisation d'au moins un composé de formule générale (I) définie dans la revendication 1, en tant qu'agent de destruction vasculaire, inhibiteur de polymérisation de tubuline, et agent antiprolifératif, pour la préparation d'un médicament destiné à la prévention et au traitement de mammifères, en particulier l'homme, atteints d'angiogénèses pathologiques, choisies parmi les rétinopathies, et les tumeurs bénignes ou malignes (cancéreuses).
Dans ce mode de réalisation, le composé de l'invention ne possède pas ou peu d'activité inhibitrice de PP1.

Encore un avantage des molécules de l'invention est qu'elles possèdent la capacité à dépolymériser les microtubules dans les cellules, conférant ainsi aux molécules de l'invention un effet anti-mitotique, permettant ainsi une action d'inhibition de la prolifération cellulaire via un mécanisme indépendant de l'inhibition de PP1.

Dans un mode de réalisation avantageux, la présente invention décrit l'utilisation d'au moins un composé de formule générale (I) définie dans la revendication 1, en tant qu'inhibiteur de protéine phosphatase 1, de polymérisation de la tubuline, agent de destruction vasculaire et agent antiprolifératif, pour la préparation d'un médicament destiné à la prévention et au traitement de mammifères, en particulier l'homme, atteints d'angiogénèses pathologiques, choisies parmi les rétinopathies, et les tumeurs bénignes ou malignes (cancéreuses).

Un autre avantage des molécules de l'invention est qu'elles possèdent, en plus des propriétés de destruction vasculaire et d'inhibition de PP1, ci-dessus décrites, la capacité à dépolymériser les microtubules dans les cellules, conférant ainsi aux molécules de l'invention un effet anti-mitotique.

Les molécules de l'invention, de par leur activité inhibitrice de PP1 et de leur activité de dépolymérisation des microtubules dans les cellules, traduit par un effet antiprolifératif, en plus de leur activité VDA, sont susceptibles d'être actives sur les tumeurs quelle que soit la taille et l'origine de ces tumeurs. La nécrose de la tumeur provoquée par cette triple activité est rapide et s'effectue en 24 heures ou moins permettant de cibler plus particulièrement les grosses tumeurs et permet aussi de cibler des mammifères, en particulier l'homme, non opérables.

La présence des quatre activités (anti-PP1, anti-tubuline et anti-proliférative et VDA) mentionnées ci-dessus confère aux molécules de l'invention des propriétés tout à fait intéressantes et notamment la possibilité de pratiquer une thérapie sur un mammifère, en particulier l'homme, sans qu'il ne développe de résistance.

Un autre avantage des molécules de l'invention, est qu'elles sont actives après l'administration par voie parentérale ou *per os.*

Encore un autre avantage des molécules de l'invention, est celui de ne pas présenter de toxicité *in vivo*, la dose maximum tolérée *per os* pouvant être estimée entre 200 et 400 mg/kg chez la souris nude.

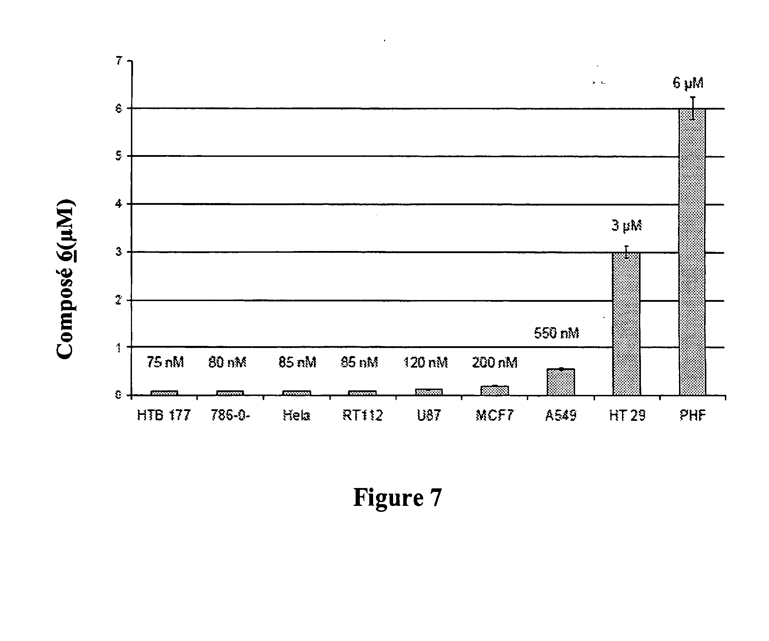

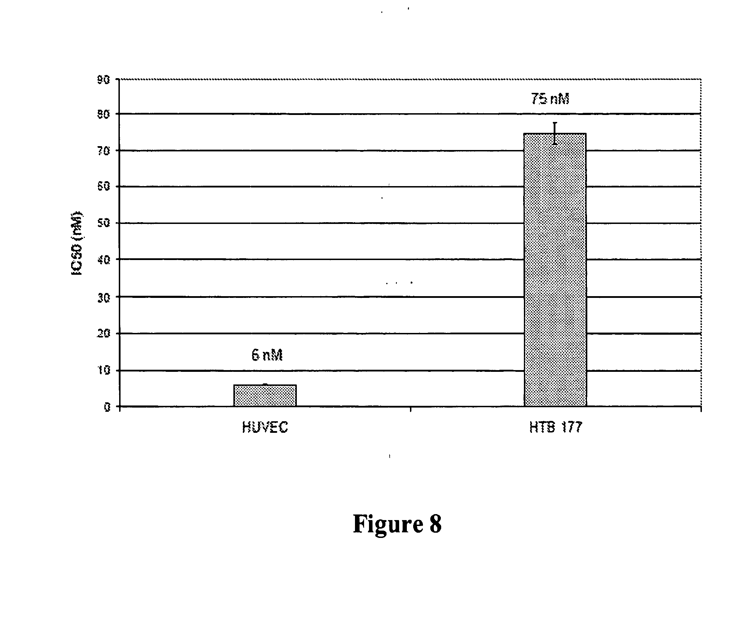

Les composés de l'invention sont très actifs *in vitro* sur les cellules endothéliales de veine de cordon ombilical humain (HUVEC) ainsi que sur les cellules endothéliales humaines microvasculaires de derme (HMEC), avec une concentration inhibant la prolifération cellulaire de moitié (G150) très inférieure à celle des lignées cancéreuses ou à celle des fibroblastes primaires de peau (figures 7 et 8 et tableau I).

Les résultats obtenus avec les cellules HMEC sont représentatifs de l'activité VDA. (Pasquier E., et al., Mol Cancer Ther ; 9(5) 2010. Pp 1408-18: ENMD-1198, a new analogue of 2-methoxyestradiol, displays both antiangiogenic and vascular-disrupting properties).

Les composés de l'invention sont capables de cibler préférentiellement les cellules endothéliales tumorales et de distinguer les cellules endothéliales « matures » des tissus sains, des cellules endothéliales tumorales « jeunes » récemment acheminées dans la tumeur par le flux sanguin, c'est-à-dire d'avoir seulement une action sur les cellules « jeunes » et pas sur les cellules endothéliales « matures ».

Dans un mode de réalisation avantageux, la présente invention décrit également l'utilisation d'au moins un composé de formule (I), dans laquelle :
lorsque l'un au moins des groupes X, R2, R4 et R5 représente un groupement phosphate :
   OPO₃H₂, alkyle phosphate: OPO-(O-(C₁-C₂)alkyle)₂, et/ou
   le groupe R3 représente un groupement CH₂OPO₃H₂ ou CH₂OPO-(O-(C₁-C₂)alkyle)₂, et/ou
   l'un au moins des groupes R1 et R2 représente un peptide ou un acide aminé lié par sa fonction acide, en particulier une sérine.

Dans ce cas, les molécules de l'invention sont considérées comme des prodrogues, c'est-à-dire que la molécule active va être libérée dans l'organisme par hydrolyse à l'aide d'une enzyme, par exemple d'une phosphatase, ou en raison du pH, pour conduire à une fonction OH libre ou CH₂OH, et/ou par hydrolyse à l'aide d'une aminopeptidase pour conduire à une fonction NH₂ libre, après administration de la prodrogue. A noter ici, que l'environnement anormal des vaisseaux tumoraux est associé avec l'expression élevée de la phosphatase alkaline à la surface des cellules endothéliales, ce qui permet la déphosphorylation des prodrogues, tels que la CA4P (Combretastatin A4 Disodium Phosphate).

Dans un mode de réalisation avantageux, l'invention décrit notamment l'utilisation d'au moins un des composés de formule générale (I) définis dans la revendication 1 ou de leurs sels pharmaceutiquement acceptables,
en tant qu'inhibiteur de protéine phosphatase 1 et/ou de polymérisation de la tubuline, et/ou agent de destruction vasculaire et/ou agent antiprolifératif, pour la préparation d'un médicament destiné à la prévention et/ou au traitement de mammifères, en particulier l'homme, atteints de tumeurs bénignes ou malignes (cancéreuses), lesdits mammifères, en particulier l'homme, atteints de tumeurs étant résistants aux traitements conventionnels ou n'étant pas susceptibles de développer une résistance après traitement par ledit médicament.

Par « traitement conventionnel » il faut comprendre tout traitement antitumoral bien connu par l'homme du métier, tels que et sans être limités à ceux-ci : la chirurgie, la radiothérapie, la chimiothérapie, la thérapie dite « ciblée », la thérapie génique, la thérapie par les bioproduits, y compris par les produits pour l'immunisation passive ou active.

Par la « chimiothérapie » il faut comprendre une méthode de traitement par substances chimiques pour traiter une maladie, en particulier les tumeurs. Par « substance chimique pour la chimiothérapie », il faut comprendre les composés suivants et, sans être limités à ceux-ci, tels que le 5-fluorouracile, la gemcitabine, l'hydroxyurée, les agents alkylants, le cisplatine, le paclitaxel, les vinca alkaloïdes tels que la vinblastine ou la vincristine, le methotrexate, les camptothecines, l'étoposide, la daunorubicine et la doxorubicine, et d'autres composés bien connus de l'homme du métier.

Par la « thérapie ciblée » il faut comprendre le produit ou la stratégie thérapeutique avec une stratégie focalisée, qui agit sur une cible bien définie ou une voie de signalisation biologique, qui, une fois inactivée, entraîne la régression ou la destruction du cancer. Par « substance pour la thérapie ciblée » il faut comprendre des substances d'origine chimique ou protéique/peptidique, sans être limités à celles-ci, telles que le mesylate d'imatinib (Gleevec®), le gefinitib (Iressa®), le bevacizumab (Avastin®), le trastuzumab (Herceptin®), le cétuximab (Erbitux®), le tositumomab marqué à l'iode ¹³¹ (Bexxar®), le rituximab (Rituxan®), l'alemtuzumab (Campath®), l'ibritumomab tiuxetan (Zevalin®) ou le gemtuzumab ozogamicin (Mylotarg®); les produits antihormonaux tels que l'anastrozole, le leuprolide, le bicalutamide.

Par la « radiothérapie » il faut comprendre une méthode de traitement locorégional des tumeurs, utilisant des radiations pour détruire les cellules tumorales en bloquant leur capacité à se multiplier.

L'expression « mammifères, en particulier l'homme, ...étant résistants aux traitements conventionnels » désigne des mammifères, en particulier l'homme, qui étaient d'emblée résistants aux traitements conventionnels ou sont devenus résistants aux traitements conventionnels après avoir été traités.

Par exemple, les composés de l'invention peuvent être utilisés pour :
- la prévention de la résistance à la chimiothérapie sur des mammifères, en particulier l'homme, n'ayant jamais été traités par un agent chimiothérapique,
- le traitement de la résistance à la chimiothérapie sur des mammifères, en particulier l'homme, déjà résistants à un agent chimiothérapique,
- le traitement de la résistance à la chimiothérapie sur des mammifères, en particulier l'homme, déjà résistants à un agent chimiothérapique au moyen de la combinaison entre un produit de l'invention et un agent chimiothérapique identique ou différent de celui pour lesquelles les mammifères, en particulier l'homme, sont résistants.

La modulation ou traitement de la chimiorésistance, en particulier de la chimiorésistance au paclitaxel, par les composés de l'invention, en particulier le composé 6, ou de la combinaison entre un composé de l'invention et un agent chimiothérapie identique ou différent de celui pour lesquelles les mammifères, en particulier l'homme, sont résistants, peut être évaluée par le protocole de l'exemple 14.

La présence des trois activités mentionnées ci-dessus confère donc également aux molécules de l'invention la possibilité de cibler des mammifères en particulier l'homme, résistants aux traitements conventionnels.

Dans un mode de réalisation avantageux, la présente invention décrit également l'utilisation d'au moins un composé de formule (I) tel que défini à la revendication 1, en tant qu'inhibiteur de polymérisation de tubuline et/ou agent de destruction vasculaire et agent antiprolifératif" pour la préparation d'un médicament destiné à la prévention et au traitement de mammifères, en particulier l'homme, atteints d'angiogénèses pathologiques, choisies parmi les rétinopathies, et les tumeurs bénignes ou malignes (cancéreuses), lesdits mammifères, en particulier l'homme, atteints de tumeurs bénignes ou malignes étant résistants aux traitements conventionnels ou n'étant pas susceptibles de développer une résistance après traitement par ledit médicament.

Dans un mode de réalisation avantageux, la présente invention décrit également l'utilisation d'au moins un composé de formule (I) tel que défini à la revendication 1, en tant qu'inhibiteur de polymérisation de la tubuline, agent de destruction vasculaire et agent antiprolifératif, pour la préparation d'un médicament destiné à la prévention et au traitement de mammifères, en particulier l'homme, atteints d'angiogénèses pathologiques, choisies parmi les rétinopathies, et les tumeurs bénignes ou malignes (cancéreuses), lesdits mammifères, en particulier l'homme, atteints de tumeurs bénignes ou malignes (cancéreuses) étant résistants aux traitements conventionnels ou n'étant pas susceptibles de développer une résistance après le traitement par ledit médicament.

Dans un mode de réalisation avantageux, la présente invention décrit également l'utilisation d'au moins un composé de formule (I) tel que défini à la revendication 1, en tant qu'inhibiteur de protéine phosphatase 1 et/ou agent de destruction vasculaire et agent antiprolifératif, pour la préparation d'un médicament destiné à la prévention et au traitement des mammifères, en particulier l'homme, atteints d'angiogénèses pathologiques, choisies parmi les rétinopathies, et les tumeurs bénignes ou malignes (cancéreuses), lesdits mammifères, en particulier l'homme, atteints de tumeurs bénignes ou malignes (cancéreuses) étant résistants aux traitements conventionnels ou n'étant pas susceptibles de développer une résistance après le traitement par ledit médicament.

Dans un mode de réalisation avantageux, la présente invention décrit également l'utilisation d'au moins un composé de formule (I) tel que défini dans la revendication 1, en tant qu'inhibiteur de protéine phosphatase 1, agent de destructions vasculaire et agent antiprolifératif, pour la préparation d'un médicament destiné à la prévention et au traitement des mammifères, en particulier l'homme, atteints d'angiogénèses pathologiques, choisies parmi les rétinopathies, et les tumeurs bénignes ou malignes (cancéreuses), lesdits mammifères, en particulier l'homme, atteints de tumeurs bénignes ou malignes (cancéreuses) étant résistants aux traitements conventionnels ou n'étant pas susceptibles de développer une résistance après le traitement par ledit médicament.

Dans un mode de réalisation avantageux, la présente invention décrit également l'utilisation d'au moins un composé de formule (I) tel que défini dans la revendication 1, en tant qu"inhibiteur de protéine phosphatase 1, et/ou de polymérisation de la tubuline et agent antiprolifératif, pour la préparation d'un médicament destiné à la prévention et au traitement de mammifères, en particulier l'homme, atteints d'angiogénèses pathologiques, choisies parmi les rétinopathies, et les tumeurs bénignes ou malignes (cancéreuses), lesdits mammifères, en particulier l'homme, atteints de tumeurs bénignes ou maligne (cancéreuses) étant résistants aux traitements conventionnels ou n'étant pas susceptibles de développer une résistance après le traitement par ledit médicament.

Dans un mode de réalisation avantageux, la présente invention décrit également l'utilisation d'au moins un composé de formule (I) tel que défini dans la revendication 1, en tant qu'inhibiteur de protéine de phosphatase 1, de polymérisation de la tubuline et agent antiprolifératif, pour la préparation d'un médicament destiné à la prévention et au traitement de mammifères, en particulier l'homme, atteints d'angiogénèses pathologiques, choisies parmi les rétinopathies, et les tumeurs bénignes ou malignes (cancéreuses), lesdits mammifères, en particulier l'homme, atteints de tumeurs bénignes ou malignes (cancéreuses) étant résistants aux traitements conventionnels ou n'étant pas susceptibles de développer une résistance après le traitement par ledit médicament.

Dans un mode de réalisation avantageux, la présente invention décrit également l'utilisation d'au moins un composé de formule (I) tel que défini dans la revendication 1, en tant qu'inhibiteur de protéine de phosphatase 1, de polymérisation de la tubuline et agent de destruction vasculaire et agent antiprolifératif, pour la préparation d'un médicament destiné à la prévention et au traitement de mammifères, en particulier l'homme, atteints d'angiogénèses pathologiques, choisies parmi les rétinopathies, et les tumeurs bénignes ou malignes (cancéreuses), lesdits mammifères, en particulier l'homme, atteints de tumeurs bénignes ou malignes (cancéreuses) étant résistants aux traitements conventionnels ou n'étant pas susceptibles de développer une résistance après le traitement par ledit médicament

Dans un mode de réalisation avantageux, la présente invention décrit l'une des utilisation ci-dessus définies d'au moins un composé de formule générale (I) tel que défini à la revendication 1, pour la préparation d'un médicament destiné à la prévention et au traitement de mammifères, en particulier l'homme, atteints d'angiogénèses pathologiques, choisies parmi les rétinopathies, et les tumeurs bénignes ou malignes (cancéreuses), quelle que soit leur taille ou leur origine.

Dans un mode de réalisation avantageux, les molécules définies dans la revendication 1 sont utilisées dans la préparation d'un médicament destiné à la prévention et/ou au traitement de mammifères, en particulier l'homme, atteints de tumeurs bénignes ou malignes (cancéreuses) choisies parmi les suivantes : carcinome de poumon à larges cellules, carcinome de cellules rénales, adénocarcinome épithélial de l'utérus, carcinome de la prostate, glioblastome, carcinome du sein, carcinome colorectal et adénocarcinome colorectal.

Dans un mode de réalisation avantageux, la présente invention décrit l'utilisation d'au moins un composé de formule (I) définie dans la revendication 1, dans laquelle le groupe R1 de la formule générale (I) est choisi parmi, NO₂ ou pyrrole.

Les composés de ce mode de réalisation possèdent essentiellement une activité VDA et sont inhibiteurs de PP1.

Dans un mode de réalisation avantageux, la présente invention décrit l'utilisation d'au moins un composé de formule (I) définie dans la revendication 1, dans laquelle le groupe R1 de la formule générale (1) est tel que défini ci-dessus, à l'exclusion de NO2 et/ou R2 correspond à un phényle substitué.

Les substituants sont choisis par exemple, sans être limité à ceux-ci, un OH, OPO₃H₂, un alkyle en C₂-C₃, un O-alkyle en C₁-C₃.

Les composés de ce mode de réalisation possèdent une activité VDA, ne sont pas ou peu inhibiteurs de PP1 et possèdent une activité inhibitrice de tubuline.
Par l'expression «pas ou peu inhibiteurs de PP1 », il faut comprendre une IC50≥ 50µM telle que mesurée par la méthode décrite dans l'exemple 9.

Dans un mode de réalisation avantageux, la présente invention décrit l'utilisation d'au moins un composé de formule (I) définie dans la revendication 1, dans laquelle le groupe R2 de la formule générale (I) représente notamment un phényle substitué en position para.

Les substituants sont choisis parmi ORe, Re étant tel que défini dans la revendication 1, un halogène, un groupe alcynyle en C₂-C₄ substitué ou non, en particulier par un triméthylsilyle, un tert-butyle, un hydroxy-2-propyle ou un isopropyle.

Les composés de ce mode de réalisation possèdent essentiellement une activité VDA, ne sont pas ou peu inhibiteurs de PP1 et ne possèdent pas ou peu d'activité inhibitrice de tubuline.

Par l'expression « pas ou peu d'activité inhibitrice de tubuline », on entend un pourcentage d'inhibition inférieur à 5%, tel que déterminé par le test de l'example 6.

Dans un mode de réalisation avantageux, la présente invention décrit l'utilisation d'au moins un composé de formule (I) définie dans la revendication 1, en tant qu'inhibiteur de polymérisation de tubuline, agent de destruction vasculaire et agent antiprolifératif, ou en tant qu'inhibiteur de protéine phosphatase 1, de polymérisation de la tubuline, agent de destruction vasculaire et agent antiprolifératif, dans laquelle le groupe R2 de la formule générale (I) représente un phényle substitué en position para par un groupement choisi parmi ORe, Re étant tel que défini ci-dessus, un halogène, un groupe alcyne en C2-C4 substitué ou non, en particulier par un triméthylsilyle, un tert-butyle, un hydroxy-2-propyle ou un isopropyle.

Les composés de ce mode de réalisation possèdent essentiellement une activité VDA et possèdent une activité inhibitrice de tubuline.

Dans un mode de réalisation avantageux, la présente invention décrit l'utilisation d'au moins un composé de formule (I) ci-dessus définie, en tant qu'agent de destruction vasculaire, ou en tant qu'inhibiteur de protéine phosphatase 1 et agent de destruction vasculaire et agent antiprolifératif, ou en tant qu'inhibiteur de polymérisation de la tubuline, agent de destruction vasculaire et agent antiprolifératif,
dans laquelle le groupe R1 de la formule générale (I) est choisi parmi , NO2 ou pyrrole et le groupe R2 de la formule générale (I) représente un phényle substitué par un groupement à fort encombrement stérique.

Les composés de ce mode de réalisation appartiennent à deux catgories de composés :
ceux possédant essentiellement une activité VDA, qui sont inhibiteurs de PP1 et possédant ou pas une activité inhibitrice de tubuline, et ceux possédant essentiellement une activité VDA, qui ne sont pas ou peu inhibiteurs de PP1 et ne possédant pas ou peu d'activité inhibitrice de tubuline.

Dans un mode de réalisation avantageux, la présente invention décrit l'utilisation d'au moins un composé de formule (I) définie dans la revendication 1,
en tant qu'agent de destruction vasculaire, ou
en tant qu'inhibiteur de protéine phosphatase 1 et agent de destruction vasculaire et agent antiprolifératif, ou
en tant qu'inhibiteur de polymérisation de la tubuline, agent de destruction vasculaire et agent antiprolifératif, ou
en tant qu'inhibiteur de protéine phosphatase 1, de polymérisation de la tubuline, agent de destruction vasculaire et agent antiprolifératif,
dans laquelle le groupe R1 de la formule générale (I) est choisi parmi, NO₂ ou pyrrole et le groupe R2 de la formule générale (I) représente un phényle substitué en position para par un groupement choisi parmi ORe, Re étant tel que défini ci-dessus, un halogène, un groupe alcyne en C₂-C₄ substitué ou non, en particulier par un triméthylsilyle, un tert-butyle, un hydroxy-2-propyle ou un isopropyle

Les composés de ce mode de réalisation appartiennent à deux catégories de composés :
ceux possédant essentiellement une activité VDA, qui sont inhibiteurs de PP1 et possédant ou pas une activité inhibitrice de tubuline, et
ceux possédant essentiellement une activité VDA, qui ne sont pas inhibiteurs de PP1 et possédant une activité inhibitrice de tubuline.

Dans un mode de réalisation avantageux, la présente invention décrit l'utilisation d'au moins un composé de formule (I) définie dans la revendication 1,
en tant qu'agent de destruction vasculaire, ou
en tant qu'inhibiteur de protéine phosphatase 1 et agent de destruction vasculaire et agent antiprolifératif, ou
en tant qu'inhibiteur de polymérisation de la tubuline, agent de destruction vasculaire et agent antiprolifératif, ou
en tant qu'inhibiteur de protéine phosphatase 1, de polymérisation de la tubuline, agent de destruction vasculaire et agent antiprolifératif,
dans laquelle le groupe R3 de la formule générale (I) représente H.

Dans ce mode de réalisation, le composé de l'invention est plus particulièrement utilisé pour la préparation d'un médicament destiné à la prévention et au traitement de mammifères, en particulier l'homme, atteints d'angiogénèses pathologiques, en particulier de rétinopathies.

Dans un mode de réalisation avantageux, la présente invention décrit l'utilisation d'au moins un composé de formule (I) ci-dessus définie,
en tant qu'agent de destruction vasculaire, ou
en tant qu'inhibiteur de protéine phosphatase 1 et agent de destruction vasculaire et agent antiprolifératif, ou
en tant qu'inhibiteur de polymérisation de la tubuline, agent de destruction vasculaire et agent antiprolifératif, ou
en tant qu'inhibiteur de protéine phosphatase 1, de polymérisation de la tubuline, agent de destruction vasculaire et agent antiprolifératif,
dans laquelle le groupe R3 de la formule générale (I) est tel que défini ci-dessus à l'exclusion de H.

Dans un mode de réalisation avantageux, la présente invention décrit l'utilisation d'au moins un composé de formule générale (I) définie ci-dessus, correspondant à un composé de formule générale (IIa) suivante : 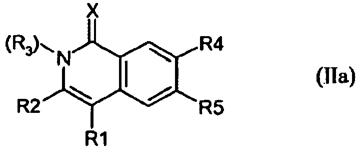 dans laquelle :
1) X représente :
   O, S, NH, N-(C1-C6)alkyle,
2) R2 représente :
   H, un halogène, OH, OPO₃H₂, OPO-(O-(C₁-C₂)alkyle)₂, l'alkyle étant éventuellement substitué par un ou plusieurs fluors,
   CF₃, CH₂OR', R' représentant H ou (C₁-C₆) alkyle, l'alkyle étant éventuellement substitué par un ou plusieurs fluors, CHO,
   CO₂R", R" représentant H, (C₁-C₆) alkyle, l'alkyle étant éventuellement substitué par un ou plusieurs fluors,
   CONR_{c}R_{d}, CH₂SO₂NR_{c}R_{d}, CH₂NHSO₂Rc R_{c} et R_{d} représentant indépendamment l'un de l'autre : H, un alkyle en C₁-C₆, et NR_{c}R_{d} représente un acide aminé lié par sa fonction amine, en particulier une sérine ou une thréonine, un cycloalkyle en C₃-C₆, ou R_{c} et R_{d} représentent ensemble un alkyle en C₂-C₆,
   NO₂, N₃, ≡, CN, C(=N)NH₂, SO₃H, SO₂NH₂, SO₂NHCH₃, NHSO₂CH₃, CH₂SO₂NR_{c}R_{d}, CH₂NHSO₂Rc SO₂-NRaRb, SO₂-imidazole,
   NRₐR_{b}, où
      Rₐ et R_{b} représentent indépendamment l'un de l'autre : H, un alkyle en C₁-C₆, un cycloalkyle en C₃-C₆, ou
   Ra est H, un alkyle en C₁-C₆, un cycloalkyle en C₃-C₆, et Rb = COR_{f}, COOR_{f} ou
   CONR_{f}R_{f} R_{f} et R_{f} représentant H, un alkyle en C₁-C₆, un cycloalkyle en C₃-C₆ ou une chaîne d'un amino acide (telle que CH(CH₂OH)NH₂ pour la sérine) Rₐ et R_{b} représentent ensemble un alkyle en C₂-C₆,
   Rₐ et R_{b} peuvent former un cycle en C₅ à C₇, notamment une pyrrolidine, une pipérazine, une morpholine, une thiomorpholine,
   un hétéroaryle substitué ou non, en particulier une pyridine, un imidazole, un oxazole, un triazole, un pyrrole, un tétrazole.
3) R2 représente :
   un phényle substitué ou non par un à trois substituants choisis parmi :
      un halogène, un OH, NHRa,
      ORe, Re représentant un benzyle, un triazole méthylène substitué ou non, notamment par un alkyle en C₁-C₆,
      OPO₃H₂, OPO-(O-(C₁-C₂)alkyle)₂, l'alkyle étant éventuellement substitué par un ou plusieurs fluors, un NH₂,
      un groupe NH-CORc dans lequel Rc représente H, un alkyle en C₁-C₆, un cycloalkyle en C₃-C₆ ou une chaîne d'un amino acide (telle que CH(CH₂OH)NH₂ pour la sérine),
      un O-alkyle en C₁-C₆, en particulier OCH₃, l'alkyle étant éventuellement substitué par un ou plusieurs fluors,
      un O-COR1 où R1 représente O-alkyle en C₁-C₆, ou NRiRii, Ri et Rii pouvant être alkyle en C₁-C₆.
      un groupe alkyle en C₂-C₄, le groupe alkyle étant éventuellement substitué par un ou plusieurs fluors, un groupement alcényle en C₂-C₄ substitué ou non,
      un groupe alcynyle en C₂-C₄ substitué ou non, en particulier par un triméthylsilyle, un tert-butyle, un hydroxy-2-propyle ou un isopropyle
      un hétéroaryle substitué ou non, en particulier une pyridine, un imidazole, un oxazole, un triazole, un pyrrole, un benzofurane, un thiophène, un benzothiophène, un indole, un cyclohéxyle, une pipérazine, une morpholine, une thiomorpholine, une pipéridine, un groupe 4-(NH₂-(CH₂-CH₂O)ₚ)Ph dans lequel p est un entier de 1 à 6
4) R3 représente :
   H. (C₁-C₆)alkyle, l'alkyle étant éventuellement substitué par un ou plusieurs fluors, (C₃-C₆)cycloalkyle, OH, OPO₃H₂, OPO-(O-(C₁-C₂)alkyle)₂,OSO₃H, SO₃H, O-alkyle en C₁ à C₆, NH₂, NH-(C₁-C₆)alkyle, N-[(C₁-C₆)alkyle]₂, NH-(C₃-C₆)cycloalkyle, N-[(C₃-C₆)cycloalkyle]₂, un groupe propargyle, CH₂CN, (CH₂)ₚOH, p variant de 1 à 6, CH₂OPO₃H₂, CH₂OPO-(O-(C₁-C₂)alkyle)₂, CH₂CH₂F, CH₂CHF₂, CH₂CF₃,
5) R4 et R5 représentent indépendamment l'un de l'autre :
   H, OH, OPO₃H₂, OPO-(O-(C₁-C₂)alkyle)₂, l'alkyle étant éventuellement substitué par un ou plusieurs fluors, un O-alkyle en C₁-C₆, en particulier OCH₃, l'alkyle étant éventuellement substitué par un ou plusieurs fluors, un alkyle en C₁-C₆, l'alkyle étant éventuellement substitué par un ou plusieurs fluors, un cycloalkyle en C₃-C₆, un halogène,
   R4 et R5 représentent ensemble un alcényle (en C₁-C₂) dioxy éventuellement substitué par un ou plusieurs fluors,

Dans un mode de réalisation avantageux, la présente invention décrit l'utilisation d'au moins un composé de formule générale (I) définie ci-dessus, correspondant à un composé de formule générale (IIb) suivante : 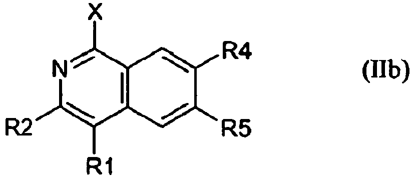 dans laquelle :
1) X représente :
   OH, OPO₃H₂, OPO-(O-(C₁-C₂)alkyle)₂, l'alkyle étant éventuellement substitué par un ou plusieurs fluors, O-(C₁-C₆)alkyle, en particulier OCH₃, l'alkyle étant éventuellement substitué par un ou plusieurs fluors, O(C₃-C₆)cycloallcyle, NH2, NH-(C₁-C₆)alkyle, N-[( C₁-C₆)alkyle]₂, NH-[(C₃-C₆)cycloalkyle, N-[(C₃-C₆)cycloalkyle]₂, SH, S-(C₁-C₆)alkyle, l'alkyle étant éventuellement substitué par un ou plusieurs fluors, en particulier SCH₃, S(C₃-C₆)cycloalkyle, SO₃H, NH-CH₂-aryl ou heteroalyl, OPh, SPh, OCH₂Ph, SCH₂Ph, le phényle de ces quatre groupes pouvant être substitué ou non par un ou plusieurs (C₁-C₆)alkyle, l'alkyle étant éventuellement substitué par un ou plusieurs fluors, 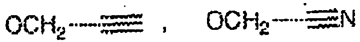
2) R1 représente :
   H, un halogène, OH, OPO₃H₂, OPO-(O-(C₁-C₂)alkyle)₂, l'alkyle étant éventuellement substitué par un ou plusieurs fluors,
   CF₃, CH2OR', R' représentant H ou (C₁-C₆)alkyle, l'alkyle étant éventuellement substitué par un ou plusieurs fluors, CHO,
   CO2R", R" représentant H, (C₁-C₆)alkyle, l'alkyle étant éventuellement substitué par un ou plusieurs fluors,
   CONR_{c}R_{d}, CH2SO2NR_{c}R_{d}, CH2NHSO2Rc, R_{c} et R_{d} représentant indépendamment l'un de l'autre : H, un alkyle en C₁-C₆ et NR_{c}R_{d} représente un acide aminé lié par sa fonction amine, en particulier une sérine ou une thréonine, un cycloalkyle en C₃-C₆, ou R_{c} et R_{d} représentent ensemble un alkyle en C₂-C₆,
   NO₂, N₃, ≡, CN, C(=N)NH₂, SO₃H, SO₂NH₂, SO₂NHCH₃, NHSO₂CH₃, CH₂SO₂NR_{c}R_{d}, CH₂NHSO₂Rc, SO₂-NRaRb, SO₂-imidazole, NRₐR_{b}, où :
      Rₐ et R_{b} représentent indépendamment l'un de l'autre : H, un alkyle en C₁-C₆, un cycloalkyle en C₃-C₆, ou
   Rₐ est H, un alkyle en C₁-C₆, un cycloalkyle en C₃-C₆, et Rb = COR_{f}, COOR_{f} ou CONR_{f}R_{f} R_{f} et R_{f} représentent H, un alkyle en C₁-C₆, un cycloalkyle en C₃-C₆ ou une chaîne d'un amino acide (telle que CH(CH₂OH)NH₂ pour la sérine) Rₐ et R_{b} représentent ensemble un alkyle en C₂-C₆,
   Rₐ et R_{b} peuvent former un cycle en C₅ à C₇, notamment une pyrrolidine, une pipérazine, une morpholine, une tiomorpholine,
   un hétéroaryle substitué ou non, en particulier une pyridine, un imidazole, un oxazole, un triazole, un pyrrole, un tétrazole.
3) R2 représente :
   un phényle substitué ou non par un à trois substituants choisis parmi :
      un halogène, un OH, NHRa,
      ORe, Re représentant un benzyle, un triazole méthylène substitué ou non, notamment par un alkyle en C₁-C₆,
      OPO₃H₂, OPO-(O-(C₁-C₂)alkyle)₂, l'alkyle étant éventuellement substitué par un ou plusieurs fluors, un NH₂
      un groupe NH-CORc dans lequel Rc représente H, un alkyle en C₁-C₆, un cycloalkyle en C₃-C₆ ou une chaîne d'un amino acide (telle que CH(CH₂OH)NH₂ pour la sérine),
      un O-alkyle en C₁-C₆, en particulier OCH₃, l'alkyle étant éventuellement substitué par un ou plusieurs fluors,
      un O-COR1 où R1 représente O-alkyle en C₁-C₆, ou NRiRii, Ri et Rii pouvant être un alkyle en C₁-C₆,
      un groupe alkyle en C₂-C₄, le groupe alkyle étant éventuellement substitué par un ou plusieurs fluors, un groupement alcényle en C₂-C₄ substitué ou non,
      un groupe alcynyle en C₂-C₄ substitué ou non, en particulier par un triméthylsilyle, un tert-butyle, un hydroxy-2-propyle ou un isopropyle
      un hétéroaryle substitué ou non, en particulier une pyridine, un imidazole, un oxazole, un triazole, un pyrrole, un benzofurane, un thiophène, un benzothiophène, un indole,
      un cyclohéxyle, une pipérazine, une morpholine, une thiomorpholine, une pipéridine, un groupe 4-(NH₂-(CH₂-CH₂O)ₚ)Ph dans lequel p est un entier de 1 à 6
4) R3 représente :
   H, (C₁-C₆)alkyle, l'alkyle étant éventuellement substitué par un ou plusieurs fluors, (C₃-C₆)cycloalkyle, OH, OPO₃H₂, OPO-(O-(C₁-C₂)alkyle)₂,OSO₃H, SO₃H, O-alkyle en C₁ à C₆, NH₂, NH-(C₁-C₆)alkyle, N-[(C₁-C₆)alkyle]₂, NH-(C₃-C₆)cycloalkyle, N-[(C₃-C₆)cycloalkyle]₂, un groupe propargyle, CH₂CN, (CH₂)ₚOH, p variant de 1 à 6, CH₂OPO₃H₂, CH₂OPO-(O-(C₁-C₂)alkyle)₂, CH₂CH₂F, CH₂CHF₂, CH₂CF₃,
5) R4 et R5 représentent indépendamment l'un de l'autre :
   H, OH, OPO₃H₂, OPO-(O-(C₁-C₂)alkyle)₂, l'alkyle étant éventuellement substitué par un ou plusieurs fluors, un O-alkyle en C₁-C₆, en particulier OCH₃, l'alkyle étant éventuellement substitué par un ou plusieurs fluors, un alkyle en C₁-C₆, l'alkyle étant éventuellement substitué par un ou plusieurs fluors, un cycloalkyle en C₃-C₆, un halogène,
   R4 et R5 représentent ensemble un alcényle (en C₁-C₂) dioxy éventuellement substitué par un ou plusieurs fluors,

Dans un mode de réalisation avantageux, la présente invention décrit l'utilisation d'au moins un composé choisi parmi les suivantes : 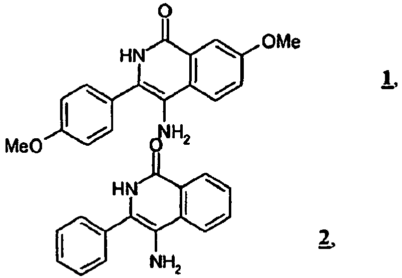 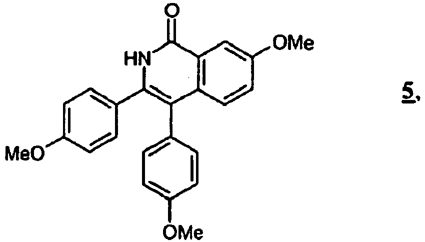 (non revendiquée)

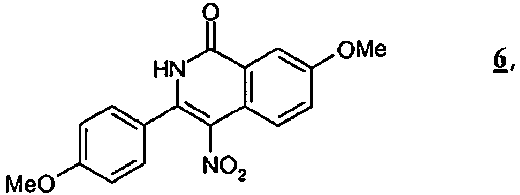

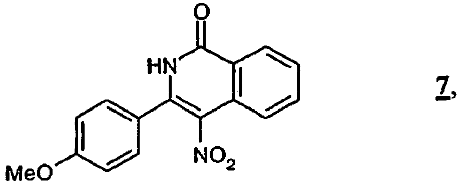

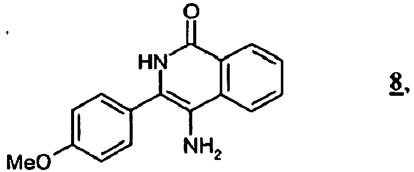

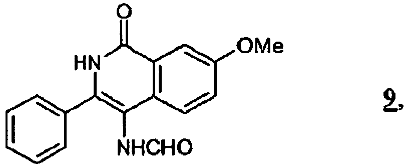

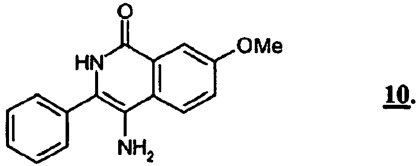

Dans un autre mode de réalisation avantageux, la présente invention décrit l'utilisation d'au moins un composé, choisi parmi les suivants :

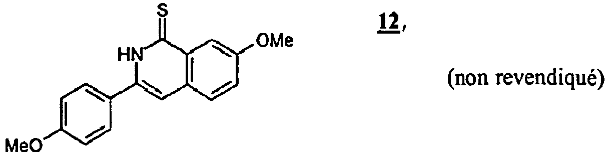

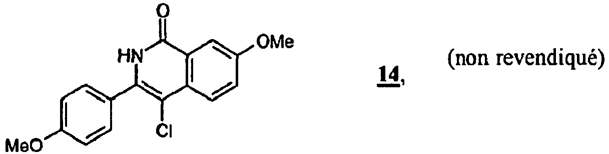

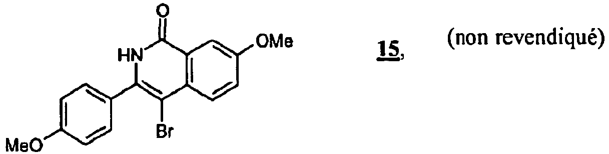

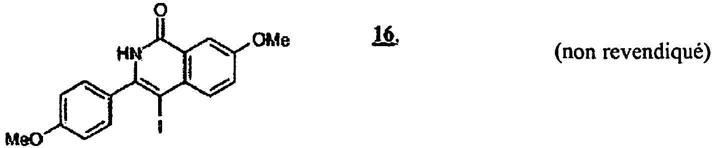

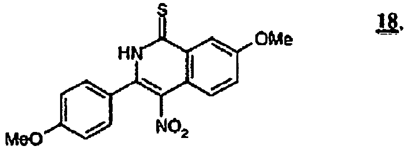

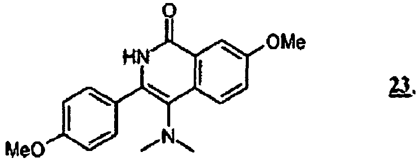

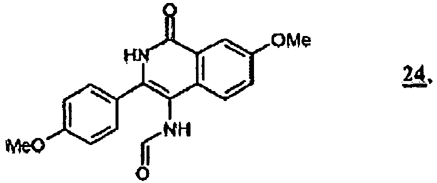

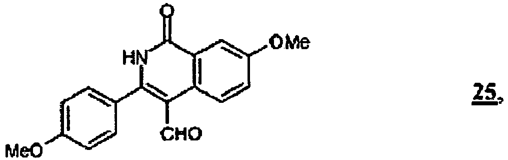

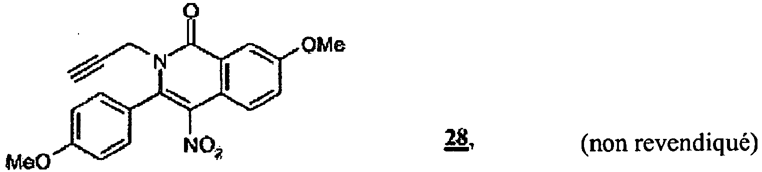

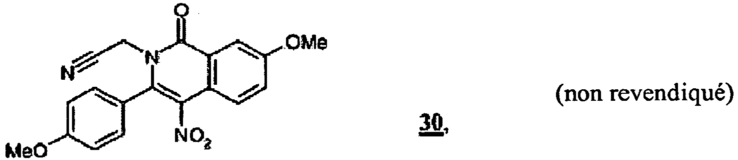

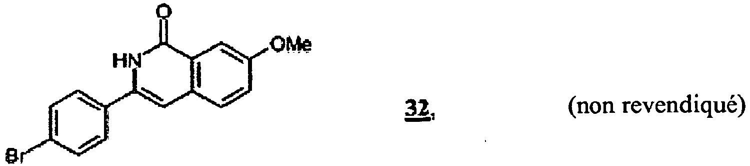

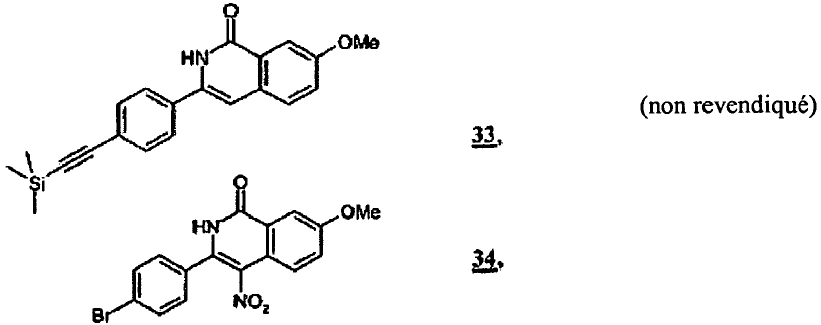

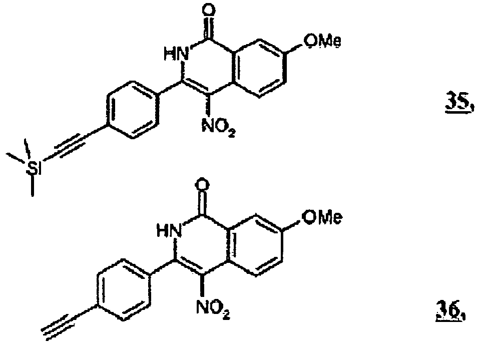

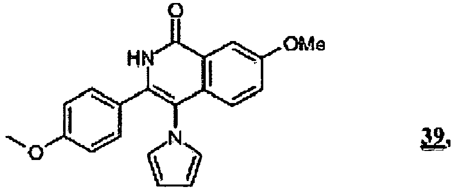

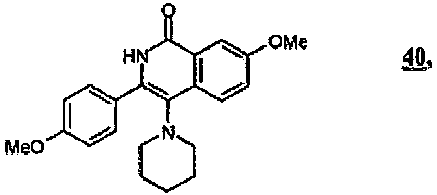

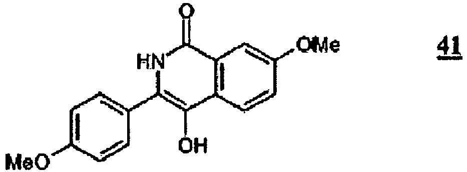

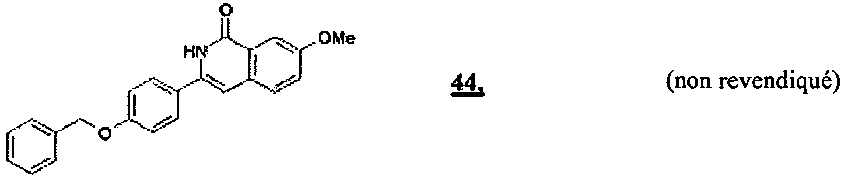

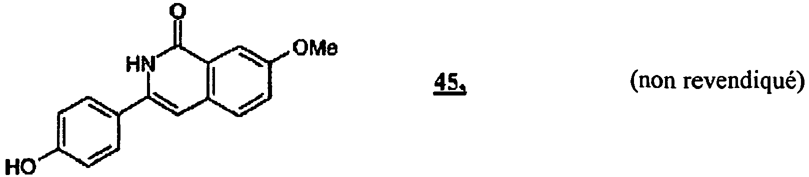

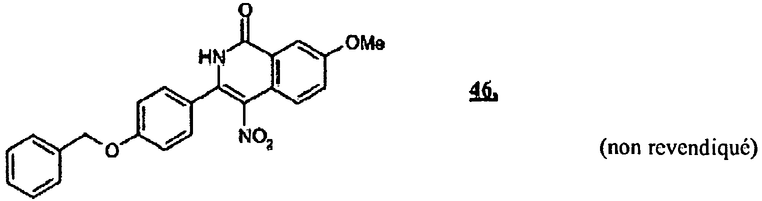

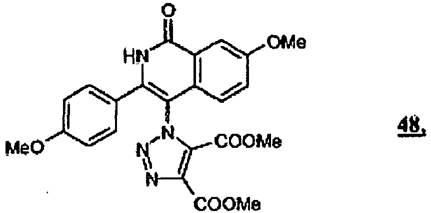

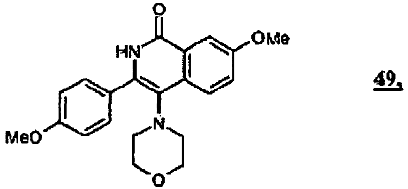

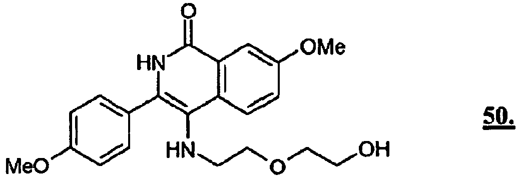

Les composés **12, 14-16, 18, 23-25, 28, 30, 32-36, 39-41, 44-46, 48-50** sont des composés nouveaux.

Dans un autre mode de réalisation avantageux, la présente invention décrit l'utilisation d'au moins composé, choisi parmi les suivants :

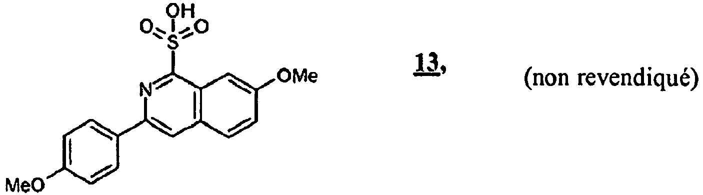

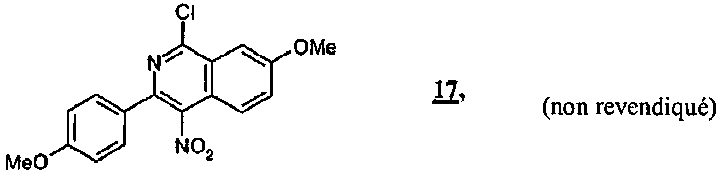

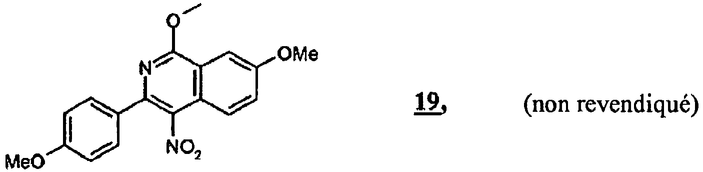

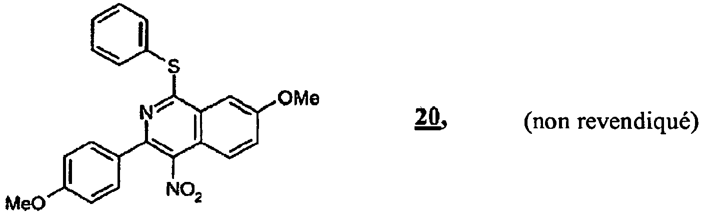

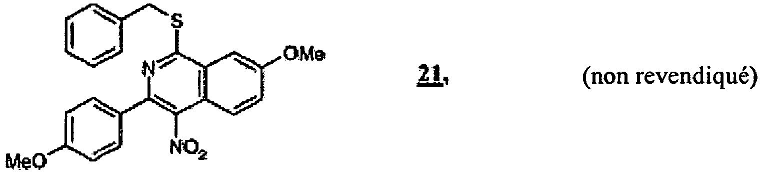

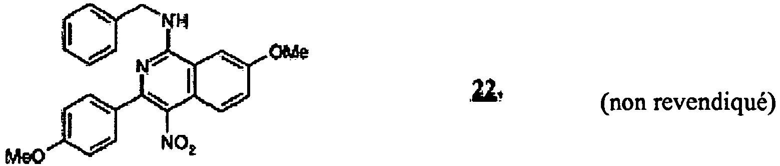

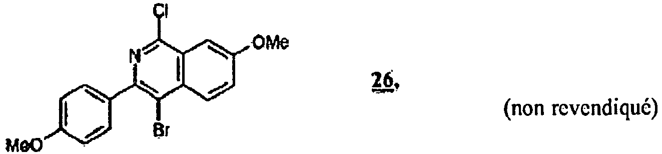

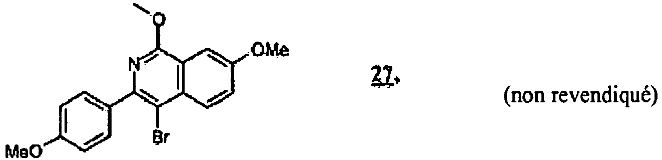

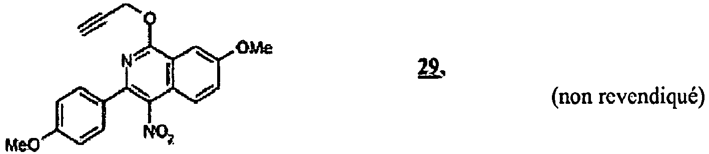

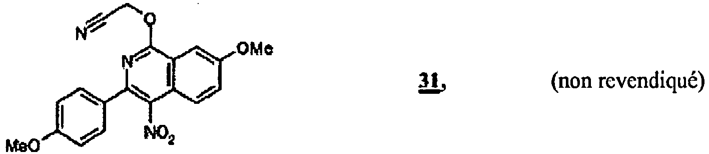

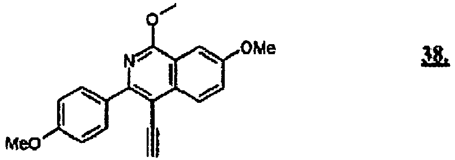

Dans un mode de réalisation avantageux, la présente invention décrit l'utilisation d'au moins un composé, en tant qu'agent de destruction vasculaire, choisi parmi les suivants :

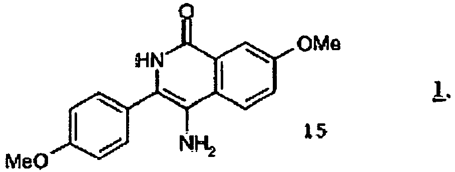

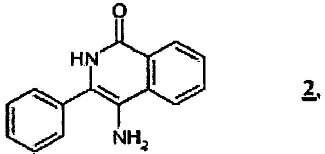

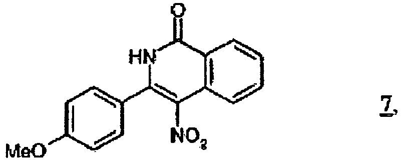

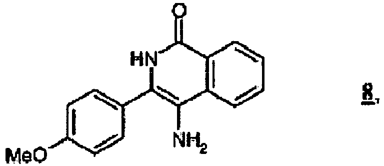

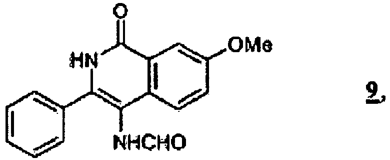

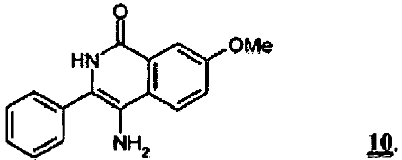

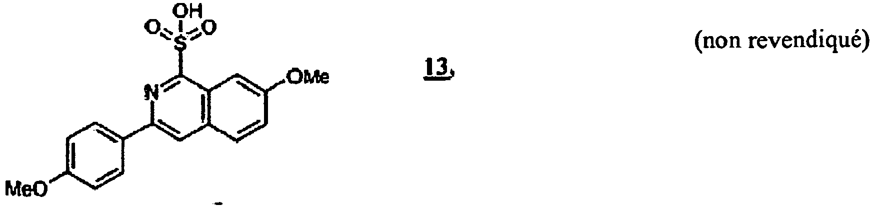

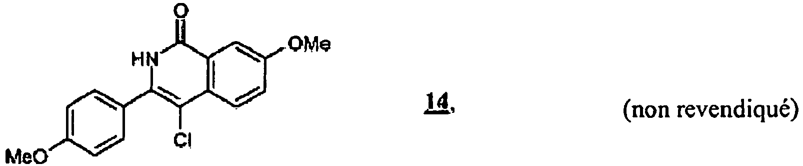

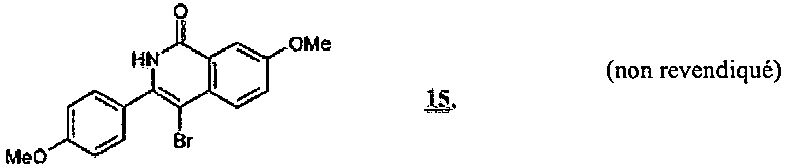

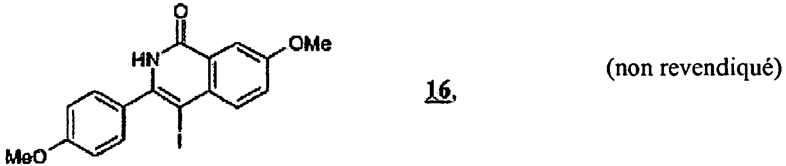

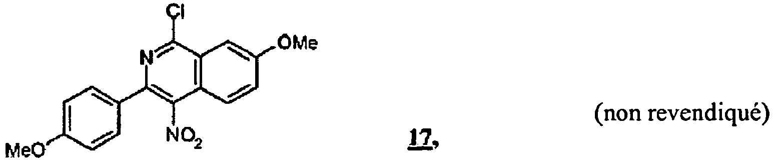

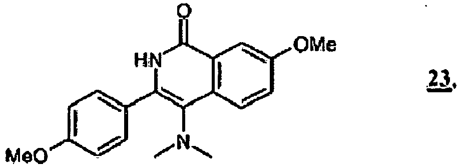

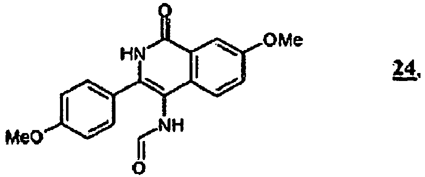

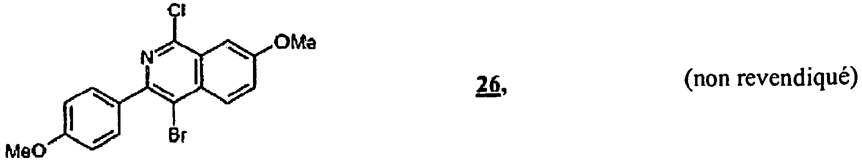

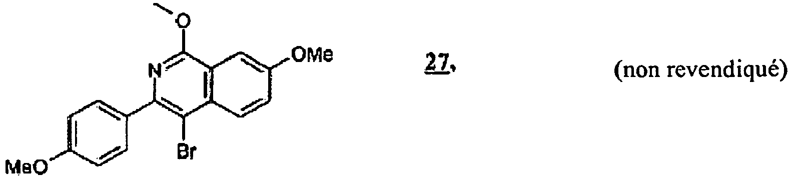

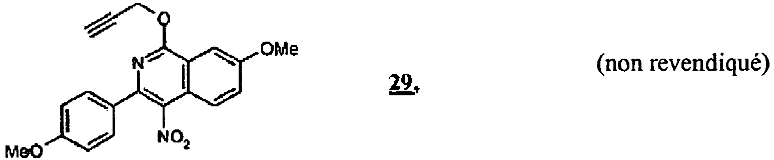

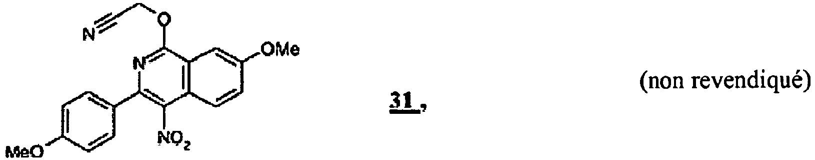

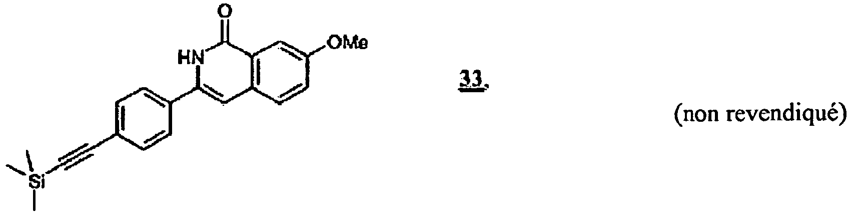

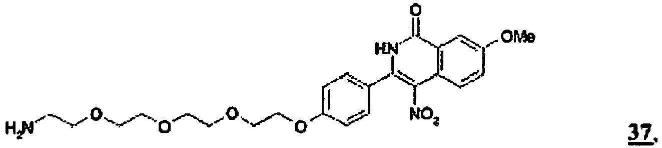

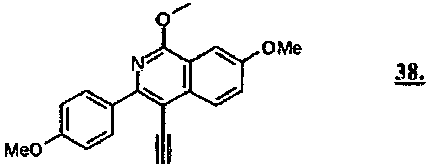

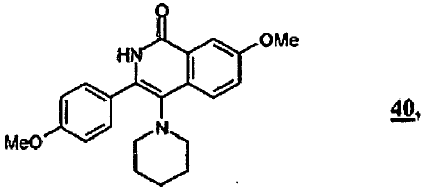

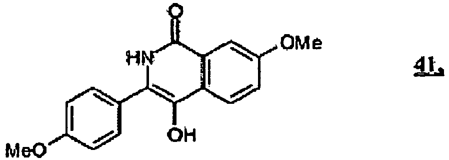

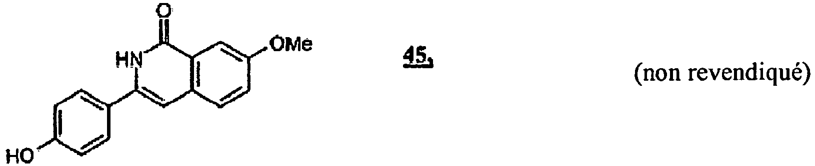

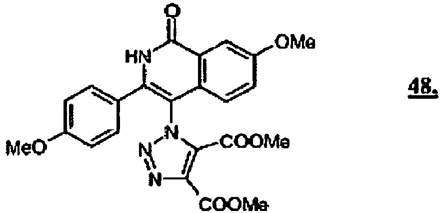

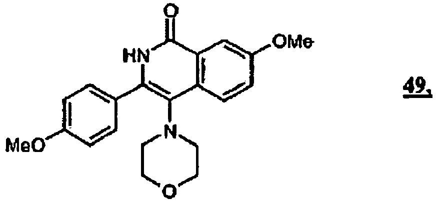

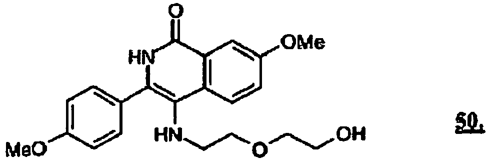

Les composés de ce mode de réalisation possèdent essentiellement une activité VDA et ne sont pas inhibiteurs de PP1.

Dans un mode de réalisation avantageux, la présente invention décrit l'utilisation d'au moins un composé, en tant qu'inhibiteur de protéine phosphatase 1, agent de destruction vasculaire et agent antiprolifératif, dans laquelle les composés sont choisis parmi les suivants :

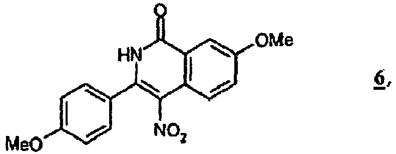

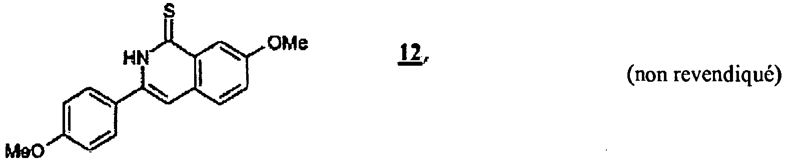

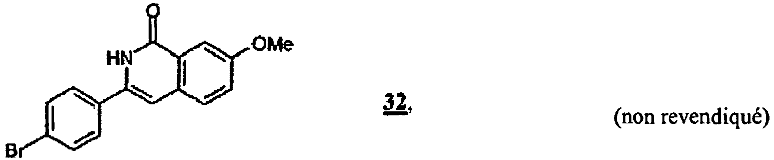

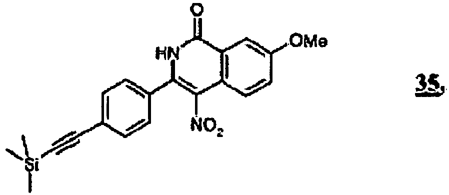

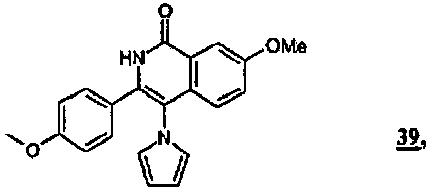

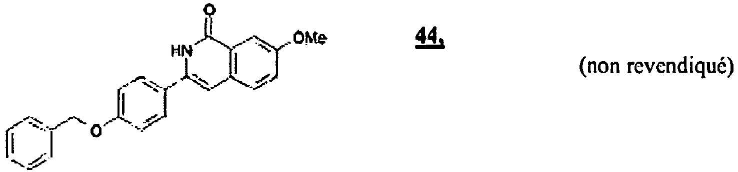

Les composés de ce mode de réalisation possèdent une activité VDA et sont inhibiteurs de PP1 mais ne possèdent pas ou peu d'activité inhibitrice de la tubuline.

Dans ce mode de réalisation avantageux, la présente invention décrit l'utilisation d'au moins un composé, en tant qu'inhibiteur de protéine phosphatase 1, agent de destruction vasculaire, inhibiteur de polymérisation de la tubuline, et agent antiprolifératif, dans laquelle les composés sont choisis parmi les suivants :

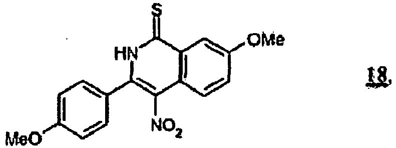

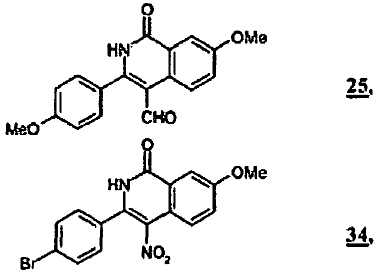

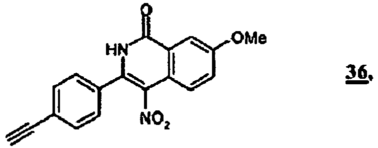

Dans un mode de réalisation avantageux, la présente invention décrit l'utilisation d'au moins un composé, en tant qu'agent de destruction vasculaire et agent antiprolifératif, dans laquelle le composé est choisi parmi les suivants :

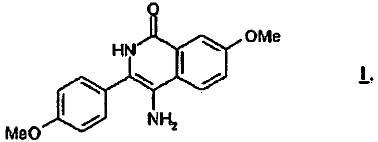

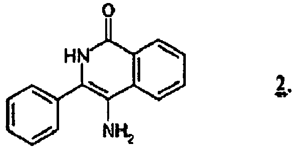

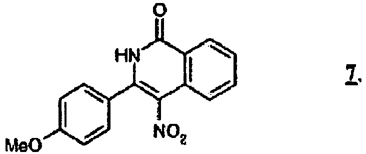

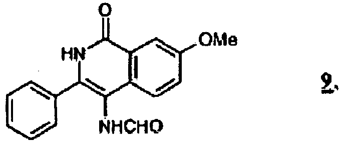

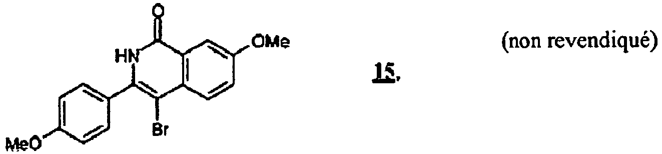

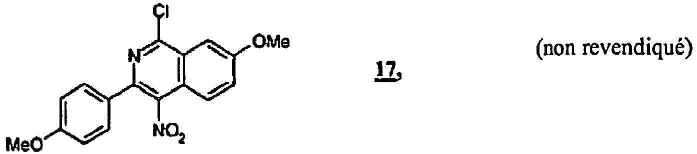

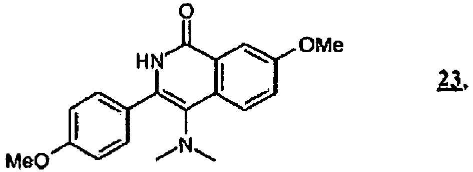

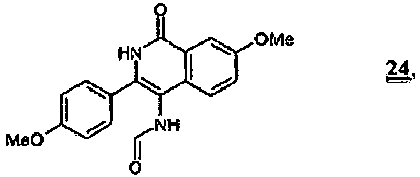

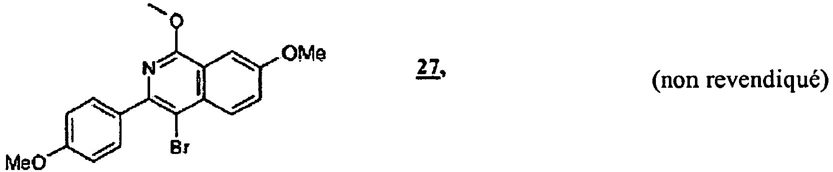

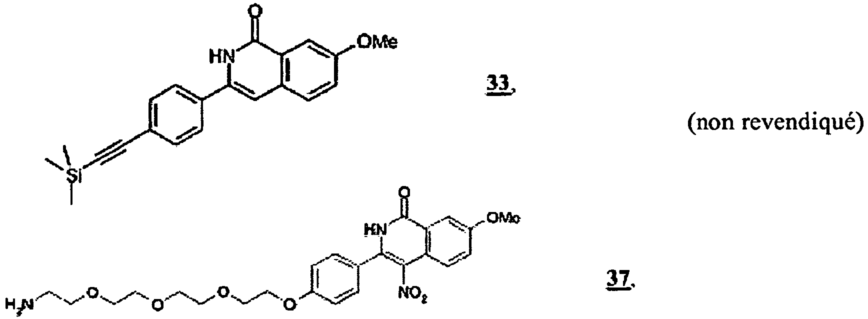

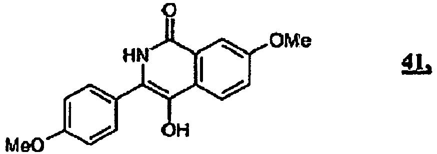

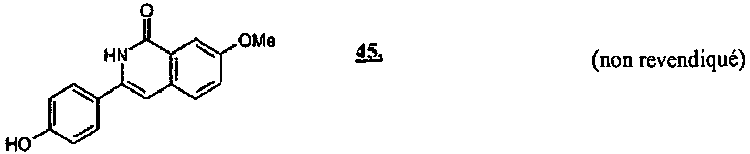

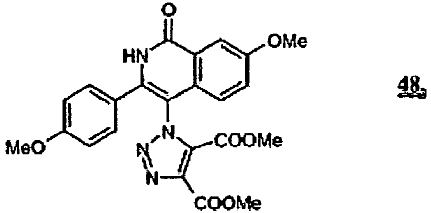

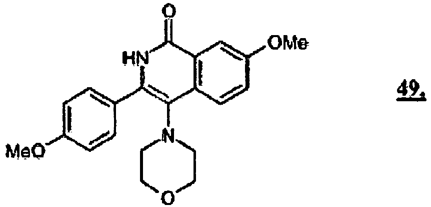

Les composés de ce mode de réalisation possèdent une activité VDA, ne sont pas ou peu inhibiteurs de PP1 et ne possèdent pas ou peu d'activité inhibitrice de la tubuline.

Dans un mode de réalisation avantageux, la présente invention décrit l'utilisation d'au moins un composé, en tant qu'agent de destruction vasculaire, inhibiteur de la polymérisation de la tubuline et agent antiprolifératif, dans laquelle le composé est choisi parmi les suivants :

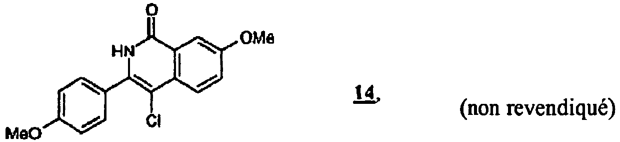

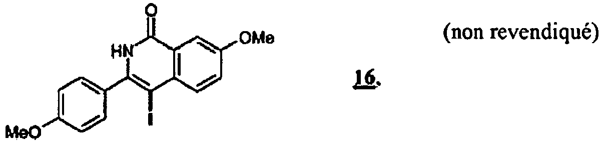

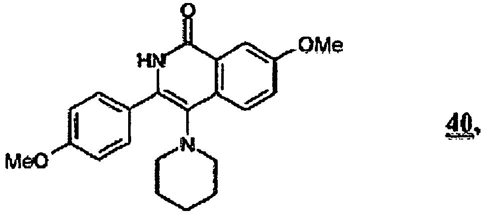

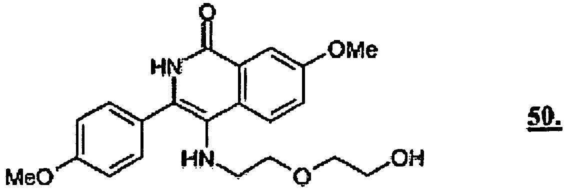

Les composés de ce mode de réalisation possèdent une activité VDA, ne sont pas ou peu inhibiteurs de PP1 et possèdent une activité inhibitrice de la tubuline.

Dans un mode de réalisation avantageux, la présente invention décrit l'utilisation d'au moins un composé de formule (I) ci-dessus définie, dans laquelle l'un des groupes groupe R4 et R5 des composés de formule (I) sont tels que définis ci-dessus à l'exception d'un halogène.

Dans un autre mode de réalisation avantageux, la présente invention décrit l'utilisation d'au moins un composé de formule (I), (IIa) ou (IIb)définie ci-dessus, ledit composé de formule (I), ou (IIa) ou (IIb) provoquant une nécrose *in vivo* au centre de la tumeur, observable en 24 heures ou moins après administration par voie orale ou parenterale d'une dose active dudit composé, une telle nécrose est observée et mesurée sur plusieurs coupes histologiques de tumeurs (à différent niveaux en profondeur) colorées à l'hématoxyline-éosine. (Salmon et aL, Eur. J. Cancer, 2007, v43 (10) : pp1622-29).

Les composés de l'invention povoquent une asphyxie à l'intérieur de la tumeur qui est difficile d'accès et qui n'est pas habituellement détruite par les produits conventionnellement utilisés, surtout pas par les produits anti-mitotiques, en particulier parce qu'il y a très peu de cellules mitotiques dans le centre des tumeurs, puisque lesdites cellules mitotiques se trouvent majoritairement à la périphérie des tumeurs

Les composés de l'invention provoquent aussi une inflammation des tissus autour des zones nécrotiques pouvant entrainer ainsi la destruction des masses tumorales non seulement par l'asphyxie mais aussi par l'inflammation non-spécifique.

Dans un autre mode de réalisation avantageux, la présente invention décrit l'utilisation d'au moins un composé de formule (I), (IIa) ou (IIb)définie ci-dessus, ledit composé de formule (1), (IIa) ou (IIb)ne possédant pas de propriétés anti-inflammatoires.

L'absence de propriétés anti-inflammatoires des composés de l'invention peut être évaluée selon le protocole de l'exemple 15.

Les produis de l'invention ne possèdent par conséquent pas de propriétés anti-inflammatoires contrairement aux composés décrits dans la demande de brevet WO 98/51307.

Dans un autre mode de réalisation avantageux, la présente invention concerne l'utilisation d'au moins un composé de formule (I), tel que défini dans la revendication 1, ledit composé étant administré par voie orale, à une dose comprise d'environ 1mg/kg à environ 200 mg/kg, préférentiellement d'environ 10 mg/kg à environ 100 mg/kg, plus préférentiellement de 20 mg/kg à 50 mg/kg, en particulier 40 mg/kg.

Dans un autre mode de réalisation avantageux, la présente invention décrit l'utilisation d'au moins un composé de formule (I), tel que défini dans la revendication 1, ledit composé étant administré par voie orale, réparti en une ou plusieurs doses d'environ 3 mg/m² à environ 600 mg/m², préférentiellement d'environ 30 mg/m² à environ 300 mg/m², plus préférentiellement de 60 mg/m² à 150 mg/m², en particulier 120 mg/m².

Dans un mode de réalisation avantageux, la dose du composé de formule (I), définie dans la revendication 1, utilisée pour le traitement, peut être d'environ 1 mg/kg, d'environ 2 mg/kg, d'environ 5 mg/kg, d'environ 7 mg/kg, d'environ 10 mg/kg, d'environ 20 mg/kg, ou elle peut être d'environ 30 mg/kg, d'environ 40 mg/kg, d'environ 50 mg/kg, d'environ 60 mg/kg, d'environ 80 mg/kg, ou encore d'environ 100 mg/kg, d'environ 120 mg/kg, d'environ 140 mg/kg, d'environ 150 mg/kg, d'environ 160 mg/kg, d'environ 180 mg/kg, d'environ 200 mg/kg.

Dans un mode de réalisation avantageux, la composé de formule (I), définie dans la revendication 1, utilisée pour le traitement, peut être administré en une ou plusieurs doses répartie(s) d'environ 3 mg/m², d'environ 6 mg/m², d'environ 15 mg/m², d'environ 21 mg/m², d'environ 30 mg/m², d'environ 60 mg/m², ou elle peut être d'environ 90 mg/m², d'environ 120 mg/m², d'environ 150 mg/m², d'environ 180 mg/m², d'environ 240 mg/m², ou encore d'environ 300 mg/m², d'environ 360 mg/m², d'environ 420 mg/m², d'environ 450 mg/m², d'environ 480 mg/m², d'environ 540 mg/m², d'environ 600 mg/m².

Dans un mode de réalisation avantageux, la dose du composé de formule (I), définie dans la revendication 1, utilisée pour le traitement, peut être comprise d'environ 200 mg/kg à environ 400 mg/kg, en particulier d'environ 200 à environ 300 mg/kg, et préférentiellement, environ 200 mg/kg, environ 210 mg/kg, environ 220 mg/kg, environ 230 mg/kg, environ 240 mg/kg, environ 250 mg/kg, environ 260 mg/kg, environ 270 mg/kg, environ 280 mg/kg, environ 290 mg/kg ou environ 300 mg/kg, en fonction de la dose efficace et/ou maximum tolérée par le mammifère, en particulier l'homme.

Dans un mode de réalisation avantageux, le composé de formule (I), définie dans la revendication 1, utilisée pour le traitement, peut être administré en une ou plusieurs doses répartie(s) comprise d'environ 600 mg/m² à environ 1200 mg/m², en particulier d'environ 600 à environ 900 mg/m², et préférentiellement, environ 600 mg/m², environ 630 mg/m², environ 660 mg/m², environ 690 mg/m², environ 720 mg/m², environ 750 mg/m², environ 780 mg/m², environ 810 mg/m², environ 840 mg/m², environ 870 mg/m² ou environ 900 mg/m², en fonction de la dose efficace et/ou maximum tolérée par le mammifère, en particulier l'homme.

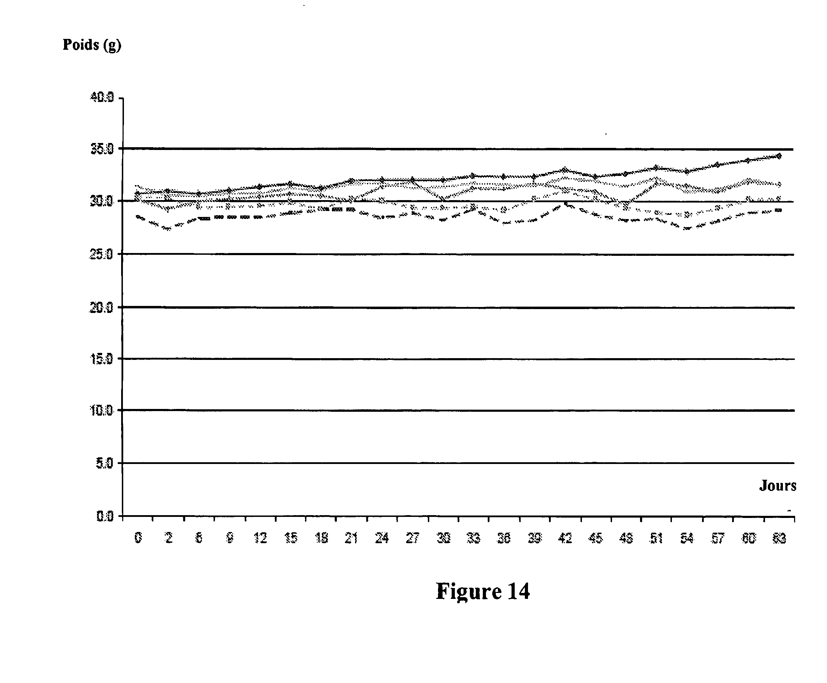

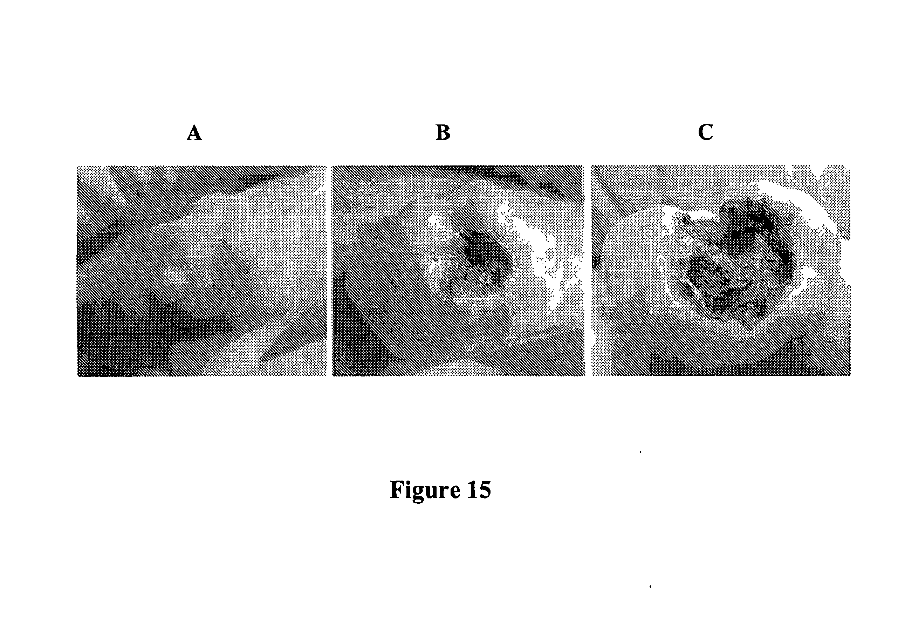

Par ailleurs, comme montré dans les exemples présentant l'activité des composés de l'invention et en particulier du composé 6, *in vivo*, ou par la figure 14 présentant l'évidence de l'absence de toxicité majeure *in vivo* des composés de l'invention et en particulier du composé 6, *in vivo*, les composés de l'invention et en particulier le composé 6 présentent un effet (figure 15) confirmant de facto la biodisponibilité des produits de l'invention et en particulier du composé 6.
Dans un mode de réalisation avantageux, la présente invention décrit l'utilisation d'au moins un composé de formule (I), définie dans la revendication 1, ledit composé étant administrable par voie intraveineuse à une dose comprise d'environ 0,1 mg/kg/j à environ 100 mg/kg/j, notamment 50 mg/kg/j, en particulier 10 mg/kg/j.

Dans un autre mode de réalisation avantageux, ledit composé de formule (I), définie dans la revendication 1, est en association avec au moins un antitumoral choisi parmi les suivants, sans être limité à :
l'abraxane, l'abarelix, l'aldesleukine, l'alemtuzumab, l'alitretinoine, l'allopurinol, l'altrétamine, l'anastrozole, l'arsenic trioxide, l'asparaginase, l'azacitidine, le bevacizumab, le bexarotene, le bicalutamide, la bleomycine, le bortezomib, le busulfan intraveineux, le busulfan oral, la calustérone, la capécitabine, le carboplatin, la carmustine, le cetuximab, le chlorambucil, le cisplatine, la cladribine, la clofarabine, le cyclophosphamide, la cytarabine, la dacarbazine, la dactinomycine, la daltéparine sodium, la dasatinib, la daunorubicine, la décitabine, la dénileukine, la dénileukine diftitox, le dexrazoxane, le docetaxel, la doxorubicine, la dromostanolone propionate, l'eculizumab, l'epirubicine, l'erlotinib, l'estramustine, l'etoposide phosphate, l'etoposide, l'exemestane, le fentanyl citrate, le filgrastime, la floxuridine, la fludarabine, le 5-fluorouracyl, le fulvestrant, le gefitinib, la gemcitabine, le gemtuzumab ozogamicine, la gosereline acetate, l'histrelin acetate, l'ibritumomab tiuxetan, l'idarubicine, l'ifosfamide, l'imatinib mesylate, l'interferon alfa 2a, l'irinotecan, le lapatinib ditosylate, le lenalidomide, le letrozole, leucovorine, le leuprodile acetate, le levamisole, la lomustine, la meclorethamine, le megestrol acetate, le melphalan, la mercaptopurine, le methotrexate, le methoxsalen, la mitomycine C, le mitotane, mitoxantrone, la nandrolone phenpropionate, la nelarabine, le nofetumomab, l'oxaliplatine, le paclitaxel, le pamidronate, le panitumumab, la pegaspargase, le pegfilgrastim, le pemetrexed disodium, la pentostatine, le pipobroman, la plicamycine, le procarbazine, la quinacrine, la rasburicase, le rituximab, le sorafenib, la streptozocine, le sunitinib, le sunitinib maleate, le tamoxifène, le taxol, le taxotère, la temozolomide, le teniposide, la testolactone, la thalidomide, la thioguanine, le thiotepa, le topotecan, le toremifene, le tositumomab, le trastuzumab, le tretinoine, le tykerb, l'uracil moutarde, la valrubicine, la vinblastine, la vincristine, la vinorelbine, le vorinostat, et le zoledronate.

Dans la plupart des cas, pour le traitement des tumeurs, bénines ou malignes, il peut être avantageux d'associer les composés de l'invention avec un(des) traitement(s) conventionnel(s), comme défini ci-dessus, de manière à obtenir l'effet additionnel ou synergique entre les composés de l'invention et le(s) traitement(s) conventionnel(s).

Dans un autre mode de réalisation avantageux, ledit composé de formule (I), définie dans la revendication 1 peut être associé à une quantité efficace de radiations ionisantes.

Le composé de l'invention et l'antitumoral classique ou la quantité efficace de radiation ionisante peuvent être administrés concommitamment et/ou non concommitamment, c'est-à-dire séquentiellement.

Dans un autre mode de réalisation avantageux, ledit composé de formule (I), définie dans la revendication 1, peut être associés à une chirurgie.

Le composé de l'invention peut alors être administré avant, pendant ou après le(s) traitement(s) conventionnel(s).

Dans un autre mode de réalisation avantageux, ledit composé de formule (I), définie dans la revendication 1, est en association avec au moins un traitement contre la rétinopathie, tels que sans être limités à ceux-ci, le laser ou l'Avastin.

L'activité de l'Avastin dans la rétinopathie est notamment décrite dans: Spaide R.F. and Fisher YL, Retina. 2006, v26(3): pp 275-8.

Selon un autre aspect, l'invention décrit une composition pharmaceutique comprenant comme principe actif un composé de formule (I), tel que défini ci-dessus, en association avec un véhicule pharmaceutiquement acceptable, à l'exclusion des composés suivants :
- lorsque X=O, n = 1, R3 = H, et que la liaison entre le carbone 1 et le groupement X est double et, R1 = NH2, R2 = phényle ou 4-OH-phényle substitué ou non, R4 = H, OH, un O-alkyle en C₁-C₆, un alkyle en C₁-C₆, un cycloalkyle en C₃-C₆, et R5 = H, OH, un O-alkyle en C₁-C₆, un alkyle en C₁-C₆, un cycloalkyle en C₃-C₆, et
- lorsque X = O ou S, n = 1, R3 = H, et lorsque la liaison entre le carbone 1 et le groupement X est double, R2 ayant la même signification que ci-dessus et l'un des R4 et R5 représentent un halogène.

L'invention concerne une composition pharmaceutique telle que définie dans les revendications.

Dans ce mode de réalisation, la formule générale (I) et les pointillés correspondent aux trois structures définies ci-dessus, à l'exclusion des composés de formule IV suivante : 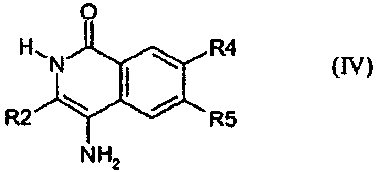 dans laquelle R2 = phényle ou 4-OH-phényle substitué ou non, R4 = H, OH, un O-alkyle en C₁-C₆, un alkyle en C₁-C₆, un cycloalkyle en C₃-C₆, et R5 = H, OH, un O-alkyle en C₁-C₆, un alkyle en C₁-C₆, un cycloalkyle en C₃-C₆.

Les véhicules pharmaceutiquement acceptables pouvant être utilisés dans le cadre des compositions pharmaceutiques de l'invention sont bien connus de l'homme de l'art.

Dans un mode de réalisation avantageux, la composition pharmaceutique définie ci-dessus, comprend comme principe actif un composé de formule I, dans laquelle :
- n=0, la liaison entre le carbone 1 et le groupement X est simple et X est choisi parmi, OCH3, , ou
- n = 1, la liaison entre le carbone 1 et le groupement X est double, X = O ou S et R2 représente un phényle substitué en position 4 par un groupement choisi parmi OH, OCH3, O-benzyle, Br, un groupe alcynyle en C2-C4 substitué ou non en particulier par un triméthylsilyle, un tert-butyle, un hydroxy-2-propyle ou un isopropyle.

Dans un mode de réalisation avantageux, l'invention décrit une composition pharmaceutique comprenant comme principe actif un composé de formule I, tel que défini ci-dessus, dans laquelle R1 = H.

Dans un mode de réalisation avantageux, l'invention décrit une composition pharmaceutique comprenant comme principe actif un composé de formule I, à l'exclusion des composés suivants :
- lorsque X = O ou S, n = 1, R3 = H, et lorsque la liaison entre le carbone 1 et le groupement X est double, R2, R4 et R5 ayant la même signification que ci-dessus, les composés dans lesquels R1 = H.

Dans un mode de réalisation décrit, la composition pharmaceutique définie ci-dessus, comprend comme principe actif un composé de formule (IIa), tel que défini ci-dessus, en association avec un véhicule, diluent ou excipient pharmaceutiquement acceptable.

Dans ce mode de réalisation, la formule générale (IIa et IIb)) et les pointillés correspondent aux deux structures définies ci-dessus, les mêmes composés que ci-dessus étant exclus des formules (IIa et IIb).

Dans un mode de réalisation décrit, la composition pharmaceutique définie ci-dessus, comprend comme principe actif un composé de formule IIa, tel que défini ci-dessus, en association avec un véhicule, diluent ou excipient p pharmaceutiquement acceptable

Dans ce mode de réalisation, les mêmes composés que ceux exclus ci-dessus sont également exclus de la formule (IIa).

Dans un mode de réalisation décrit, la composition pharmaceutique définie ci-dessus, comprend comme principe actif un composé de formule IIb, tel que défini ci-dessus.

Dans un mode de réalisation décrit, la composition pharmaceutique définie ci-dessus, comprend comme principe actif un composé de formule IIa, choisi parmi les composés **1**, **2**, **4-10** défini ci-dessus.

Dans un mode de réalisation décrit, la composition pharmaceutique définie ci-dessus, comprend comme principe actif un composé de formule IIa, choisi parmi les composés **12**, **14-16, 18, 23-25, 28, 30, 32-36, 39-41, 44-46,** et **48-50** définis ci-dessus.

Dans un mode de réalisation décrit, la composition pharmaceutique définie ci-dessus, comprend comme principe actif un composé de formule IIb, choisi parmi les composés **13, 17, 19-22, 26-27, 29, 31** et **38** définis ci-dessus.

Dans un mode de réalisation décrit, la composition pharmaceutique définie ci-dessus, comprend comme principe actif un composé choisi parmi les composés **1, 2, 7, 8, 9, 10, 13-17, 23-24, 26-27, 29, 31, 33, 37-38, 40-41, 45** et **48-50** définis ci-dessus.

Dans un mode de réalisation décrit, la composition pharmaceutique définie ci-dessus, comprend comme principe actif un composé choisi parmi les composés **6, 12, 19, 20, 22, 28, 30, 32, 35, 39, 44,** et **46** définis ci-dessus.

Dans un mode de réalisation décrit, la composition pharmaceutique définie ci-dessus, comprend comme principe actif un composé choisi parmi les composés **18, 21, 25, 29, 34,** et **36** définis ci-dessus.

Dans un mode de réalisation décrit, la composition pharmaceutique définie ci-dessus, comprend comme principe actif un composé choisi parmi les composés **1, 2, 7, 8**, **9**, **10**, **13**, **15**, **17,** **23-24, 26-27, 29, 31, 33, 37-38**, **41, 45,** et **48-49** définis ci-dessus.

Dans un mode de réalisation décrit, la composition pharmaceutique définie ci-dessus, comprend comme principe actif un composé choisi parmi les composés **14, 16, 24, 26-40,** et **50** définis ci-dessus.

Dans un mode de réalisation décrit, la composition pharmaceutique définie ci-dessus, comprend comme principe actif une prodrogue, telle que définie ci-dessus, dans laquelle l'un au moins des groupes X, R2, R4 et R5 représente un groupement phosphate : OPO₃H₂, alkyle phosphate: OPO-(O-(C₁-C₂)alkyle)₂, et/ou
le groupe R3 représente un groupement CH₂OPO₃H₂ ou CH₂OPO-(O-(C₁-C₂)alkyle)₂ et/ou
l'un au moins des groupes R1 et R2 représente un acide aminé lié par sa fonction acide, en particulier une sérine.

Dans un mode de réalisation avantageux, la composition pharmaceutique comprenant un composé de formule (I), définie dans la revendication 1, est administrable par voie orale à une dose comprise d'environ 1 mg/kg à environ 200 mg/kg, préférentiellement d'environ 10 mg/kg à environ 100 mg/kg, plus préférentiellement de 20 mg/kg à 50 mg/kg, en particulier 40 mg/kg.

Dans un autre mode de réalisation avantageux, la composition pharmaceutique comprenant un composé de formule (I), définie dans la revendication 1, est administrable par voie orale, réparti en une ou plusieurs doses d'environ 3 mg/m² à environ 600 mg/m², préférentiellement d'environ 30 mg/m² à environ 300 mg/m², plus préférentiellement de 60 mg/m² à 150 mg/m², en particulier 120 mg/m².

Dans un autre mode de réalisation avantageux, la composition pharmaceutique comprenant un composé de formule (I), définie dans la revendication 1, est administrable par voie orale, à une comprise d'environ 1 mg/kg, d'environ 2 mg/kg, d'environ 5 mg/kg, d'environ 7 mg/kg, d'environ 10 mg/kg, d'environ 20 mg/kg, ou elle peut être d'environ 30 mg/kg, d'environ 40 mg/kg, d'environ 50 mg/kg, d'environ 60 mg/kg, d'environ 80 mg/kg, ou encore d'environ 100 mg/kg, d'environ 120 mg/kg, d'environ 140 mg/kg, d'environ 150 mg/kg, d'environ 160 mg/kg, d'environ 180 mg/kg, d'environ 200 mg/kg,

Dans un autre mode de réalisation avantageux, la composition pharmaceutique comprenant un composé de formule (I), définie dans la revendication 1, est administrable par voie orale, réparti en une ou plusieurs doses d'environ 3 mg/m², d'environ 6 mg/m², d'environ 15 mg/m², d'environ 21 mg/m², d'environ 30 mg/m², d'environ 60 mg/m², ou elle peut être d'environ 90 mg/m², d'environ 120 mg/m², d'environ 150 mg/m², d'environ 180 mg/m², d'environ 240 mg/m², ou encore d'environ 300 mg/m², d'environ 360 mg/m², d'environ 420 mg/m², d'environ 450 mg/m², d'environ 480 mg/m², d'environ 540 mg/m², d'environ 600 mg/m².

Dans un autre mode de réalisation avantageux, la composition pharmaceutique comprenant un composé de formule (I), définie dans la revendication 1, est administrable par voie orale, réparti en une ou plusieurs doses d'environ 600 mg/m² à environ 1200 mg/m², en particulier d'environ 600 mg/m² à environ 900 mg/m², et préférentiellement, environ 600 mg/m², environ 630 mg/m², environ 660 mg/m², environ 690 mg/m², environ 720 mg/m², environ 750 mg/m², environ 780 mg/m², environ 810 mg/m², environ 840 mg/m², environ 870 mg/m² ou environ 900 mg/m², en fonction de la dose maximum tolérée par le mammifère, en particulier l'homme.

Dans un autre mode de réalisation avantageux, la composition pharmaceutique comprenant un composé de formule (I), définie dans la revendication 1, est administrable par voie orale, réparti en une ou plusieurs doses d'environ 3 mg/m² à environ 600 mg/m², préférentiellement d'environ 30 mg/m² à environ 300 mg/m², plus préférentiellement de 60 mg/m² à 150 mg/m², en particulier 120 mg/m² en fonction de la dose maximum tolérée par le mammifère, en particulier l'homme.

L'utilisation d'un véhicule pharmaceutiquement acceptable tel que la bêta cyclodextrine (β-CD) permet d'augmenter la solubilité des composés de l'invention, et par conséquent d'administrer les composés de l'invention par voie orale.
La bêta cyclodextrine possède la structure suivante : 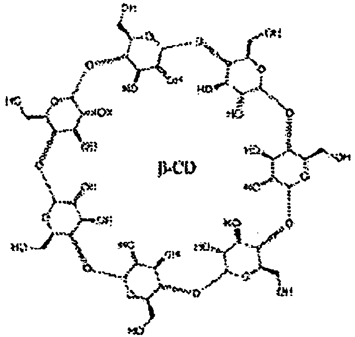 mais de l'alpha cyclodextrine ou de la gamma cyclodextrine peuvent aussi être utilisées comme un moyen d'augmenter la solubilité aqueuse du produit.

Il est bien entendu que d'autres véhicules, diluents ou excipients pharmaceutiquement acceptables peuvent être utilisés, même ceux n'améliorant pas la solubilité. Un autre exemple de véhicule pharmaceutique utilisable avec les produits de l'invention est l'albumine ou une autre protéine et/ou un autre macromolécule non protéique pharmaceutiquement acceptable, chargée du produit de l'invention à l'état natif ou sous forme de nanoparticules.

Dans un mode de réalisation avantageux, la composition pharmaceutique comprenant un composé de formule (I), définie dans la revendication 1, est administrable par exemple et sans être limité à celle-ci : par voie parentérale, intraveineuse, à une dose comprise d'environ 0,1mg/kg/j à environ 100mg/kg/j, notamment 0,1 mg/kg à 50 m/kg/j, en particulier 10 mg/kg/j.

Selon un autre aspect, l'invention décrit un composé tel que défini ci-dessus, de formule générale (I) suivante : 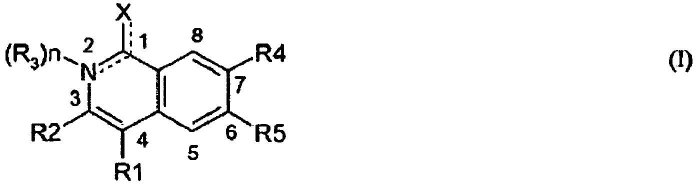 dans laquelle :
1) la liaison entre le carbone 1 et le groupement X est simple ou double, la liaison entre l'azote 2 et le carbone 1 pourra être simple ou double
   étant entendu que :
   a. lorsque la liaison entre X et ledit carbone 1 est double, alors la liaison entre l'azote 2 et le carbone 1 est simple :
      et
   b. lorsque la liaison entre X et ledit carbone 1 est simple, alors la liaison entre l'azote 2 et le carbone 1 est double et la formule I correspond à un système aromatique de formule I-A suivante : 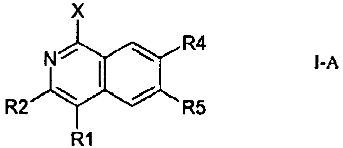
2) n représente 0 ou 1 ;
3) X représente :
   a) lorsque n = 1 :
      O, S, NH, N-(C1-C6)alkyle,
   b) lorsque n = 0 :
      OH, OPO₃H₂, OPO-(O-(C₁-C₂)alkyle)₂, l'alkyle étant éventuellement substitué par un ou plusieurs fluors, O-(C₁-C₆)alkyle, en particulier OCH₃, l'alkyle étant éventuellement substitué par un ou plusieurs fluors, O(C₃-C₆)cycloalkyle, NH2, NH-(C₁-C₆)alkyle, N-[(C₁-C₆)alkyle]₂, NH-(C₃-C₆)cycloalkyle, N-[(C₃-C₆)cycloalkyle]₂, SH, S-(C₁-C₆)alkyle, l'alkyle étant éventuellement substitué par un ou plusieurs fluors, en particulier SCH₃, S(C₃-C₆)cycloalkyle, SO₃H, NH-CH2-aryl ou heteroaryl,
      OPh, SPh, OCH₂Ph, SCH₂Ph, le phényle de ces quatre groupes pouvant être substitué ou non par un ou plusieurs (C₁-C₆)alkyle, l'alkyle étant éventuellement substitué par un ou plusieurs fluors, 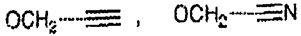
4) R1 représente :
   H, un halogène, OH, OPO₃H₂, OPO-(O-(C₁-C₂)alkyle)₂, l'alkyle étant éventuellement substitué par un ou plusieurs fluors,
   CF₃, CH₂OR', R' représentant H ou (C₁-C₆)alkyle, l'alkyle étant éventuellement substitué par un ou plusieurs fluors, CHO,
   CO₂R", R" représentant H, (C₁-C₆)alkyle, l'alkyle étant éventuellement substitué par un ou plusieurs fluors,
   CONR_{c}R_{d}, CH₂SO₂NR_{c}R_{d}, CH₂NHSO₂Rc, R_{c} et R_{d} représentant indépendamment l'un de l'autre : H, un alkyle en C₁-C₆, et NR_{c}R_{d} représente un acide aminé lié par sa fonction aminé, en particulier une sérine ou une thréonine, un cycloalkyle en C₃-C₆, ou R_{c} et R_{d} représentent ensemble un alkyle en C₂-C₆,
   NO₂ N₃, ≡, CN, C(=N)NH₂, SO₃H, SO₂NH₂, SO₂NHCH₃, NHSO₂CH₃, CH₂SO₂NR_{c}R_{d}, CH₂NHSO₂Rc, SO₂-NRaRb, SO₂-imidazole, NRₐR_{b}, où
      Rₐ et R_{b} représentent indépendamment l'un de l'autre : H, , un alkyle en C₁-C₆, un cycloalkyle en C₃-C₆, ou
   Ra est H, un alkyle en C₁-C₆, un cycloalkyle en C₃-C₆, et Rb = COR_{f}, COOR_{f} ou CONR_{f}R_{f}, R_{f} et R_{f} représentant H, un alkyle en C₁-C₆, un cycloalkyle en C₃-C₆ ou une chaîne d'un amino acide (telle que CH(CH₂OH)NH₂ pour la sérine) Rₐ et R_{b} représentent ensemble un alkyle en C₂-C₆,
   Rₐ et R_{b} peuvent former un cycle en C₅ à C₇, notamment une pyrrolidine, une pipérazine, une morpholine, une thiomorpholine,
   un hétéroaryle substitué ou non, en particulier une pyridine, un imidazole, un oxazole, un triazole, un pyrrole, un tétrazole.
5) R2 représente :
   un phényle substitué ou non par un à trois substituants choisis parmi :
      un halogène, un OH, NHRa,
      ORe, Re représentant un benzyle, un triazole méthylène substitué ou non, notamment par un alkyle en C₁-C₆,
      OPO₃H₂, OPO-(O-(C₁-C₂)alkyle)₂, l'alkyle étant éventuellement substitué par un ou plusieurs fluors, un NH₂,
      un groupe NH-CORc dans lequel Rc représente H, un alkyle en C₁-C₆, un cycloalkyle en C₃-C₆ ou une chaîne d'un amino acide (telle que CH(CH₂OH)NH₂ pour la sérine),
      un O-alkyle en C₁-C₆, en particulier OCH₃, l'alkyle étant éventuellement substitué par un ou plusieurs fluors,
      un O-COR1 où R1 représente O-alkyle en C₁-C₆, ou NRiRii, Ri et Rii pouvant être alkyle en C₁-C₆,
      un groupe alkyle en C₂-C₄, le groupe alkyle étant éventuellement substitué par un ou plusieurs fluors, un groupement alcényle en C₂-C₄ substitué ou non,
      un groupe alcynyle en C₂-C₄ substitué ou non, en particulier par un triméthylsilyle, un tert-butyle, un hydroxy-2-propyle ou un isopropyle
      un hétéroaryle substitué ou non, en particulier une pyridine, un imidazole, un oxazole, un triazole, un pyrrole, un benzofurane, un thiophène, un benzothiophène,
      un indole, un cyclohéxyle, une pipérazine, une morpholine, une thiomorpholine, une pipéridine, un groupe 4-(NH₂-(CH₂-CH₂O)ₚ)Ph dans lequel p est un entier de 1 à 6
6) R3 représente :
   H, (C₁-C₆)alkyle, l'alkyle étant éventuellement substitué par un ou plusieurs fluors, (C₃-C₆)cycloalkyle, OH, OPO₃H₂, OPO-(O-(C₁-C₂)alkyle)₂, OSO₃H, SO₃H, O-alkyl en C₁ à C₆, NH₂, NH-(C₁-C₆)alkyle, N-[(C₁-C₆)alkyle]₂, NH-(C₃-C₆)cycloalkyle, N-[(C₃-C₆)cycloalkyle]₂, un groupe propargyle, CH₂CN, (CH₂)ₚOH, p variant de 1 à 6, CH₂OPO₃H₂, CH₂OPO-(O-(C₁-C₂)alkyle)₂, CH₂CH₂F, CH₂CHF₂, CH₂CF₃,
7) R4 et R5 représentent indépendamment l'un de l'autre :
   H, OH, OPO₃H₂, OPO-(O-(C₁-C₂)alkyle)₂, l'alkyle étant éventuellement substitué par un ou plusieurs fluors, un O-alkyle en C₁-C₆, en particulier OCH₃, l'alkyle étant éventuellement substitué par un ou plusieurs fluors, un alkyle en C₁-C₆, l'alkyle étant éventuellement substitué par un ou plusieurs fluors, un cycloalkyle en C₃-C₆, un halogène,
   R4 et R5 représentent ensemble un alcényle (en C₁-C₂) dioxy éventuellement substitué par un ou plusieurs fluors,
   à l'exclusion des composés suivants :
   lorsque X=O, R3 = H, et que la liaison entre le carbone 1 et le groupement X est double, :
      a. R1 = NH₂, R2 = 4-méthoxy-phényle, R4 = OCH₃ et R5 = H,
      b. R1 = NH₂, R2 = phényle, R4 = H et R5 = H,
      c. R1 = NO₂, R2 = 4-méthoxy-phényle, R4 = OCH₃ et R5 = H,
      d. R1 = NO₂, R2 = 4-méthoxy-phényle, R4 = H et R5 = H,
      e. R1 = NH₂, R2 = 4-méthoxy-phényle, R4 = H et R5 = H,
      f. R1 = NHCHO, R2 = phényle, R4 = OCH₃ et R5 = H,
      g. R1 = NH₂, R2 = phényle, R4 = OCH₃ et R5 = H,
      h. R1 = NH₂, R2 = phényle ou 4-OH-phényle substitué ou non, R4 = H, OH, un O-alkyle en C₁-C₆, un alkyle en C₁-C₆, un cycloalkyle en C₃-C₆, et R5 = H, OH, un O-alkyle en C₁-C₆, un alkyle en C₁-C₆, un cycloalkyle en C₃-C₆,
      i. les composés dans lesquels l'un des R4 et R5 représentent un halogène.

L'invention concerne les composés objets de la revendication 27 ou 28.

Dans un mode de réalisation, la présente invention décrit un composé de formule générale (I), tel que défini ci-dessus, dans laquelle : n=0, la liaison entre le carbone 1 et le groupement X est simple et X est choisi parmi SO₃H, OCH₃, 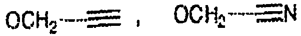 et R1 est choisi parmi H, NO₂ ou Br, ou n = 1, la liaison entre le carbone 1 et le groupement X est double, X = O ou S et R2 représente un phényle substitué en position 4 par un groupement choisi parmi OH, OCH₃, O-benzyle, Br, un groupe alcynyle en C₂-C₄ substitué ou non, en particulier par un triméthylsilyle, un tert-butyle, un hydroxy-2-propyle ou un isopropyle.

Dans un mode de réalisation, la présente invention décrit un composé de formule générale (I), tel que défini ci-dessus, dans laquelle R1 = H.

Dans un mode de réalisation, la présente invention décrit un composé tel que défini ci-dessus, de formule générale (I) suivante : 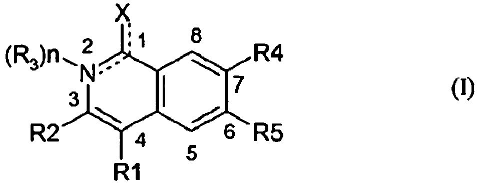 dans laquelle :
1) la liaison entre le carbone 1 et le groupement X est simple ou double, la liaison entre l'azote 2 et le carbone 1 pourra être simple ou double
   étant entendu que :
   a. lorsque la liaison entre X et ledit carbone 1 est double, alors la liaison entre l'azote 2 et le carbone 1 est simple :
      et
   b. lorsque la liaison entre X et ledit carbone 1 est simple, alors la liaison entre l'azote 2 et le carbone 1 est double et la formule I correspond à un système aromatique de formule I-A suivante : 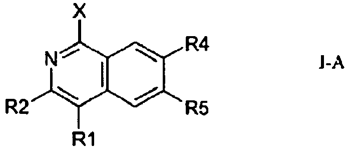
2) n représente 0 ou 1 ;
3) X représente :
   a) lorsque n = 1 :
      O, S, NH, N-(C1-C6)alkyle,
   b) lorsque n = 0 :
      OH, OPO₃H₂, OPO-(O-(C₁-C₂)alkyle)₂, l'alkyle étant éventuellement substitué par un ou plusieurs fluors, O-(C₁-C₆)alkyle, en particulier OCH₃, l'alkyle étant éventuellement substitué par un ou plusieurs fluors, O(C₃-C₆)cycloalkyle, NH2, NH-(C₁-C₆)alkyle, N-[(C₁-C₆)alkyle]₂, NH-(C₃-C₆)cycloallcyle, N-[(C₃-C₆)cycloalkyle]₂, SH, S-(C₁-C₆)alkyle, l'alkyle étant éventuellement substitué par un ou plusieurs fluors, en particulier SCH₃, S(C₃-C₆)cycloalkyle, SO₃H, NH-CH₂-aryl ou heteroaryl, OPh, SPh, OCH₂Ph, SCH₂Ph, le phényle de ces quatre groupes pouvant être substitué ou non par un ou plusieurs (C₁-C₆)alkyle, l'alkyle étant éventuellement substitué par un ou plusieurs fluors, 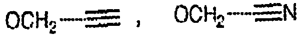
4) R1 représente :
   un halogène, OH, OPO₃H₂, OPO-(O-(C₁-C₂)alkyle)₂, l'alkyle étant éventuellement substitué par un ou plusieurs fluors,
   CF₃, CH₂OR', R' représentant H ou (C₁-C₆)alkyle, l'alkyle étant éventuellement substitué par un ou plusieurs fluors, CHO,
   CO₂R", R" représentant H, un acide aminé, (C₁-C₆)alkyle, l'alkyle étant éventuellement substitué par un ou plusieurs fluors, CONR_{c}R_{d}, R_{c} et R_{d} représentant indépendamment l'un de l'autre : H, un alkyle en C₁-C₆, un acide aminé, un cycloalkyle en C₃-C₆, ou R_{c} et R_{d} représentent ensemble un alkyle en C2-C6,
   NO₂, N₃, ≡, CN, C(=N)NH₂, SO₃H, SO₂NH₂, SO₂NHCH₃, NHSO₂CH₃, CH₂SO₂NR_{d}, CH₂NHSO₂Rc, SO₂-NRaRb, SO₂-imidazole, NRₐR_{b}, où
      Rₐ et R_{b} représentent indépendamment l'un de l'autre : H, CHO, un alkyle en C₁-C₆, un cycloalkyle en C₃-C₆, un acide aminé lié par sa fonction acide, en particulier une sérine,
      Rₐ et R_{b} représentent ensemble un alkyle en C₂-C₆,
   Rₐ et R_{b} peuvent former un cycle en C₅ à C₇, notamment une pyrrolidine, une pipérazine, une morpholine, une thiomorpholine,
   un hétéroaryle substitué ou non, en particulier une pyridine, un imidazole, un oxazole, un triazolen, un pyrrole, un tétrazole.
5) R2 représente :
   un phényle substitué ou non par un à trois substituants choisis parmi :
      un halogène, un OH, NHRa,
      ORe, Re représentant un benzyle, un triazole méthylène substitué ou non,
      OPO₃H₂, OPO-(O-(C₁-C₂)alkyle)₂, l'alkyle étant éventuellement substitué par un ou plusieurs fluors, un NH₂,
      un groupe NH-Rc dans lequel Rc représente un acide aminé lié par sa fonction acide, en particulier une sérine,
      un O-alkyle en C₁-C₆, en particulier OCH₃, l'alkyle étant éventuellement substitué par un ou plusieurs fluors,
      un groupe alkyle en C₂-C₄, le groupe alkyle étant éventuellement substitué par un ou plusieurs fluors, un groupement alcényle en C2-C4 substitué ou non,
      un groupe alcynyle en C₂-C₄ substitué ou non, en particulier par un triméthylsilyle, un tert-butyle, un hydroxy-2-propyle ou un isopropyle
      un hétéroaryle substitué ou non, en particulier une pyridine, un imidazole, un oxazole, un triazole, un pyrrole, un benzofurane, un thiophène, un benzothiophène, un indole,
      un cyclohéxyle, une pipérazine, une morpholine, une thiomorpholine, une pipéridine, un groupe 4-(NH₂-(CH₂-CH₂O)ₚ)Ph dans lequel p est un entier de 1 à 6.
6) R3 représente :
   H, (C₁-C₆)alkyle, l'alkyle étant éventuellement substitué par un ou plusieurs fluors, (C₃-C₆)cycloalkyle, OH, OPO₃H₂, OPO-(O-(C₁-C₂)alkyle)₂, O-alkyle en C₁ à C₆, NH₂, NH-(C₁-C₆)alkyle, N-[(C₁-C₆)alkyle]₂, NH-(C₃-C₆)cycloalkyle, N-[(C₃-C₆)cycloalkyle]₂, un groupe propargyle, CH₂CN, (CH₂)ₚOH, p variant de 1 à 6, CH₂OPO₃H₂, CH₂OPO-(O-(C₁-C₂)alkyle)₂, CH₂CH₂F, CH₂CHF₂, CH₂CF₃,
7) R4 et R5 représentent indépendamment l'un de l'autre :
   H, OH, OPO₃H₂, OPO-(O-(C₁-C₂)alkyle)₂, l'alkyle étant éventuellement substitué par un ou plusieurs fluors, un O-alkyle en C₁-C₆, en particulier OCH₃, l'alkyle étant éventuellement substitué par un ou plusieurs fluors, un alkyle en C₁-C₆, l'alkyle étant éventuellement substitué par un ou plusieurs fluors, un cycloalkyle en C₃-C₆, un halogène,
   R4 et R5 représentent ensemble un alcényle (en C₁-C₂) dioxy éventuellement substitué par un ou plusieurs fluors,

Dans un autre mode de réalisation, le composé ci-dessus défini de formule générale (IIa) est choisi parmi les suivants :

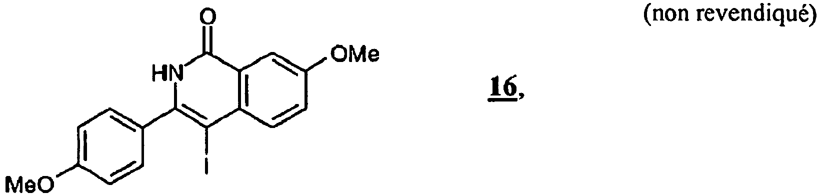

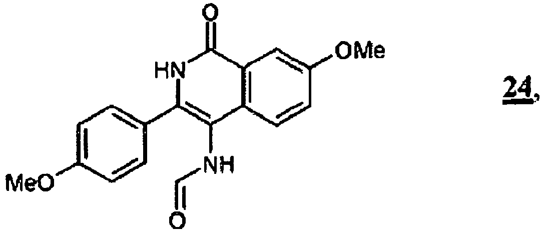

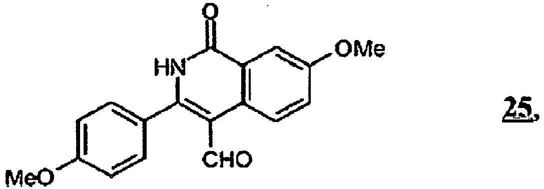

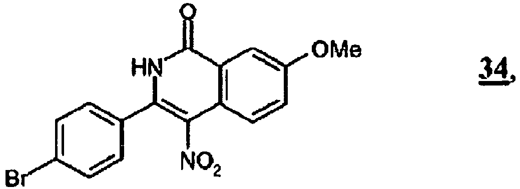

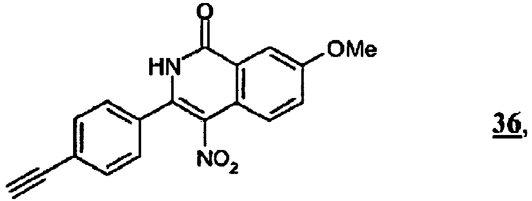

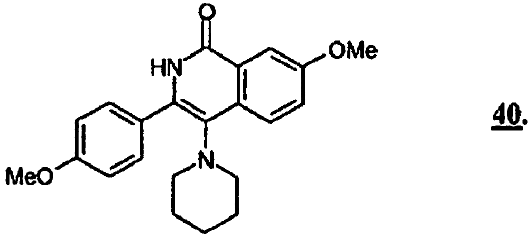

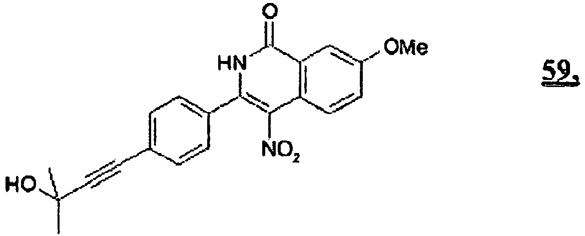

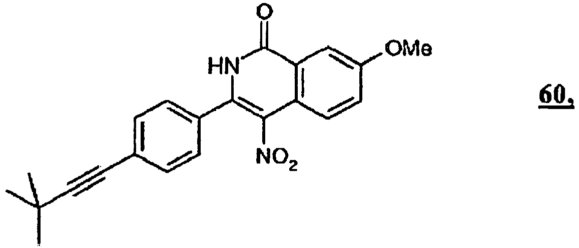

Dans un autre mode de réalisation avantageux, l'invention concerne un nouveau produit comprenant une première préparation pharmaceutique de formule (I) définie dans la revendication 1, et une seconde préparation pharmaceutique comprenant au moins l'un des antitumoraux suivants et sans être limité à ceux-ci :
l'abraxane, l'abarelix, l'aldesleukine, l'alemtuzumab, l'alitretinoine, l'allopurinol, l'altrétamine, l'anastrozole, l'arsenic trioxide, l'asparaginase, l'azacitidine, le bevacizumab, le bexarotene, le bicalutamide, la bleomycine, le bortezomib, le busulfan intraveineux, le busulfan oral, la calustérone, la capécitabine, le carboplatin, la carmustine, le cetuximab, le chlorambucil, le cisplatine, la cladribine, la clofarabine, le cyclophosphamide, la cytarabine, la dacarbazine, la dactinomycine, la daltéparine sodium, la dasatinib, la daunorubicine, la décitabine, la dénileukine, la dénileukine diftitox, le dexrazoxane, le docetaxel, la doxorubicine, la dromostanolone propionate, l'eculizumab, l'epirubicine, l'erlotinib, l'estramustine, l'etoposide phosphate, l'etoposide, l'exemestane, le fentanyl citrate, le filgrastime, la floxuridine, la fludarabine, le 5-fluorouracyl, le fulvestrant, le gefitinib, la gemcitabine, le gemtuzumab ozogamicine, la gosereline acetate, l'histrelin acetate, l'ibritumomab tiuxetan, l'idarubicine, l'ifosfamide, l'imatinib mesylate, l'interferon alfa 2a, l'irinotecan, le lapatinib ditosylate, le lenalidomide, le letrozole, leucovorine, le leuprodile acetate, le levamisole, la lomustine, la meclorethamine, le megestrol acetate, le melphalan, la mercaptopurine, le methotrexate, le methoxsalen, la mitomycine C, le mitotane, mitoxantrone, la nandrolone phenpropionate, la nelarabine, le nofetumomab, l'oxaliplatine, le paclitaxel, le pamidronate, le panitumumab, la pegaspargase, le pegfilgrastim, le pemetrexed disodium, la pentostatine, le pipobroman, la plicamycine, le procarbazine, la quinacrine, la rasburicase, le rituximab, le sorafenib, la streptozocine, le sunitinib, le sunitinib maleate, le tamoxifène, le taxol, le taxotère, la temozolomide, le teniposide, la testolactone, la thalidomide, la thioguanine, le thiotepa, le topotecan, le toremifene, le tositumomab, le trastuzumab, le tretinoine, l'uracil moutarde, la valrubicine, la vinblastine, la vincristine, la vinorelbine, le vorinostat, et le zoledronate.
en tant que préparation combinée pour une utilisation simultanée, séparée ou séquentielle dans le traitement de mammifères, en particulier l'homme, atteints de tumeurs bénignes ou malignes (cancéreuses).
Dans un mode de réalisation avantageux, l'invention concerne un nouveau produit comprenant une première préparation pharmaceutique de formule (I) définie dans la revendication 1, et une seconde préparation pharmaceutique comprenant l'un au moins des antitumoraux ci-dessus définis en tant que préparation combinée pour une utilisation simultanée, séparée ou séquentielle dans le traitement de mammifères, en particulier l'homme, atteints de tumeurs bénignes ou malignes (cancéreuses).

Selon un autre aspect, l'invention décrit un procédé de préparation de composés de formule IIa tel que définis ci-dessus, comprenant les étapes suivantes :
∘ pour X=O:
   a) condensation d'un acide arylacétique sur un arylcarboxaldéhyde pour former un acide 2,3-diarylacrylique intermédiaire,
   b) transformation du groupement acide en azide correspondant par NaN₃ via un anhydride mixte,
   c) cyclisation par voie thermique de l'azide pour conduire à la 3-arylisoquinolin-1(2H)-one,
   d) aménagement des groupements fonctionnels selon les protocoles détaillés plus loin.
∘ pour X=S,
   a) à partir de la 3-arylisoquinolin-1(2H)-one, la synthèse d'isoquinolin-1(2H)-thione est réalisée avec le réactif de Lawesson,
   b) à partir de la 3-aryl-4-nitroisoquinolin-1(2H)-one, la méthode de synthèse d'isoquinolin-1(2H)-thione comporte les étapes principales suivantes :
      i. chloruration par l'oxyde chorure de phosphore ou par le réactif de Vilsmeier pour former la 1-chloroisoquinoléine intermédiaire,
      ii. transformation du groupement chloro en groupement thione avec la thiourée.

Selon un autre aspect, l'invention concerne un procédé de préparation de composés de formule IIb tels que définis ci-dessus, comprenant les étapes suivantes :
∘ à partir de la 3-arylisoquinolin-1(2H)-one : la synthèse d'isoquinoline IIb est réalisée avec l'haloakyle voulu en présence d'une base telle que K₂CO₃;
∘ à partir de la 1-chloro-4-nitroisoquinoléine dont sa synthèse a été mentionnée plus haut : la synthèse d'isoquinoline IIb est réalisée avec le nucléophile voulu en milieu basique.

### DESCRIPTION DES FIGURES

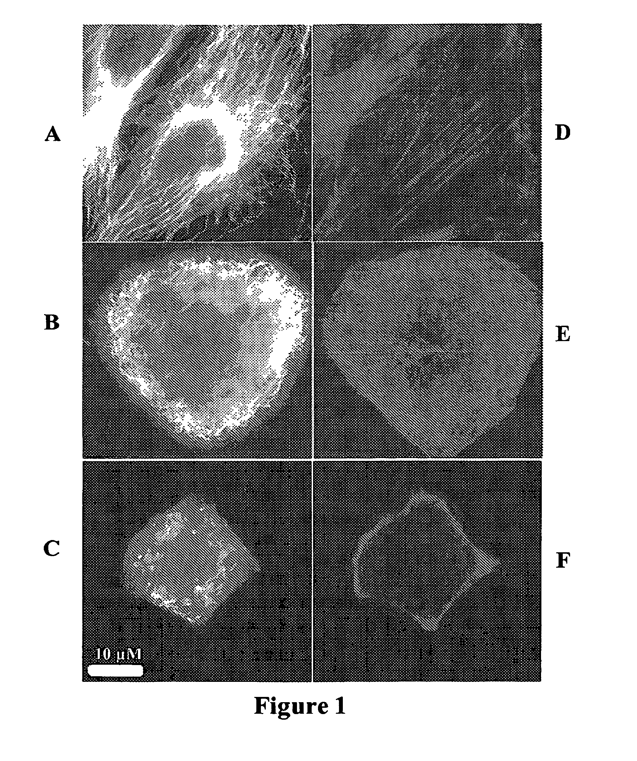

Les figures 1A à 1F représentent les images d'immunofluorescence indirecte montrant les phénotypes induits par le composé 6 sur les microtubules et le cytosquelette d'actine des cellules HeLa en interphase. Ce composé induit une dépolymérisation des microtubules et une réorganisation du cytosquelette d'actine (renforcement de l'actine au niveau du cortex cellulaire et diminution des fibres de stress).
Les figures 1A à 1C représentent les microtubules marqués par un anticorps anti α-tubuline et les figures 1D à 1F représentent la F-actine marquée par un conjugué de phalloïdine Alexa Fluor® 568.
Les figures 1A et 1D représentent les cellules contrôles incubées avec du DMSO.
Les figures 1B et 1E représentent les cellules incubées avec le composé 6 à une concentration de 1µM pendant 24h.
Les figures 1C et 1F représentent les cellules incubées avec le composé 6 à une concentration de 10µM pendant 24h.
Les figures 2A à 2I représentent les images d'immunofluorescence indirecte montrant les phénotypes induits par le composé 6 sur les microtubules et le cytosquelette d'actine des cellules HeLa en mitose. Ce composé induit une dépolymérisation des microtubules du fuseau et des bougeonnements d'actine au niveau du cortex (« blebbing »).

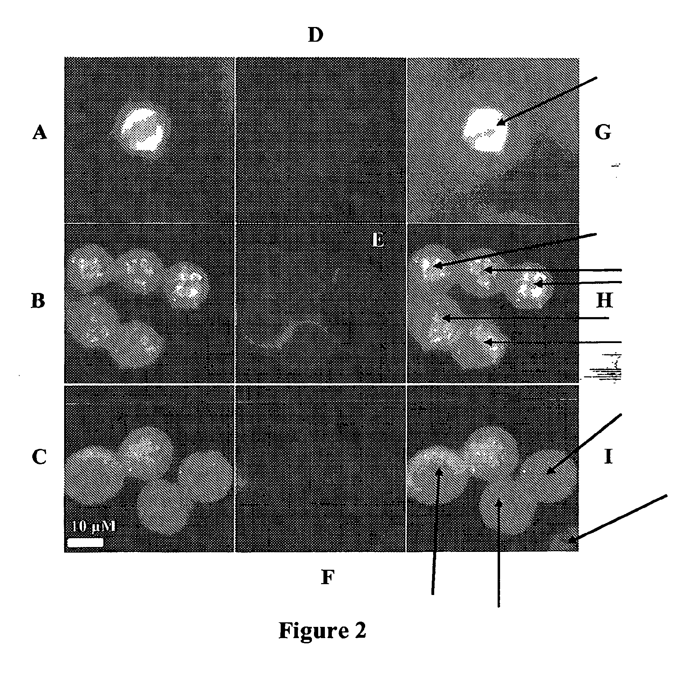

Les figures 2A à 2C représentent les microtubules marqués par un anticorps anti α-tubuline. Les figures 2D à 2F représentent la F-actine marquée par un conjugué de phaltoïdine Alexa Fluor® 568. Les figures 2G à 2I représentent la superposition des 2 images montrant la tubuline et l'actine, avec en plus l'ADN mitotique (flèches noires correspondant à l'ADN marqué avec du Hoechst 33258).
Les figures 2A, 2D et 2G représentent les cellules contrôles, incubées avec du DMSO.
Les figures 2B, 2E et 2H représentent les cellules incubées avec le composé 6 à une concentration de 1µM pendant 24h.
Les figures 2C, 2F et 2I représentent les cellules incubées avec le composé 6 à une concentration de 10µM pendant 24h.

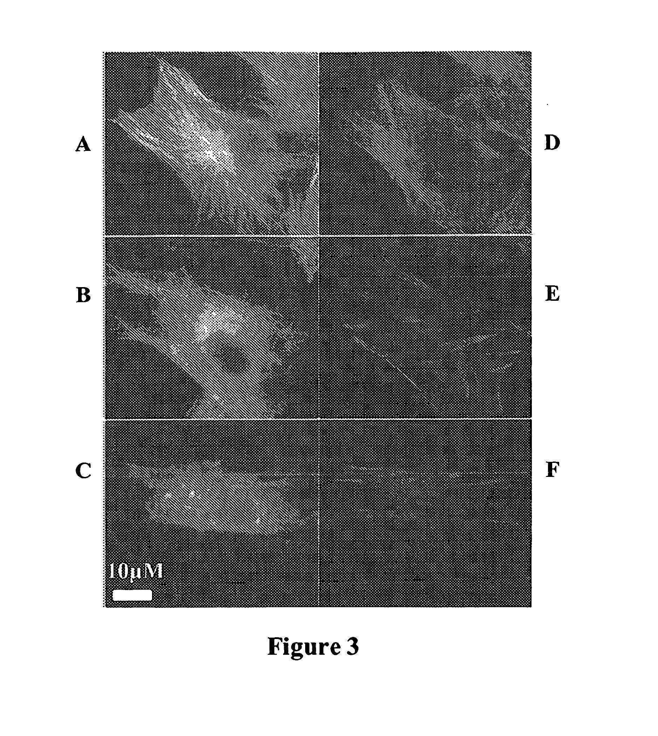

Les figures 3A à 3F représentent les images d'immunofluorescence indirecte montrant les phénotypes induits par le composé 6 sur les microtubules et le cytosquelette d'actine des fibroblastes humains primaires en interphase. Ce composé induit une dépolymérisation des microtubules et une réorganisation du cytosquelette d'actine (léger renforcement de l'actine au niveau du cortex cellulaire).
Les figures 3A à 3C représentent les microtubules marqués par un anticorps anti α-tubuline et les figures 3D à 3F représentent la F-actine marquée par un conjugué de phalloïdine Alexa Fluor® 568.
Les figures 3A et 3D représentent les cellules contrôles, incubées avec du DMSO.
Les figures 3B et 3E représentent les cellules incubées avec le composé 6 à une concentration de 5uM pendant 24h.
Les figures 3C et 3F représentent les cellules incubées avec le composé 6 à une concentration de 10µM pendant 24h.

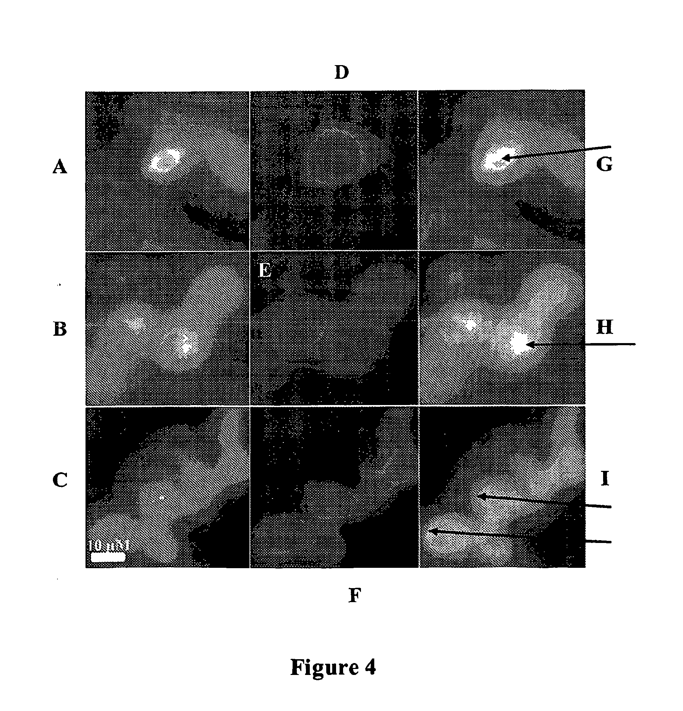

Les figures 4A à 4I représentent les images d'immunofluorescence indirecte montrant les phénotypes induits par le composé 6 sur les microtubules et le cytosquelette d'actine des fibroblastes humains primaires en mitose. Ce composé induit une dépolymérisation des microtubules du fuseau et la formation de sillons ectopiques.
Les figures 4A à 4C représentent les microtubules marqués par un anticorps anti α-tubuline, les figures 4D à 4F représentent la F-actine marquée par un conjugué de phalloïdine Alexa Fluor® 568. Les figures 4G à 4I représentent la superposition des 2 images montrant la tubuline et l'actine, avec en plus l'ADN mitotique (flèches noires correspondant à l'ADN marqué avec du Hoechst 33258).
Les figures 4A, 4D et 4G représentent les cellules contrôles, incubées avec du DMSO.
Les figures 4B, 4E et 4H représentent les cellules incubées avec le composé 6 à une concentration de 5µM pendant 24h.
Les figures 4C, 4F et 4I représentent les cellules incubées avec le composé 6 à une concentration de 10µM pendant 24h.

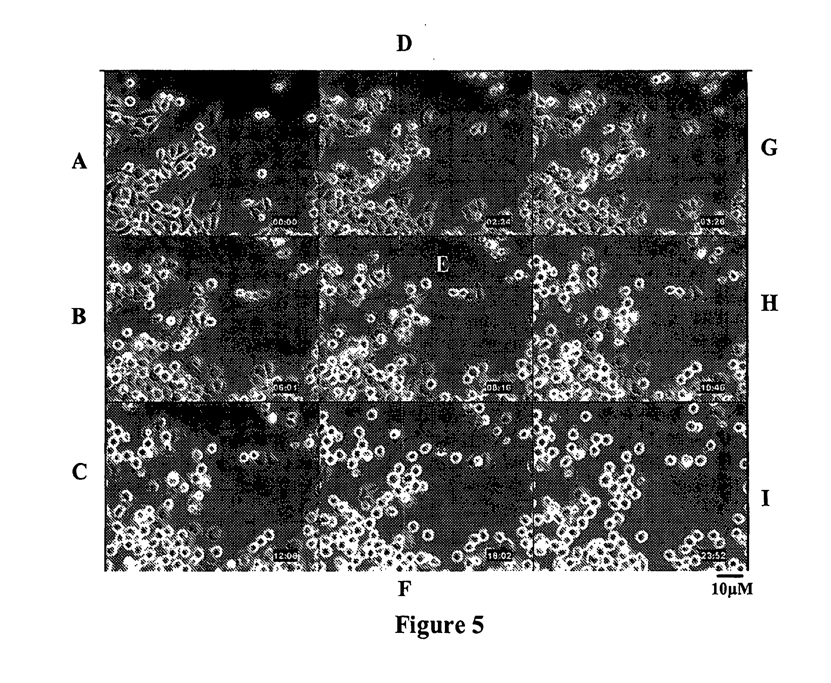

Les figures 5A à 5I illustrent l'arrêt mitotique induit par le composé 6 sur des cellules HeLa, en microscopie à contraste de phase pendant 24h (vidéomicroscopie).

Elles montrent l'accumulation de cellules HeLa arrondies en présence du composé 6 (0,5µM).

Les images ont été prises dans le même champ, toutes les 5 minutes pendant 24h en utilisant un objectif Neofluar 20x (Zeiss).

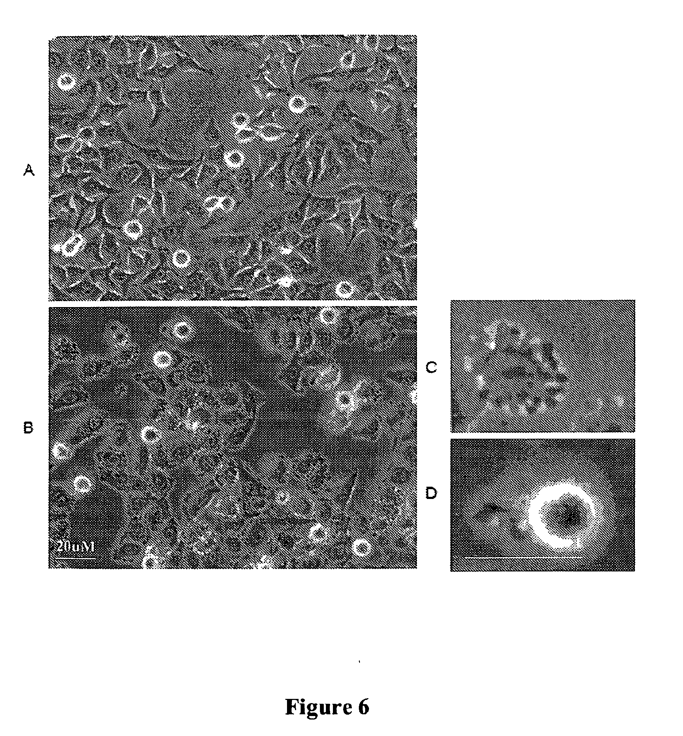

Les figures 6A à 6D présentent les images de microscopie à contraste de phase montrant les bourgeonnements corticaux induits par le composé 6 (0,5µM) sur des cellules HeLa (en interphase et en mitose).
Figure 6A : Cellules contrôles, incubées avec du DMSO.
Figure 6B : Cellules après 3h d'incubation avec le composé 6.
Figure 6C : zoom d'une cellule adhérente interphasique après 3h d'incubation avec le composé 6 (provenant de la figure 6B).
Figure 6D : zoom d'une cellule arrondie après 3h d'incubation avec le composé 6 (provenant de la figure 6B).

Acquisition des images avec un objectif Neofluar 20x (Zeiss).

La figure 7 présente le graphe montrant l'activité antiproliférative du composé 6 sur différents types cellulaires.

Elle montre la concentration du composé 6 nécessaire pour inhiber de 50% (GI50 pour Growth Inhibition 50) la prolifération de différentes cellules ou lignées cellulaires.

Les valeurs sont indiquées en moyenne ± SEM de deux expériences effectuées en triplicat.

La figure 8 présente le graphe montrant l'activité antiproliférative du composé 6 sur les HUVEC et la lignée cellulaire HTB177.

Elle montre la concentration du composé 6 nécessaire pour inhiber de 50% la prolifération des cellules HTB177 (lignée cellulaire la plus sensible au composé 6) et les HUVEC (cellules endothéliales de veine ombilicale humaine).

Les valeurs sont indiquées en moyenne ± SEM de deux expériences effectuées en triplicat.

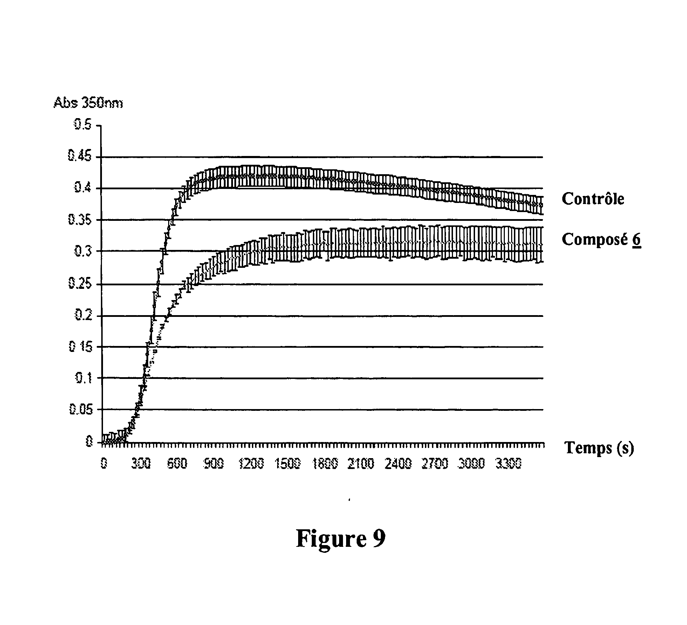

La figure 9 présente le graphe montrant l'effet du composé 6 (50µM) sur la polymérisation de la tubuline *in vitro.*

Le contrôle est effectué avec du DMSO 0,5% ce qui correspond à la quantité de DMSO ajoutée avec la molécule 6 car cette dernière est solubilisée dans du DMSO.

Les valeurs sont indiquées en moyenne ± SEM de deux expériences effectuées en triplicat.

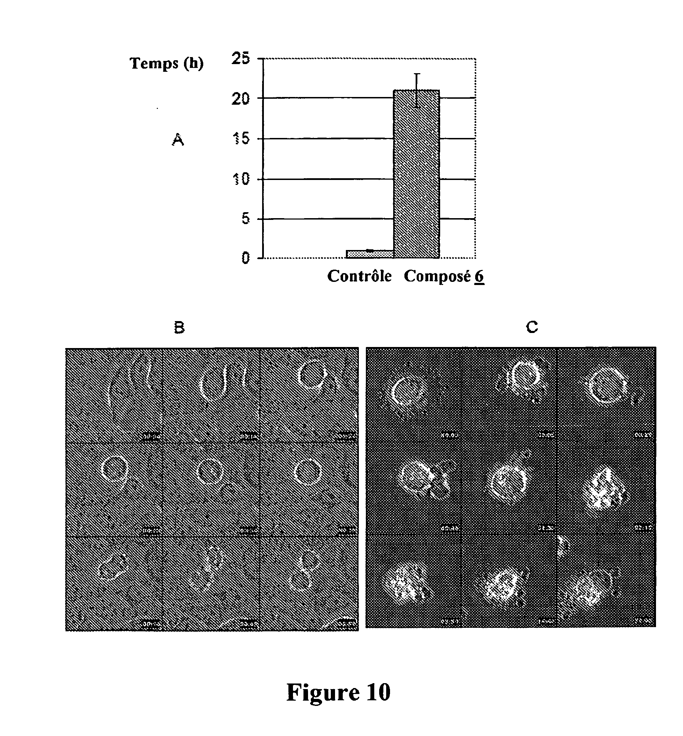

Les figures 10A à 10C présentent le graphe montrant la durée de la mitose de cellules HeLa en présence du composé 6 ainsi que l'illustration en microscopie à contraste de phase du devenir de ces cellules arrêtées en mitose.

Le composé 6 augmente la durée de la mitose dans les cellules HeLa par rapport au contrôle (DMSO).
Figure 10A : la mitose dure 22h en présence de 0,5µM de composé 6 alors qu'elle dure 1h dans les conditions contrôles.
Figure 10B : contrôle. Au bout d'1h la cellule se divise en 2 cellules-filles.
Figure 10C : suivi par vidéomicroscopie (à contraste de phase) d'une mitose en présence de 0,5µM du composé 6. La mitose dure 22h avec une forte activité de bourgeonnement membranaire jusqu'à ce que la cellule meurt. 80% des cellules mitotiques observées montrent un bourgeonnement intense, 20% des cellules restant arrondies.

Les cellules ont été filmées pendant 48h (acquisition toute les 5 minutes) avec un objectif Neofluar 40x (Zeiss).

Les valeurs de la figure 10A sont des moyennes ± SEM de deux expériences dans lesquelles 50 cellules mitotiques ont été suivies.

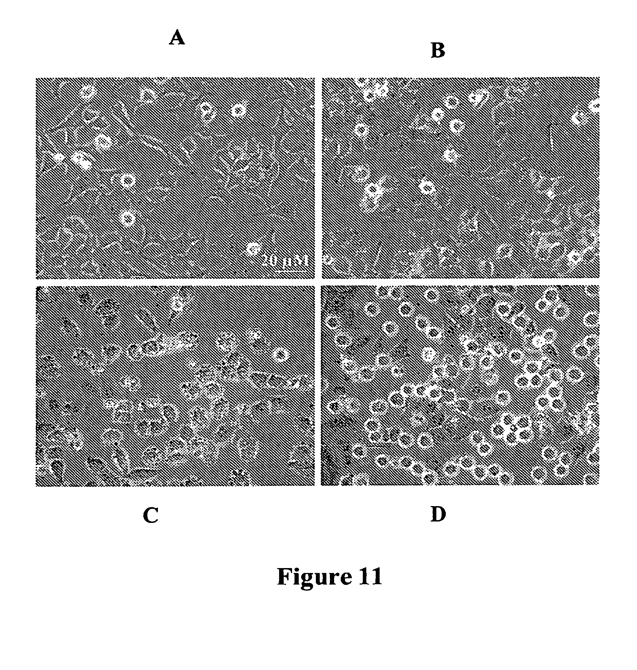

Les figure 11A à 11D présentent les images de microscopie à contraste de phase montrant que la suppression simultanée des trois isoformes de PP1 (α+β+γ) dans des cellules HeLa, par des siRNA, reproduit le phénotype induit par le composé 6.

La supression simultannée des trois isoformes de PP1 par les siRNA induit un bourgeonnement membranaire des cellules HeLa 48h après transfection.
Figure 11A : cellules contrôle.
Figure 11B : cellules HeLa incubées avec le composé 6 à 0,5µM pendant 3h.
Figure 11C : la suppression des trois sous unités catalytiques de PP1 conduit à l'apparition d'un phénotype bourgeonnement membranaire identique à celui de la figure 11B.
Figure 11D : la suppression du domaine catalytique de PP2A par le siRNA correspondant, induit une accumulation de cellules arrondies.

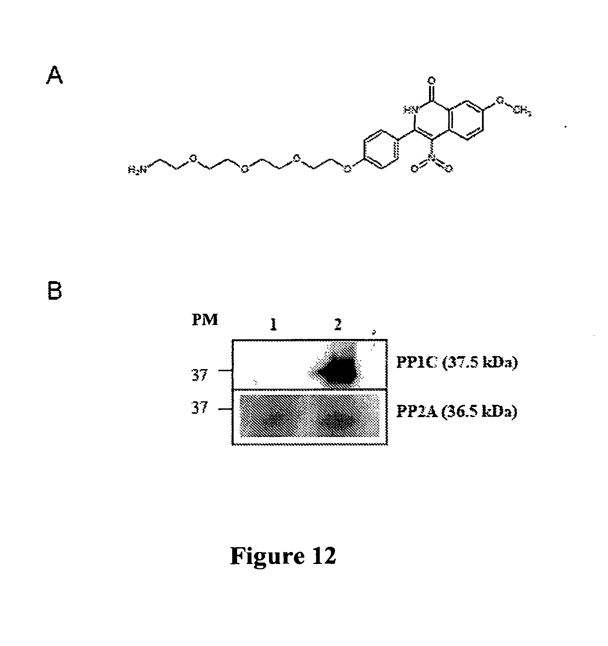

Les figures 12A et 12B présentent le composé 37 qui retient spécifiquement le(s) domaine(s) catalytique (s) de PP1.
   (A) La structure du composé 37 (correspond au composé 6 avec un groupe PEG).
   (B) Le composé 37 immobilisé sur une résine de sépharose retient spécifiquement PP1C.

Immunoblot des éluats des conditions 1 et 2 avec des anticorps anti-PP1C et anti-PP2A.
1 : Sépharose N-HydroxySuccinimide bloquée par la monoéthanolamine (contrôle).
2 : Le composé 37 couplé à la Sépharose NHS.
Il faut noter l'abscence de PP1C dans le contrôle et des quantités similaires de PP2A (à l'état de traces), dans les deux conditions (PM : poids moléculaire).

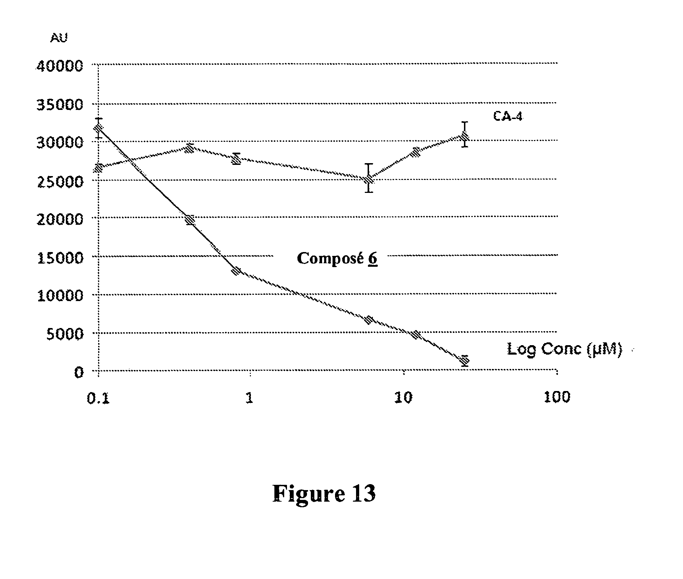

La figure 13 présente le graphe montrant l'inhibition de l'activité PP1Cα par le composé 6 *in vitro.* PP1Cα (New England Biolabs, 30 mU/puit) a été incubée en présence du composé 6 à différentes concentrations et d'un substrat fluorescent, le diFMUP.

Il faut noter que la combretastatine A-4 (CA-4) (substance utilisée comme contrôle négatif) n'inhibe pas l'activité de PP1.

Les valeurs sont indiquées en moyenne ± SEM de deux expériences effectuées en triplicat. AU : Unités arbitraires.

La figure 14 présente l'évolution du poids de souris nude durant 63 jours. Les souris ont reçu une administration du composé 6 (200 mg/kg) *per os* deux fois par semaine pendant 2 mois.

Le composé 6 a été dissout à 8mg/ml dans une solution aqueuse de beta-cyclodextrine à 60 mg/ml (véhicule).

Le composé 6 n'a pas d'effet significatif sur le poids des souris nude.
Les figures 15A à 15C présentent les images de tumeurs humaines (HTB-177 : carcinome pulmonaire) xenogreffées sur des souris nude après traitement avec le composé 6 (40 mg/kg administré 2 fois par semaine *per os*) ou le véhicule seul.
Figure 15A : tumeur contrôle au jour 7.
Figure 15B : tumeur traité avec le composé 6 au jour 7.
Figure 15C : tumeur traité avec le composé 6 au jour 20.

Les animaux xénogreffés par HTB-177 traités oralement avec le composé 6 montrent une nécrose centrale déjà après un cycle d'administration du composé 6 (jour 7). Il faut noter que les animaux contrôles n'ont pas de signe de nécrose.

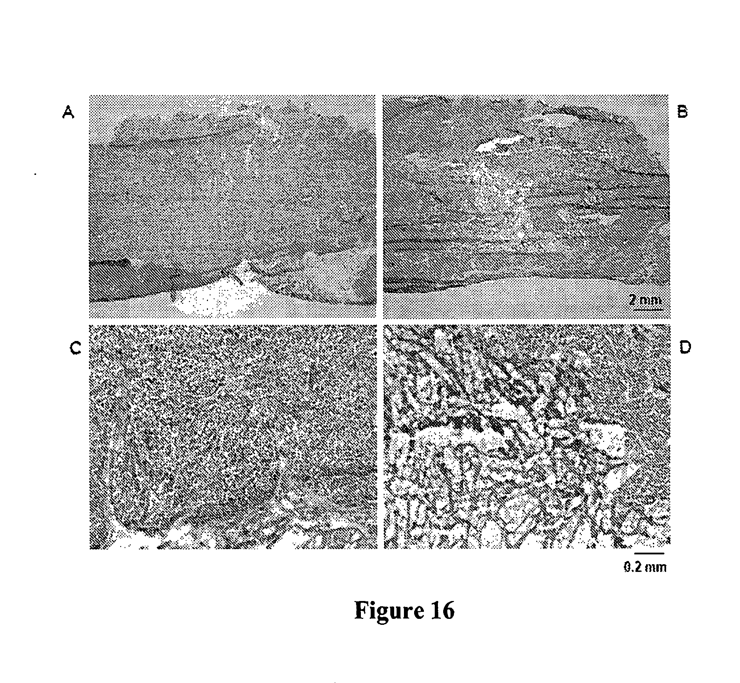

Les figures 16A à 16D présentent les photographies montrant la nécrose de tumeurs xenogreffées (HTB177) 48h après administration du composé 6 (40 mg/kg). Les coupes de tumeurs (10µm d'épaisseur) ont été colorées à l'H&E (heamatoxiline-eosine). Les coupes de tumeurs provenant d'animaux traités avec le composé 6 montrent des zones étendues de nécrose alors que les tissus tumoraux contrôles sont intacts.
Les figures 16A et 16C présentent des coupes de tumeurs obtenues à partir d'animaux contrôles.
Les figures 16B et 16D présentent des coupes de tumeurs après traitement avec le composé 6.

### Matériel et Méthodes

### Exemple 1 : Culture cellulaire

Tous les milieux de culture ont été enrichis avec du Sérum Bovin Foetal (10%), de la Pénicilline (100U/ml) et de la Streptomycine (100 mg/ml). Les cellules sont cultivées en étuve à 37°C en présence de 5% de CO₂.

Les fibroblastes humains primaires (PHF) ont été préparés à partir de prépuce de nouveau-né comme décrit dans (Nahm WK et al., J Dermatol Sci. 2002, 28(2): pp 152-8).

Les lignées cellulaires suivantes : HeLa (ATCC # CCL-2, carcinome cervical humain), NCI-H460 (ATCC # HTB-177, carcinome pulmonaire humain à cellules larges), 786-O (ATCC # CRL-1932, adénocarcinome rénale humain), RT-112 (Benham F et al., J Histochem Cytochem. 1977, 25: pp 266-74, carcinome de vessie humain), HMEC (Pasquier E et al., Mol Cancer Ther 9(5) 2010, pp 1408-18: ENMD-1198, a new analogue of 2-methoxyestradiol, displays both antiangiogenic and vascular-disrupting properties) ont été cultivées dans un milieu RPMI 1640.

Les lignées cellulaires suivantes : U87 (ATCC # HTB-14, glioblastome humain), HT-29 (ATCC # HTB-38, adénocarcinome du colon humain de grade II), MCF7 (ATCC # HTB-22, adénocarcinome du sein humain) ont été cultivées dans un milieu DMEM. Pour les MCF7, le milieu a été enrichi avec de l'insuline (0,01 mg/ml).

Les cellules A549 (ATCC #CCL-185, carcinome de poumon humain) ont été cultivées dans un milieu F-12K.
Les HUVEC (Cellules Endothéliales de veine ombilical Humaine, PromoCell # c-12203) ont été cultivées dans un milieu de croissance de cellule endothéliale recommandé par le fournisseur (Promocell # c-22010).

### Exemple 2: Tests in vitro et in vivo des molécules de l'invention

Pour les études *in vitro,* toutes les molécules ont été resuspendues dans du DMSO anhydre pour donner une solution stock à 10mM et stockées en aliquots à -20°C.

Pour les expérimentations *in vivo,* la molécule 6 (8mg/ml) a été préparée dans 60 mg/ml de β-cyclodextrine (Sigma # 4805) dissoute dans l'eau et vortexée.

### Exemple 3 : Extraits d'oeuf de Xénope

Les extraits cytoplasmiques d'oeufs de Xénope ont été préparés à partir d'oeufs infertilisés de *Xenopus laevis* selon la méthode decrite (Desai A et al., Methods Cell Biol. 1999; 61: pp 385-412).

### Exemple 4 : Analyse des cellules par immunofluorescence indirecte

Les cellules ont été cultivées sur des lamelles dans des plaques 24 puits et incubées pendant 24 heures avec la molécule 6 à différentes concentrations de 1 à 10 µM.

Des quantités correspondantes de DMSO ont été ajoutées au milieu de culture dans les puits contrôles.

Les cellules ont été fixées dans une solution de PBS avec 4% de paraformaldéhyde (PFA) et 0,1% de glutaraldéhyde (GA), pendant 15 minutes et centrifugées à 300g pendant les 10 dernières minutes de la fixation. Les lamelles ont été rincées dans du PBS et les groupes aldéhydes libres ont été réduits par deux incubations successives de 5 minutes dans du PBS contenant 1 mg/ml de NaBH4.

Après un lavage avec du PBS puis un autre avec du PBST (PBS avec 0,5% de triton-X-100), les cellules sont incubées 30 minutes à température ambiante avec la solution d'anticorps monoclonal anti α-tubuline (DM1A clone, Sigma # T6199) dilué à 1:200 dans du PBST. Après 3 lavages de 10 minutes au PBST, les cellules sont incubées 30 minutes à température ambiante dans une solution d'anticorps polyclonaux de chèvre anti souris couplés à l'Alexa 488 (1:1000, Molecular Probes # A-11029) et de phalloidine couplé au Texas-red pour marquer l'actine (1 :2000, Molecular Probes # T7471), dans du PBST contenant aussi du Hoechst 33258 (1 µg/ml) pour le marquage de l'ADN. Après 3 lavages de 10 minutes dans du PBST, les lamelles sont montées sur lames dans du milieu de montage Fluorsave™ (Calbiochem # 345789) et sont observées et analysées à l'aide d'un microscope à fluorescence inversé Axiovert 200M (Zeiss®). Les images sont réalisées à l'objectif à huile Plan-Apochromat 63x (Zeiss®) grâce à une caméra Coolsnap HQ (Roper scientifics®) et au logiciel d'acquisition Metamorph (Universal Imaging™ Corporation).

Afin d'évaluer l'effet des analogues du composé 6 sur les PHF et les cellules HeLa, la technique d'immunofluorescence décrite ci-dessus à été utilisée et adaptée à un format de boite 96 puits. Les cellules ont été encemensées dans des boites 96 puits à fond de verre et incubées 24h en présence de différentes concentrations de molécules de 100 µM à 50 nM. Et de la même façon que décrite ci-dessus, les cellules ont été fixées et immunomarquées. L'analyse des phénotypes ainsi que le comptage des cellules mitotiques ont été réalisés avec le même microscope mais en utilisant un objectif x40 (Olympus). Les molécules ont été comparées entre elles par rapport à l'arrêt mitotique induit sur les PHF et les cellules HeLa à 25 et 0,3 µM.

Pour la vidéomicroscopie en z séries, les cellules HeLa ont été cultivées dans des boites à fond de verre (Iwaki, Milian # BY-3910035). Le système comporte : un microscope inversé AXIOVERT 200M (Zeiss®), une chambre d'incubation thermostatée qui maintient une température de 37°C et un taux constant de 5% de CO₂, une plateforme motorisée et une caméra Cool SNAP HQ (Roper Scientifics®) couplée au système d'acquisition et d'analyse Metamorph (Universal Imaging™ Corporation). Les études ont été réalisées avec les objectifs x20 ou x40 (Neofluar Zeiss).

Dans un premier temps, les champs d'acquisitions ont été choisis et gardés en mémoire, puis les paramètres d'acquisition ont été définis, comme la durée de l'expérience (48 heures), l'intervalle entre les prises d'images (5 minutes),ou le nombre de coupes en profondeur z (9 coupes). A la fin des 48 heures d'enregistrement, les piles d'acquisitions ont été reconstituées avec l'image en z ayant le meilleur focus, grâce au logiciel Metamorph.

### Exemple 5 : Test de prolifération cellulaire

Les cellules ont été encemensées dans des plaques 96 puits (1000 par puit) et incubées avec différentes concentrations du composé 6 (1 nM à 100µM), durant 5 jours (120 heures). A la fin de cette incubation, du Hoechst 33342 (1 µg/ml) a été ajouté dans le milieu de culture et les cellules vivantes ont été comptabilisées sous un microscope à fluorescence (Zeiss Axiovert 200M). Les données représentent la moyenne de deux expériences indépendantes durant lesquelles 10 champs choisis de façon aléatoire ont été analysés pour chaque concentration de composé 6, elles-mêmes réalisées en triplicat.

### Exemple 6 : Purification de la tubuline de cerveau de boeuf et test de Polymérisation in vitro

La tubuline a été purifiée à partir de cerveau de boeuf en utilisant le procédé de polymérisation-dépolymérisation décrit par (Castoldi M and Popov AV. Protein Expr Purif. 2003, 32(1): pp 83-8).

Les tests de polymérisation *in vitro* de la tubuline (à 50 µM) ont été réalisés en présence de GTP 1mM dans du tampon BRB80 (80 mM K-PIPES pH 6,8, 1 mM MgCl2, 1 mM EGTA) à 37°C. Les tests ont été effectués dans des microplaques 96 puits (demi-puits) (Costar plates, Corning # 3695), en triplicats avec un volume final de 70 µl. L'assemblage de la tubuline à été réalisée en présence du composé 6 ou de ses analogues (tous utilisés à une concentration de 50 µM) et a été suivi par mesure de la turbidimétrie à 350 nm au spectrophotomètre (Spectramax Plus, Molecular Devices).

Les résultats obtenus *in vitro* dans les exemples 4, 6 9 et avec les composés de l'invention sont répertoriés dans le tableau I ci-dessous

**TABLEAU I**

| **N°** | **Code** | **Structure** | **Formule brute** | **PM** | **Inhibition PP1 IC50** | **Inhibition de la polymérisation de la Tubuline** | **Effet sur les Hela à 25 µM** | **G150 sur HMEC** |
|---|---|---|---|---|---|---|---|---|
| 1 | ICC128-L-004-H09 | 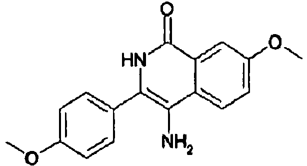 | C₁₇H₁₆N₂O₃ | 296,32 | - | - | *** | ++ |
| 2 | ICC108-L-006-C05- | 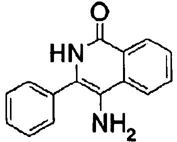 | C₁₅H₁₂N₂O | 236,28 | - | - | - | + |
| 4 EC | ICC123-L-022-C04- | 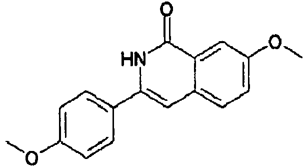 | C₁₇H₁₅NO₃ | 281,3 | - | - | - | |
| | | | | | | | mais effet ** à 50 et 100 µM | |
| 5 EC | ICC103-L-030-D05- | 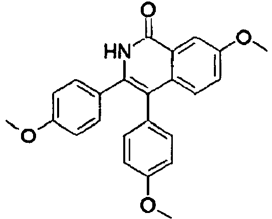 | C₂₄H₂₁NO₄ | 387,4 | - | +++ | *** | +++ |
| 6 | ICC128-L-013-B06- | 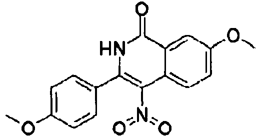 | C₁₇H₁₄N₂O₅ | 326,3 | ++++ | + | **** | ++++ |
| 7 | ICC108-L-006-G06- | 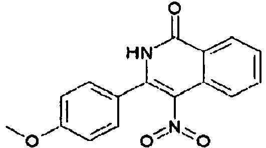 | C₁₆H₁₂N₂O₄ | 296,27 | - | + | *** | ++ |
| 8 | ICC108-L-006-G08- | 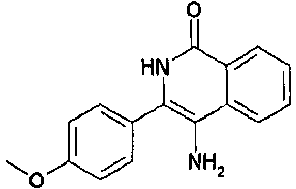 | C₁₆H₁₄N₂O₂ | 266,29 | - | - | - | - |
| 9 | ICC108-L-006-H06- | 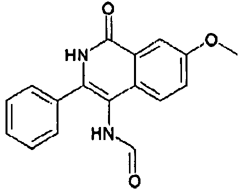 | C₁₇H₁₄N₂O₃ | 294,3 | - | - | - | + |
| | | | | | | | mais effet ** à 50 et 100 µM | |
| 10 | ICC108-L-006-H03- | 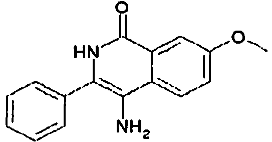 | C₁₆H₁₄N₂O₂ | 266,29 | - | + | - | - |
| | | | | | | | mais effet * à 50 et 100 µM | |
| 12 EC | CH1583A | 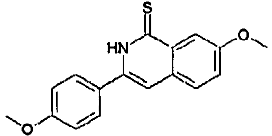 | C₁₇H₁₅NO₂S | 297,37 | ++++ | ++ | - | ++ |
| | | | | | | | mais effet * à 100 µM | |
| 13 EC | CH1585 | 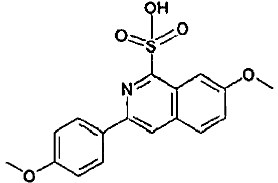 | C₁₇H₁₅NO₅S | 345,37 | - | ++ | - | - |
| | | | | | | | mais effet * à 100 µM | |
| 14 EC | CH1587A | 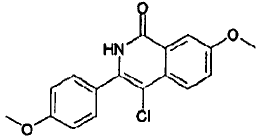 | C₁₇H₁₄CLNO₃ | 315,75 | - | +++ | - | + |
| 15 EC | CH1584A | 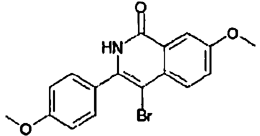 | C₁₇H₁₄BrNO₃ | 360,2 | - | ++ | *** | +++ |
| 16 EC | CH1586 | 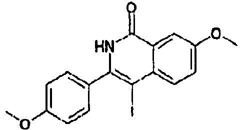 | C₁₇H₁₄INO₃ | 407,2 | - | +++ | *** | +++ |
| 17 EC | CH1576 | 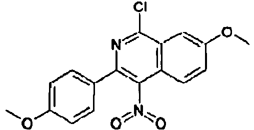 | C₁₇H₁₃ClN₂O₄ | 344,75 | - | ++ | - | + |
| 18 | CH1590 | 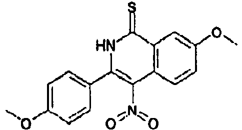 | C₁₇H₁₄N₂O₄S | 342,4 | +++ | +++ | **** | ++ |
| 19 | CH1577 | 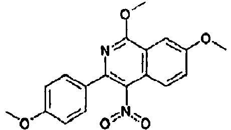 | C₁₈H₁₆N₂O₅ | 340,33 | ++ | + | - | - |
| | | | | | | | mais effet ** à 100 µM | |
| 20 EC | CH1591 | 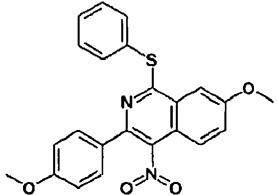 | C₂₃H₁₈N₂O₄S | 418,46 | ++ | ++ | - | - |
| | | | | | | | mais effet * 50 et 100 µM | |
| 21 EC | CH1592 | 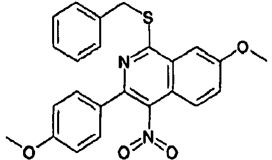 | C₂₄H₂₀N₂O₄S | 432,49 | ++ | +++ | - | - |
| 22 EC | CH1594 | 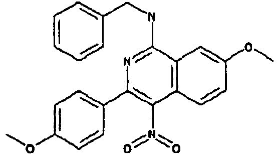 | C₂₄H₂₁N₃O₄ | 415,44 | ++++ | + | - | - |
| 23 | DN78-2 | 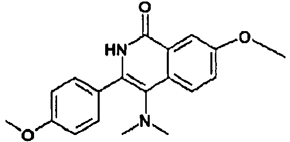 | C₁₉H₂₀N₂O₃ | 324,37 | - | + | *** | ++ |
| 24 | DN86 | 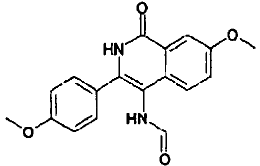 | C₁₈H₁₆N₂O₄ | 324,33 | - | + | **** | ++ |
| 25 | DN77-1 | 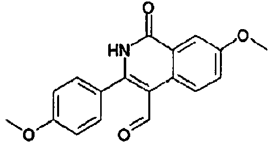 | C₁₈H₁₅NO₄ | 309,3 | + | +++ | **** | +++ |
| 26 EC | PJ23 | 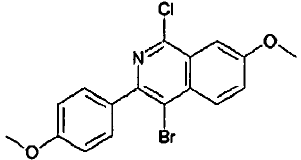 | C₁₇H₁₃BrClN O₂ | 378,65 | - | - | - | - |
| 27 | PJ24 | 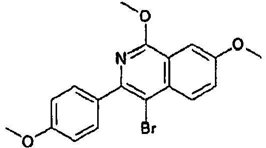 | C₁₈H₁₆BrNO₃ | 374,23 | - | + | - | + |
| 28 EC | PJ38f2 | 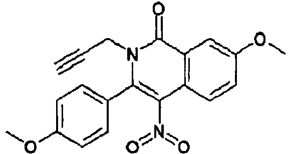 | C₂₀H₁₆N₂O₅ | 364,35 | ++ | - | - | - |
| | | | | | | | mais effet ** à 50 et 100 µM | |
| 29 EC | PJ38f1 | 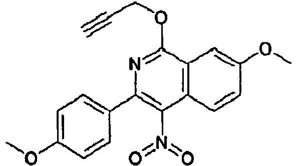 | C₂₀H₁₆N₂O₅ | 364,35 | + | + | - | - |
| 30 | PJ46f2 | 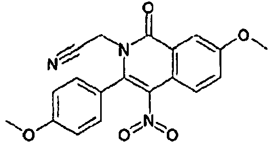 | C₁₉H₁₅N₃O₅ | 365,34 | + | + | - | - |
| 31 EC | PJ46f1 | 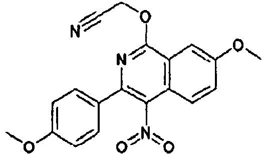 | C₁₉H₁₅N₃O₅ | 365,34 | - | ++ | - | - |
| 32 | PJ37 | 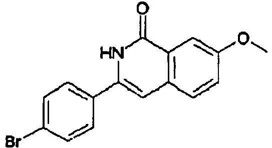 | C₁₆H₁₂BrNO₂ | 330,18 | +++ | - | **** | +++ |
| 33 EC | PJ39 | 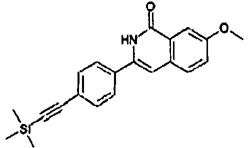 | C₂₁H₂₁NO₂Si | 347,48 | - | - | - | + |
| 34 | PJ47 | 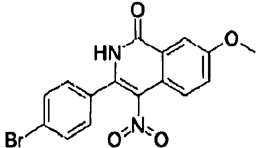 | C₁₆H₁₁BrN₂O₄ | 375,17 | ++ | +++ | *** | ++++ |
| 35 | PJ54 | 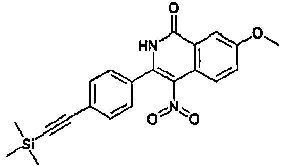 | C₂₁H₂₀N₂O₄Si | 392,48 | +++ | - | *** | ++++ |
| 36 | PJ56 | 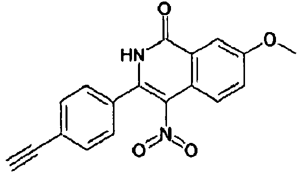 | C₁₈H₁₂N₂O₄ | 320,30 | +++ | +++ | **** | ++++ |
| 37 | CLG1963 | 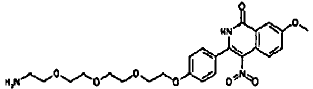 | C₂₄H₂₉N₃O₈ | 487.5 | - | - | - | + |
| 38 | PJ-58 | 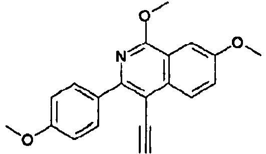 | C₂₀H₁₇NO₃ | 319.35 | - | + | - | - |
| 39 | DN103-1 | 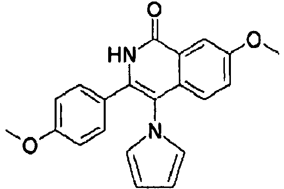 | C₂₁H₁₈N₂O₃ | 346,38 | +++ | + | - | +++ |
| 40 | DN114 | 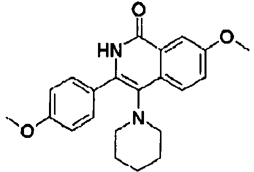 | C₂₂H₂₄N₂O₃ | 364,43 | - | +++ | - | +++ |
| 41 | DN122 | 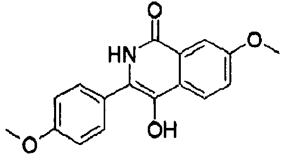 | C₁₇H₁₅NO₄ | 297.31 | - | - | - | ++ |
| 44 EC | CLG 1929 | 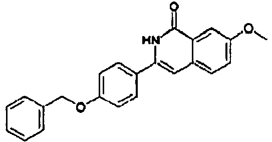 | C₂₃H₁₉NO₃ | 357,4 | +++ | - | - | + |
| 45 EC | CLG 1930 | 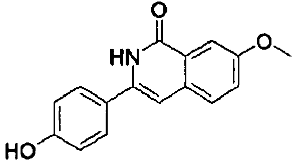 | C₁₆H₁₃NO₃ | 267,28 | - | ++ | **** | +++ |
| 46 EC | CLG 1939 | 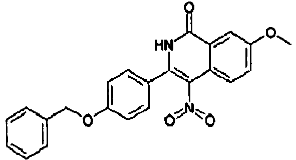 | C₂₃H₁₈N₂O₅ | 402,40 | +++ | + | - | |
| 48 | DN141-2 | 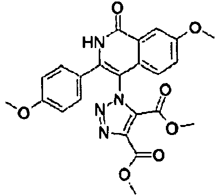 | C₂₃H₂₀N₄O₇ | 464,42 | - | - | - | + |
| | | | | | | | mais effet * à 50 µM | |
| 49 | DN145-1 | 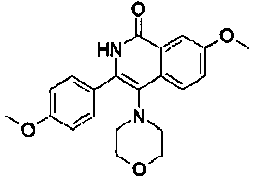 | C₂₁H₂₂N₂O₄ | 366,4 | - | + | ** | +++ |
| 50 | DN145-3 | 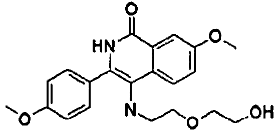 | C₂₁H₂₄N₂O₅ | 384,42 | - | +++ | - | + |
| 56 | DN174-2 | 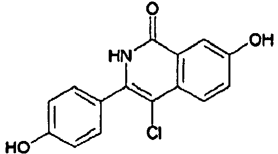 | C₁₅H₁₀ClNO₃ | 287,7 | +++ | - | - | - |
| | | | | | | | mais effet * à 100 µM | |
| 57 | DN 171 | 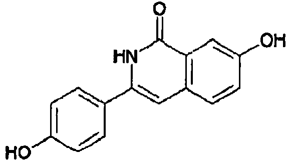 | C₁₅H₁₁NO₃ | 253,25 | - | + | *** | ++ |
| 59 | ADV 1-116 | 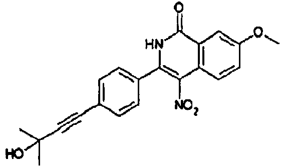 | C₂₁H₁₈N₂O₅ | 378,38 | ++ | - | - | - |
| 60 | ADV 1-112 | 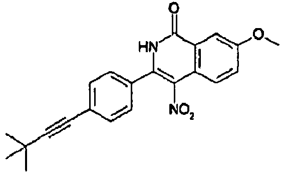 | C₂₂H₂₀N₂O₄ | 376,41 | ++++ | - | *** | ++ |
| 61 | DN228-1 | 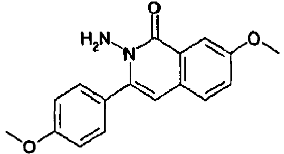 | C₁₇H₁₆N₂O₃ | 296,12 | - | - | - | - |
| 62 | DN282 | 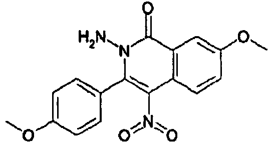 | C₁₇H₁₅N₃O₅ | 341,10 | - | - | *** | ++ |
| 64 | DN263-1 | 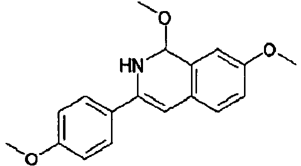 | C₁₈H₁₉NO₃ | 297.36 | +++ | - | - | - |
| 65 | DN263-2 | 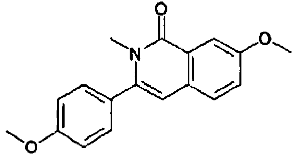 | C₁₈H₁₇NO₃ | 295,12 | + | + | * | ++ |
| 66 | DN264 | 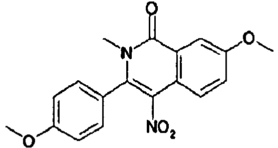 | C₁₈H₁₆N₂O₅ | 340,11 | ++++ | + | * | ++ |
| 67 | DN267 | 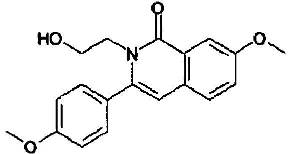 | C₁₉H₁₉NO₄ | 325,13 | ++ | - | - | + |
| 68 | DN281-2 | 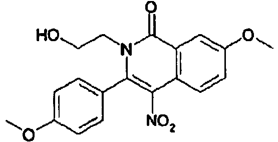 | C₁₉H₁₈N₂O₆ | 370,12 | - | - | - | + |
| 69 | DN274 | 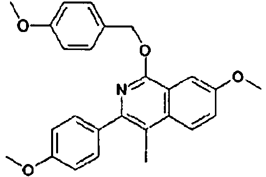 | C₂₅H₂₂INO₄ | 527.06 | ++ | + | - | + |
| 70 | DN279 | 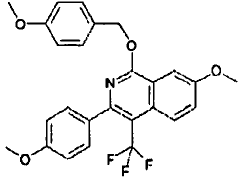 | C₂₆H₂₂F₃NO₄ | 469,15 | +++ | + | - | + |
| 71 | DN278 | 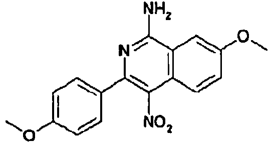 | C₁₇H₁₅N₃O₄ | 325,11 | - | + | - | + |
| 72 | ADV2-029 | 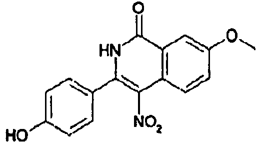 | C₁₆H₁₂N₂O₅ | 312,28 | +++ | ++ | **** | +++ |
| 73 | ADV2-046 | 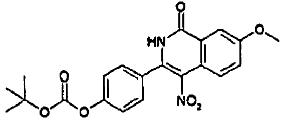 | C₂₁H₂₀N₂O₇ | 412,39 | +++ | - | *** | ++ |
| 74 | ADV2-035 | 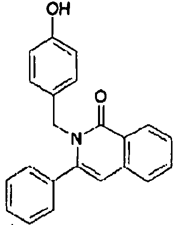 | C₂₃H₁₈N₂O₆ | 418.39 | +++ | + | - | + |
| | | | | | | | mais effet * à 50 µM | |

| | | | | | | | | |
|---|---|---|---|---|---|---|---|---|
| EC = exemple comparatif Nota Bene : AM : Arrêt mitotique ; PHF : Fibroblastes humains primaires ; MTs : Microtubules | | | | | | | | |

### 1. Effet sur l'activité de PP1CA in vitro

La détermination de l'IC50 a été réalisée avec la PP1CA à 80 mU en présence d'un substrat fluorescent (50 µM) et de différentes concentrations de molécules de 50µM à 50 nM.
(-) aucune activité inhibitrice de PP1 même à 50 µM.
(+) inhibition de PP1 faible : IC50 ≥ 50 µM.
(++) inhibition de PP1 modérée : IC50 entre 25 et 50 µM.
(+++) inhibition de PP1 prononcée : IC50 entre 10 et 24 µM.
(++++) inhibition de PP1 forte : IC50 ≤ 9 µM.
NT : molécule non testée

### 2. Effet sur la polymérisation de la tubuline in vitro

La mesure de l'effet d'une molécule sur la polymérisation a été calculée à t= 3000s en prenant la valeur du contrôle au même temps égale à 100%.
(-) signifie que la molécule n'induit pas ou très peu d'inhibition de l'assemblage de la tubuline *in vitro :* 0 + ou - -5% du contrôle DMSO.
(+) signifie que la molécule induit une inhibition de l'assemblage de la tubuline *in vitro* comprise entre 6 et 25% du contrôle DMSO.
(++) signifie que la molécule induit une inhibition de l'assemblage de la tubuline *in vitro* comprise entre 26 et 50% du contrôle DMSO.
(+++) signifie que la molécule induit une inhibition de l'assemblage de la tubuline *in vitro* de plus de 50% par rapport au contrôle DMSO.
NT : molécule non testée.

### 3. Effet sur les cellules HeLa

L'index mitotique et/ou la dépolymérisation des MTs ont été évalués à 25 µM.
- : aucune activité sur les cellules à 25 µM (si effet à des concentrations supérieures, précision dans le tableau).
   * : faible activité (AM et/ou dépolymérisation des MTs légère et/ou inhibition de prolifération légère),
   ** : activité moyenne (AM et/ou dépolymérisation des MTs modérée et/ou inhibition de prolifération modérée),
   *** : forte activité (AM et/ou dépolymérisation des MTs prononcée et/ou inhibition de prolifération prononcée),
   **** : très forte activité (AM et/ou dépolymérisation des MTs très prononcée et/ou inhibition de prolifération forte).
   NT : molécule non testée.

### 4. GI50 sur les cellules HMEC

Les HMEC ont été ensemencées à 10% de confluence et incubées avec les molécules à différentes concentrations (de 50 µM à 1 nM) pendant 5 jours. Le GI50 pour Growth Inhibition 50% définit la concentration induisant une baisse de la prolifération de 50% par rapport au contrôle.
(-) aucune activité inhibitrice sur les cellules à 50 µM.
(+) inhibition de prolifération légère : GI50 entre 25 et 50 µM.
(++) inhibition de prolifération modérée : GI50 entre 1 et 25 µM.
(+++) inhibition de prolifération prononcée : IC50 entre 0.1 et 1 µM.
(++++) inhibition de prolifération forte : IC50 ≤ 0.1 µM.
NT : molécule non testée.

### Exemple 8 : Chromatographie d'affinité et immunoblotting

La molécule 37 a été immobilisée sur une résine de sépharose activée par un groupe N-hydroxysuccinimide (NHS) (GE Healthcare # 17-0906-01) comme suit: 100 µg de la molécule 37 par ml de NHS-Sépharose dans du PBS pH 8,3, pendant 1h à température ambiante. La sépharose couplée avec la molécule 37 a été dénommée résine « composé 6 », alors qu'en parallèle, une autre résine « contrôle », a été réalisée avec une incubation dans 0,5 M de Monoéthanolamine (MEA) pH 8,3, dans les mêmes conditions, afin de neutraliser les fonctions NHS. Après plusieurs lavages avec le PBS pH 8,3, les fonctions NHS restées libres ont été bloquées avec du MEA 0,5 M pendant 30 minutes. Une fois bien lavées au PBS, les résines « contrôle » et « composé 6 » ont été incubées avec 1 ml d'extraits d'oeuf de Xénope dilué 1:1 dans du tampon CSF-XB contenant des inhibiteurs de protéases (#1 873 580 Roche®), pendant 1 heure sous agitation à 4°C. Après 2 lavages au PBST, les protéines de l'extrait restées accrochées, ont été éluées avec 100 µl de tampon de charge pour électrophorèse SDS (Laemmli, U.K., Cleavage of structural proteins during the assembly of the head of bacteriophage T4. Nature 1970; 227, 680-685) enrichi avec de l'urée 6M. Les échantillons ainsi dénaturés ont été résolus sur un gel de polyacrylamide (gradient de 6 à 18%), puis transférés sur une membrane de nitrocellulose.

L'immunodétection a été effectuée en utilisant des anticorps anti-PPI de souris (Sigma # 7979) ou anti-PP2A de lapin (Upstate # 07-324) à une dilution de 1:1000, et les anticorps secondaires correspondant couplés avec un anticorps HRP0 (1 : 5000, Ig de chèvre anti-souris, Sigma # A3683; Ig de chèvre anti-lapin, Sigma # A0545).

Pour détecter le signal HRPO un kit ECL plus Amersham (GE Healthcare # RPN2132) a été utilisé.

### Exemple 9 : Test de la Protéine phosphatase

Pour les tests d'activité, nous avons utilisé la PP1α (New England Biolabs # P0754) à une concentration finale de 30 mU ou de 80 mU pour le criblage de tous les analogues. Le phosphate 6,8-difluoro-4-methylumbelliferyl (DiFMUP, Invitrogen # D6567) a été utilisé comme substrat fluorescent à une concentration de 60 µM. Tous les tests ont été effectués dans un volume final de 100 µl, à 37°C dans des microplaques 96 puits (Greiner # 655096).

Le composé 6, la combretastatine A4 (CA-4) ainsi que tous les analogues ont été dilués dans 50 µl de tampon PP1 (Tris-HCl 100 mM pH 7,5, DTT 4 mM, EDTA 0,2 mM, MnCl₂ 0,5 mM) de 50µM à 50nM. Des quantités correspondantes de DMSO ont été additionnées dans des puits contrôles. PP1α (30 ou 80 mU dans 30 µl de tampon PP1) a été ajoutée dans chaque puit. Après 15 minutes d'incubation à TA, le substrat a été ajouté (dans 20 µl de tampon PP1). Après 20 minutes d'incubation à 37°C, la fluorescence a été mesurée dans un fluorimètre à plaques à 355-460 nm pour doser l'activité phosphatase. Le test a été effectué trois fois en duplicat pour chaque condition.

### Exemple 10 : Expérimentations In vivo

Toutes les expérimentations animales ont été effectuées selon les recommandations du comité d'éthique local. Les souris nude (femelles NCr-nu/nu (laboratoires Harlan), ont été hébergées dans une pièce à air filtré à 22°C avec une alternance de 12h de jour et 12 h de nuit.

Des cellules HTB177 (1 × 10⁶) ont été implantées en sous-cutanée dans les flancs des souris. La croissance tumorale a été contrôlée deux fois par semaine en mesurant la taille des tumeurs au pied à coulisse nous permettant ainsi d'estimer le volume de la tumeur, calculé en mm³ selon la formule suivante: longueur x largeur x hauteur x π/6.

Le traitement a débuté quand les tumeurs ont atteint approximativement la taille de 250mm³. Le poids de l'animal, le volume de la tumeur et l'extension des nécroses ont été mesurés deux fois par semaine.

Les animaux ont été sacrifiés quand la tumeur a atteint 2000 mm³ ou plus tôt si un signe clinique de souffrance, défini par le comité d'éthique, a été observé.

Les souris ont reçu le composé 6 *per os* à des doses et intervalles décrits dans la légende des figures. Les souris contrôles ont reçu le véhicule seul (volume identique de béta-cyclodextrine à 60 mg/ml))

### Exemple 11 : Histologie

Après l'euthanasie des animaux, les tumeurs ont été retirées, coupées de manière sagittale le long de la ligne médiane, congelées rapidement dans de l'azote liquide et conservés à -80°C. Des coupes de tumeur (10µm d'épaisseur) ont été réalisées puis colorées avec de l'haematoxyline-éosine (H&E) suivant les procédures standard.

Des images des coupes de tumeur ont été réalisées sous un microscope optique (objectif x10) avec une caméra Olympus BX41.

### Exemple 12 : ESSAIS COMPARATIFS AVEC INH2BP :

Le produit composé 5-iodo-6-amino-1,2-benzopyrone (INH2BP) décrit dans la demande de brevet WO 98/51307 ne présente pas d'activité antiproliférative à 50µM sur les cellules HeLa.

### Exemple 13 : SYNTHESE DES MOLECULES

Les composés **1, 2, 4, 6** à **8** et **10** ont été synthétisés selon Bisagni et al., Tetrahedron, 1996, 52, 10427-10440.

Le composé **5** a été synthétisé selon Croisy-Delsey et al., Bioorg. Med. Chem., 200, 8, 2629-2641.

Les composés de formule générale IIa peuvent être synthétisés de la manière suivante :
Protocole général :
   - pour X=O, la méthode de synthèse est effectuée selon Bisagni et al., Tetrahedron 1996, 52, 10427-10440 et comporte les étapes principales suivantes :
      a) condensation d'un acide arylacétique sur un arylcarboxaldéhyde pour former un acide 2,3-diarylacrylique intermédiaire,
      b) transformation du groupement acide en azide correspondant par NaN₃ via un anhydride mixte,
      c) cyclisation par voie thermique de l'azide pour conduire à la 3-arylisoquinolin-1(2H)-one,
      d) aménagement des groupements fonctionnels selon les protocoles détaillés plus loin.
   - pour X=S, la synthèse comprend les étapes suivantes
      a) à partir de la 3-arylisoquinolin-1(2H)-one, la synthèse d'isoquinolin-1(2H)-thione est réalisée avec le réactif de Lawesson,
      b) à partir de la 3-aryl-4-nitroisoquinolin-1(2H)-one, la méthode de synthèse d'isoquinolin-1(2H)-thione comporte les étapes principales suivantes :
         o chloruration par l'oxyde chorure de phosphore ou par le réactif de Vilsmeier pour former la 1-chloroisoquinoléine intermédiaire,
         o transformation du groupement chloro en groupement thione avec la thiourée.

Les composés de formule générale IIb peuvent être synthétisés de la manière suivante :
Protocole général :
   ∘ à partir de la 3-arylisoquinolin-1(2H)-one: la synthèse d'isoquinoline IIb est réalisée avec l'haloakyle voulu en présence d'une base telle que K₂CO₃ ;
   o à partir de la 1-chloro-4-nitroisoquinoléine dont sa synthèse a été mentionnée plus haut: la synthèse d'isoquinoline IIb est réalisée avec le nucléophile voulu en milieu basique.

### Composé 9: N-(7-Méthoxy-1-oxo-3-phényle-1,2-dihydroisoquinoline-4-yl)méthanamide:

Une solution d'acide formique (2.40 mL; 63 mmol) et de composé **10** (500 mg, 1.88 mmol) dans le toluène (50 mL) est chauffée au reflux pendant 5 h et le liquide est évaporé à sec sous pression réduite. Le résidue est ensuite trituré dans le mélange d'acide formique et d'eau (80/20), puis laissé refroidir pour donner le composé attendu 9 (450 mg; 77%) sous forme de microcristaux rose pâle; ¹H NMR (CDCl₃): δ 3.90 ( 3 H, s), 7.39 (1 H, dd), 7.42-7.5 ( 5 H, m), 7.52 ( 1 H, d), 7.67 (1 H, d), 8.18 (1 H, s), 9.43 (1 H, br s), 11.51 (1 H, br s); Anal. Cal. pour C₁₇H₁₄N₂O₃ · 0.17 HCO₂H: C, 68.47; H, 4.76; N, 9.30; Trouvé: C, 68.43; H, 4.89; N, 9.35.

### Composé 12: 7-Méthoxy-3-(4-méthoxyphényle)isoquinoline-1(2H)-thione

Le réactif de Lawesson (975 mg, 2.4 mmol) est ajouté sous atmosphère d'argon à la suspension de lactame **4** (584 mg, 2.1 mmol) dans du toluène anhydre (25 mL) à 85°C. Le mélange réactionnel est agité à cette température pendant 20 h. De l'eau (150 mL) est ajoutée et le mélange est extrait avec de l'acétate d'éthyl (2*150 mL). La phase organique est lavée au saumure (2*150 mL), séchée sur MgSO₄ et le résidu solide obtenu après l'évaporation du solvant est recristallisé dans l'éthanol absolu pour donner le composé thiolactame **12** sous forme d'aiguilles jaunes vif (550 mg, 89%), mp 188-190°C ; ¹H NMR (DMSO-*d₆*) δ 13.21 (1 H, br s), 8.21 (1 H, d), 7.80-7.70 (3 H, m), 7.45 (1 H, dd), 7.30 (1 H, s), 7.06 (2 H, d), 3.91 (3 H, s), 3.83 (3 H, s); MS 298 (M+H); Anal. Cal. pour C₁₇H₁₅NO₂S · 0.5 H₂O: C, 66.66; H, 5.22; N, 4.57; Trouvé: C, 66.31; H, 4.87; N, 5.05.

### Composé 13: Acide 7-méthoxy-3-(4-méthoxyphényle)isoquinoline-1-sulfonique

Une solution d'acide nitrique (d 1.33 ; 146 µL) dans AcOH (650 µL) est ajoutée goutte-à-goutte à la suspension de thiolactame **12** (275 mg, 0.9 mmol) dans AcOH (4.3 mL) et AcOEt (1.2 mL) à 0°C. Le mélange est ensuite laissé revenir à température ambiante et on maintient l'agitation encore 1 h avant d'y ajouter de l'eau (30 mL). Le précipité est filtré, lavé à l'eau, séché puis chromatographié sur colonne de gel de silice utilisant CH₂Cl₂ comme éluant pour donner le composé **13** sous forme de microcristaux jaune pâle (180 mg; 56%), mp 226-228°C; ¹H NMR (DMSO-*d₆*) δ 8.14 (1 H, s), 8.00 (1 H, d), 7.83 (2 H, d), 7.68 (1 H, d), 7.54 (1 H, dd), 6.72 (2 H, d), 3.97 (3 H, s), 3.71 (3 H, s); MS 344 (M-H).

### Composé 14: 4-Chloro-7-méthoxy-3-(4-méthoxyphényle)isoquinoline-1(2H)-one

A température ambiante, le N-chlorosuccinimide (67 mg ; 0.5 mmol) est ajouté en une seule fois à la solution de lactame **4** (128 mg, 0.45 mmol) dissous dans AcOH (1.2 mL) et AcOEt (1.2 mL). Le mélange réactionnel est agité pendant 24 h et une nouvelle portion de N-chlorosuccinimide (67 mg ; 0.5 mmol) est ajoutée. De nouveau le mélange réactionnel est agité pendant 24 h, puis de l'eau (10 mL) est ajoutée et le précipité formé est fitré, lavé avec de l'eau et séché. Il est ensuite chromatographié sur colonne de gel de silice utilisant un gradient d'éthanol (0 à 1.5%) dans CH₂Cl₂ comme éluant pour donner le composé attendu **14** (140 mg; 97%) sous forme de gomme jaune pâle; ¹H NMR (CDCl₃) δ 7.96 (1 H, d), 7.75 (1 H, d), 7.51 (2 H, d), 7.17 (1 H, dd), 6.85 (2 H, d), 6.66 (1 H, br s), 3.98 (3 H, s), 3.77 (3 H, s); MS 316 et 318 (M+H).

### Composé 15: 4-Bromo-7-méthoxy-3-(4-méthoxyphényle)isoquinoline-1(2H)-one

A température ambiante, le N-bromosuccinimide (92 mg ; 0.5 mmol) est ajouté en une seule fois à la solution de lactame **4** (108 mg, 0.38 mmol) dissous dans AcOH (1.0 mL) et AcOEt (1.0 mL). Le mélange réactionnel est agité pendant 4 h, de l'eau (10 mL) est ajoutée et le précipité formé est fitré, lavé avec de l'eau et séché. Il est ensuite chromatographié sur colonne de gel d'alumine neutre utilisant un gradient d'éthanol (0 à 1 %) dans CH₂Cl₂ comme éluant pour donner le composé attendu **15** (80 mg; 58%) sous forme de microcristaux blancs, mp 240-242°C; ¹H NMR (DMSO-d₆) δ 11.72 (1 H, br s), 7.89 (1 H, d), 7.70 (1 H, d), 7.50-7.43 (3 H, m), 7.05 (2 H, d), 3.91 (3 H, s), 3.83 (3 H, s); MS 359 et 361 (M+H) ); Anal. Cal. pour C₁₇H₁₄BrNO₃: C, 56.69; H, 3.92; N, 3.89; trouvé: C, 56.58; H, 3.92; N, 3.66.

### Composé 16: 4-Iodo-7-méthoxy-3-(4-méthoxyphényle)isoquinoline-1(2H)-one

A température ambiante, le *N*-iodosuccinimide (127 mg ; 0.5 mmol) est ajouté en une seule fois à la solution de lactame **4** (130 mg, 0.46 mmol) dissous dans AcOH (1.2 mL) et AcOEt (1.2 mL). Le mélange réactionnel est agité pendant 2.5 h, de l'eau (10 mL) est ajoutée et le précipité formé est fitré, lavé avec de l'eau et séché. Il est ensuite chromatographié sur colonne de gel d'alumine neutre utilisant un gradient d'éthanol (0 à 1 %) dans CH₂Cl₂ comme éluant pour donner le composé attendu **16** (130 mg; 69%) sous forme de microcristaux blanc, mp 216-218°C; ¹H NMR (DMSO-*d₆*) δ 11.72 (1 H, br s), 7.89 (1 H, d), 7.70 (1 H, d), 7.50-7.43 (3 H, m), 7.05 (2 H, d), 3.91 (3 H, s), 3.83 (3 H, s); MS 408 (M+H); Anal. Cal pour C₁₇H₁₄BINO₃: C, 50.14; H, 3.47; N, 3.44; Trouvé: C, 50.32; H, 3.48; N, 3.45.

### Composé 17: 1-Chloro-7-méthoxy-3-(4-méthoxyphényle)-4-nitroisoquinoline

Sous atmosphère d'argon, l'oxyde chlorure de phosphore (3.2 mL) est ajouté en une seule fois à la solution de nitrolactame **6** (1.33 g, 40 mmol) et de chlorure de benzyltriéthylammonium dans l'acétonitrile (27 mL). Le mélange réactionnel est ensuite chauffé au reflux pendant 3 h puis évaporé sous pression réduite. De l'eau froide (50 mL) est ajoutée et le pH est ajusté à 7-8 par NH₄OH à 14%. Le précipité formé est fitré, lavé avec de l'eau, séché et chromatographié sur colonne de gel de silice utilisant CH₂Cl₂/acetone : 8-2 comme éluant pour donner le composé attendu 17 (1.18 g; 83%) sous forme de paillettes jaune brillant, mp 196-198°C; ¹H NMR (CDCl₃) δ 7.71 (1 H, d), 7.70-7.64 (2 H, m), 7.63 (1 H, d), 7.53 (1 H, dd), 7.00 (2 H, d), 4.03 (3 H, s), 3.86 (3 H, s); MS 345 et 347 (M+H) Anal. Cal. pour C₁₇H₁₃ClN₂O₄: C, 59.23; H, 3.80; N, 8.13; Trouvé: C, 59.17; H, 3.75; N, 7.88.

### Composé 18: 7-Méthoxy-3-(4-méthoxyphényle)-4-nitroisoquinoline-1(2H)-thione

Sous atmosphère d'argon, le mélange formé par le dérivé chloronitro **17** (230 mg, 0.66 mmol), thiourée (164 mg, 2.1 mmol) et l'éthanol absolu (7 mL) est chauffé au reflux pendant 4 h. Le précipité formé est filtré, lavé avec de l'éthanol, séché et chromatographié sur colonne de gel d'alumine neutre utilisant CH₂Cl₂ comme éluant pour donner le composé attendu **18** (150 mg; 65%) sous forme de microcristaux orange pâle, mp 226-228°C; ¹H NMR (DMSO-*d₆*) δ 13.83 (1 H, br s), 8.31 (1 H, s), 7.61 (2 H, br s), 7.47 (2 H, d), 7.07 (2 H, d), 7.00 (2 H, d), 3.95 (3 H, s), 3.83 (3 H, s); MS 343 (M+H). Anal. Cal. pour C₁₇H₁₄N₂O₄S˙0.25 H₂O: C, 58.87; H, 4.18; N, 8.08; Trouvé: C, 58.83; H, 4.05; N, 7.94.

### Composé 19: 1,7-Diméthoxy-3-(4-méthoxyphényle)-4-nitroisoquinoline

Sous atmosphère d'argon, le mélange formé par le dérivé chloronitro **17** (209 mg, 0.60 mmol), DMF (2 mL) et solution de méthoxyde de sodium à 30% dans le méthanol (9 mL) est chauffé au reflux pendant 18 h. De l'eau froide (40 mL) est ajoutée et le précipité formé est filtré, lavé avec de l'eau, séché et chromatographié sur colonne de gel de silice utilisant CH₂Cl₂ comme éluant pour donner le composé attendu **19** (158 mg; 76%) sous forme de paillettes jaune brillant, mp 176-178°C; ¹H NMR (CDCl₃) δ 7.70-7.65 (3 H, m), 7.57 (1 H, d), 7.42 (1 H, dd), 6.98 (2 H, d), 4.22 (3 H, s), 3.97 (3 H, s), 3.86 (3 H, s); MS 363 (M+Na); Anal. Cal. pour C₁₈H₁₆N₂O₅: C, 63.52; H, 4.74; N, 8.23; Trouvé: C, 63.46; H, 4.78; N, 7.99.

### Composé 20: 7-Méthoxy-3-(4-méthoxyphényle)-4-nitro-1-(phénylethio)isoquinoline

Sous atmosphère d'argon, le mélange formé par le dérivé chloronitro **17** (110 mg, 0.32 mmol), éthanol absolu (5 mL), triéthylamine (0.1 mL) et thiophenol (70 mg, 0.6 mmol) est chauffé au reflux pendant 18 h. Le précipité formé est filtré, lavé avec de l'éthanol, séché pour donner le composé attendu **20** (120 mg; 90%) sous forme d'aiguilles jaune brillant, mp 169-171°C; ¹H NMR (CDCl₃) δ 7.70 (1 H, d), 7.67-7.62 (2 H, m), 7.57 (1 H, d), 7.50-7.44 (4 H, m), 7.39 (2 H, d), 6.82 (2 H, d), 4.02 (3 H, s), 3.80 (3 H, s); MS 419 (M+H); Anal. Cal. pour C₂₃H₁₈N₂O₄S: C, 66.01; H, 4.34; N, 6.39; Trouvé: C, 65.85; H, 4.15; N, 6.61.

### Composé 21: 1-(Benzylthio)-7-méthoxy-3-(4-méthoxyphényle)-4-nitroisoquinoline

Sous atmosphère d'argon, le mélange formé par le dérivé chloronitro **17** (138 mg, 0.40 mmol), éthanol absolu (4 mL), triéthylamine (0.1 mL) et benzylmercaptan (60 mg, 0.48 mmol) est chauffé au reflux pendant 24 h. Le précipité formé est filtré, lavé avec de l'éthanol, séché et chromatographié sur colonne de gel de silice utilisant CH₂Cl₂ comme éluant pour donner le composé attendu **21** (120 mg; 69%) sous forme de microcristaux orange pâle, mp 148-150°C; ¹H NMR (CDCl₃) δ 7.72-7.62 (3 H, m), 7.48-7.42 (4 H, m), 7.36-7.27 (3 H, m), 6.99 (2 H, d), 4.68 (2 H, s), 3.96 (3 H, s), 3.87 (3 H, s); MS 433 (M+H); Anal. Cal. pour C₂₄H₂₀N₂O₄S: C, 66.65; H, 4.66; N, 6.48; Trouvé: C, 66.85; H, 4.58; N, 6.51.

### Composé 22: N-Benzyl-N-[7-méthoxy-3-(4-méthoxyphényle)-4-nitroisoquinoline-1-yl]-amine

Sous atmosphère d'argon, le mélange formé par le dérivé chloronitro **17** (145 mg, 0.42 mmol), éthanol absolu (4 mL), triéthylamine (0.1 mL) et benzylamine (136 mg, 1.30 mmol) est chauffé au reflux pendant 18 h. Le précipité formé est filtré, lavé avec de l'éthanol, séché et chromatographié sur colonne de gel de silice utilisant CH₂Cl₂ comme éluant pour donner le composé attendu **22** (110 mg; 62%) sous forme de microcristaux orange pâle, mp 199-201°C; ¹H NMR (CDCl₃) δ 7.81 (1 H, d), 7.62 (2 H, d), 7.47-7.30 (6 H, m), 7.02-6.92 (3 H, m), 5.75-5.67 (1 H, m), 4.93 (2 H, d), 3.93 (3 H, s), 3.84 (3 H, s); MS 414 (M-H); Anal. Cal. pour C₂₄H₂₁N₃O₄·0.25 H₂O: C, 68.65; H, 5.12; N, 10.01; Trouvé: C, 68.59; H, 4.92; N, 9.95.

### Composé 23: 4-(Dimethylamino)-7-méthoxy-3-(4-méthoxyphényle)isoquinoline-1(2H)-one

Au mélange de 4-amino-7-methoxy-3-(4-methoxyphenyl)isoquinolin-1(2*H*)-one (composé **1**) (200 mg, 0.67 mmol), de formaldehyde aqueux à 37% (0.54 mL, 6.7 mmol) et d'acétonitrile (5 mL), le cyanoborohydrure 126 mg ( 2.01 mmol) est ajouté à température anbiante. De l'acide acétique glacial (116µL, 2.01 mmol) est ensuite ajouté lentement et l'ensemble est laissé sous agitation pendant 5h. De nouveau, une deuxième quantité d'acide acetique glacial (116 µL, 2.01mmol) est introduite, l'agitation est maintenue encore 30 min puis l'ensemble est évaporé sous pression réduite et de l'ether (15 mL) est ajouté. La phase éthérée est lavée successivement avec une solution normale de KOH (3 * 30 mL), de la saumure (3 mL), puis séchée sur MgSO₄ et évaporée sous pression réduite. Le résidu est chromatographié sur colonne de gel de silice utilisant un gradient d'éthanol (0 à 2%) dans CH₂Cl₂ comme éluant pour donner le composé attendu **23** (54 mg, 25%) sous forme de solide orange, mp 183-185°C; ¹H NMR (CDCl₃): δ 8.30 (1 H, br s), 7.85 (1 H, d), 7.81 (1 H, d), 7.38 (2 H, d), 7.33 (1 H, dd), 6.99 (2 H, d), 3.94 (3 H, s), 3.88 (3 H, s), 2.64 (6 H, s); MS 325 ( M+H); Anal. Cal. pour C₁₉H₂₀N₂O₃·1.1 H₂O: C, 66.2; H, 6.44; N, 8.13. Trouvé: C, 66.24; H, 5.81; N, 8.13.

### Composé 24: N-(7-Méthoxy-3-(4-méthoxyphényle)-1-oxo-1,2-dihydroisoquinoline-4-yl)méthanamide

Au mélange de 4-amino-7-methoxy-3-(4-methoxyphenyl)isoquinolin-1(2*H*)-one (composé **1**) (100 mg, 0.33mmol) et de formate d'éthyle (7 mL, préalablement distillé sur l'hydrure de calcium) est ajouté de l'acide formique (1.6 mL) et le mélange est chauffé au reflux pendant 12 h. L'ensemble est ensuite évaporé à sec sous pression réduite et le résidu obtenu est lavé à l'éthanol, filtré, séché sous vide pour donner 36 mg du composé attendu **24**. Le filtrat est concentré puis chromatographié sur colonne de gel de silice utilisant un gradient d'éthanol (0 à 5%) dans CH₂Cl₂ comme éluant pour donner une deuxième fraction de bon produit **24** 15 mg (46% de rendement global) sous forme de solide blanc, mp >270°C; ¹H NMR (CDCl₃): δ 11.44 (1 H, br s), 9.41 (1 H, br s), 8.18 (1 H, s), 7.65 (1 H, s), 7.51 (1 H, d), 7.42 (2 H, d), 7.38 (1 H, dd), 7.00 (2 H, d), 3.89 (3 H, s), 3.81 (3 H, s); MS 347 (M+Na); Anal. Cal. pour C₁₈H₁₆N₂O₄: C, 66.66; H, 4.97; N, 8.64; Trouvé: C, 66.75; H, 4.92; N, 8.53.

### Composé 25: 7-Méthoxy-3-(4-méthoxyphényle)isoquinoline-1(2H)-one-4-carbaldéhyde

A -78°C, le n-BuLi (1.6M dans l'hexane, 1.8 mL, 1.1 mmol) est ajouté sur une solution de 4-bromo-7-methoxy-3-(4-methoxyphenyl)isoquinolin-1(2*H*)-one (composé **15**, 200 mg, 0.55 mmol) et de THF (10 mL). La suspension jaune obtenue est agitée 15 min supplémentaires à la même température puis du DMF (85 µL, 1.1mmol) est introduit. Après 1h à -78°C, de l'eau (10 mL) est ajoutée puis l'ensemble est extrait au dichlorométhane (3*10 mL). Les phases organiques réunies sont lavées à l'eau, séchées sur MgSO₄ et le résidu obtenu après l'évaporation du solvant est chromatographié sur colonne de gel de silice utilisant un gradient d'éthanol (0 à 2%) dans CH₂Cl₂ comme éluant pour donner le composé attendu **25** (46 mg, 27%) sous forme de solide blanc, mp 232°C, ¹H NMR (CDCl₃): δ 3.91 (3 H, s), 3.95 (3 H, s), 7.06 (2 H, d), 7.4 (1 H, dd), 7.48 (2H, d), 7.81 (1 H, d), 8.77 (1 H, br s), 9.15 (1 H, d), 9.79 (1 H, s); MS 310 (M+H); Anal. Cal. pour C₁₈H₁₅NO₄ · 0.2 H₂O: C, 69.07; H, 4.92; N, 4.47. Trouvé: C, 68.68; H, 4.98; N, 4.34.

### Composé 26 : 4-Bromo-1-chloro-7-méthoxy-3-(4-méthoxyphényl)isoquinoline

Une suspension du réactif de Vilsmeier (1.7 g, 13.2 mmol) dans 13 mL de dichloroéthane est agitée pendant quelques minutes puis le composé 4-bromo-7-méthoxy-3-(4-méthoxyphényl)isoquinolin-1(2*H*)-one (2.1 g, 6 mmol) est ajouté. Le mélange est porté à reflux. Au bout d'une heure, on laisse revenir à température ambiante. Puis la solution est diluée dans 750 mL de chloroforme, lavée avec 3x200 mL d'eau. La phase organique est séchée sur MgSO₄, filtrée et évaporée sous pression réduite. Le solide blanc obtenu est cristallisé dans le méthanol. On obtient sous forme de cristaux blancs le composé **26** attendu (1.78 g, 95 %), mp 164-166 °C, MS 378 [M+H]⁺, ¹H NMR (CDCl₃): δ 3.86 (s, 3H), 4.00 (s, 3H), 6.95-7.02 (m, 2H), 7.47 (dd, J = 2.6 et J = 9.2 Hz, 1H), 7.54 (d, J = 2.5 Hz, 1H), 7.65-7.70 (m, 2H), 8.24 (d, J = 9.3 Hz, 1H); ¹³C NMR (CDCl₃): δ 55.4, 55.8, 104.2, 113.4, 117.0, 125.2, 127.7, 129.3, 131.4, 131.8, 133.0, 148.0, 148.5, 159.7, 159.8.

### Composé 27 : 4-Bromo-1,7-Diméthoxy-3-(4-méthoxyphényl)isoquinoline

A une suspension du composé 4-bromo-1-chloro-7-méthoxy-3-(4-méthoxyphényl)isoquinoline **26** (1.78 g, 4.7 mmol) dans 70 mL de méthanol est ajouté le méthylate de sodium (1.26 g, 23.5 mmol). Le mélange réactionnel est porté à reflux pendant 2 jours. Puis le solvant est évaporé, le brut réactionnel est remis en solution dans 100 mL d'eau et extrait avec 300 mL de chloroforme. La phase organique est séchée sur MgSO₄, filtrée et concentrée sous pression réduite. Le solide blanc obtenu est purifié par chromatographie sur colonne de silice (CH₂Cl₂/Cyclohexane 1/1 puis 7/3). On obtient le composé **27** attendu sous forme d'une poudre blanche (1.55 g, 88 %), mp 138-140 °C, MS 375 et 377 [M+H]⁺, ¹H NMR (CDCl₃): δ 3.89 (s, 3H), 3.97 (s, 3H), 4.13 (s, 3H), 6.97-7.04 (m, 2H), 7.39 (dd, J = 2.7 et J = 9.2 Hz, 1H), 7.53 (d, J = 2.6 Hz, 1H), 7.74-7.81 (m, 2H), 8.15 (d, J = 9.2 Hz, 1H); ¹³C NMR (CDCl₃, 300 MHz): δ 53.8, 55.2, 55.5, 102.4, 109.3, 113.0, 120.5, 123.4, 128.4, 131.4, 132.5, 133.1, 145.8, 158.2, 158.4, 159.3.

### Composé 28: 7-Méthoxy-3-(4-méthoxyphényl)-4-nitro-2-(propargyl)isoquinolin-1(2H)-one

Un mélange du composé **6** (1.36 g, 4.17 mmol), K₂CO₃ (576 mg,4.17 mmol) dans 42 mL de DMF est agité à température ambiante pendant 15 minutes. Puis le bromure de propargyle (450 µL, 4.17 mmol) est additionné et la solution est agitée à température ambiante. Au bout de 24h, 80 mL d'eau sont ajoutés et le mélange est extrait avec 4x40 mL d'acétate d'éthyle. Les phases organiques sont réunies, lavées avec 2x20 mL d'une solution aqueuse saturée de NaCl, séchées sur MgSO₄, filtrées et concentrées sous pression réduite. L'huile jaune obtenue est purifiée par chromatographie sur colonne de silice (cyclohexane/acétate d'éthyle 95/5). On récupère 1.1 g (75 %) du composé **28** sous forme d'une poudre jaune : mp 162-164 °C; MS 365 [M+H]⁺; ¹H NMR (CDCl₃): δ 2.24 (t, 1H), 3.87 (s, 3H), 3.95 (s, 3H), 4.55 (d, J = 2.4 Hz, 2H), 6.98-7.04 (m, 2H), 7.36 (dd, J = 2.7 et J = 9.0 Hz, 1H), 7.40-7.47 (m, 3H), 7.90 (d, J = 2.7 Hz, 1H); ¹³C NMR (CDCl₃, 300 MHz): δ 36.2, 55.5, 56.0, 72.4, 78.4, 108.8, 114.6, 121.4, 122.7, 123.2, 124. 125.9, 130.9, 134.0, 136.7,160.1, 160.7, 161.3.

### Composé 29: 7-Méthoxy-3-(4-méthoxyphényl)-4-nitro-1-(prop-2ynyloxy) isoquinoline

Ce composé se forme en même temps que le composé 7-méthoxy-3-(4-méthoxyphényl)-4-nitro-2-(propargyl)isoquinolin-1(2*H*)-one **28** dont la préparation a déjà été décrite précédemment. Il est obtenu sous forme d'un solide jaune-orangé **29** (228 mg, 11 %), mp 177-179 °C, par chromatographie sur colonne de silice et correspond au dérivé le moins polaire, MS 365 [M+H]⁺; ¹H NMR (CDCl₃) δ 2.55 (t, J = 2.4 Hz, 1H), 3.85, 3.96 (2xs, 2x3H), 5.26 (d, J = 2.4 Hz, 2H), 6.96-7.02 (m, 2H), 7.42 (dd, J = 2.6 et J = 9.2 Hz, 1H), 7.56 (d, J = 2.6 Hz, 1H), 7.64-7.72 (m, 3H); ¹³C NMR (CDCl₃): δ 54.6, 55.5, 55.9, 75.0, 78.8, 102.8, 114.3, 119.3, 122.8, 125.3, 126.0, 128.5, 129.8, 138.2, 139.9, 157.4, 159.2, 160.7.

### Composé 30: 2-(7-Méthoxy-3-(4-méthoxyphényl)-4-nitro-1-oxoisoquinolin-2(1H)-yl)ethanenitrile

Un mélange du composé **6** (1.47 g, 4.5mmol), K₂CO₃ (622 mg, 4.5 mmol) dans 45 mL de DMF est agité à température ambiante pendant 15 minutes. Puis le bromoacétonitrile (313 µL, 4.5 mmol) est additionné et la solution est agitée à température ambiante. Au bout de 3h, 100 mL d'eau sont ajoutés et le mélange est extrait avec 4x100 mL d'acétate d'éthyle. Les phases organiques sont réunies, lavées avec 2x20 mL d'une solution aqueuse saturée de NaCl, puis séchées sur MgSO₄, filtrées et concentrées sous pression réduite. Le solide jaune obtenu est purifié par chromatographie sur colonne de silice (cyclohexane/acétate d'éthyle 95/5). On récupère 980 mg (60%) du composé **30** sous forme d'une poudre jaune : mp 194-196 °C; MS 366 [M+H]⁺; ¹H NMR (DMSO-*d₆*): δ 3.85 (s, 3H), 3.95 (s, 3H), 4.65 (s, 2H), 7.14-7.19 (m, 2H), 7.46-7.61 (m, 4H), 7.80 (d, J = 2.4 Hz, 1H); ¹³C NMR (DMSO, 300 MHz): δ 34.6, 55.4, 55.9, 108.9, 114.8, 115.6, 120.6, 121.9, 123.3, 124.1, 124.9, 130.9, 133.8, 136.5, 159.7, 159.8, 161.0.

### Composé 31: 2-(7-Méthoxy-3-(4-méthoxyphényl)-4-nitroisoquinolin-1-yloxy)éthanenitrile

Ce composé se forme en même temps que le composé 2-(7-méthoxy-3-(4-méthoxyphényl)-4-nitro-1-oxoisoquinolin-2(1*H*)-yl)éthanenitrile **30** dont la synthèse a été décrite précédemment. Il est obtenu sous forme d'un solide jaune (118 mg, 11 %), mp 192-194 °C, par chromatographie sur colonne de silice et correspond au dérivé le moins polaire, MS 366 [M+H]⁺; ¹H NMR (CDCl₃) δ 3.86 (s, 3H), 3.98 (s, 3H), 5.27 (s, 2H), 6.98-7.02 (m, 2H), 7.46-7.51 (m, 2H), 7.67-7.71 (m, 3H); ¹³C NMR (CDCl₃): δ 51.2, 55.5, 56.0, 102.3, 114.5, 115.4, 118.8, 123.0, 125.9, 126.2, 127.8, 129.8, 138.9, 139.5, 156.0,159.6, 160.9.

### Composé 32: 3-(4-Bromophényl)-7-méthoxyisoquinolin-1(2H)-one

La synthèse de la molécule **32** est réalisée selon un protocole décrit dans la littérature par Bisagni et al, Tetrahedron 1996, 52, 10427-10440. Seul l'acide 4-méthoxyphényl acétique est remplacé par l'acide 4-bromophényl acétique. Le composé **32** (7.18 g, 90 %) est obtenu sous forme d'un solide blanc : mp >250 °C; MS 330 et 332 [M+H]⁺; ¹H NMR (DMSO-*d₆*): δ 3.88 (s, 3H), 6.93 (s, 1H), 7.35 (dd, J = 2.6 et J = 8.6 Hz, 1H), 7.61-7.74 (m, 6H), 11.54 (br s, 1H).

### Composé 33: 7-Méthoxy-3-(4-((triméthylsilyl)éthynyl)phényl)isoquinolin-1(2H)-one

Un mélange du dérivé bromé **32** (1 g, 3.03 mmol), d'iodure de cuivre (29 mg, 0.15mmol), de Pd(PPh₃)₂Cl₂ (53mg, 0.076mmol,) et de triméthylsilylacétylène (915 µL, 6.1mmol) dans 45 mL de DMF et 12 mL de diisopropyléthylamine est agitée sous argon à 80°C pendant 24h. Le mélange réactionnel est filtré sur Celite et le filtrat est dissous dans 100 mL d'eau et 500 mL d'acétate d'éthyle. La phase organique est lavée avec une solution aqueuse saturée de NH₄Cl jusqu'à neutralité, séchée sur MgSO₄ et évaporée sous pression réduite. Le solide obtenu est purifié par chromatographie sur colonne de silice (cyclohexane/acétate d'éthyle, 9/1). On récupère 400 mg (40 %) du produit **33** sous forme d'une poudre blanche : mp 133-135 °C; MS 348 [M+H]⁺; ¹H NMR (CDCl₃, 300 MHz): δ 0.27 (s, 9H), 3.95 (s, 3H), 6.78 (s, 1H), 7.30 (dd, J = 2.7 et J = 5.8 Hz, 2H), 7.51-7.67 (m, 5H), 7.80 (d, J = 2.6 Hz, 1H), 9.9 (br s, 1H).

### Composé 34: 3-(4-Bromophényl)-7-méthoxy-4-nitroisoquinolin-1(2H)-one

A un mélange du composé **32** (4 g, 12.1 mmol) en suspension dans 20 mL d'acétate d'éthyle et 40 mL d'acide acétique est ajouté une solution aqueuse d'acide nitrique à 53% (6 mL, 50 mmol) diluée dans 25 mL d'acétate d'éthyle. La suspension est chauffée à 50°C pendant 2h. En présence de 100 mL d'eau, il y a formation d'un précipité qui est filtré, séché et recristallisé dans le toluène. On obtient 4 g (89 %) du composé **34** sous forme d'un solide orangé: mp 222-224 °C; MS 375 et 377 [M+H]⁺; ¹H NMR (DMSO-*d₆*): δ 3.93 (s, 3H), 7.45-7.55 (m, 3H), 7.67 (d, J = 9.0 Hz, 1H), 7.69-7.75 (m, 3H), 10.5 (br s, 1H).

### Composé 35: 7-Méthoxy-4-nitro-3-(4-((triméthylsilyl)éthynyl)phényl)isoquinolin-1(2H)-one

La préparation de la molécule **35** est réalisée à partir du composé **34** selon le protocole décrit précédemment pour synthétiser la molécule **33**. Dans ces conditions, on récupère le produit attendu **35** (2.45 g, 75 %) sous forme d'une poudre jaune : mp 178-180 °C; MS 393 [M+H]⁺; ¹H NMR (DMSO, 300 MHz): δ 0.25 (s, 9H), 3.93 (s, 3H), 7.47-7.6 (m, 5H), 7.65 (d, J = 9.1 Hz, 1H), 7.72 (d, J = 2.7 Hz, 1H), 12.3 (br s, 1H); ¹³C NMR (DMSO-*d₆*):δ 54.9, 55.7, 92.0, 108.2, 123.2, 123.5, 123.6, 123.9, 125.9, 128.7, 130.0, 131.2, 131.8, 136.9, 159.2, 160.6.

### Composé 36: 3-(4-Ethynylphényl)-7-méthoxy-4-nitroisoquinolin-1(2H)-one

A une solution du composé **35** (2 g, 5.1 mmol) dans 100 mL de THF distillé est ajouté le fluorure de tributylammonium sur silice (5g, 7.61mmol). Le mélange est agité à température ambiante pendant 4h. Puis la solution est filtrée sur Celite et le filtrat est évaporé sous pression réduite. Le produit brut est alors purifié par chromatographie sur colonne de silice (cyclohexane/acétate d'éthyle 7/3). On récupère sous forme d'une poudre jaune le composé **36** (1.33 g, 81 %): mp 246-248 °C; MS 321 [M+H]⁺; ¹H NMR (DMSO-*d₆*): δ 3.91 (s, 3H), 4.37 (s, 1H), 7.49-7.54 (m, 3H), 7.61 (m, 2H), 7.68 (d, J = 9.1 Hz, 1H), 7.72 (d, J = 2.8 Hz, 1H), 12.2 (br s, 1H); ¹³C NMR (DMSO-*d₆*): δ 55.7, 82.7, 108.2, 123.1, 123.5, 123.6, 125.9, 128.7, 130.2, 131.2, 132.0, 136.9, 159.1, 160.5.

### Composé 37: 3-(4-(2-(2-(2-(2-Aminoéthoxy)éthoxy)éthoxy)éthoxy)phényl)-7-méthoxy-4-nitroisoquinolin-1(2H)-one

A une solution de diméthanesulfonate de tétraéthylèneglycol (5.25 g, 15 mmol), obtenu selon A. W. Chwabacher *et al.* (J. Org. Chem. 1998, 65(5), 1717), dans 18 mL d'éthanol à 95° est ajouté l'azoture de sodium (1.0 g). Le mélange réactionnel est porté à reflux pendant 24 h. Puis le solvant est évaporé, le brut réactionnel est mis dans 100 mL de saumure et extrait avec de l'éther 3*100 mL. La phase organique est séchée sur MgSO₄, filtrée et concentrée sous pression réduite. Le résidu obtenu est purifié par chromatographie sur colonne de silice en utilisant un gradient d'AcOEt dans l'hexane (1/1 à 3/1) comme éluants. On obtient le mono-méthanesulfonate de 2-(2-(2-(2-azidoéthoxy)éthoxy)éthoxy)éthyl 47 (2,10 g, 47%) qui est directement utilisé dans l'étape suivante.

Au mélange de 3-(4-hydroxyphényl)-7-méthoxyisoquinolin-1(2H)-one (composé 45) (130 mg, 0.48 mmol) dans du DMF (6 mL) est ajouté le méthanesulfonate de 2-(2-(2-(2-azidoéthoxy)éthoxy)éthoxy)éthyl (175 mg, 0.58 mmol) et du Cs₂CO₃ (190 mg, 0.58 mmol). Le mélange réactionnel est chauffé à 80°C pendant 24 h. Puis le solvant est évaporé, le brut réactionnel est mis dans l'eau (100 mL) et extrait au CH₂Cl₂ (3*100 mL). La phase organique est séchée sur MgSO₄, filtrée et concentrée sous pression réduite. Le résidu obtenu est purifié par chromatographie sur colonne de silice en utilisant un gradient d'EtOH (1 à 3%) dans CH₂Cl₂ comme éluants. On obtient l'intermédiaire 3-(4-(2-(2-(2-(2-azidoethoxy)ethoxy)ethoxy)ethoxy)phenyl)-7-methoxyisoquinolin-1(2*H*)-one (52 mg, 23%) qui est directement utilisé dans l'étape suivante.

La nitration du composé 3-(4-(2-(2-(2-(2-azidoethoxy)ethoxy)ethoxy)ethoxy)phenyl)-7-methoxyisoquinolin-1(2*H*)-one, obtenu ci-dessus, est réalisée selon les conditions décrites par E. Bisagni et al. (Tetrahedron, 1996, 52, 10427-10440) et fournit le composé 3-(4-(2-(2-(2-(2-azidoéthoxy)éthoxy)éthoxy)éthoxy)phényl)-7-méthoxy-4-nitroisoquinolin-1(2*H*)-one avec un rendement de 50%, MS 512 [M-H]⁺; ¹H NMR (CDCl₃) δ 8.63 (1H, br s), 7.82 (1H, br s), 7.63 (1H, d), 7.42 (3H, m), 7.03 (2H, d), 4.19 (2H, t), 3.96 (3H, s), 3.89 (2H, t), 3.71 (10H, m), 3.38 (2H, t), Anal. Cal. pour C₂₄H₂₇N₅O₈˙H₂O, C, 54.23; H, 5.50, Trouvé: C, 54.49; H, 5.21.

A température ambiante, le mélange de 3-(4-(2-(2-(2-(2-azidoéthoxy)éthoxy)éthoxy)éthoxy)phényl)-7-méthoxy-4-nitroisoquinolin-1(2*H*)-one (56 mg, 0.11 mmol), de triphénylphosphine (52 mg) et de THF-Eau (1/4 v/v, 1 mL) est agité pendant 4 jours. L'ensemble est évaporé sous pression réduite et le résidu obtenu est purifié par chromatographie sur colonne de silice en utilisant le mélange CH₂Cl₂/EtOH/NH₄OH 90/10/5 comme éluant pour donner le composé attendu **37** (20 mg, 38%), MS 488 (M+H), ¹H NMR (CDCl₃) δ 7.75 (1H, d), 7.57 (1H, d), 7.35 (1H, d), 7.30 (1H, dd), 6.98 (1H, d), 4.16 (2H, t), 3.88 (3H, s), 3.81 (2H, t), 3.66 (2H, m), 3.57 (4H, m), 3.48 (2H, m), 3.36 (2H, t), Anal. Cal. pour C₂₄H₂₉N₃O₈·H₂O, C, 57.02; H, 6.18, Trouvé: C, 57.41; H, 6.39.

### Composé 38: 4-Ethynyl-1,7-dimethoxy-3-(4-methoxyphenyl)isoquinoline

Un mélange du composé 4-bromo-1,7-diméthoxy-3-(4-méthoxyphényl)isoquinoline (composé **26**) (187 mg, 0.5 mmol), de (triméthylsilyl)éthynyltrifluoroborate de potassium (133 mg, 0.65 mmol), de diacétate de palladium (3.5 mg, 0.015 mmol), de RuPhos (14 mg, 0.03 mmol) et de carbonate de potassium (318 mg, 1.5 mmol) dissous dans 2 mL d'un mélange THF distillé/H₂O dégazée (10/1) est mis sous argon à reflux pendant 2 h. Le milieu réactionnel est filtré sur silice puis condensé sous vide. Le brut est purifié sur colonne de silice (cyclohexane/dichlorométhane, 7/3). 182 mg (93 %) de l'intermédiaire 1,7-diméthoxy-3-(4-méthoxyphényl)-4-((trimethylsilyl)ethynyl)isoquinoline sont obtenus sous forme d'un solide marron qui est directement utilisé dans l'étape suivante,

A une solution de 1,7-diméthoxy-3-(4-méthoxyphényl)-4-((triméthylsilyl)éthynyl)isoquinoline (175 mg, 0.447 mmol) dans 25 mL de THF distillé est ajouté (450 mg, 0.67 mmol) de fluorure de tributylammonium adsorbé sur silice. Le mélange est agité à température ambiante pendant une nuit. La solution réactionnelle est filtrée sur Célite, puis le filtrat est concentré sous pression réduite. Le brut est alors purifié sur colonne de silice (cyclohexane/dichlorométhane, 1/1). On obtient 133 mg (90 %) du composé **38** attendu sous forme d'une poudre vert-de-gris, mp 209-210 °C; MS 320 [M+H]⁺; ¹H NMR (CDCl₃) δ 3.52 (s, 1H), 3.88 (s, 3H), 3.96 (s, 3H), 4.20 (s, 3H), 7.00 (m, 2H), 7.38 (dd, J= 2.7 et J = 9.1 Hz, 1H), 7.53 (d, J = 2.6 Hz, 1H), 8.14-8.24 (m, 3H); ¹³C NMR (CDCl₃) δ 53.8, 55.3, 55.6, 80.1, 84.9, 102.5, 104.1, 113.2, 118.6, 123.3, 127.1, 131.0, 132.3, 133.2, 134.3, 150.4, 158.3, 158.7, 160.

### Composé 39: 4-(Pyrrol-1-yl)-7-méthoxy-3-(4-méthoxyphényle)isoquinoline-1(2H)-one

Le diméthoxytétrahydrofurane (42 µL, 0.32 mmol) est ajouté à 0°C sous agitation à la suspension de 4-amino-7-methoxy-3-(4-methoxyphenyl)isoquinolin-1(2*H*)-one (compound **1**, 80 mg, 0.27 mmol) dans HOAc (5 mL) puis le mélange est chauffé au reflux pendant 2h. De l'eau (10 mL) et NH₄OH à 14% (2 mL) sont introduits au mélange réactionnel refroidi. Le précipité formé est filtré, lavé à l'eau et séché. Le filtrat est extrait au CH₂Cl₂ puis la phase organique est séchée sur MgSO₄ et le résidu obtenu après l'évaporation du solvant est réuni au précipité obtenu précédemment et l'ensemble est chromatographié sur colonne de gel de silice utilisant un gradient d'éthanol (0 à 5%) dans CH₂Cl₂ comme éluant pour donner le composé attendu **39** (26 mg, 28 %) sous forme de solide blanc, mp 238°C, ¹H NMR (CDCl₃): δ 9.42 (1H, br s), 7.82 (1H, d, *J*=3Hz), 7.25 (1H, dd, *J*=6Hz, *J*=3Hz), 7.18 (2H, d, *J*=9Hz), 7.05 (1H, d, *J*=9Hz), 6.85 (2H, d, *J*= 9Hz), 6.66 (2H, d, *J*=1.8Hz), 6.29 (2H, d, *J*=1.8Hz), 6.29 (2H, d, *J*=1.8Hz), 3.96 (3H, s), 3.81 (3H, s); MS 347 (M+H). Anal. Cal. pour C₂₁H₁₈N₂O₃ ·0.5 H₂O: C, 70.98; H, 5.07; N, 7.88. trouvé: C, 70.99; H, 5.41; N, 7.57.

### Composé 40: 4-(Piperidin-1-yl)-7-méthoxy-3-(4-méthoxyphényle)isoquinoline-1(2H)-one

Le cyanoborohydrure de sodium (21 mg, 0.33 mmol) est ajouté lentement sur la solution de 4-amino-7- methoxy-3-(4-methoxyphenyl)isoquinolin-1(2*H*)-one (composé **1**, 100mg, 0.33 mmol) et de glutaraldehyde (25% in H₂O, 0.377 mL, 0.99 mmol) dissous dans CH₃CN (5 mL). De l'acide acétique (3 gouttes) est ensuite introduit et le mélange réactionnel est laissé sous agitation à température ambiante pendant 2h avant de verser dans l'eau (15 mL). Le précipité obtenu est filtré, lavé à l'eau et séché sous vide pour le composé attendu **40** (80 mg, 66%) sous forme de solide blanc, mp 206°C; 1H NMR (CDCl₃) δ 8.16 (1H, br s), 7.95 (1H, d), 7.82 (1H, d), 7.36 (2H, d), 7.34 (1H, dd), 7.01 (2H, d), 3.96 (3H, s), 3.90 (3H, s), MS 387 (M+Na). Anal. Cal. pour C₂₁H₁₈N₂O₃ ·0.5 H₂O: C, 70.77; H, 6.7; N, 7.5. Trouvé: C, 70.63; H, 6.41; N, 7.63.

### Composé 41: 4-Hydroxy-7-méthoxy-3-(4-méthoxyphényl)isoquinolin-1(2H)-one

A 0°C, une solution de nitrite de sodium (24.8 mg, 0.36 mmol) dans l'eau (0.5mL) est ajoutée lentement à la solution de 4-amino-7-methoxy-3-(4-methoxyphenyl)isoquinolin-1(2*H*)-one (100 mg, 0.33 mmol) dissous dans l'acide acétique (1 mL) et l'acide sulfurique (0.5 mL). Le mélange est laissé sous l'agitation à froid pendant 30 min puis l'azoture de sodium (25.7mg, 0.39 mmol) dissous dans un minimun d'eau (0.2 mL) est introduit lentement. On maintient l'agitation toujours à froid encore 3 h, puis de l'eau (10 mL) est versée sur le mélange réactionnel et le résidu solide formé est recueilli par filtration. La chromatographie sur colonne de gel de silice utilisant un gradient d'éthanol (0 à 2%) dans CH₂Cl₂ comme éluant permet d'isoler le composé possédant la plus grande mobilité qui correspond au composé **41** attendu (31 mg, 31%) sous forme de solide blanc, ¹H NMR (CDCl₃) δ 7.91 (1H, d), 7.74 (1H, d), 7.41 (2H, d), 7.16 (1H, dd), 6.85 (2H, d), 6.75 (1H, br s), 4.60 (1H, br s), 3.98 (3H, s), 3.78 (3H, s); MS 295 (M-2H).

### Composé 44: 3-(4-(Benzyloxy)phenyl)-7-methoxyisoquinolin-1(2H)-one

La synthèse de ce composé a été réalisée selon la méthode décrite par E. Bisagni et al. (Tetrahedron, 1996, 52, 10427-10440) dans laquelle l'acide 4-méthoxyphényl acétique est remplacé par l'acide 4-benzyloxyphényl acétique. Toutefois, en raison de l'instabilité de l'intermédiaire, le 2-(4-(benzyloxy)phényl)-3-(4-méthoxyphényl)prop-2-enoyl azide, ce dernier est immédiatement transformé en carbamate par chauffage au reflux dans l'éthanol absolu. La cyclisation de l'ethyl 1-(4-(benzyloxy)phényl)-2-(4-méthoxyphényl)vinylcarbamate est effectuée par chauffage au reflux dans le diphényl éther en présence de tributyl amine (3%, v/v) pendant 3,5 h et fournit le produit **44** attendu avec un rendement de 51%, mp > 260°C, MS 380 [M+Na]⁺, ¹H NMR (DMSO-d₆): δ 11.45 (1H, br s), 7.76 (2H, d), 7.66 (2H, m), 7.42 (6H, m), 7.15 (2H, d), 6.87 (1H, s), 5.22 (2H, s), 3.91 (3H, s), Anal. Cal. pour C₂₃H₁₉NO₃·1/3 H₂O: C, 76.02; H, 5.45; N, 3.85, Trouvé: C, 76.01; H, 5.47; N, 3.93.

### Composé 45: 3-(4-Hydroxyphenyl)-7-methoxyisoquinolin-1(2H)-one

A température ambiante, le mélange de 3-(4-hydroxyphényl)-7-méthoxyisoquinolin-1(2*H*)-one 3-(4-(benzyloxy)phenyl)-7-methoxyisoquinolin-1(2*H*)-one (150 mg, 0.42 mmol), de Pd/C (10%, 70 mg) et d'éthanol absolu (60 mL) est agité sous pression ordinaire d'hydrogène pendant 18 h. Le catalyseur est ensuite filtré, lavé à l'acool puis le solvant est évaporé sous pression réduite. Au résidu est ajouté de l'éther éthylique (50 mL), le solide gris obtenu est ensuite récolté et correspond au composé 3-(4-hydroxyphenyl)-7-methoxyisoquinolin-1(2*H*)-one attendu **45** (90 mg, 80%), mp 255°C, ¹H NMR (DMSO-d₆): δ 11.36 (1H, br s), 9.88 (1 H, br s), 7.68-7.57 (4H, m), 7.36 (1H, dd), 6.88 (2H, d), 6.80 (1H, s), 3.98 (3H, s), 3.90 (3H, s), MS 268 (M+H); Anal. Cal. pour C₁₆H₁₃NO₃·1/3 H₂O: C, 70.32; H, 5.04; N, 5.13. Trouvé: C, 69.92; H, 4.95; N, 5.09.

### Composé 46: 3-(4-(Benzyloxy)phenyl)-7-methoxy-4-nitroisoquinolin-1(2H)-one

La synthèse de ce composé est réalisée selon la méthode de nitration décrite par E. Bisagni et al. (Tetrahedron, 1996, 52, 10427-10440) dans laquelle 7-methoxy-3-(4-methoxyphenyl)isoquinolin-1(2*H*)-one est remplacé par 3-(4-(benzyloxy)phenyl)-7-methoxyisoquinolin-1(2*H*)-one. Il est obtenu sous forme d'une poudre jaune (77% de rendement), mp 252-254 °C, MS 401 [M-H]⁺; ¹H NMR (DMSO-d₆): δ 12.11 (1 H, s), 7.75 (1H, d), 7.63 (1H, d), 7.55-7.35 (8H, m), 5.21 (2H, s), 3.36 (3H, s), Anal. Cal. pour C₂₃H₁₈N₂O₅: C, 68.65; H, 4.51; N, 6.96, Trouvé: C, 68.72; H, 4.49; N, 6.72.

### Composé 47: 3-(4-(2-(2-(2-(2-Azidoéthoxy)éthoxy)éthoxy)éthoxy)phényl)-7-méthoxy-4-nitroisoquinolin-1(2H)-one

A une solution de diméthanesulfonate de tétraéthylèneglycol (5.25 g, 15 mmol), obtenu selon A. W. Chwabacher et al. (J. Org. Chem. 1998, 65(5), 1717), dans 18 mL d'éthanol à 95° est ajouté l'azoture de sodium (1.0 g). Le mélange réactionnel est porté à reflux pendant 24 h. Puis le solvant est évaporé, le brut réactionnel est mis dans 100 mL de saumure et extrait avec de l'éther 3*100 mL. La phase organique est séchée sur MgSO₄, filtrée et concentrée sous pression réduite. Le résidu obtenu est purifié par chromatographie sur colonne de silice en utilisant un gradient d'AcOEt dans l'hexane (1/1 à 3/1) comme éluants. On obtient le mono-méthanesulfonate de 2-(2-(2-(2-azidoéthoxy)éthoxy)éthoxy)éthyl **47** (2,10 g, 47%) qui est directement utilisé dans l'étape suivante.

Au mélange de 3-(4-hydroxyphényl)-7-méthoxyisoquinolin-1(2*H*)-one (composé 45) (130 mg, 0.48 mmol) dans du DMF (6 mL) est ajouté le méthanesulfonate de 2-(2-(2-(2-azidoéthoxy)éthoxy)éthoxy)éthyl (175 mg, 0.58 mmol) et du Cs₂CO₃ (190 mg, 0.58 mmol). Le mélange réactionnel est chauffé à 80°C pendant 24 h. Puis le solvant est évaporé, le brut réactionnel est mis dans l'eau (100 mL) et extrait au CH₂Cl₂ (3*100 mL). La phase organique est séchée sur MgSO₄, filtrée et concentrée sous pression réduite. Le résidu obtenu est purifié par chromatographie sur colonne de silice en utilisant un gradient d'EtOH (1 à 3%) dans CH₂Cl₂ comme éluants. On obtient l'intermédiaire 3-(4-(2-(2-(2-(2-azidoethoxy)ethoxy)ethoxy)ethoxy)phenyl)-7-methoxyisoquinolin-1(2*H*)-one (52 mg, 23%) qui est directement utilisé dans l'étape suivante.

La nitration du composé 3-(4-(2-(2-(2-(2-azidoethoxy)ethoxy)ethoxy)ethoxy)phenyl)-7-methoxyisoquinolin-1(2*H*)-one, obtenu ci-dessus, est réalisée selon les conditions décrites par E. Bisagni et al. (Tetrahedron, 1996, 52, 10427-10440) et fournit le produit attendu 3-(4-(2-(2-(2-(2-azidoéthoxy)éthoxy)éthoxy)éthoxy)phényl)-7-méthoxy-4-nitroisoquinolin-1(2*H*)-one **47** avec un rendement de 50%, MS 512 [M-H]⁺; ¹H NMR (CDCl₃) δ 8.63 (1H, br s), 7.82 (1H, br s), 7.63 (1H, d), 7.42 (3H, m), 7.03 (2H, d), 4.19 (2H, t), 3.96 (3H, s), 3.89 (2H, t), 3.71 (10H, m), 3.38 (2H, t), Anal. Cal. pour C₂₄H₂₇N₅O₈H₂O, C, 54.23; H, 5.50, Trouvé: C, 54.49; H, 5.21.

### Composé 48: Dimethyl 1-[7-methoxy-3-(4-methoxyphenyl)-1-oxo-1,2-dihydroisoquinolin-4-yl]-1H-[1,2,3]triazole-4,5-dicarboxylate.

A 0°C, une solution de nitrite de sodium (12 mg, 0.17 mmol) dans le DMF ( 0.2ml) est ajouté goutte à goutte à une solution de 4-amino-7-methoxy-3-(4-methoxyphenyl)isoquinolin-1(2*H*)-one (composé **1**) (47 mg, 0.16 mmol) dans AcOH ( 0.7 mL). Le mélange est agité à 0°C pendant 30 min. puis l'azoture de sodium (12.3 mg, 0.19 mmol) est ajouté à la réaction et le mélange est agité à 0°C pendant 1 h. La réaction est arrêtée par l'ajout de 10 mL d'eau et le milieu est extrait avec 3*10 ml de dichlorométhane. Les phases organiques sont lavées avec 3*10 mL d'eau, séchées sur MgSO₄ et concentrées sous vide. Le résidu obtenu, placé sous atmosphère d'argon, est dissous dans 1 mL de dioxane puis le diméthyl acétylènedicarboxylate (77.7 µl, 0.63 mmol) est ajouté à la solution et le mélange est alors chauffé à 50°C pendant 12 h. Les solvants sont ensuite évaporés, de l'eau est ajoutée et le milieu est extrait trois fois avec EtOAc. La phase organique est séchée sur MgSO₄, filtrée puis évaporée. Le résidu est purifié par chromatographie sur colonne de gel de silice en utilisant un mélange CH₂Cl₂ - éthanol comme éluant (100% à 98/2) pour donner le composé attendu **48** (10 mg, 13 %) sous forme d'un solide jaune; mp 220°C; ¹H NMR (CDCl₃): δ 9.76 (1H, br s), 7.82 (1H, s), 7.28 (1H, dd), 7.23 (2H, d), 6.86 (2H, d), 6.75 (1H,d), 3.98 (3H, s), 3.95 (3H, s), 3.81 (3H, s), 3.69 (3H, s) ; MS 487 (M+23).

### Composé 49: 7-Méthoxy-3-(4-méthoxyphényl)-4-morpholinoisoquinolin-1(2H)-one

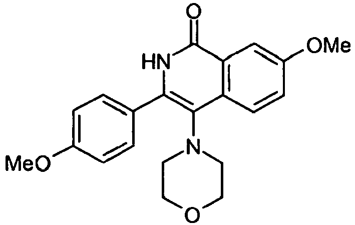

et Composé **50**: 4-(2-(2-hydroxyethoxy)ethylamino)-7-methoxy-3-(4-methoxyphenyl)isoquinolin-1(2*H*)-one

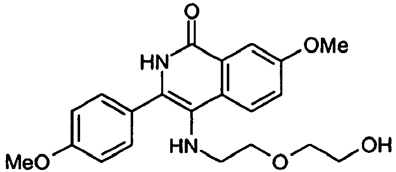

Dans un ballon contenant le 1,4-anhydroerythritol (34 mg, 0.33 mmol) dissous dans un mélange eau/CH₃CN (1:1 ; 4 mL) est additionné NaIO4 (66 mg, 0.33 mmol) et le mélange est maintenu sous agitation pendant 18h. Le 4-amino-7-methoxy-3-(4-methoxyphenyl)isoquinolin-1(2*H*)-one (composé **1**) (100 mg, 0.33 mmol) et NaBH₃CN (63 mg, 1 mmol) sont ensuite ajoutés à la solution de 2,2'-oxybis(acétaldéhyde) préparée précédemment. Ensuite, 3 gouttes d'acide acétique sont additionnées et le milieu est agité pendant 3h à température ambiante. La réaction est arrêtée par l'ajout de 10 mL d'eau. Le milieu est extrait avec 3*10 mL de dichlorométhane. Les phases organiques sont lavées avec 3*10 mL d'eau, séchées sur MgS04 et concentrées sous vide. Le résidu est purifié par colonne chromatographique de gel de silice avec du dichlorométhane-éthanol (100/0 à 98/2) comme éluant pour donner successivement i) le composé 7-méthoxy-3-(4-méthoxyphényl)-4-morpholinoisoquinolin-1(2*H*)-one **49** (10 mg, 8%) sous forme d'un solide blanc, mp >270 °C ; ¹H NMR (CDCl₃): δ 8.18 (1H, br s), 7.99 (1H, d), 7.83 (1H, dd), 7.37 (3H, m), 7.03 (2H, d), 3.97 (3H, s), 3.91 (3H, s), 3.72 (4H, m), 2.90 (2H, m), 2.75 (2H, m) ; MS 367 (M+H) ; et ii) le composé 4-(2-(2-hydroxyethoxy)ethylamino)-7-methoxy-3-(4-methoxyphenyl)isoquinolin-1(2*H*)-one **50** (15 mg, 12%) sous forme d'un solide blanc, mp 139 °C ; ¹H NMR (CDCl₃): δ 8.71 (1H, br s), 7.89 (1H, d), 7.82 (1H, s), 7.45 (2H, d), 7.34 (1H,dd), 7.01 (2H, d), 3.94 (3H, s), 3.87 (3H, s), 3.60 (2H, t), 3.49 (2H, t), 3.38 (2H, t), 3.03 (2H, t), 1.75 (1H, br s), MS 385 (M+H).

### Composé 56 : 4-Chloro-7-hydroxy-3-(4-hydroxyphényl)isoquinoline-1(2H)-one

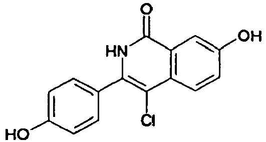

Dans un schlenk, le 7-méthoxy-3-(4-méthoxyphényl)-4-nitroisoquinoline-1(2*H*)-one (composé 6) ( 150 mg, 0.46 mmol) et le chlorure de benzyltriéthylamonium (732 mg, 3.22 mmol) sont dissous dans l'acide chlorhydrique à 37% (13 mL). Le mélange est chauffé à 130°C pendant 20 h. Après l'évaporation sous pression réduite, de l'eau (20 ml) est ajoutée et le précipité formé est filtré, lavé avec de l'eau, séché et chromatographié sur colonne de gel de silice utilisant un gradient de CH2Cl2/éthanol : 100/0 à 98/2 comme éluant pour donner le composé **56** (6 mg, 4%) sous forme de solide noir.
1H NMR (DMSO-*d*6) δ 11.42 (s, 1 H), 10.20 (s, 1H), 9.82 (s, 1H), 7.79 (d, 1 H), 7.58 (d, 1H), 7.36 (d, 2H), 7.33 (s, 1H), 6.85 (d, 2H).

### Composé 57 : 7-Hydroxy-3-(4-hydroxyphényl)isoquinoline-1(2H)-one:

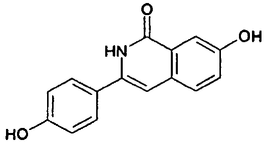

Dans un tube scellé on introduit le composé 7-méthoxy-3-(4-méthoxyphényl)isoquinoline-1(2*H*)-one (300 mg, 1 mmol), le chlorure de benzyltriéthylammonium (1.68 g, 7 mmol) et l'acide chlorhydrique à 37 % (20 mL). Le milieu est chauffé pendant 20 h à 140°C puis l'ensemble est évaporé à sec sous pression réduite. Après l'ajout de l'eau (20 mL) au résidu obtenu, un précipité se forme. Il est filtré et séché sous pour donner 7-hydroxy-3-(4-hydroxyphényl)isoquinoline-1(2*H*)-onze **57** attendu (200 mg, 74%) sous forme d'un solide marron.
1H NMR (DMSO-*d*6) δ 11.14 (br s, 1H), 9.86 (br s, 1H), 9.74 (br s, 1H), 7.51 (m, 4H), 7.16 (s, 1H), 6.82 (s, 2H), 6.67 (s, 1H) ; MS 252 [M+H]+ ;
Anal. Cal. pour C15H11NO3 :
C, 71.14; H, 4.38; N, 5.53. Trouvé : C, 69.77 ; H, 4.53 ; N, 5.86.

### Composé 58 : 6-Méthoxy-2-(4-méthoxyphényl)quinoline-4(1H)-one

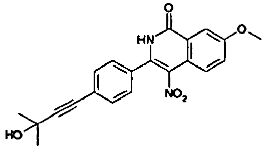

Dans un ballon de 100 mL, la *p*-anisidine (300 mg, 2.4 mmol) est dissoute dans de 2-methoxyethanol (20 mL). L'éthyl 3-(4-méthoxyphényl)-3-oxopropanoate (459 µl, 2.4 mmol) est additionné à la solution précédente puis 3 gouttes d'acide acétique sont également ajoutées au milieu réactionnel. Le mélange est chauffé pendant 24h à 120°C. Ce mélange est ensuite évaporé sous pression réduite et le résidu obtenu est dilué dans le diphénylether (10 mL) puis ajouté dans un tricol de 100 mL contenant du diphényléther (25 mL) bouillant. Le milieu est chauffé au reflux pendant 1h et la solution refroidie est ensuite versée dans l'hexane (100 mL). Le précipité formé est filtré, lavé avec de l'hexane, séché et chromatographié sur colonne de gel de silice utilisant un gradient de CH₂Cl₂/éthanol : 100/0 à 98/2 comme éluant pour donner le composé **58** (100 mg, 15%) sous forme d'une poudre marron.
1H NMR (DMSO-*d*6)
δ 11.55 (s, 1H), 7.76 (d, 2H), 7.69 (d, 1H), 7.47 (s, 1H), 7.28 (d, 1H), 7.10 (d, 2H), 6.26 (s, 1H), 3.82 (s, 6H); MS 282 [M+H]+ ;
Anal. Cal. pour C₁₇H₁₅ NO₃. 0.1 H2O: C, 72.05; H, 5.36;
N, 4.64; Trouvé : C, 71.85 ; H, 5.27 ; N, 4.94.

### Composé 59 : :3-(4-(3,3-dimethylbut-1-ynyl)phenyl)-7-methoxy-4-nitroisoquinolin-1(2H)-one

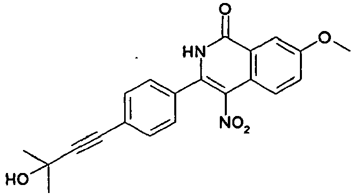

Le composé **59** est synthétisé comme le composé **60** mais à partir de 2-methylbut-3-yn-2-ol et de 32 dont la synthèse est indiquée précédemment.
**¹H NMR** (300 mHz, DMSO): 12.21 (1H), 7.26 (1H), 7.65 (1H), 7.52 (1H), 7.50 (4H), 5.54 (1H), 3.92 (1H), 1.48 (6H)

### Composé 60: 3-(4-(3,3-dimethylbut-1-ynyl)phenyl)-7-methoxy-4-nitroisoquinolin-1(2H)-one

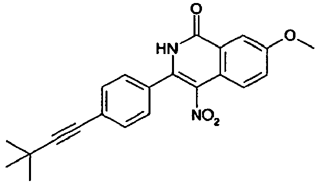

A une solution de **32** (100 mg, 0.26 mmol, 1.0) dans un mélange THF/TEA (1 ml/ 0.75 ml) est ajouté l'alkyne (87.6 mg, 1.06 mmol, 4.0 eq.) le PdCl₂(PPh₃) (15 mg, 0.021 mmol, 0.08 mol %) et CuI (2.0 mg, 0.011 mmol, 0.04 mol %) en tube scellé. Le milieu réactionnel est agité à 70 °C pendant 18h. Après retour à t.a., le milieu réactionnel est concentré sous pression réduite, dilué dans l'acétate d'éthyle, filtré sur celite, lavé à l'acétate d'éthyle. Le brut est ensuite purifié par chromatographie sur gel de sillice (c-hexane/acétate d'éthyle. 3 :2) pour obtenir de 3-(4-(3,3-dimethylbut-1-ynyl)phenyl)-7-methoxy-4-nitroisoquinolin-1(2*H*)-one 60 sous frome d'un solide jaune (70 mg, rdt = 71 %).
¹H NMR (300 mHz, DMSO): 9.02 (1H), 7.8 (1H), 7.65 (1H), 7.42 (2H), 7.38 (3H), 3.96(3H), 133 (9H)

### Composé 61 : 2-Amino-7-méthoxy-3-(4-méthoxyphényl)isoquinoline-1(2H)-one

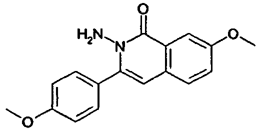

Dans un ballon de 25 mL sous argon, 7-méthoxy-3-(4-méthoxyphényl)isoquinoline-1(2*H*)-one (100 mg, 0.38 mmol) est dissous dans le DMF (5 mL). A 0°C, l'hydrure de sodium ( 60% dans l'huile, 30mg, 0.76 mmol) est ajouté. Après 30 min d'agitation à 0°C, le O-mésitylènesulfonylhydroxylamine (fraîchement préparé selon la litérature Y. Tamura et al. Synthesis 1977, 1) (82 mg, 0.38 mmol) est additionné à température ambiante. L'agitation est maintenue pendant 12h puis de l'eau (20 mL) est ajoutée au milieu réactionnel. Ce milieu est extrait avec du dichorométhane. Les phases organiques sont lavées avec de l'eau, séchées sur MgSO₄ et concentrées sous vide. Le résidu obtenu est chromatographié sur colonne de gel de silice utilisant un gradient de CH₂Cl₂/éthanol : 100/0 à 98/2 comme éluant pour donner le composé **61** (51 mg, 46%) sous forme d'un solide orange.
1H NMR CDCl3 δ 7.82 (s, 1H), 7.50 (t, 3H), 7.29 (d, 1 H), 7.01 (d, 2H), 6.50 (s, 1H), 5.18 (s, 2H), 3.93 (d, 6H);
MS 297 [M+H]+.

### Composé 62 : 2-Amino-7-méthoxy-3-(4-méthoxyphényl)-4-nitroisoquinoline-1(2H)-one

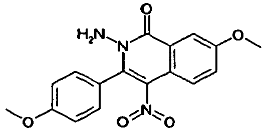

Dans un ballon de 25 mL sous argon, le 7-méthoxy-3-(4-méthoxyphényl)-4-nitroisoquinoline-1(2*H*)-one (composé D5, 150 mg, 0.46 mmol) est dissous dans 5 mL de diméthylformamide. A 0°C, le carbonate de potassium (317 mg, 2.3 mmol) est ajouté. Après 30 min d'agitation à 0°C, le O-mesitylenesulfonylhydroxylamine (fraîchement préparé selon la litérature Y. Tamura et al. Synthesis 1977, 1) (93 mg, 0.46 mmol) est additionné. L'agitation est maintenue pendant 12h à température ambiante. Le milieu est ensuite neutralisé avec 20 mL d'une solution d'acide chlorhydrique N puis extrait avec 3*10 mL de dichorométhane. Les phases organiques sont lavées avec 3*10 ml d'H₂O, séchées sur MgSO₄ et concentrées sous vide. Le résidu obtenu est chromatographié sur colonne de gel de silice utilisant un gradient de CH₂Cl₂/éthanol : 100/0 à 97/3 comme éluant pour donner le composé **62** (65 mg, 41%) sous forme d'un solide jaune.
1H NMR CDC13 δ 7.88 (s, 1H), 7.56 (d, 1H), 7.40 (d, 3H), 7.04 (d, 2H), 5.10 (s, 2H), 3.99 (s, 3H), 3.88 (s, 3H) ;
MS 342 [M+H]+.

### Composé 64 : 1,7-Diméthoxy-3-(4-méthoxyphényl)isoquinoline et composé 65, 7-Méthoxy-3-(4-méthoxyphényl)-2-méthylisoquinoline-1(2H)-one

Dans un ballon de 25 mL sous argon, le 7-méthoxy-3-(4-méthoxyphényl)isoquinoline- 1(2H)-one (200 mg, 0.71 mmol) est dissous dans 5 mL de diméthylformamide. A 0°C, le carbonate de potassium (491mg, 3.5 mmol) est ajouté. Après 30 min d'agitation à 0°C, le iodométhane (444 µL, 7.1 mmol) est additionné. L'agitation est maintenue pendant 12h à température ambiante. Le milieu est neutralisé avec 20 mL d'une solution d'acide chlorhydrique 1N puis extrait avec 3*10 mL de dichorométhane. Les phases organiques sont lavées avec 3*10 ml d'H₂O, séchées sur MgSO₄ et concentrées sous vide. Le résidu obtenu est chromatographié sur colonne de gel de silice utilisant un gradient de CH₂Cl₂/éthanol : 100/0 à 95/5 comme éluant pour donner le composé **64** (39 mg, 18%) sous forme d'un solide blanc et le composé **65** (122 mg, 58%) sous forme d'un solide beige.
Composé **64** :

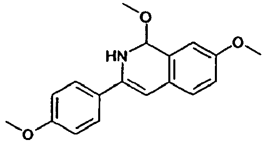

1H NMR CDCl3 δ 8.11 (d, 2H), 7.70 (d, 1H), 7.58 (s, 1H), 7.53 (s, 1H), 7.31 (d, 1H), 7.03 (d, 2H), 4.25 (s, 3H), 3.97 (s, 3H), 3.90 (s, 3H);
MS 296 [M+H]+.

### Composé 65 : 7-Méthoxy-3-(4-méthoxyphényl)-2-méthylisoquinoline-1(2H)-one

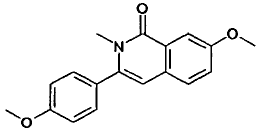

1H NMR CDCl3 δ 7.84 (s, 1H), 7.40 (d, 1H), 7.31 (d, 2H), 7.24 (d, 1H), 6.97 (d, 2H), 6.40 (s, 1H), 3.93 (s, 3H), 3.86 (s, 3H), 3.44 (s, 3H);
MS 296 [M+H]+.

### Composé 66 :7-Méthoxy-3-(4-méthoxyphényl)-2-méthyl-4-nitroisoquinoline-1(2H)-one

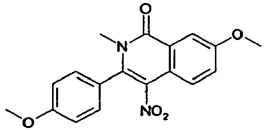

Dans un ballon de 25 mL, le 7-méthoxy-3-(4-méthoxyphényl)-2-méthylisoquinoline-1(2*H*)- one (composé **65**, 50 mg, 0,17 mmol) est dissous dans 0,5 mL d'acétate d'éthyle et 1,5mL d'acide acétique. A 0°C, l'acide nitrique ( à 52.5%, 38µl, 0,425mmol) est ajouté au milieu et l'agitation est maintenue pendant 3 h. 10 ml d'eau glacée sont ajoutés et le précipité jaune obtenu est ensuite filtré, lavé avec de l'eau et séché pour donner le composé **66** (25 mg, 44%) sous forme d'un solide jaune.
1H NMR CDCl3 δ 7.91 (s, 1H), 7.50 (d, 1H), 7.38 (d, 1 H), 7.32 (d, 2H), 7.03 (d, 2H), 3.98 (s, 3H), 3.88 (s, 3H), 3.35 (s, 3H) ;
MS 341 [M+H]+.

### Composé 67: 2-(2-Hydroxyéthyl)-7-méthoxy-3-(4-méthoxyphényl)isoquinoline-1(2H)-one

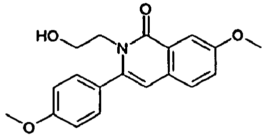

Dans un ballon de 25 mL sous argon, le composé 7-méthoxy-3-(4-méthoxyphényl)isoquinoline-1(2*H*)-one (300 mg, 1.06 mmol) est dissous dans 10 mL de DMF. Le carbonate de potassium (737mg, 5.3 mmol) est ensuite ajouté. Après 30 min d'agitation, le 2-bromoéthanol (746 µl, 10.6 mmol) est additionné. L'agitation est maintenue pendant 24 h à 100°C. Une fois revenu à température ambiante, le milieu est neutralisé avec 20 mL d'une solution d'acide chlorhydrique N puis extrait avec 3*10 mL de dichorométhane. Les phases organiques sont lavées avec 3*10 mL d'H2O, séchées sur MgS04 et concentrées sous vide. Le résidu est purifié par colonne chromatographique de gel de silice avec du cyclohexane-acétate d'éthyle (60/40) comme éluant pour donner **67** (35 mg, 10%) sous forme d'un solide blanc. 1H NMR CDC13 δ 7.98 (d, 2H), 7.68 (d, 1H), 7.54 (d, 2H), 7.32 (d, 1H), 7.02 (d, 2H), 4.84 (d, 2H), 4.23 (br s, 1H), 4.13 (d, 2H), 3.96 (s, 3H), 3.87 (s, 3H),
MS 326 [M+H]+.

### Composé 68 : 2-(2-Hydroxyéthyl)-7-méthoxy-3-(4-méthoxyphényl)-4-nitroisoquinoline-1(2H)-one

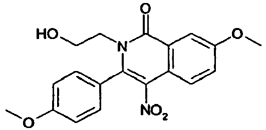

Dans un ballon de 25 mL sous argon, le 7-méthoxy-3-(4-méthoxyphényl)-4-nitroisoquinoline-1(2*H*)-one (composé D5, 150 mg, 0.46 mmol) est dissous dans 5 mL de DMF. Le carbonate de potassium (317 mg, 2.3 mmol) est ensuite ajouté. Après 30 min d'agitation, le 2- bromoéthanol (329 µl, 4.6 mmol) est additionné. L'agitation est maintenue pendant 24 h à 50°C. Une fois revenu à température ambiante, le milieu est neutralisé avec 20 mL d'une solution d'acide chlorhydrique 1N puis extrait avec 3*10 mL d'acétate d'éthyle. Les phases organiques sont lavées avec 3*10 ml d'H2O, séchées sur MgSO₄ et concentrées sous vide. Le résidu obtenu est chromatographié sur colonne de gel de silice utilisant le mélange cyclohexane-acétate d'éthyle (60/40) comme éluant pour donner 2-(2-hydroxyéthyl)-7- méthoxy-3-(4-méthoxyphényl)-4-nitroisoquinoline-1(2*H*)-one **68** attendu (73 mg, 43%) sous forme d'un solide jaune.
1H NMR CDCl3 δ 7.90 (s, 1H), 7.49 (d, 1H), 7.42 (d, 1H), 7.34 (d, 2H), 7.02 (d, 2H), 4.12 (d, 2H), 3.99 (s, 3H), 3.89 (s, 3H), 3.80 (d, 2H), 2.80 (br s, 1 H);
MS 393 [M+Na]+.

### Composé 69 : 4-Iodo-7-méthoxy-1-(4-méthoxybenzyloxy)-3-(4-méthoxyphényl)isoquinoline

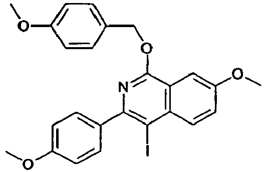

Dans un ballon de 25 mL sous argon, le 4-iodo-7-methoxy-3-(4-methoxyphenyl)isoquinolin 1(2*H*)-one (500 mg, 1.23 mmol) est dissous dans 15 mL de THF anhydre. Le 4- méthoxybenzylalcool ( 217µl, 1.84 mmol), la triphénylphosphine (482 mg, 1.84 mmol) et le diisopropyl azodicarboxylate (261 µl, 1.84 mmol) sont ajoutés à la solution précédente. Après une nuit d'agitation à température ambiante, le milieu est hydrolyse avec 20 mL d'eau et extrait avec 3* 20 mL de dichlorométhane. Les phases organiques sont lavées avec 3*10 mL d'eau, séchées sur MgSO₄ et concentrées sous vide. Le composé **69** est obtenu par recristallisation dans l'éther (358 mg, 55%). 1H NMR CDC13 δ 7.94 (s, 1H), 7.89 (d, 1H), 7.34 (d, 1H), 6.98 (d, 2H), 6.91 (d, 2H), 6.81 (d, 2H), 6.72 (d, 2H), 5.19 (s, 2H), 3.97 (s, 3H), 3.88 (s, 3H), 3.74 (s, 3H);
MS 528 [M+H]+.

### Composé 70 :7-Méthoxy-1-(4-méthoxybenzyloxy)-3-(4-méthoxyphényl)-4-(trifluorométhyl) isoquinoline

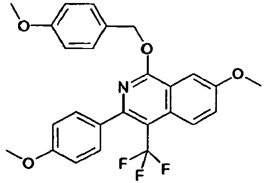

Dans un ballon de 10 mL, le 4-iodo-7-methoxy-1-((4-methoxybenzyl)oxy)-3-(4-methoxyphenyl)isoquinoline (100 mg, 0.2 mmol) est dissous dans 2 mL de DMF anhydre. L'iodure de cuivre (95.2 mg, 0.5 mmol) et le méthyl 2,2-difluoro-2-(fluorosulfonyl)acetate (119 µl, 0.94 mmol) sont ajoutés à la solution précédente. Après une nuit d'agitation à 80°C, le milieu réactionnel est filtré, lavé avec 30 mL de dichlorométhane. Le filtrat obtenu est lavé avec 3* 10 mL d'eau, séché sur MgSO₄ et concentré sous vide pour donner le composé **70** attendu (75 mg, 80 %). 1H NMR CDCl3 δ 7.97 (s, 1H), 7.87 (d, 1H), 7.37 (dd, 1H), 6.98 (d, 2H), 6.85 (d, 2H), 6.80 - 6.62 (m, 4H), 5.04 (s, 2H), 3.96 (s, 3H), 3.86 (s, 3H), 3.75 (s, 3H) ;
MS 470 [M+H]+.

### Composé 71 : 1-Amino-7-méthoxy-3-(4-méthoxyphényl)-4-nitroisoquinoline

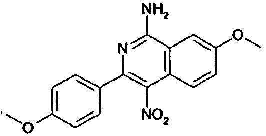

Dans un schlenk de 25 mL, sous argon, le 1-chloro-7-méthoxy-3-(4-méthoxyphényl)-4-nitroisoquinoline (100 mg, 0.43 mmol) est dissous dans 20 mL d'une solution saturée d'ammoniaque dans l'éthanol. Après 4 jours d'agitation à 80°C, le milieu est concentré sous vide puis purifié par colonne chromatographique de gel de silice avec du dichlorométhane-éthanol (95/5) comme éluant pour donner le composé aminé **71** attendu (79 mg, 56%) sous forme d'un solide jaune. 1H NMR CDCl3 δ 7.81 (d, 1H), 7.60 (d, 2H), 7.45 (d, 1H), 7.07 (s, 1H), 6.99 (d, 2H), 5.44 (s, 2H), 3.99 (s, 3H), 3.87 (s, 3H) ; MS 326 [M+H]+.

### Composé 72 :3-(4-hydroxyphenyl)-7-methoxy-4-nitroisoquinolin-1(2H)-one :

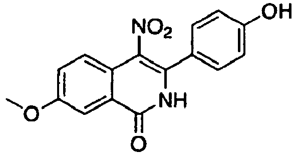

La molécule a été préparé selon la procédure décrite par Bisagni (Bisagni, E.; Landras, C.; Thirot, S.; Huel, C., Tetrahedron 1996, 52 (31), 10427-10440.), a partir de l'acide 2-(4-(benzyloxy)phenyl)ethanoique (Mannekens, E.; Tourwé, D.; Lubell, W. D., Synthesis 2000, 2000 (09), 1214,1216) et de 4-methoxybenzaldehyde. L'intermédiaire OBenzylé est ensuite debenzylé selon la méthode décrite par Mannekens (Mannekens, E.; Tourwé, D.; Lubell, W. D., Synthesis 2000, 2000 (09), 1214,1216).
**¹H NMR** (300 mHz, CdCl₃): δ 12.01 (s, 1H), 10.01 (s, 1H), 7.70 (d, 1H), 7.56 (d, 1H), 7.49 (dd, 1H), 7.31 (d, 1H), 6.86 (d, 2H), 3.91 (s, 3H)

### Composé 73 : tert-butyl 4-(7-methoxy-4-nitro-1-oxo-1,2-dihydroisoquinolin-3-yl)phenyl carbonate

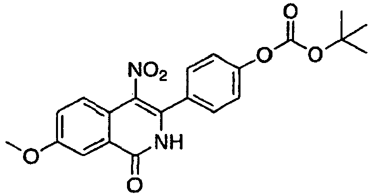

Une solution de 3-(4-hydroxyphenyl)-7-methoxy-4-nitroisoquinolin-1(2*H*)-one (50.0 mg, 0.16 mmol, 1.0 eq.), K₂CO₃ (22.15 mg, 0.16 mmol, 1.0 eq.), et Boc2 (34.9 mg, 0.16 mmol, 1.0 eq.) dans le DMF (1 ml) dégazé à l'argon dans un tube scellé est agité à t.a. pendant 18h. Le milieu réactionnel est dilué avec H₂O. Les phases organiques sont extraites avec de l'acétate d'éthyle (2 fois), lavé avec une solution de NaCl saturée (2 fois), séchée sur MgSO4 puis concentré sous pression réduite, pour obtenir un produit pur (rdt= 100 %).
¹H NMR (300 mHz, DMSO): δ 12.21 (s, 1H), 7.70 (d, 1H), 7.63 (d, 1H), 7.56 (d, 1H), 7.50 (dd, 1H), 7.33 (d, 1H), 3.92 (s, 3H), 1.51 (s, 9H)

### Composé 74 : :2-(p-hydroxybenzyle)-3penylisoquinolin-1(2H)-one

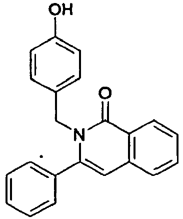

Dans un ballon séché sous vide est ajouté NaOtBu (6823 mg, 0.71 mmol, 3.0 eq.), Xantphos (6.8 mg, 0.012 mmol, 5 mol %), Pd₂dba₃ (10.8 mg, 0.012 mmol, 5 mol %). Les réactifs sont dissout dans du toluène distillé sur CaH₂ sous argon (0.8 ml). La solution rouge est dégazé sous argon en sonicant pendant 30 secondes. Une solution de (*Z*)-1-bromo-2-(2-bromo-2-phenylvinyl)benzene (80 mg, 0.24 mmol, 1.0 eq) et p-methoxybenzyle amine (114 mg, 0.83 mmol, 3.5 eq.) dans du toluene distillé sur CaH₂ sous argon (0.8 ml) est ajouté au système catalytique. Après addition, le milieu réactionnel est dégazé à l'argon, et agité pendant 1h30 à 55°C. Ensuite, le milieu réactionnel est dégazé par du monoxyde de carbone (en ballon, 1 atm.) pendant 25 min à 55°C. Ensuite, le milieu réactionnel est agité à 90°C pendant 16h. Après refroidissement à t.a., le milieu réactionnel est quenché par une solution de HCl 1N (5 ml). Les phases organiques sont extraites avec du dichloromethane (2 fois), lavé avec une solution de NaCl saturée (2 fois), séchée sur MgSO4 puis concentré sous pression réduite. Le brut est ensuite purifié par chromatographie sur gel de silice (pentane/ diethyle éther (7/3)) pour obtenir de 2-(4-methoxybenzyl)-3-phenylisoquinolin-1(2*H*)-one (45 mg, rdt=54 %) sous forme d'un solide jaunâtre.

¹H NMR (300 mHz, CdCl₃): δ 8.49 (d, 1H), 7.67 (t, 1H), 7.50 (m, 2H), 7.44-7.35 (m, 3H), 7.22 (d, 2H), 6.83 (d, 2H), 6.69 (d, 2H), 5.20 (s, 2H), 3.76 (s, 3H)
¹³C NMR (75 mHz, CdCl₃): δ 1632 (CO); 158.6 (C); 143.9 (C); 136.5 (C); 136.0 (C); 132.5 (CH); 129.8 (CH); 129.3 (CH); 128.8 (CH); 128.4 (CH); 128.3 (CH, CH); 126.7 (CH); 125.6 (CH); 125.3 (C); 113.7 (CH); 108.2 (CH); 55.2 (-OMe); 48.0 (-CH₂-)
A une solution de 2-(4-methoxybenzyl)-3-phenylisoquinolin-1(2*H*)-one obtenue ci-dessus(30 mg, 0,087 dans du CH₂Cl₂(1ml) à-78°C est ajouté une solution de BBr3 (170 µL d'un solution (1M) dans le CH₂Cl₂), La solution orange est agitée de 0°C à température ambiante pendant 1h. Une CCM (cyclohexane/Ethyl acetate/triethyleamine 1%) indique une réaction complète. De l'EtOH (2ml), une solution aqueuse saturée NaHPO₄ et du CH₂Cl₂ (2ml) sont ajoutés. Après extraction, les phases organiques collectées sont séchées (MgSO₄) et évaporées sous vide. Le résidu obtenu est purifié par chromatographie sur colonne de SiO₂ (cyclohexane-ethyle acetate, 1 :1). On obtient 25 mg de **74** (93%).
¹H NMR (CDCl₃, 300 MHz): δ 9,27 (S, H) ; 8,41 (d,1H, J= 14) ; 7,66-7,52 (m, H) ; 7,50-7,40 (m, 1H) ; 7,46-7,32 (m, 3H) ; 7,26-7,21 (m, 2H) ; 6,72 (d,J=7, 1H) ; 6, (d, J=7, 1H) ; 6,46 (s,1H).

### Exemple 14 : Modulation de la chimiorésistance au Paclitaxel par les composés de l'invention

Comme décrit dans "Kyu-Ho Han et al., Modulation of drug résistance by α-tubulin in paclitaxel-resistant human lung cancer cell lines European Journal of Cancer 2000 36 (12) : 1565-1571", les clones de la lignée NCI-H460 (ATCC # HTB-177, carcinome pulmonaire humain à cellules larges), résistants au Paclitaxel sont sélectionnés et dénommés NCI-H460/R.

Ces cellules résistantes, ainsi que des cellules NCI-H460 « normales » sont encemencées dans des plaques 96 puits (1000 par puit) et incubées avec différentes concentrations du composé 6 ou de paclitaxel ou de vinblastine (de 0.01 nM à 100µM), durant 5 jours (120 heures).

A la fin de cette incubation, du Hoechst 33342 (1 µg/ml) est ajouté dans le milieu de culture et les cellules vivantes sont comptabilisées sous un microscope à fluorescence (Zeiss Axiovert 200M), afin d'évaluer la concentration diminuant de 50% le nombre de cellules à 120h par rapport au contrôle, pour chaque composé testé.

### Exemple 15 : Evaluation de l'activité anti-inflammatoire des composés de l'invention

Pour induire un oedème inflammatoire de la patte de rat ou souris, une injection intradermique de carraghénine (CAR) (1%, v/v) est réalisée et évaluée selon la méthode de Winter, C. A., Risley E. A. and Nuss G.W. (Carrageenan-induced edema in the hindpaw of rats as an assay for anti-inflammatory drugs. Proc. Soc. Axp. Biol. Med, 1962, v.l11 : pp 544-7).

Les composés de l'invention sont donnés oralement de 1 à 6 heures avant l'injection de CAR. Une solution de CAR à 1% dans une solution saline est injectée par voie s. c. dans la partie sous-plantaire de la patte postérieure droite de rat (souris). Ensuite, le volume de la réaction inflammatoire est mesuré par pléthysmographie après entre 1 et 5 heures de l'injection de CAR. Le volume de la réaction inflammatoire est comparé avec celui du contrôle (injection CAR sans traitement ou après le traitement avec le véhicule).

## Revendications

1. Composé de formule générale (I) suivante : 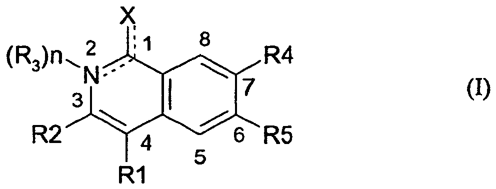 dans lequel :
1) la liaison entre le carbone 1 et le groupement X est simple ou double, la liaison entre l'azote 2 et le carbone 1 pourra être simple ou double
étant entendu que :
a) lorsque la liaison entre X et ledit carbone 1 est double, alors la liaison entre l'azote 2 et le carbone 1 est simple:
et,
b) lorsque la liaison entre X et ledit carbone 1 est simple, alors la liaison entre l'azote 2 et le carbone 1 est double et la formule 1 correspond à un système aromatique de formule I-A suivante : 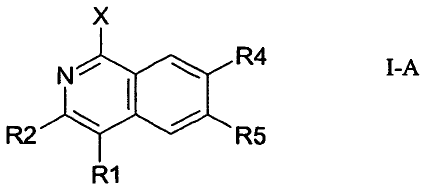
2) n représente 0 ou 1,
3) X représente :
a) lorsque n =1:
O, S,
b) lorsque n = 0 :
OPO₃H₂, OPO-(O-(C₁-C₂)alkyle)₂, l'alkyle étant éventuellement substitué par un ou plusieurs fluors, O-(C₁-C₆)alkyle, en particulier OCH₃, l'alkyle étant éventuellement substitué par un ou plusieurs fluors, NH-(C₁-C₆)alkyle, S(C₃-C₆)cycloalkyle,
4) R1 représente :
, OH, OPO₃H₂, OPO-(O-(C₁-C₂)alkyle)₂, l'alkyle étant éventuellement substitué par un ou plusieurs fluors,
CF₃, CH₂OR', R' représentant H ou (C₁-C₆)alkyle, l'alkyle étant éventuellement substitué par un ou plusieurs fluors, CHO,
CONR_{c}R_{d}, R_{c} et R_{d} représentant indépendamment l'un de l'autre : H, un alkyle en C₁-C₆, et NR_{c}R_{d} représente un acide aminé lié par sa fonction amine, en particulier une sérine ou une thréonine, un cycloalkyle en C₃-C₆, ou R_{c} et R_{d} représentent ensemble un alkyle en C₂-C₆,
NO₂, N₃, ≡ , CN, C(=N)NH₂, SO₃H, SO₂NH₂, SO₂NHCH₃, NHSO₂CH₃, CH₂SO₂NR_{c}R_{d}, CH₂NHSO₂Rc, SO₂-NRaRb, SO₂-imidazole, NRₐR_{b}, où :
Rₐ et R_{b} représentent indépendamment l'un de l'autre: H,un alkyle en C₁-C₆, un cycloalkyle en C₃-C₆, ou
Ra est H, un alkyle en C₁-C₆, un cycloalkyle en C₃-C₆, et Rb = COR_{f}, COOR_{f} ou CONR_{f}R_{f} R_{f} et R_{f} représentant H, un alkyle en C₁-C₆, un cycloalkyle en C₃-C₆ ou une chaîne d'un amino acide (telle que CH(CH₂OH)NH₂ pour la sérine) Rₐ et R_{b} représentent ensemble un alkyle en C2-C6,
Rₐ et R_{b} peuvent former un cycle en C5 à C7, notamment une pyrrolidine,une pipérazine, une morpholine, une thiomorpholine,
un hétéroaryle substitué ou non, en particulier une pyridine, un imidazole, un oxazole, un triazole, un pyrrole, un tétrazole.
5) R2 représente :
un phényle substitué ou non par un à trois substituants choisis parmi :
un halogène, un OH, NHRa,
ORe, Re représentant un benzyle, un triazole méthylène substitué ou non, notamment par un alkyle en C1-C6 ,
OPO₃H₂, OPO-(O-(C₁-C₂)alkyle)₂, l'alkyle étant éventuellement substitué par un ou plusieurs fluors, un NH₂,
un groupe NH-CORc dans lequel Rc représente H, un alkyle en C₁-C₆, un cycloalkyle en C₃-C₆ ou une chaîne d'un amino acide (telle que CH(CH₂OH)NH₂ pour la sérine),
un O-alkyle en C₁-C₆, en particulier OCH₃, l'alkyle étant éventuellement substitué par un ou plusieurs fluors,
un O-COR₁ où R₁ représente O-alkyle en C₁-C₆, ou NRiRii, Ri et Rii pouvant être alkyle en C₁-C₆,
un groupe alkyle en C₂-C₄, le groupe alkyle étant éventuellement substitué par un ou plusieurs fluors, un groupement alcényle en C₂-C₄ substitué ou non,
un groupe alcynyle en C₂-C₄ substitué ou non, en particulier par un triméthylsilyle, un tert-butyle, un hydroxy-2-propyle ou un isopropyle,
un hétéroaryle substitué ou non, en particulier une pyridine, un imidazole, un oxazole, un triazole, un pyrrole, un benzofurane, un thiophène, un benzothiophène, un indole,
un cyclohéxyle, une pipérazine, une morpholine, une thiomorpholine, une pipéridine, un groupe 4-(NH₂-(CH₂-CH₂O)p)Ph dans lequel p est un entier de 1 à 6
6) R3 représente :
H, (C₁-C₆)alkyle, l'alkyle étant éventuellement substitué par un ou plusieurs fluors, (C₃-C₆)cycloalkyle, O-alkyle en C₁ à C₆, NH₂, un groupe propargyle, CH₂CN,
7) R4 et R5 représentent indépendamment l'un de l'autre :
H, OH, OPO₃H₂, OPO-(O-(C₁-C₂)alkyle)₂, l'alkyle étant éventuellement substitué par un ou plusieurs fluors, un O-alkyle en C₁-C₆, en particulier OCH₃, l'alkyle étant éventuellement substitué par un ou plusieurs fluors, un alkyle en C₁-C₆, l'alkyle étant éventuellement substitué par un ou plusieurs fluors, un cycloalkyle en C₃-C₆, un halogène,
R4 et R5 représentent ensemble un alcényle (en C₁-C₂) dioxy éventuellement substitué ² par un ou plusieurs fluors,
et les sels pharmaceutiquement acceptables,
pour utilisation chez les mammifères en tant qu'agent de destruction vasculaire dans la prévention et le traitement d'angiogénèses pathologiques choisies parmi lesrétinopathies et les tumeurs bénignes ou malignes (cancéreuses).

2. Composé selon la revendication 1 pour son utilisation chez les mammifères en tant qu'inhibiteur de protéine phosphatase 1, agent de destruction vasculaire et agent antiprolifératif dans la prévention et le traitement d'angiogénèses pathologiques choisies parmi les rétinopathies et lestumeurs bénignes ou malignes (cancéreuses).

3. Composé selon la revendication 1 pour son utilisation chez les mammifères en tant qu'inhibiteur de polymérisation de tubuline, agent de destruction vasculaire et agent antiprolifératif dans la prévention et le traitement d'angiogénèses pathologiques choisies parmi lesrétinopathies et les tumeurs bénignes ou malignes (cancéreuses).

4. Composé selon la revendication 1 pour son utilisation chez les mammifères en tant qu'inhibiteur de protéine phosphatase 1, de polymérisation de la tubuline, agent de destruction vasculaire et agent antiprolifératif dans la prévention et le traitement d'angiogénèses pathologiques choisies parmi les rétinopathieset les tumeurs bénignes ou malignes cancéreuses

5. Composé destiné à être utilisé selon l'une des revendications 2 à 4, dans lequel le groupe R1 de la formule générale (I) est choisi parmi NO₂ ou pyrrole.

6. Composé destiné à être utilisé selon l'une des revendications 2 à 4, dans lequel le groupe R2 de la formule générale (I) représente un phényle substitué en position para par un groupement choisi parmi ORe, Re étant tel que défini dans la revendication 1, un halogène, un groupe alcyne en C2-C4 substitué ou non, en particulier par un triméthylsilyle, un tert-butyle, un hydroxy-2-propyle ou un isopropyle.

7. Composé destiné à être utilisé selon l'une des revendications 1 à 4, dans lequel le groupe R1 de la formule générale (I) est choisi parmi NO2 ou pyrrole et le groupe R2 de la formule générale (I) représente un phényle substitué en position para par un groupement choisi parmi ORe, Re étant tel que défini dans la revendication 1, un halogène, un groupe alcyne en C2-C4 substitué ou non, en particulier par un triméthylsilyle, un tert-butyle, un hydroxy-2-propyle ou un isopropyle.

8. Composé destiné à être utilisé selon la revendication 7, dans lequel le groupe R3 de la formule générale (I) représente H.

9. Composé destiné à être utilisé selon la revendication 1, dans laquelle les composés de formule (I) sont choisis parmi les suivants :

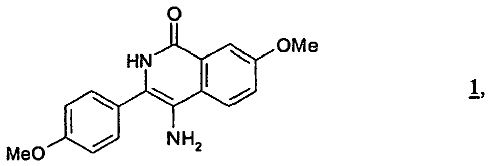

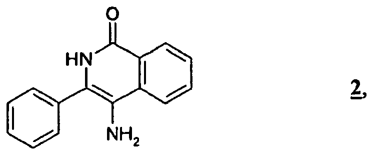

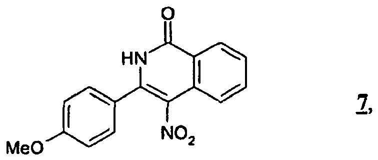

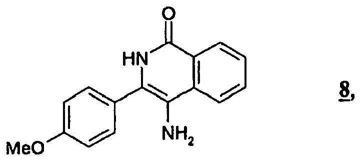

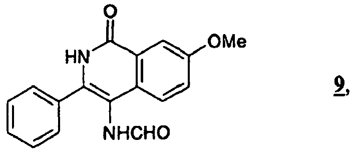

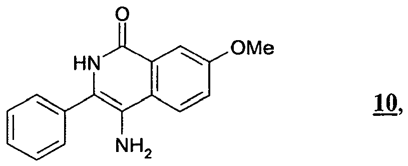

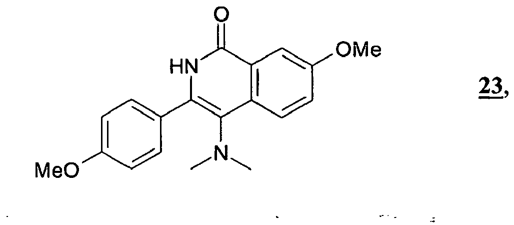

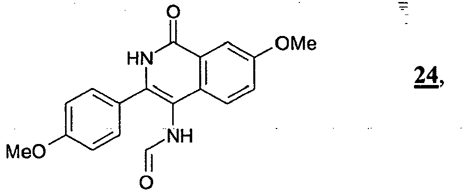

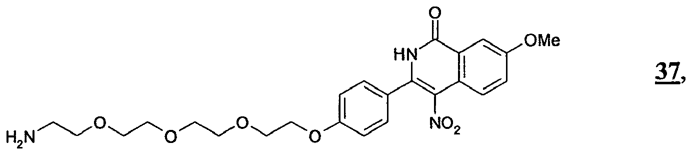

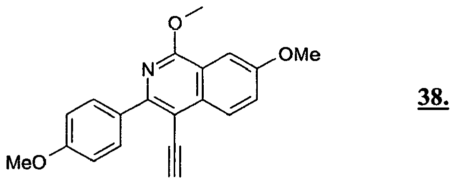

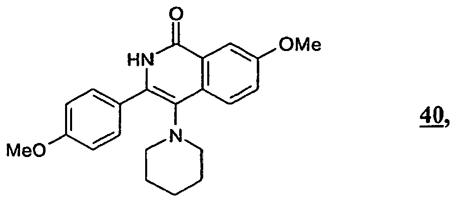

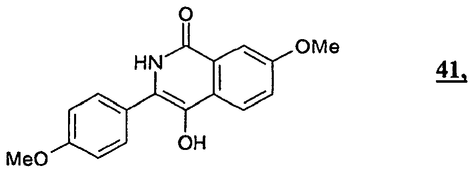

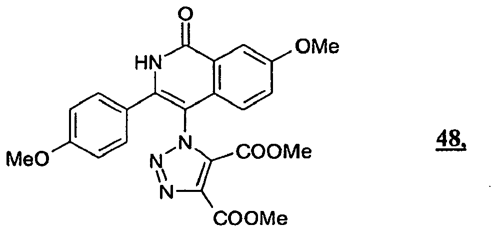

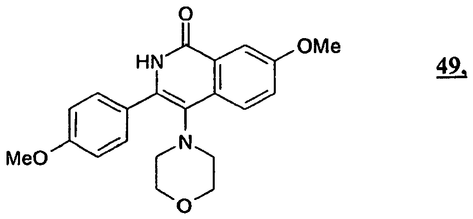

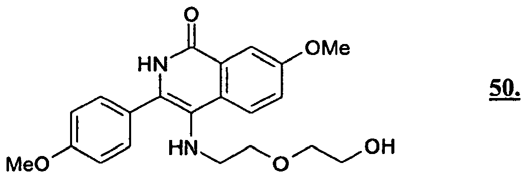

10. Composé destiné à être utilisé selon l'une des revendications 1 à 8, dans lequel les composés de formule (I) sont choisis parmi les suivants :

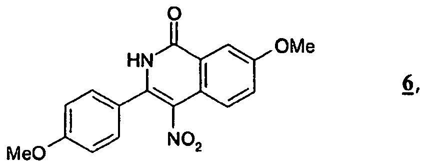

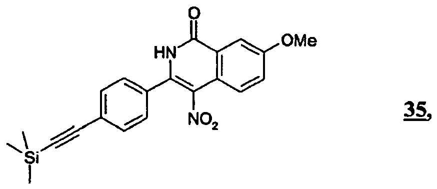

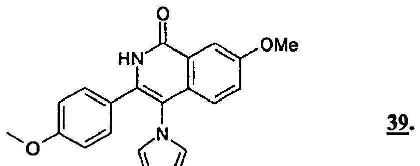

11. Composé destiné à être utilisé selon l'une des revendications 1 à 8, dans lequel les composés de formule (I) sont choisis parmi les suivants :

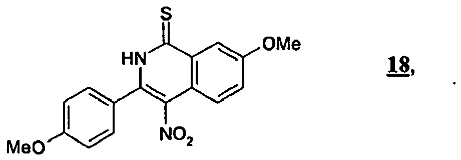

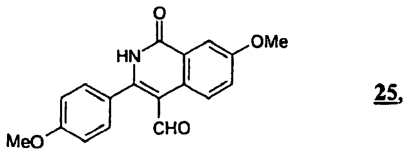

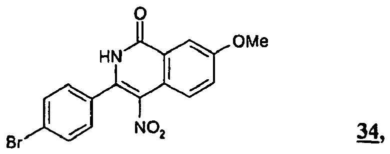

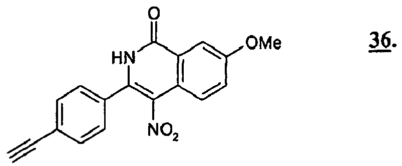

12. Composé destiné à être utilisé selon l'une des revendications 1 à 8, dans lequel les composés de formule (I) sont choisis parmi les suivants :

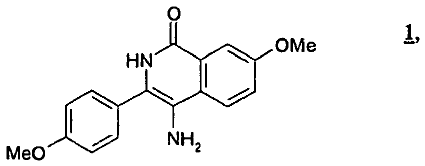

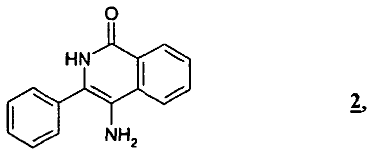

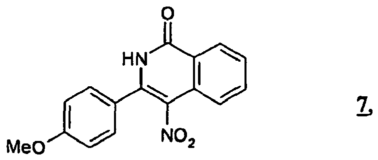

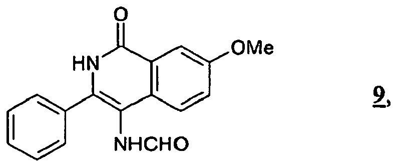

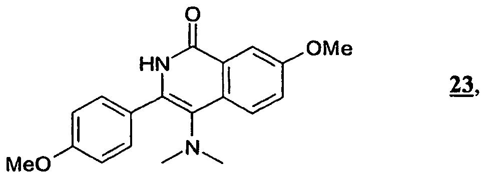

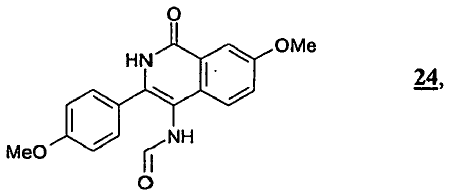

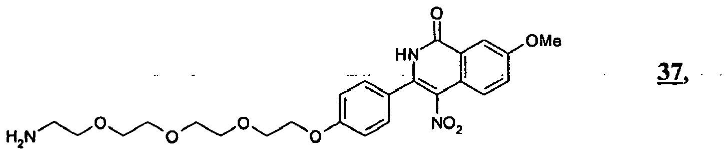

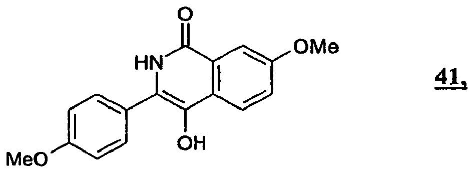

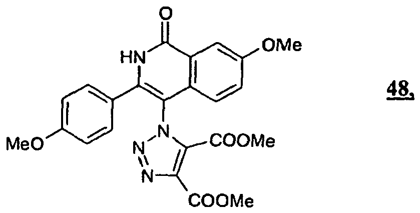

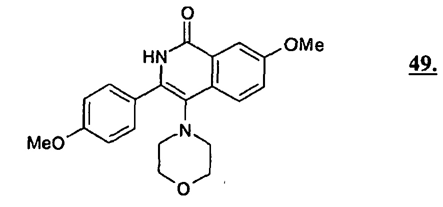

13. Composé destiné à être utilisé selon l'une des revendications 1 à 8, dans lequel les composés de formule (I) sont choisis parmi les suivants :

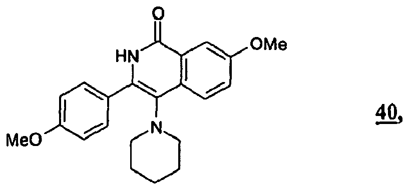

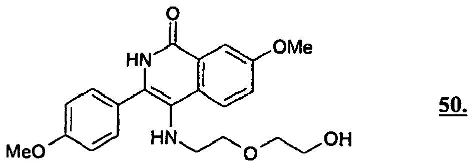

14. Composé destiné à être utilisé selon l'une des revendications 1 à 13 administré par voie orale, à une dose comprise d'environ 1 mg/kg à environ 200 mg/kg, préférentiellement d'environ 10 mg/kg à environ 100 mg/kg, plus préférentiellement de 20 mg/kg à 50 mg/kg, en particulier 40 mg/kg.

15. Composé destiné à être utilisé selon l'une des revendications 1 à 13 administré par voie parentérale, intraveineuse; à une dose comprise d'environ 0,1mg/kg/j à environ 100mg/kg/j, notamment 0,1 mg/kg à 50 mg/kg/j, en particulier 10 mg/kg/j

16. Composé destiné à être utilisé selon l'une des revendications 1 à 15, en association avec un antitumoral.

17. Composé destiné à être utilisé selon la revendication 16 dans lequel l'antitumoral est choisi parmi les suivants : l'abraxane, l'abarelix, l'aldesleukinee, l'alemtuzumab, l'alitretinoine, l'allopurinol, l'altrétamine, l'anastrozole, l'arsenic trioxide, l'asparaginase, l'azacitidine, le bevacizumab, le bexarotene, le bicalutamide, la bleomycine, le bortezomib, le busulfan intraveineux, le busulfan oral, la calustérone, la capécitabine, le carboplatin, la carmustine, le cetuximab, le chlorambucil, le cisplatine, la cladribine, la clofarabine, le cyclophosphamide, la cytarabine, la dacarbazine, la dactinomycine, la daltéparine sodium, le dasatinib, la daunorubicine, la décitabine, la dénileukine, la dénileukine diftitox, le dexrazoxane, le docetaxel, la doxorubicine, la dromostanolone propionate, l'eculizumab, l'epirubicine, l'erlotinib, l'estramustine, l'etoposide phosphate, l'etoposide, l'exemestane, le fentanyl citrate, le filgrastime, la floxuridine, la fludarabine, le 5-fluorouracyl, le fulvestrant, le gefitinib, la gemcitabine, le gemtuzumab ozogamicine, la gosereline acetate, l'histrelin acetate, l'ibritumomab tiuxetan, l'idarubicine, l'ifosfamide, l'imatinib mesylate, l'interferon alfa 2a, l'irinotecan, le lapatinib ditosylate, le lenalidomide, le letrozole, leucovorine, le leuprolide acetate, le levamisole, la lomustine, la meclorethamine, le megestrol acetate, le melphalan, la mercaptopurine, le methotrexate, le methoxsalen, la mitomycine C, le mitotane, mitoxantrone, la nandrolone phenpropionate, la nelarabine, le nofetumomab, l'oxaliplatine, le paclitaxel, le pamidronate, le panitumumab, la pegaspargase, le pegfilgrastim, le pemetrexed disodium, la pentostatine, le pipobroman, la plicamycine, le procarbazine, la quinacrine, la rasburicase, le rituximab, le sorafenib, la streptozocine, le sunitinib, le sunitinib maleate, le tamoxifène, le taxol, le taxotère, la temozolomide, le teniposide, la testolactone, la thalidomide, la thioguanine, le thiotepa, le topotecan, le toremifene, le tositumomab, le trastuzumab, le tretinoine, l'uracil moutarde, la valrubicine, la vinblastine, la vincristine, la vinorelbine, le vorinostat, et le zoledronate.

18. Composition pharmaceutique comprenant comme principe actif un composé de formule I, tel que défini dans la revendication 1, en association avec un véhicule pharmaceutiquement acceptable pour utilisation chez les mammifères dans la prévention et le traitement d'angiogénèses pathologiques choisies parmi les rétinopathies et les tumeurs bénignes ou malignes (cancéreuses).

19. Composition pharmaceutique selon la revendication 18, comprenant comme principe actif un composé de formule I, dans laquelle:
- n=0, la liaison entre le carbone 1 et le groupement X est simple et X est choisi parmi OCH3, ou
- n = 1, la liaison entre le carbone 1 et le groupement X est double, X = O ou S et R2 représente un phényle substitué en position 4 par un groupement choisi parmi OH, OCH₃, O-benzyle, Br, un groupe alcynyle en C2-C4 substitué ou non en particulier par un triméthylsilyle, un tert-butyle, un hydroxy-2-propyle ou un isopropyle.

20. Composition pharmaceutique selon la revendication 18, comprenant comme principe actif un composé, tel que défini dans la revendication 9, en association avec un véhicule pharmaceutiquement acceptable.

21. Composition pharmaceutique selon la revendication 18, comprenant comme principe actif un composé tel que défini dans la revendication 10.

22. Composition pharmaceutique selon la revendication 18, comprenant comme principe actif un composé tel que défini dans la revendication 11.

23. Composition pharmaceutique selon la revendication 18, comprenant comme principe actif un composé tel que défini dans la revendication 12.

24. Composition pharmaceutique selon la revendication 18, comprenant comme principe actif un composé tel que défini dans la revendication 13.

25. Composition pharmaceutique selon l'une des revendications 18 à 24, administrable par voie orale à une dose comprise d'environ 1 mg/kg à environ 200 mg/kg, préférentiellement d'environ 10 mg/kg à environ 100 mg/kg, plus préférentiellement de 20 mg/kg à 50 mg/kg, en particulier 40 mg/kg.

26. Composition pharmaceutique selon l'une des revendications 18 à 24, administrable par voie intraveineuse à une dose comprise d'environ 0,1 mg/kg/j à environ 100 mg/kg/j, notamment 50 mg/kg/j, en particulier 10 mg/kg/j.

27. Composé de formule générale (I) suivante : 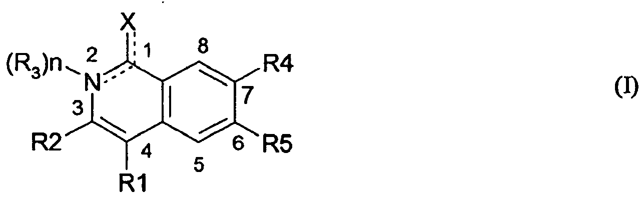 dans laquelle :
1) la liaison entre le carbone 1 et le groupement X est simple ou double, la liaison entre l'azote 2 et le carbone 1 pourra être simple ou double
étant entendu que :
a. lorsque la liaison entre X et ledit carbone 1 est double, alors la liaison entre l'azote 2 et le carbone 1 est simple :
et,
b. lorsque la liaison entre X et ledit carbone 1 est simple, alors la liaison entre l'azote 2 et le carbone 1 est double et la formule 1 correspond à un système aromatique de formule I-A suivante : 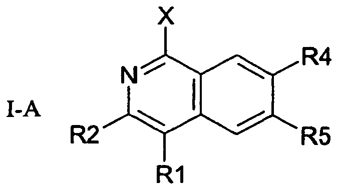
2) n représente 0 ou 1,
3) X représente:
a) lorsque n =1:
O, S,
b) lorsque n = 0 :
OPO₃H₂, OPO-(O-(C₁-C₂)alkyle)₂, l'alkyle étant éventuellement substitué par un ou plusieurs fluors, O-(C₁-C₆)alkyle, en particulier OCH₃, l'alkyle étant éventuellement substitué par un ou plusieurs fluors, NH-(C₁-C₆)alkyle, S(C₃-C₆)cycloalkyle,
4) R1 représente :
OPO₃H₂, OPO-(O-(C₁-C₂)alkyle)₂, l'alkyle étant éventuellement substitué par un ou plusieurs fluors,
CF₃, CH₂OR', R' représentant H ou (C₁-C₆)alkyle, l'alkyle étant éventuellement substitué par un ou plusieurs fluors, CHO,
CONR_{c}R_{d}, R_{c} et R_{d} représentant indépendamment l'un de l'autre : H, un alkyle en C₁-C₆, et NR_{c}R_{d} représente un acide aminé lié par sa fonction amine, en particulier une sérine ou une thréonine, un cycloalkyle en C₃-C₆, ou R_{c} et R_{d} représentent ensemble un alkyle en C₂-C₆,
NO₂, N₃, ≡, C(=N)NH₂, SO₃H, SO₂NH₂, SO₂NHCH₃, NHSO₂CH₃, CH₂SO₂NR_{c}R_{d}, CH₂NHSO₂Rc, SO₂-NRaRb, SO₂-imidazole, NRₐR_{b}, où :
Rₐ et R_{b} représentent indépendamment l'un de l'autre : H, CHO, un alkyle en C₁-C₆, un cycloalkyle en C₃-C₆, un acide aminé lié par sa fonction acide, en particulier une sérine ou
Ra est H, un alkyle en C₁-C₆, un cycloalkyle en C₃-C₆, et Rb = COR_{f}, COOR_{f} ou CONR_{f}R_{f},_{'} R_{f} et R_{f} représentant H, , un cycloalkyle en C₃-C₆ ou une chaîne d'un amino acide (telle que CH(CH₂OH)NH₂ pour la sérine), ou
Rₐ et R_{b} représentent ensemble un alkyle en C₂-C₆,
Rₐ et R_{b} peuvent former un cycle en C₅ à C₇, notamment une pyrrolidine,une pipérazine, une morpholine, une thiomorpholine,
un hétéroaryle substitué ou non, en particulier une pyridine, un imidazole, un oxazole, un triazole, un pyrrole, un tétrazole.
5) R2 représente :
un phényle substitué par un à trois substituants choisis parmi :
un halogène, un OH, NHRa,
ORe, Re représentant un benzyle, un triazole méthylène substitué ou non, notamment par un alkyle en C₁-C₆,
OPO₃H₂, OPO-(O-(C₁-C₂)alkyle)₂, l'alkyle étant éventuellement substitué par un ou plusieurs fluors, un NH₂,
un groupe NH-CORc dans lequel Rc représente H, un alkyle en C₁-C₆, un cycloalkyle en C₃-C₆ ou une chaîne d'un amino acide (telle que CH(CH₂OH)NH₂ pour la sérine),
un O-alkyle en C₁-C₆, en particulier OCH₃, l'alkyle étant éventuellement substitué par un ou plusieurs fluors,
un groupe NH-Rc dans lequel Rc représente un acide aminé lié par sa fonction acide, en particulier une sérine,
un O-COR1 où R1 représente O-alkyle en C₁-C₆, ou NRiRii, Ri et Rii pouvant être alkyle en C₁-C₆,
un groupe alkyle en C₂-C₄, le groupe alkyle étant éventuellement substitué par un ou plusieurs fluors, un groupement alcényle en C₂-C₄ substitué ou non,
un groupe alcynyle en C₂-C₄ substitué ou non, en particulier par un triméthylsilyle, un tert-butyle, un hydroxy-2-propyle ou un isopropyle
un hétéroaryle substitué ou non, en particulier une pyridine, un imidazole, un oxazole, un triazole, un pyrrole, un benzofurane, un thiophène, un benzothiophène, un indole,
un cyclohéxyle, une pipérazine, une morpholine, une thiomorpholine, une pipéridine,
un groupe 4-(NH₂-(CH₂-CH₂O)p)Ph dans lequel p est un entier de 1 à 6
6) R3 représente :
H, (C₁-C₆)alkyle, l'alkyle étant éventuellement substitué par un ou plusieurs fluors, (C₃-C₆)cycloalkyle, O-alkyle en C₁ à C₆, NH₂, un groupe propargyle, CH₂CN,
7) R4 et R5 représentent indépendamment l'un de l'autre :
H, OH, OPO₃H₂, OPO-(O-(C₁-C₂)alkyle)₂, l'alkyle étant éventuellement substitué par un ou plusieurs fluors, un O-alkyle en C₁-C₆, en particulier OCH₃, l'alkyle étant éventuellement substitué par un ou plusieurs fluors, un alkyle en C₁-C₆, l'alkyle étant éventuellement substitué par un ou plusieurs fluors, un cycloalkyle en C₃-C₆, un halogène,
R4 et R5 représentent ensemble un alcényle (en C₁-C₂) dioxy éventuellement substitué par un ou plusieurs fluors,
à l'exclusion des composés suivants :
lorsque X=O, R3 = H, et que la liaison entre le carbone 1 et le groupement X est double, :
a. R1 = NH₂, R2 = 4-méthoxy-phényle, R4 = OCH₃ et R5 = H,
b. R1 = NH₂, R2 = phényle, R4 = H et R5 = H,
c. R1 = NO₂, R2 = 4-méthoxy-phényle, R4 = OCH₃ et R5 = H,
d. R1 = NO₂, R2 = 4-méthoxy-phényle, R4 = H et R5 = H,
e. R1 = NH₂, R2 = 4-méthoxy-phényle, R4 = H et R5 = H,
f. R1 = NHCHO, R2 = phényle, R4 = OCH₃ et R5 = H,
g. R1 = NH₂, R2 = phényle, R4 = OCH₃ et R5 = H,
h. R1 = NH₂, R2 = phényle ou 4-OH-phényle substitué ou non, R4 = H, OH, un O-alkyle en C₁-C₆, un alkyle en C₁-C₆, un cycloalkyle en C₃-C₆, et R5 = H, OH, un O-alkyle en C₁-C₆, un alkyle en C₁-C₆, un cycloalkyle en C₃-C₆,
i. les composés dans lesquels l'un des R4 et R5 représentent un halogène.

28. Composé selon la revendication 27, de formule générale (I): 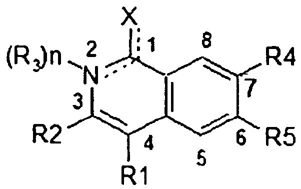 dans laquelle
- n=0, la liaison entre le carbone 1 et le groupement X est simple et X est OCH₃, et R1 est NO₂, ou
- n = 1, la liaison entre le carbone 1 et le groupement X est double, X = O ou S et R2 représente un phényle substitué en position 4 par un groupement choisi parmi OH, OCH₃, O-benzyle, Br, un groupe alcynyle en C2-C4 substitué ou non, en particulier par un triméthylsilyle, un tert-butyle, un hydroxy-2-propyle ou un isopropyle.

29. Composé selon la revendication 27, choisi parmi les suivants:

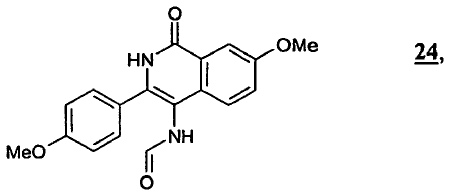

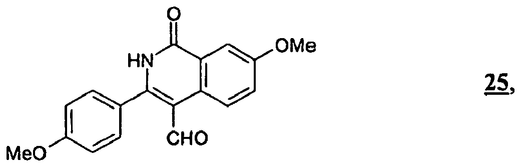

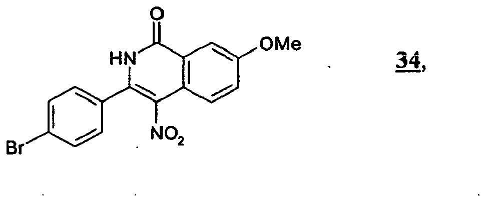

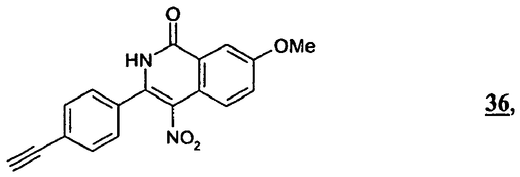

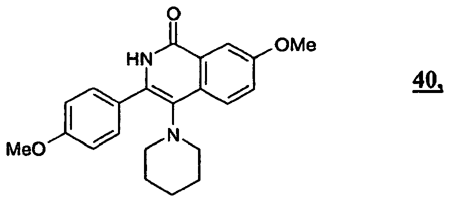

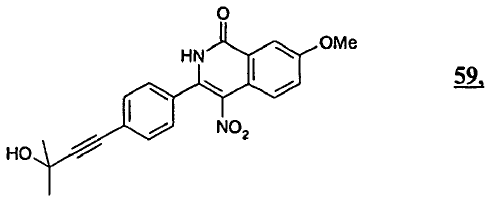

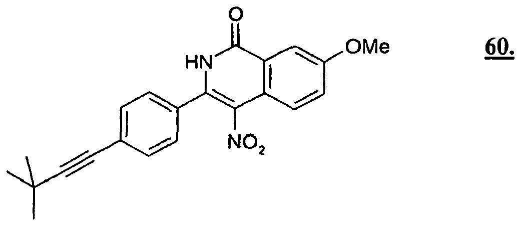

30. Produit comprenant une première préparation pharmaceutique de formule (I) selon la revendication 1, et une seconde préparation pharmaceutique comprenant au moins un antitumoral en tant que préparation combinée pour une utilisation simultanée, séparée ou séquentielle dans le traitement de mammifères, en particulier l'homme, atteints de tumeurs bégnines ou malignes (cancéreuses).

31. Produit selon la revendication 30 dans lequel au moins un antitumoral est choisi parmi l'abraxane, l'abarelix, l'aldesleukine, l'alemtuzumab, l'alitretinoine, l'allopurinol, l'altrétamine, l'anastrozole, l'arsenic trioxide, l'asparaginase, l'azacitidine, le bevacizumab, le bexarotene, le bicalutamide, la bleomycine, le bortezomib, le busulfan intraveineux, le busulfan oral, la calustérone, la capécitabine, le carboplatin, la carmustine, le cetuximab, le chlorambucil, le cisplatine, la cladribine, la clofarabine, le cyclophosphamide, la cytarabine, la dacarbazine, la dactinomycine, la daltéparine sodium, le dasatinib, la daunorubicine, la décitabine, la dénileukine, la dénileukine diftitox, le dexrazoxane, le docetaxel, la doxorubicine, la dromostanolone propionate, l'eculizumab, l'epirubicine, l'erlotinib, l'estramustine, l'etoposide phosphate, l'etoposide, l'exemestane, le fentanyl citrate, le filgrastime, la floxuridine, la fludarabine, le 5-fluorouracyl, le fulvestrant, le gefitinib, la gemcitabine, le gemtuzumab ozogamicine, la gosereline acetate, l'histrelin acetate, l'ibritumomab tiuxetan, l'idarubicine, l'ifosfamide, l'imatinib mesylate, l'interferon alfa 2a, l'irinotecan, le lapatinib ditosylate, le lenalidomide, le letrozole, leucovorine, le leuprolide acetate, le levamisole, la lomustine, la meclorethamine, le megestrol acetate, le melphalan, la mercaptopurine, le methotrexate, le methoxsalen, la mitomycine C, le mitotane, mitoxantrone, la nandrolone phenpropionate, la nelarabine, le nofetumomab, l'oxaliplatine, le paclitaxel, le pamidronate, le panitumumab, la pegaspargase, le pegfilgrastim, le pemetrexed disodium, la pentostatine, le pipobroman, la plicamycine, le procarbazine, la quinacrine, la rasburicase, le rituximab, le sorafenib, la streptozocine, le sunitinib, le sunitinib maleate, le tamoxifène, le taxol, le taxotère, la temozolomide, le teniposide, la testolactone, la thalidomide, la thioguanine, le thiotepa, le topotecan, le toremifene, le tositumomab, le trastuzumab, le tretinoine, l'uracil moutarde, la valrubicine, la vinblastine, la vincristine, la vinorelbine, le vorinostat, et le zoledronate.

## Patentansprüche

1. Verbindung der folgenden allgemeinen Formel (I) : 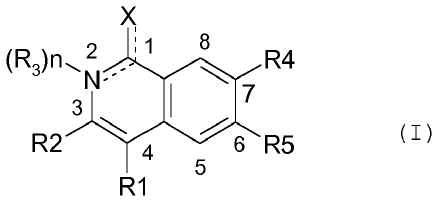 wobei:
1) die Bindung zwischen dem Kohlenstoff 1 und der Gruppe X einfach oder doppelt ist, die Bindung zwischen dem Stickstoff 2 und dem Kohlenstoff 1 einfach oder doppelt sein kann,
wobei es sich versteht, dass:
a) wenn die Bindung zwischen X und dem Kohlenstoff 1 doppelt ist, die Bindung zwischen dem Stickstoff 2 und dem Kohlenstoff 1 dann einfach ist:
und,
b) wenn die Bindung zwischen X und dem Kohlenstoff 1 einfach ist, die Bindung zwischen dem Stickstoff 2 und dem Kohlenstoff 1 dann doppelt ist und die Formel I einem aromatischen System der folgenden Formel I-A entspricht: 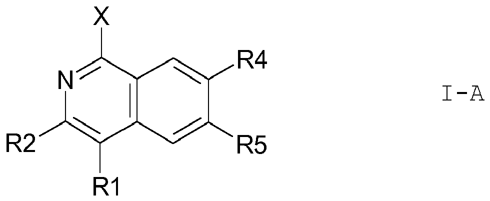
2) n 0 oder 1 darstellt,
3) X darstellt:
a) wenn n =1:
0, S,
b) wenn n = 0:
OPO₃H₂, OPO-(O-(C₁-C₂)alkyl)₂, wobei das Alkyl eventuell durch ein oder mehrere Fluore substituiert ist, O-(C₁-C₆) alkyl, insbesondere OCH₃, wobei das Alkyl eventuell durch ein oder mehrere Fluore substituiert ist, NH-(C₁-C₆)alkyl, S(C₃-C₆)cycloalkyl,
4) R1 darstellt:
OH, OPO₃H₂, OPO-(O-(C₁-C₂)alkyl)₂, wobei das Alkyl eventuell durch ein oder mehrere Fluore substituiert ist,
CF₃, CH₂OR', wobei R' H oder (C₁-C₆)alkyl darstellt, wobei das Alkyl eventuell durch ein oder mehrere Fluore substituiert ist, CHO,
CONR_{c}R_{d}, wobei R_{c} und R_{d} unabhängig voneinander darstellen: H, ein C₁-C₆-Alkyl, und NR_{c}R_{d} eine durch ihre Aminfunktion gebundene Aminosäure darstellt, insbesondere ein Serin oder ein Threonin, ein C₃-C₆Cycloalkyl, oder R_{c} und R_{d} gemeinsam ein C₂-C₆-Alkyl darstellen,
NO₂, N₃, ≡, CN, C (=N) NH₂, SO₃H, SO₂NH₂, SO₂NHCH₃, NHSO₂CH₃, CH₂SO₂NR_{c}R_{d}, CH₂NHSO₂R_{c}, SO₂-NRaRb, SO₂-imidazol, NRₐR_{b}, wobei:
Rₐ und R_{b} unabhängig voneinander darstellen:
H, ein C₁-C₆-Alkyl, ein C₃-C₆-Cycloalkyl, oder
Ra H, ein C₁-C₆-Alkyl, ein C₃-C₆-Cycloalkyl ist, und Rb = COR_{f}, COOR_{f} oder CONR_{f}R_{f}, R_{f} und R_{f}, H, ein C₁-C₆-Alkyl, ein C₃-C₆-Cycloalkyl oder eine Kette einer Aminosäure (wie CH(CH₂OH)NH₂ für Serin) darstellt Rₐ und R_{b} gemeinsam ein C₂-C₆-Alkyl darstellen,
Rₐ und R_{b} einen C₅- bis C₇-Zyklus formen können, vor allem ein Pyrrolidin, ein Piperazin, ein Morpholin, ein Thiomorpholin,
ein Heteroaryl, substituiert oder nicht, insbesondere ein Pyridin, ein Iimidazol, ein Oxazol, ein Triazol, ein Pyrrol, ein Tetrazol.
5) R2 darstellt:
ein Phenyl, substituiert oder nicht, von einem bis drei Substituenten, ausgewählt aus:
einem Halogen, einem OH, NHRa,
ORe, wobei Re ein Benzyl, ein Methylentriazol, substituiert oder nicht, vor allem durch ein C₁-C₆-Alkyl, darstellt,
OPO₃H₂, OPO- (O-(C₁-C₂) alkyl)₂, wobei das Alkyl eventuell durch ein oder mehrere Fluore, substituiert ist, ein NH₂,
eine Gruppe NH-CORc, wobei Rc H, ein C₁-C₆-Alkyl, ein C₃-C₆-Cycloalkyl oder eine Kette einer Aminosäure (wie CH(CH₂OH)NH₂ für Serin) darstellt,
ein C₁-C₆-O-Alkyl, insbesondere OCH₃, wobei das Alkyl eventuell durch ein oder mehrere Fluore substituiert ist,
ein O-COR₁, wobei R₁ C₁-C₆-O-Alkyl darstellt, wobei NRiRii, Ri und Rii C₁-C₆-Alkyl sein können,
eine C₂-C₄-Alkylgruppe, wobei die Alkylgruppe eventuell durch ein oder mehrere Fluore substituiert ist, eine C₂-C₄-Alcenylgruppe, substituiert oder nicht,
eine C₂-C₄-Alcynylgruppe, substituiert oder nicht, insbesondere durch ein Trimethylsilyl, ein tert-Butyl, ein Hydroxy-2-propyl oder ein Isopropyl,
ein Heteroaryl, substituiert oder nicht, insbesondere ein Pyridin, ein Imidazol, ein Oxazol, ein Triazol, ein Pyrrol, ein Benzofuran, ein Thiophen, ein Benzothiophen, ein Indol,
ein Cyclohexyl, ein Piperazin, ein Morpholin, ein Thiomorpholin, ein Piperidin,
eine Gruppe 4- (NH₂-(CH₂-CH₂O)ₚ) Ph, wobei p eine Ganzzahl von 1 bis 6 ist,
6) R3 darstellt:
H, (C₁-C₆) alkyl, wobei das Alkyl eventuell durch ein oder mehrere Fluore substituiert ist, (C₃-C₆)cycloalkyl, C₁ bis C₆-O-Alkyl, NH₂, eine Propargylgruppe, CH₂CN,
7) R4 und R5 unabhängig voneinander darstellen:
H, OH, OPO₃H₂, OPO- (O-(C₁-C₂) alkyl)₂, wobei das Alkyl eventuell durch ein oder mehrere Fluore substituiert ist, ein C₁-C₆-O-Alkyl, insbesondere OCH₃, wobei das Alkyl eventuell durch ein oder mehrere Fluore substituiert ist, ein C₁-C₆-Alkyl, wobei das Alkyl eventuell durch ein oder mehrere Fluore substituiert ist, ein C₃-C₆-Cycloalkyl, ein Halogen,
wobei R4 und R5 gemeinsam ein (C₁-C₂) dioxyalcenyl, darstellen, eventuell substituiert² durch ein oder mehrere Fluore,
und die pharmazeutisch akzeptablen Salze,
für die Verwendung bei den Säugetieren als Mittel zur Gefäßzerstörung bei der Prävention und der Behandlung pathologischer Angiogenesen, ausgewählt aus den Retinopathien und den benignen oder malignen (kanzerogenen) Tumoren.

2. Verbindung nach Anspruch 1 für ihre Verwendung bei den Säugetieren als Inhibitor von Proteinphosphatase 1, Mittel zur Gefäßzerstörung und antiproliferatives Mittel bei der Prävention und der Behandlung pathologischer Angiogenesen, ausgewählt aus den Retinopathien und den benignen oder malignen (kanzerogenen) Tumoren.

3. Verbindung nach Anspruch 1 für ihre Verwendung bei den Säugetieren als Inhibitor von Tubulinpolymerisation, Mittel zur Gefäßzerstörung und antiproliferatives Mittel bei der Prävention und der Behandlung pathologischer Angiogenesen, ausgewählt aus den Retinopathien und den benignen oder malignen (kanzerogenen) Tumoren.

4. Verbindung nach Anspruch 1 für ihre Verwendung bei den Säugetieren als Inhibitor von Proteinphosphatase 1, von Tubulinpolymerisation, Mittel zur Gefäßzerstörung und antiproliferatives Mittel bei der Prävention und der Behandlung pathologischer Angiogenesen, ausgewählt aus den Retinopathien und den benignen oder malignen (kanzerogenen) Tumoren.

5. Verbindung, bestimmt zur Verwendung nach einem der Ansprüche 2 bis 4, wobei die Gruppe R1 der allgemeinen Formel (I) aus NO₂ oder Pyrrol ausgewählt ist.

6. Verbindung, bestimmt zur Verwendung nach einem der Ansprüche 2 bis 4, wobei die Gruppe R2 der allgemeinen Formel (I) ein Phenyl darstellt, substituiert an Position para durch eine Gruppe, die aus ORe, wobei Re nach Anspruch 1 ist, einem Halogen, einer C₂-C₄-Alcyngruppe, substituiert oder nicht, insbesondere durch ein Trimethylsilyl, ein tert-Butyl, ein Hydroxy-2-propyl oder ein Isopropyl, ausgewählt ist.

7. Verbindung, bestimmt zur Verwendung nach einem der Ansprüche 1 bis 4, wobei die Gruppe R1 der allgemeinen Formel (I) aus NO2 oder Pyrrol ausgewählt ist und die Gruppe R2 der allgemeinen Formel (I) ein Phenyl darstellt, substituiert an Position para durch eine Gruppe, die aus ORe, wobei Re nach Anspruch 1 ist, einem Halogen, einer C₂-C₄-Alcyngruppe, substituiert oder nicht, insbesondere durch ein Trimethylsilyl, ein tert-Butyl, ein Hydroxy-2-propyl oder ein Isopropyl, ausgewählt ist.

8. Verbindung, bestimmt zur Verwendung nach Anspruch 7, wobei die Gruppe R3 der allgemeinen Formel (I) H darstellt.

9. Verbindung, bestimmt zur Verwendung nach Anspruch 1, wobei die Verbindungen der Formel (I) aus den folgenden Verbindungen ausgewählt sind:

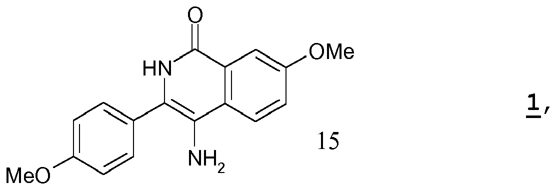

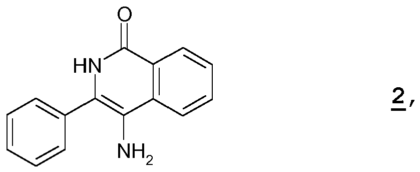

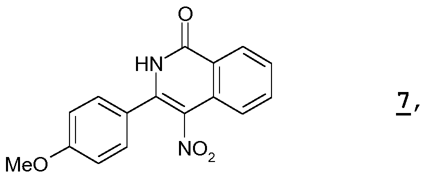

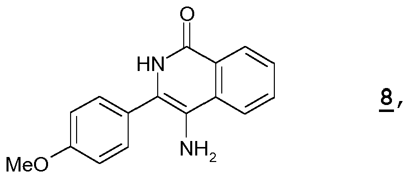

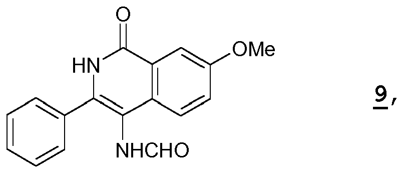

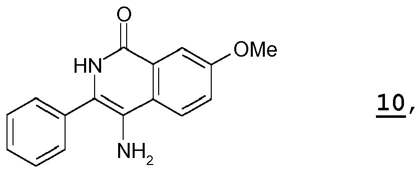

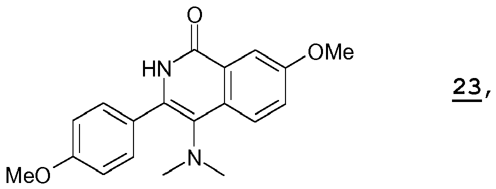

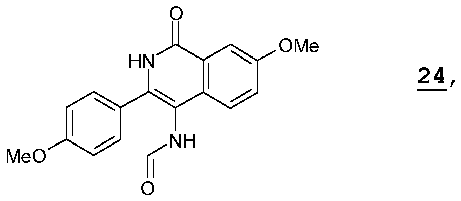

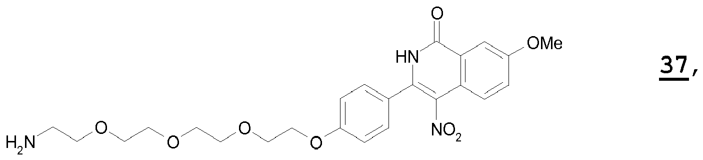

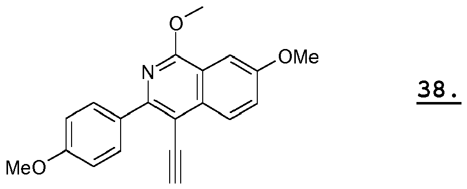

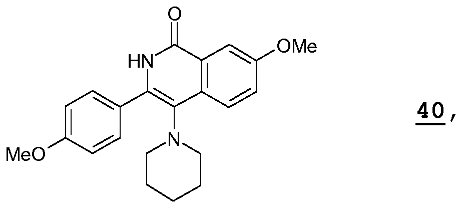

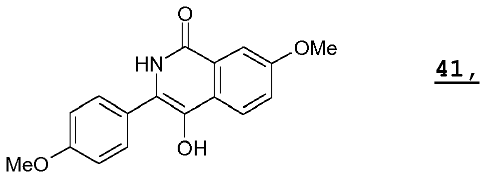

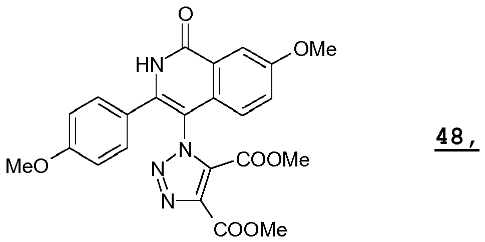

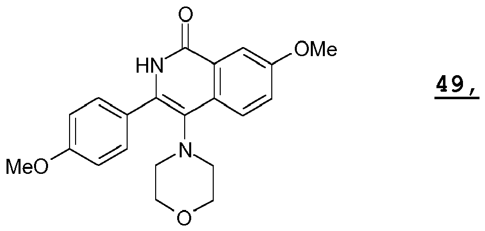

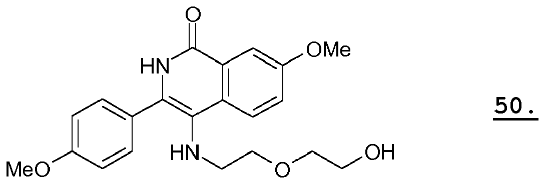

10. Verbindung, bestimmt zur Verwendung nach einem der Ansprüche 1 bis 8, wobei die Verbindungen der Formel (I) aus den folgenden Verbindungen ausgewählt sind:

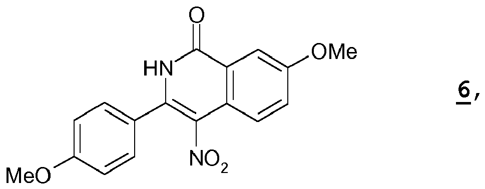

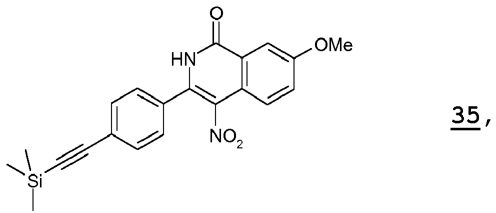

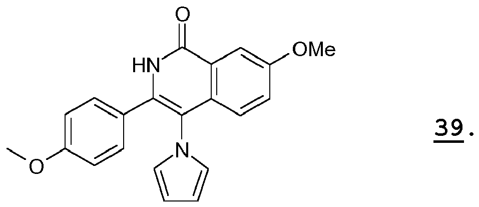

11. Verbindung, bestimmt zur Verwendung nach einem der Ansprüche 1 bis 8, wobei die Verbindungen der Formel (I) aus den folgenden Verbindungen ausgewählt sind:

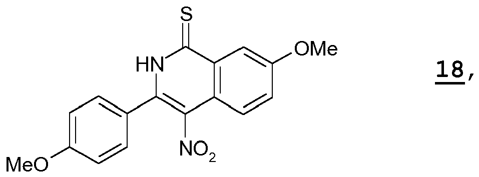

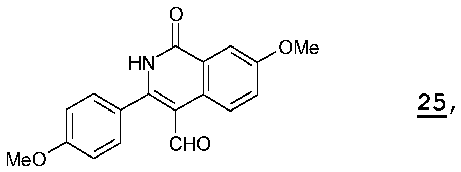

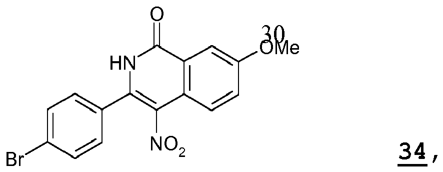

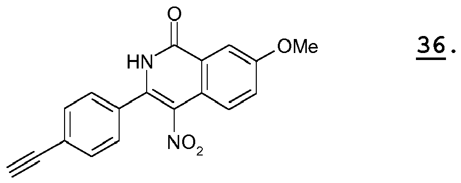

12. Verbindung, bestimmt zur Verwendung nach einem der Ansprüche 1 bis 8, wobei die Verbindungen der Formel (I) aus den folgenden Verbindungen ausgewählt sind:

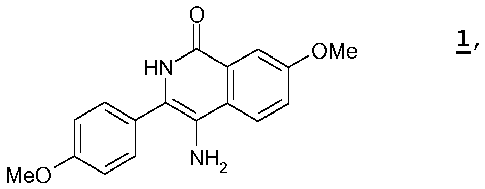

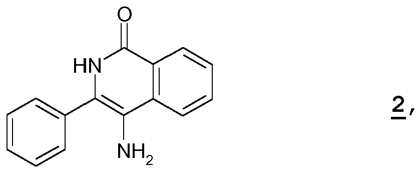

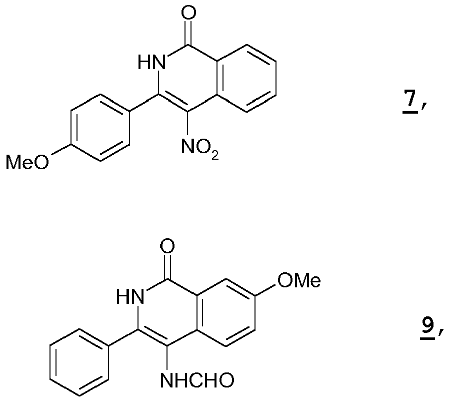

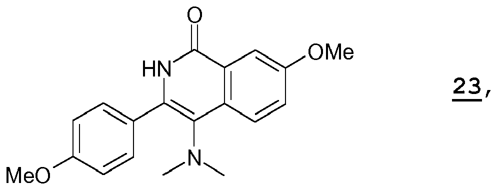

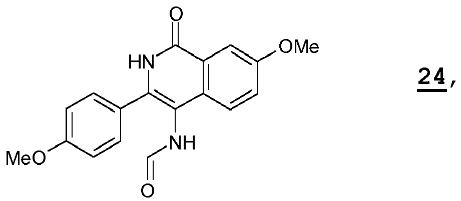

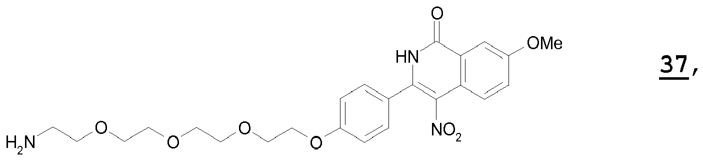

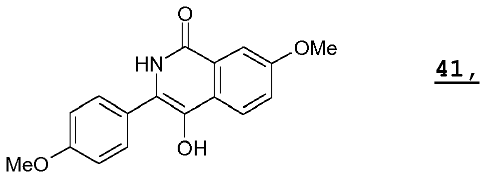

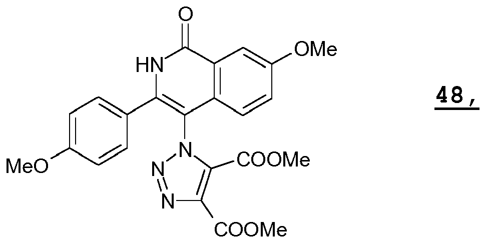

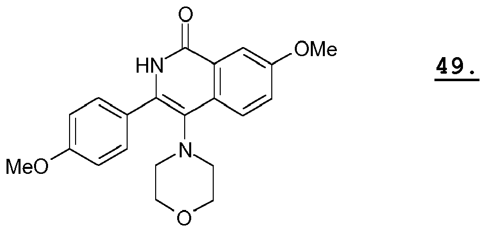

13. Verbindung, bestimmt zur Verwendung nach einem der Ansprüche 1 bis 8, wobei die Verbindungen der Formel (I) aus den folgenden Verbindungen ausgewählt sind:

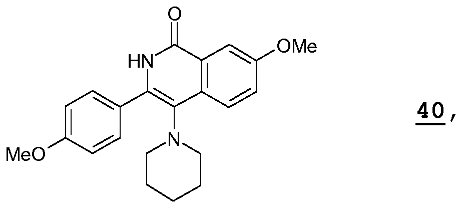

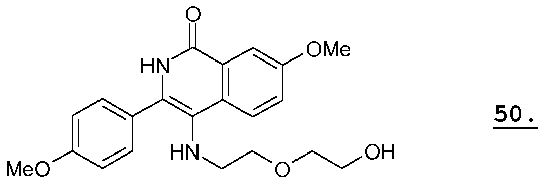

14. Verbindung, bestimmt zur Verwendung nach einem der Ansprüche 1 bis 13, die auf oralem Weg in einer Dosierung von zirka 1 mg/kg bis zirka 200 mg/kg inklusive, vorzugsweise von zirka 10 mg/kg bis zirka 100 mg/kg, in bevorzugterer Weise von 20 mg/kg bis 50 mg/kg, insbesondere von 40 mg/kg, verabreicht wird.

15. Verbindung, bestimmt zur Verwendung nach einem der Ansprüche 1 bis 13, die auf parenteralem, intravenösem Weg in einer Dosierung von zirka 0,1mg/kg/j bis zirka 100mg/kg/j inklusive, vor allem von 0,1 mg/kg bis 50 mg/kg/j, insbesondere von 10 mg/kg/j, verabreicht wird.

16. Verbindung, bestimmt zur Verwendung nach einem der Ansprüche 1 bis 15 in Kombination mit einem Antitumormittel.

17. Verbindung bestimmt zur Verwendung nach Anspruch 16, wobei das Antitumormittel aus den folgenden Mitteln ausgewählt ist: dem Abraxan, dem Abarelix, dem Aldesleukine, dem Alemtuzumab, dem Alitretinoin, dem Allopurinol, dem Altretamin, dem Anastrozol, dem Arsentrioxid, der Asparaginase, dem Azacitidin, dem Bevacizumab, dem Bexaroten, dem Bicalutamid, dem Bleomycin, dem Bortezomib, dem intravenösen Busulfan, dem oralen Busulfan, dem Calusteron, dem Capecitabin, dem Carboplatin, dem Carmustin, dem Cetuximab, dem Chlorambucil, dem Cisplatin, dem Cladribin, dem Clofarabin, dem Cyclophosphamid, dem Cytarabin, dem Dacarbazin, dem Dactinomycin, dem Natriumdalteparin, dem Dasatinib, dem Daunorubicin, dem Decitabin, dem Denileukin, dem Diftitoxdenileukin, dem Dexrazoxan, dem Docetaxel, dem Doxorubicin, dem Dromostanolon-Propionat, dem Eculizumab, dem Epirubicin, dem Erlotinib, dem Estramustin, dem Etoposidphosphat, dem Etoposid, dem Exemestan, dem Fentanylcitrat, dem Filgrastim, dem Floxuridin, dem Fludarabin, dem 5-Fluoruracyl, dem Fulvestrant, dem Gefitinib, dem Gemcitabin, dem Gemtuzumab-Ozogamicin, dem Goserelinacetat, dem Histrelinacetat, dem Ibritumomab-Tiuxetan, dem Idarubicin, dem Ifosfamid, dem Imatinibmesylat, dem Alpha 2a-Interferon, dem Irinotecan, dem Lapatinibditosylat, dem Lenalidomid, dem Letrozol, Leucovorin, dem Leuprolidacetat, dem Levamisol, dem Lomustin, dem Meclorethamin, dem Megestrolacetat, dem Melphalan, dem Mercaptopurin, dem Methotrexat, dem Methoxsalen, dem Mitomycin C, dem Mitotan, Mitoxantron, dem Nandrolon-Phenpropionat, dem Nelarabin, dem Nofetumomab, dem Oxaliplatin, dem Paclitaxel, dem Pamidronat, dem Panitumumab, der Pegaspargase, dem Pegfilgrastim, dem Dinatriumpemetrexed, dem Pentostatin, dem Pipobroman, dem Plicamycin, dem Procarbazin, dem Quinacrin, der Rasburicase, dem Rituximab, dem Sorafenib, dem Streptozocin, dem Sunitinib, dem Sunitinibmaleat, dem Tamoxifen, dem Taxol, dem Taxoter, dem Temozolomid, dem Teniposid, dem Testolacton, dem Thalidomid, dem Thioguanin, dem Thiotepa, dem Topotecan, dem Toremifen, dem Tositumomab, dem Trastuzumab, dem Tretinoin, dem Uracilsenf, dem Valrubicin, dem Vinblastin, dem Vincristin, dem Vinorelbin, dem Vorinostat und dem Zoledronat.

18. Pharmazeutische Zusammensetzung, die als Wirkstoff eine Verbindung der Formel I nach Anspruch 1 in Kombination mit einem pharmazeutisch akzeptablen Vehikel für die Verwendung bei den Säugetieren bei der Prävention und der Behandlung pathologischer Angiogenesen, ausgewählt aus den Retinopathien und den benignen oder malignen (kanzerogenen) Tumoren, umfasst.

19. Pharmazeutische Zusammensetzung nach Anspruch 18, die als Wirkstoff eine Verbindung der Formel I umfasst, wobei:
- n = 0, die Bindung zwischen dem Kohlenstoff 1 und der Gruppe X einfach ist und X aus OCH3 ausgewählt ist, oder
- n = 1, die Bindung zwischen dem Kohlenstoff 1 und der Gruppe X doppelt ist, X = 0 oder S und R2 ein Phenyl darstellt, substituiert an Position 4 durch eine Gruppe, die aus OH, OCH₃, O-benzyl, Br, einer C₂-C₄-Alcynylgruppe, substituiert oder nicht, insbesondere durch ein Trimethylsilyl, ein tert-Butyl, ein Hydroxy-2-propyl oder ein Isopropyl ausgewählt ist.

20. Pharmazeutische Zusammensetzung nach Anspruch 18, die als Wirkstoff eine Verbindung nach Anspruch 9 in Kombination mit einem pharmazeutisch akzeptablen Vehikel umfasst.

21. Pharmazeutische Zusammensetzung nach Anspruch 18, die als Wirkstoff eine Verbindung nach Anspruch 10 umfasst.

22. Pharmazeutische Zusammensetzung nach Anspruch 18, die als Wirkstoff eine Verbindung nach Anspruch 11 umfasst.

23. Pharmazeutische Zusammensetzung nach Anspruch 18, die als Wirkstoff eine Verbindung nach Anspruch 12 umfasst.

24. Pharmazeutische Zusammensetzung nach Anspruch 18, die als Wirkstoff eine Verbindung nach Anspruch 13 umfasst.

25. Pharmazeutische Zusammensetzung nach einem der Ansprüche 18 bis 24, die auf oralem Weg in einer Dosierung von zirka 1 mg/kg bis zirka 200 mg/kg inklusive, vorzugsweise von zirka 10 mg/kg bis zirka 100 mg/kg, in bevorzugterer Weise von 20 mg/kg bis 50 mg/kg, insbesondere von 40 mg/kg, verabreichbar ist.

26. Pharmazeutische Zusammensetzung nach einem der Ansprüche 18 bis 24, die auf intravenösem Weg in einer Dosierung von zirka 0,1 mg/kg/j bis zirka 100 mg/kg/j inklusive, vor allem von 50 mg/kg/j, insbesondere von 10 mg/kg/j, verabreichbar ist.

27. Verbindung der folgenden allgemeinen Formel (I) :
wobei: 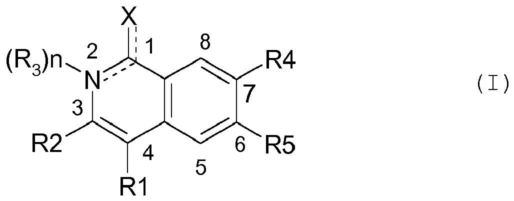
1) die Bindung zwischen dem Kohlenstoff 1 und der Gruppe X einfach oder doppelt ist, die Bindung zwischen dem Stickstoff 2 und dem Kohlenstoff 1 einfach oder doppelt sein kann,
wobei es sich versteht, dass:
a. wenn die Bindung zwischen X und dem Kohlenstoff 1 doppelt ist, die Bindung zwischen dem Stickstoff 2 und dem Kohlenstoff 1 dann einfach ist:
und,
b. wenn die Bindung zwischen X und dem Kohlenstoff 1 einfach ist, die Bindung zwischen dem Stickstoff 2 und dem Kohlenstoff 1 dann doppelt ist und die Formel I einem aromatischen System der folgenden Formel I-A entspricht: 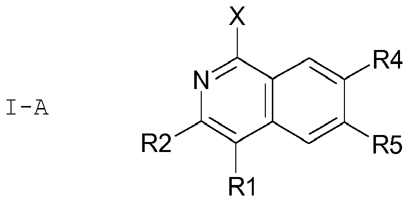
2) n 0 oder 1 darstellt,
3) X darstellt:
a) wenn n =1:
0, S,
b) wenn n = 0:
OPO₃H₂, OPO- (O-(C₁-C₂) alkyl)₂, wobei das Alkyl eventuell durch ein oder mehrere Fluore substituiert ist, O-(C₁-C₆) alkyl, insbesondere OCH₃, wobei das Alkyl eventuell durch ein oder mehrere Fluore substituiert ist, NH-(C₁-C₆) alkyl, S(C₃-C₆)cycloalkyl,
4) R1 darstellt:
OPO₃H₂, OPO- (O-(C₁-C₂) alkyl)₂, wobei das Alkyl eventuell durch ein oder mehrere Fluore substituiert ist,
CF₃, CH₂OR', wobei R' H oder (C₁-C₆) alkyl darstellt, wobei das Alkyl eventuell durch ein oder mehrere Fluore substituiert ist, CHO,
CONR_{c}R_{d}, wobei R_{c} und R_{d} unabhängig voneinander darstellen: H, ein C₁-C₆-Alkyl, und NR_{c}R_{d} eine durch ihre Aminfunktion gebundene Aminosäure darstellt, insbesondere ein Serin oder ein Threonin, ein C₃-C₆Cycloalkyl, oder R_{c} und R_{d} gemeinsam ein C₂-C₆-Alkyl darstellen,
NO₂, N₃, ≡, C (=N)NH₂, SO₃H, SO₂NH₂, SO₂NHCH₃, NHSO₂CH₃, CH₂SO₂NR_{c}R_{d}, CH₂NHSO₂Rc, SO₂-NRaRb, SO₂-imidazol,
NRₐR_{b}, wobei:
Rₐ und R_{b} unabhängig voneinander darstellen: H, CHO, ein C₁-C₆-Alkyl, ein C₃-C₆-Cycloalkyl, eine durch ihre Säurefunktion gebundene Aminosäure, insbesondere ein Serin, oder
Ra H, ein C₁-C₆-Alkyl, ein C₃-C₆-Cycloalkyl ist, und Rb = COR_{f}, COOR_{f} oder CONR_{f}R_{f'}, wobei R_{f} und R_{f}, H, ein C₃-C₆-Cycloalkyl oder eine Kette einer Aminosäure (wie CH(CH₂OH)NH₂ für Serin) darstellen, oder
Rₐ und R_{b} gemeinsam ein C₂-C₆-Alkyl darstellen,
Rₐ und R_{b} einen C₅- bis C₇-Zyklus formen können, vor allem ein Pyrrolidin, ein Piperazin, ein Morpholin, ein Thiomorpholin,
ein Heteroaryl, substituiert oder nicht, insbesondere ein Pyridin, ein Iimidazol, ein Oxazol, ein Triazol, ein Pyrrol, ein Tetrazol.
5) R2 darstellt:
ein Phenyl, substituiert, von einem bis drei Substituenten, ausgewählt aus:
einem Halogen, einem OH, NHRa,
ORe, wobei Re ein Benzyl, ein Methyltriazol, substituiert oder nicht, vor allem durch ein C₁-C₆-Alkyl, darstellt,
OPO₃H₂, OPO-(O-(C₁-C₂) alkyl)₂, wobei das Alkyl eventuell durch ein oder mehrere Fluore substituiert ist, ein NH₂,
eine Gruppe NH-CORc, wobei Rc H, ein C₁-C₆-Alkyl, ein C₃-C₆-Cycloalkyl oder eine Kette einer Aminosäure (wie CH(CH₂OH)NH₂ für Serin) darstellt,
ein C₁-C₆O-Alkyl, insbesondere OCH₃, wobei das Alkyl eventuell durch ein oder mehrere Fluore substituiert ist,
eine Gruppe NH-Rc, wobei Rc eine Aminosäure darstellt, die durch ihre Säurefunktion gebunden ist, insbesondere ein Serin,
ein O-COR1, wobei R1 C₁-C₆-O-Alkyl darstellt, wobei NRiRii, Ri und Rii C₁-C₆-Alkyl sein können,
eine C₂-C₄-Alkylgruppe, wobei die Alkylgruppe eventuell durch ein oder mehrere Fluore substituiert ist, eine C₂-C₄-Alcenylgruppe, substituiert oder nicht,
eine C₂-C₄-Alcynylgruppe, substituiert oder nicht, insbesondere durch ein Trimethylsilyl, ein tert-Butyl, ein Hydroxy-2-propyl oder ein Isopropyl,
ein Heteroaryl, substituiert oder nicht, insbesondere ein Pyridin, ein Imidazol, ein Oxazol, ein Triazol, ein Pyrrol, ein Benzofuran, ein Thiophen, ein Benzothiophen, ein Indol,
ein Cyclohexyl, ein Piperazin, ein Morpholin, ein Thiomorpholin, ein Piperidin,
eine Gruppe 4-(NH₂-(CH₂-CH₂O)p)Ph, wobei p eine Ganzzahl von 1 bis 6 ist,
6) R3 darstellt:
H, (C₁-C₆)alkyl, wobei das Alkyl eventuell durch ein oder mehrere Fluore substituiert ist, (C₃-C₆)cycloalkyl, C₁ bis C₆-O-Alkyl, NH₂, eine Propargylgruppe, CH₂CN,
7) R4 und R5 unabhängig voneinander darstellen:
H, OH, OPO₃H₂, OPO-(O-(C₁-C₂)alkyl)₂, wobei das Alkyl eventuell durch ein oder mehrere Fluore substituiert ist, ein C₁-C₆-O-Alkyl, insbesondere OCH₃, wobei das Alkyl eventuell durch ein oder mehrere Fluore substituiert ist, ein C₁-C₆-Alkyl, wobei das Alkyl eventuell durch ein oder mehrere Fluore substituiert ist, ein C₃-C₆-Cycloalkyl, ein Halogen,
wobei R4 und R5 gemeinsam ein (C₁-C₂) dioxyalcenyl darstellen, eventuell substituiert durch ein oder mehrere Fluore,
bei Ausschluss der folgenden Verbindungen:
wenn X = O, R3 = H, und wenn die Bindung zwischen dem Kohlenstoff 1 und der Gruppe X doppelt ist:
a. R1 = NH₂, R2 = 4-Methoxyphenyl, R4 = OCH₃ und R5 = H,
b. R1 = NH₂, R2 = Phenyl, R4 = H und R5 = H,
c. R1 = NO₂, R2 = 4-Methoxyphenyl, R4 = OCH₃ und R5 = H,
d. R1 = NO₂, R2 = 4-Methoxyphenyl, R4 = H und R5 = H,
e. R1 = NH₂, R2 = 4-Methoxyphenyl, R4 = H und R5 = H,
f. R1 = NHCHO, R2 = Phenyl, R4 = OCH₃ und R5 = H,
g. R1 = NH₂, R2 = Phenyl, R4 = OCH₃ und R5 = H,
h. R1 = NH₂, R2 = Phenyl oder 4-OH-phenyl, substituiert oder nicht, R4 = H, OH, ein C₁-C₆-O-Alkyl, ein C₁-C₆-Alkyl, ein C₃-C₆-Cycloalkyl, und R5 = H, OH, ein C₁-C₆-O-Alkyl, ein C₁-C₆-Alkly, ein C₃-C₆-Cycloalkyl,
i. die Verbindungen, in denen eins der R4 und R5 ein Halogen darstellt.

28. Verbindung nach Anspruch 27 der folgenden allgemeinen Formel (I): 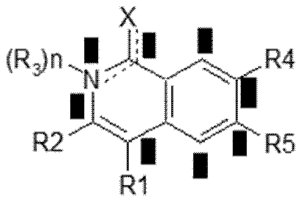 wobei
- n = 0, die Bindung zwischen dem Kohlenstoff 1 und der Gruppe X einfach ist und X OCH₃ ist, und R1 NO₂ ist, oder
- n = 1, die Bindung zwischen dem Kohlenstoff 1 und der Gruppe X doppelt ist, X = 0 oder S und R2 ein Phenyl darstellt, substituiert an Position 4 durch eine Gruppe, die aus OH, OCH₃, O-benzyl, Br, einer C2-C4-Alcynylgruppe, substituiert oder nicht, insbesondere durch ein Trimethylsilyl, ein tert-Butyl, ein Hydroxy-2-propyl oder ein Isopropyl, ausgewählt ist.

29. Verbindung nach Anspruch 27, ausgewählt aus den folgenden Verbindungen:

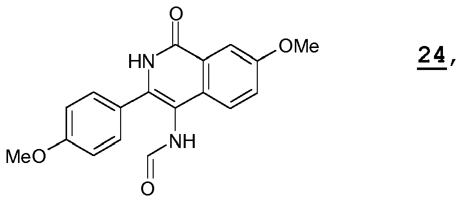

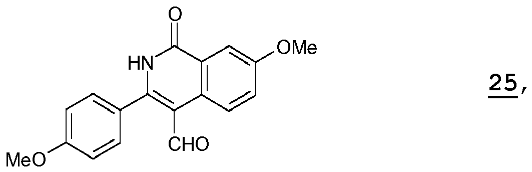

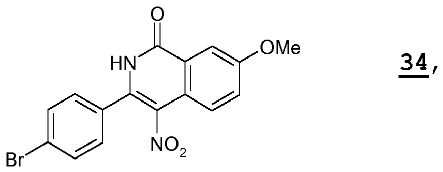

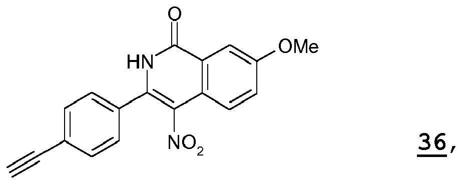

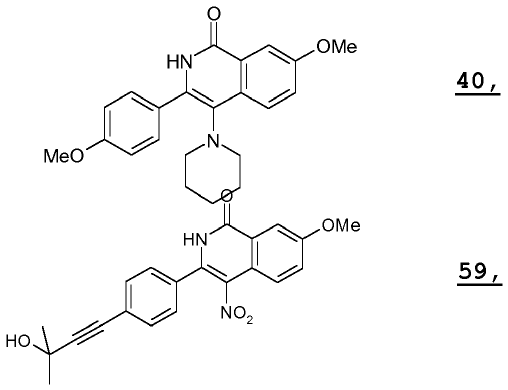

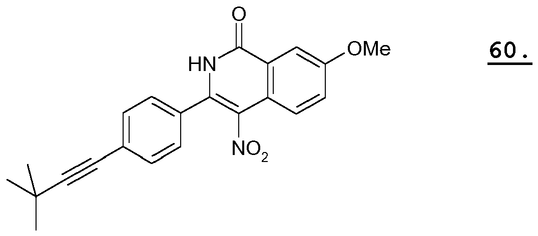

30. Produkt, das eine erste pharmazeutische Zubereitung der Formel (I) nach Anspruch 1 und eine zweite pharmazeutische Zubereitung, die mindestens ein Anti-Tumor-Mittel als kombinierte Zubereitung für eine gleichzeitige, separate oder sequentielle Verwendung bei der Behandlung von Säugetieren, insbesondere des Menschen, umfasst, die von benignen oder malignen (kanzerogenen) Tumoren befallen sind.

31. Produkt nach Anspruch 30, wobei mindestens ein Antitumormittel aus dem Abraxan, dem Abarelix, dem Aldesleukine, dem Alemtuzumab, dem Alitretinoin, dem Allopurinol, dem Altretamin, dem Anastrozol, dem Arsentrioxid, der Asparaginase, dem Azacitidin, dem Bevacizumab, dem Bexaroten, dem Bicalutamid, dem Bleomycin, dem Bortezomib, dem intravenösen Busulfan, dem oralen Busulfan, dem Calusteron, dem Capecitabin, dem Carboplatin, dem Carmustin, dem Cetuximab, dem Chlorambucil, dem Cisplatin, dem Cladribin, dem Clofarabin, dem Cyclophosphamid, dem Cytarabin, dem Dacarbazin, dem Dactinomycin, dem Natriumdalteparin, dem Dasatinib, dem Daunorubicin, dem Decitabin, dem Denileukin, dem Diftitoxdenileukin, dem Dexrazoxan, dem Docetaxel, dem Doxorubicin, dem Dromostanolon-Propionat, dem Eculizumab, dem Epirubicin, dem Erlotinib, dem Estramustin, dem Etoposidphosphat, dem Etoposid, dem Exemestan, dem Fentanylcitrat, dem Filgrastim, dem Floxuridin, dem Fludarabin, dem 5-Fluoruracyl, dem Fulvestrant, dem Gefitinib, dem Gemcitabin, dem Gemtuzumab-Ozogamicin, dem Goserelinacetat, dem Histrelinacetat, dem Ibritumomab-Tiuxetan, dem Idarubicin, dem Ifosfamid, dem Imatinibmesylat, dem Alpha 2a-Interferon, dem Irinotecan, dem Lapatinibditosylat, dem Lenalidomid, dem Letrozol, Leucovorin, dem Leuprolidacetat, dem Levamisol, dem Lomustin, dem Meclorethamin, dem Megestrolacetat, dem Melphalan, dem Mercaptopurin, dem Methotrexat, dem Methoxsalen, dem Mitomycin C, dem Mitotan, Mitoxantron, dem Nandrolon-Phenpropionat, dem Nelarabin, dem Nofetumomab, dem Oxaliplatin, dem Paclitaxel, dem Pamidronat, dem Panitumumab, der Pegaspargase, dem Pegfilgrastim, dem Dinatriumpemetrexed, dem Pentostatin, dem Pipobroman, dem Plicamycin, dem Procarbazin, dem Quinacrin, der Rasburicase, dem Rituximab, dem Sorafenib, dem Streptozocin, dem Sunitinib, dem Sunitinibmaleat, dem Tamoxifen, dem Taxol, dem Taxoter, dem Temozolomid, dem Teniposid, dem Testolacton, dem Thalidomid, dem Thioguanin, dem Thiotepa, dem Topotecan, dem Toremifen, dem Tositumomab, dem Trastuzumab, dem Tretinoin, dem Uracilsenf, dem Valrubicin, dem Vinblastin, dem Vincristin, dem Vinorelbin, dem Vorinostat und dem Zoledronat ausgewählt ist.

## Claims

1. A compound of general formula (I) below: 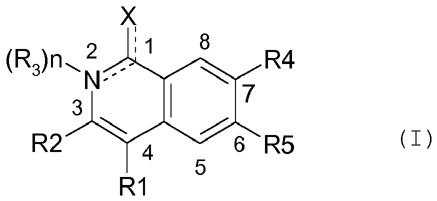 in which:
1) the bond between carbon 1 and the X group is single or double, the bond between nitrogen 2 and carbon 1 can be single or double
it being understood that:
a) when the bond between X and said carbon 1 is double, then the bond between nitrogen 2 and carbon 1 is single:
and,
b) when the bond between X and said carbon 1 is single, then the bond between nitrogen 2 and carbon 1 is double and formula I corresponds to an aromatic system of formula I-A below: 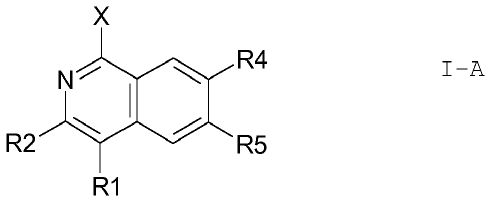
2) n is 0 or 1,
3) X is:
a) when n=1:
O, S,
b) when n=0:
OPO₃H₂, OPO-(O-(C₁-C₂) alkyl)₂, the alkyl being optionally substituted by one or more fluorines, O-(C₁-C₆)alkyl, in particular OCH₃, the alkyl being optionally substituted by one or more fluorines, NH-(C₁-C₆) alkyl, S(C₃-C₆) cycloalkyl,
4) R1 is:
OH, OPO₃H₂, OPO- (O-(C₁-C₂) alkyl)₂, the alkyl being optionally substituted by one or more fluorines,
CF₃, CH₂OR', R' being H or (C₁-C₆) alkyl, the alkyl being optionally substituted by one or more fluorines, CHO,
CONR_{c}R_{d}, R_{c} and R_{d} being independently of each other: H, a C₁-C₆ alkyl, and NR_{c}R_{d} is an amino acid bound by its amine function, in particular a serine or a threonine, a C₃-C₆ cycloalkyl, or R_{c} and R_{d} are together a C₂-C₆ alkyl,
NO₂, N₃, ≡, CN, C (=N)NH₂, SO₃H, SO₂NH₂, SO₂NHCH₃, NHSO₂CH₃, CH₂SO₂NR_{c}R_{d}, CH₂NHSO₂R_{c}, _{f} SO₂-NRₐR_{b}, SO2-imidazole, NRₐR_{b}, where:
Rₐ and R_{b} are independently of each other: H, a C1-C6 alkyl, a C₃-C₆ cycloalkyl, or
Ra is H, a C₁-C₆ alkyl, a C₃-C₆ cycloalkyl, and Rb = CORₜ, COOR_{f} or CONR_{f}R_{f'}, R_{f} and R_{f} being H, a C₁-C₆ alkyl, a C₃-C₆ cycloalkyl or an amino acid chain (such as CH(CH₂OH)NH₂ for serine) Rₐ and R_{b} are together a C₂-C₆ alkyl,
Rₐ and R_{b} can form a C₅ to C₇ ring, in particular a pyrrolidine, a piperazine, a morpholine, a thiomorpholine,
a heteroaryl, substituted or not, in particular a pyridine, an imidazole, an oxazole, a triazole, a pyrrole, a tetrazole.
5) R2 is:
a phenyl substituted or not by one to three substituents chosen from:
a halogen, an OH, NHRa,
ORe, Re being a benzyl, a methylene triazole, substituted or not, in particular by a C₁-C₆ alkyl,
OPO₃H₂, OPO-(O-(C₁-C₂) alkyl)₂, the alkyl being optionally substituted by one or more fluorines, an NH2,
an NH-CORc group in which Rc is H, a C₁-C₆ alkyl, a C₃-C₆ cycloalkyl or an amino acid chain (such as CH(CH₂OH)NH₂ for serine),
an O-(C₁-C₆)alkyl, in particular OCH₃, the alkyl being optionally substituted by one or more fluorines,
an O-COR₁ where R₁ is O-(C₁-C₆)alkyl, or NRiRii, Ri and Rii being able to be C₁-C₆ alkyl,
a C₂-C₄ alkyl group, the alkyl group being optionally substituted by one or more fluorines, a C₂-C₄ alkenyl group, substituted or not,
a C₂-C₄ alkynyl group, substituted or not, in particular by a trimethylsilyl, a tert-butyl, a hydroxy-2-propyl or an isopropyl,
a heteroaryl, substituted or not, in particular a pyridine, an imidazole, an oxazole, a triazole, a pyrrole, a benzofuran, a thiophene, a benzothiophene, an indole, a cyclohexyl, a piperazine, a morpholine, a thiomorpholine, a piperidine,
a 4- (NH₂-(CH₂-CH₂O)ₚ) Ph group in which p is an integer from 1 to 6
6) R3 is:
H, (C₁-C₆)alkyl, the alkyl being optionally substituted by one or more fluorines, (C₃-C₆) cycloalkyl, O-(C₁-C₆) alkyl, NH₂, a propargyl group, CH₂CN,
7) R4 and R5 are independently of each other:
H, OH, OPO₃H₂, OPO- (O-(C₁-C₂) alkyl)₂, the alkyl being optionally substituted by one or more fluorines, an O-(C₁-C₆) alkyl, in particular OCH₃, the alkyl being optionally substituted by one or more fluorines, a C₁-C₆ alkyl, the alkyl being optionally substituted by one or more fluorines, a C₃-C₆ cycloalkyl, a halogen,
R4 and R5 are together a (C₁-C₂) alkenyl dioxy optionally substituted by one or more fluorines,
and the pharmaceutically acceptable salts,
for use in mammals as vascular-disrupting agent for the prevention and treatment of pathological angiogenesis, selected from retinopathies and benign or malignant (cancerous) tumors.

2. The compound according to claim 1, for use in mammals as a protein phosphatase 1 inhibitor, vascular-disrupting agent and antiproliferative agent for the prevention and treatment of pathological angiogenesis, selected from retinopathies and benign or malignant (cancerous) tumors.

3. The compound according to claim 1, for use in mammals as a tubulin polymerization inhibitor, vascular-disrupting agent and antiproliferative agent for the prevention and treatment of pathological angiogenesis, selected from retinopathies and benign or malignant (cancerous) tumors.

4. The compound according to claim 1, for use in mammals as a protein phosphatase 1, tubulin polymerization inhibitor, vascular-disrupting agent and antiproliferative agent for the prevention and treatment of pathological angiogenesis, selected from retinopathies and benign or malignant (cancerous) tumors.

5. The compound for use according to one of claims 2 to 4, wherein the R1 group of general formula (I) is selected from NO₂ or pyrrole.

6. The compound for use according to one of claims 2 to 4, in which the R2 group of general formula (I) is a phenyl substituted in the para position by a group chosen from ORe, Re being as defined in claim 1, a halogen, a C₂-C₄ alkyne group, substituted or not, in particular by a trimethylsilyl, a tert-butyl, a hydroxy-2-propyl or an isopropyl.

7. The compound for use according to one of claims 1 to 4, in which the R1 group of general formula (I) is chosen from NO₂ or pyrrole and the R2 group of general formula (I) is a phenyl substituted in the para position by a group chosen from ORe, Re being as defined in claim 1, a halogen, a C₂-C₄ alkyne group, substituted or not, in particular by a trimethylsilyl, a tert-butyl, a hydroxy-2-propyl or an isopropyl.

8. The compound for use according to claim 7, in which the R3 group of general formula (I) is H.

9. The compound for use according to claim 1, in which the compounds of formula (I) are chosen from the following:

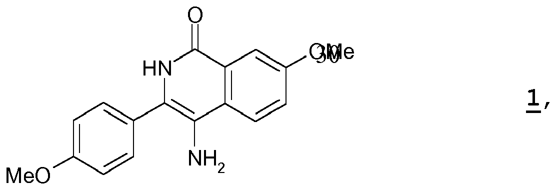

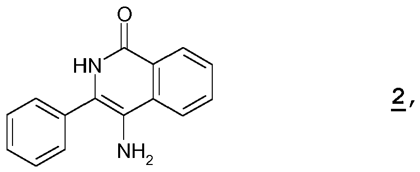

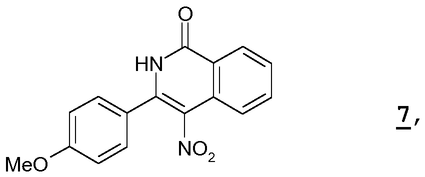

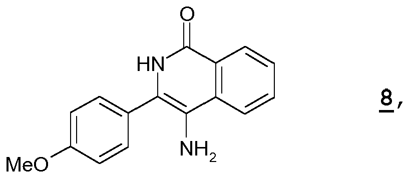

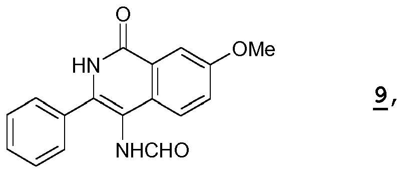

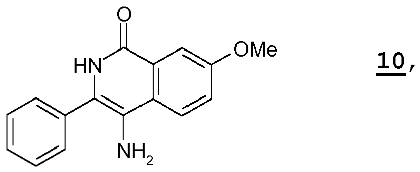

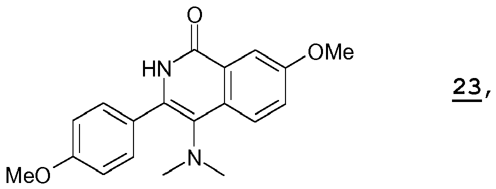

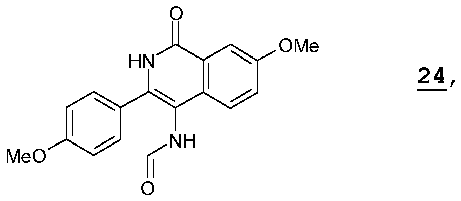

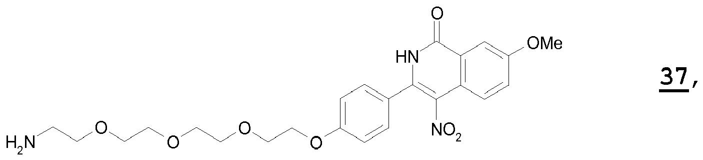

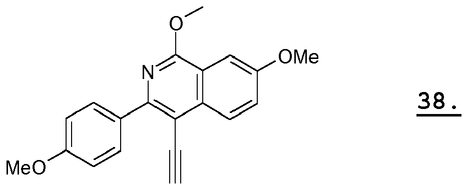

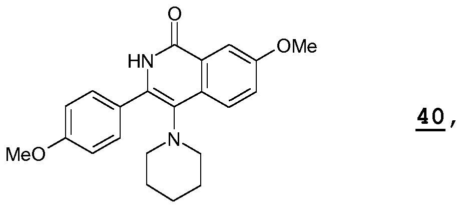

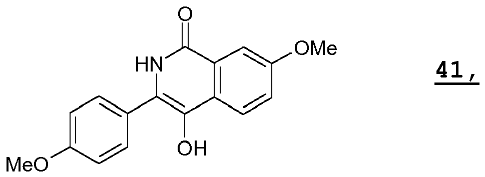

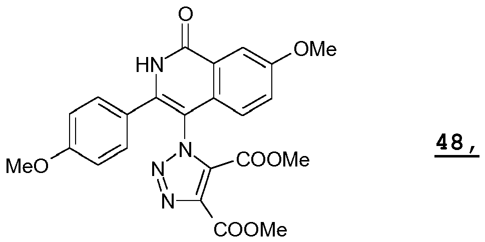

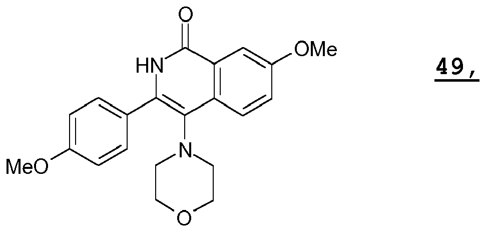

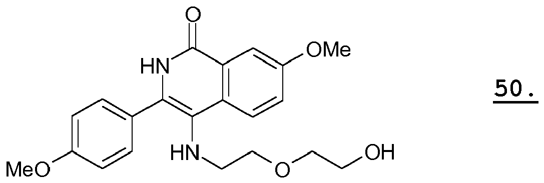

10. The compound for use according to one of claims 1 to 8, in which the compounds of formula (I) are chosen from the following:

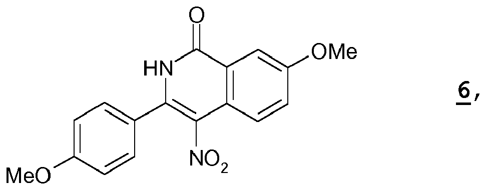

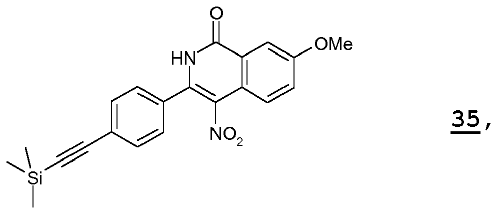

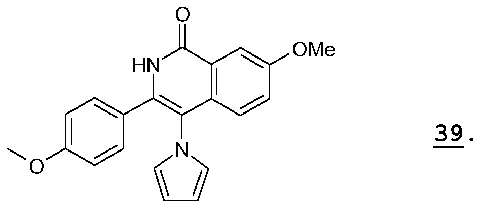

11. The compound for use according to one of claims 1 to 8, in which the compounds of formula (I) are chosen from the following:

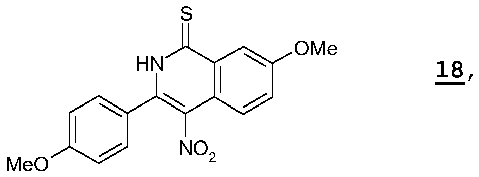

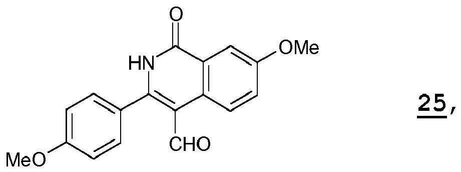

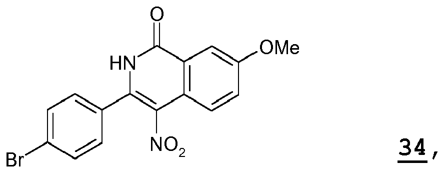

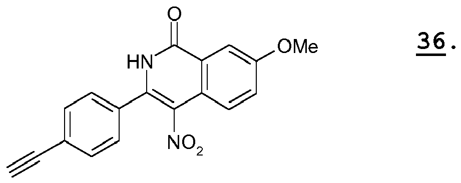

12. The compound for use according to one of claims 1 to 8, in which the compounds of formula (I) are chosen from the following:

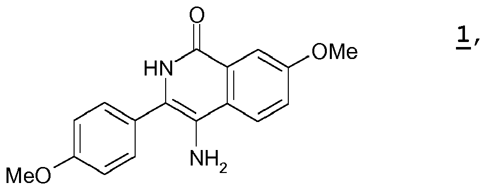

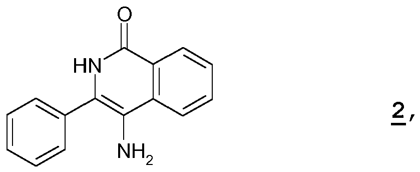

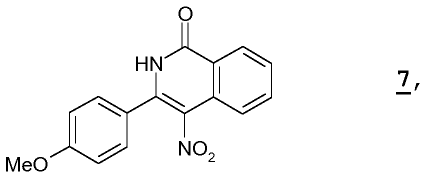

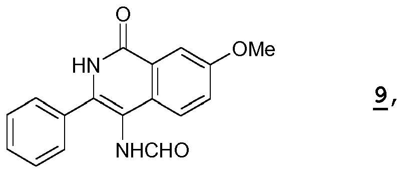

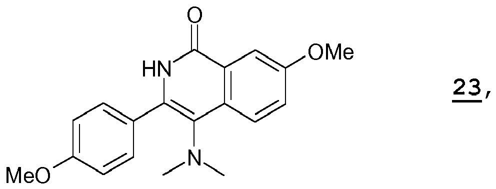

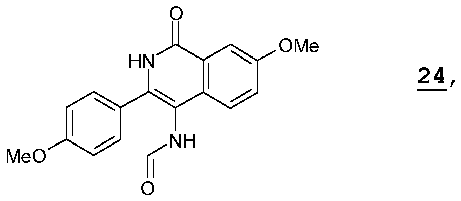

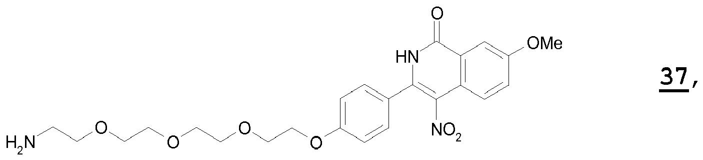

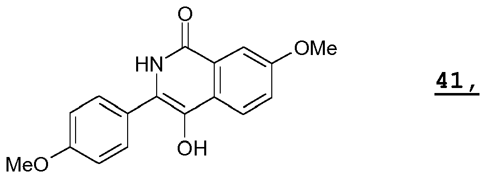

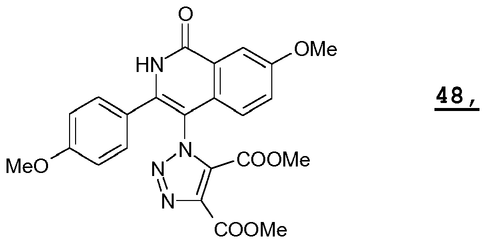

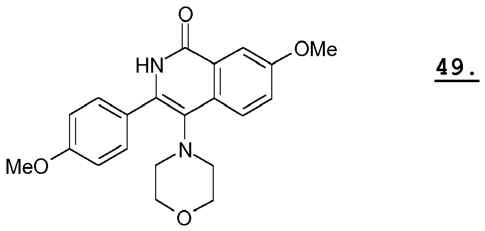

13. The compound for use according to one of claims 1 to 8, in which the compounds of formula (I) are chosen from the following:

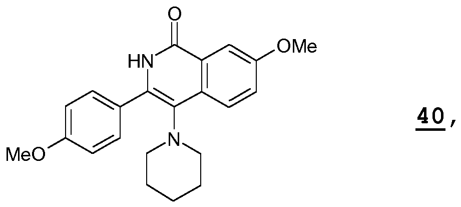

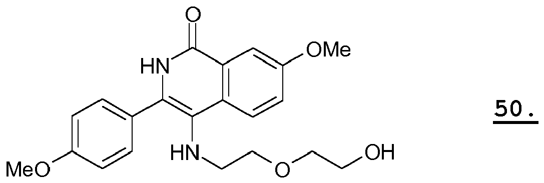

14. The compound for use according to one of claims 1 to 13, administered by oral route, at a dose comprised of from about 1 mg/kg to about 200 mg/kg, preferably from about 10 mg/kg to about 100 mg/kg, more preferably from 20 mg/kg to 50 mg/kg, in particular 40 mg/kg.

15. The compound for use according to one of claims 1 to 13, administered by parenteral, intravenous route at a dose comprised of from about 0.1 mg/kg/d to about 100 mg/kg/d, in particular 0.1 mg/kg to 50 mg/kg/d, in particular 10 mg/kg/d.

16. The compound for use according to one of claims 1 to 15, in combination with an antitumor.

17. The compound for use according to claim 16, wherein said antitumor is chosen from the following:
abraxane, abarelix, aldesleukin, alemtuzumab, alitretinoin, allopurinol, altretamine, anastrozole, arsenic trioxide, asparaginase, azacitidine, bevacizumab, bexarotene, bicalutamide, bleomycin, bortezomib, intravenous busulphan, oral busulphan, calusterone, capecitabine, carboplatin, carmustine, cetuximab, chlorambucil, cisplatin, cladribine, clofarabine, cyclophosphamide, cytarabine, dacarbazine, dactinomycin, dalteparin sodium, dasatinib, daunorubicin, decitabine, denileukin, denileukin diftitox, dexrazoxane, docetaxel, doxorubicin, dromostanolone propionate, eculizumab, epirubicin, erlotinib, estramustine, etoposide phosphate, etoposide, exemestane, fentanyl citrate, filgrastim, floxuridine, fludarabine, 5-fluorouracil, fulvestrant, gefitinib, gemcitabine, gemtuzumab ozogamicin, goserelin acetate, histrelin acetate, ibritumomab tiuxetan, idarubicin, ifosfamide, imatinib mesylate, interferon alfa 2a, irinotecan, lapatinib ditosylate, lenalidomide, letrozole, leucovorin, leuprolide acetate, levamisole, lomustine, mechlorethamine, megestrol acetate, melphalan, mercaptopurine, methotrexate, methoxsalen, mitomycin C, mitotane, mitoxantrone, nandrolone phenpropionate, nelarabine, nofetumomab, oxaliplatin, paclitaxel, pamidronate, panitumumab, pegaspargase, pegfilgrastim, pemetrexed disodium, pentostatin, pipobroman, plicamycin, procarbazine, quinacrine, rasburicase, rituximab, sorafenib, streptozocin, sunitinib, sunitinib maleate, tamoxifen, taxol, taxotere, temozolomide, teniposide, testolactone, thalidomide, thioguanine, thiotepa, topotecan, toremifene, tositumomab, trastuzumab, tretinoin, uracil mustard, valrubicin, vinblastine, vincristine, vinorelbine, vorinostat, and zoledronate.

18. A pharmaceutical composition comprising as active ingredient a compound of formula I, as defined in claim 1, in combination with a pharmaceutically acceptable vehicle for use in mammals for the prevention and treatment of pathological angiogenesis, selected from retinopathies and benign or malignant (cancerous) tumors.

19. The pharmaceutical composition according to claim 1, comprising as active ingredient a compound of formula I, in which:
- n=0, the bond between carbon 1 and the X group is single and X is chosen from OCH₃. or
- n=1, the bond between carbon 1 and the X group is double, X = 0 or S and R2 is a phenyl substituted in position 4 by a group chosen from OH, OCH₃. O-benzyl, Br, a C₂-C₄ alkynyl group substituted or not in particular by a trimethylsilyl, a tert-butyl, a hydroxy-2-propyl or an isopropyl.

20. The pharmaceutical composition according to claim 18, comprising as active ingredient a compound, as defined in claim 9, in combination with a pharmaceutically acceptable vehicle.

21. The pharmaceutical composition according to claim 18, comprising as active ingredient a compound, as defined in claim 10.

22. The pharmaceutical composition according to claim 18, comprising as active ingredient a compound, as defined in claim 11.

23. The pharmaceutical composition according to claim 18, comprising as active ingredient a compound, as defined in claim 12.

24. The pharmaceutical composition according to claim 18, comprising as active ingredient a compound, as defined in claim 13.

25. The pharmaceutical composition according to one of claims 18 to 24, administrable by oral route at a dose comprised of from about 1 mg/kg to about 200 mg/kg, preferably from about 10 mg/kg to about 100 mg/kg, more preferably from 20 mg/kg to 50 mg/kg, in particular 40 mg/kg.

26. The pharmaceutical composition according to one of claims 18 to 24, administrable by intravenous route at a dose comprised of from about 0.1 mg/kg/d to about 100 mg/kg/d, in particular 50 mg/kg/d, in particular 10 mg/kg/d.

27. A compound of general formula (I) below: 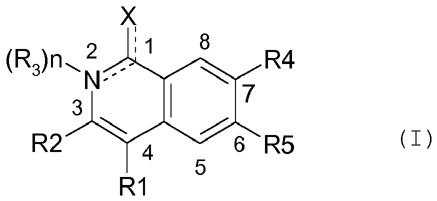 in which:
1) the bond between carbon 1 and the X group is single or double, the bond between nitrogen 2 and carbon 1 can be single or double
it being understood that:
a. when the bond between X and said carbon 1 is double, then the bond between nitrogen 2 and carbon 1 is single:
and,
b. when the bond between X and said carbon 1 is single, then the bond between nitrogen 2 and carbon 1 is double and formula I corresponds to an aromatic system of formula I-A below: 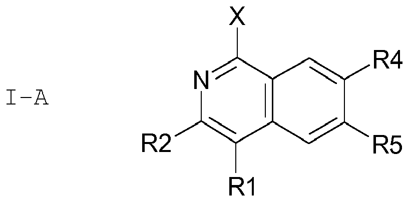
2) n is 0 or 1,
3) X is:
a) when n=1:
O, S,
b) when n=0:
OPO₃H₂, OPO-(O-(C₁-C₂)alkyl)₂, the alkyl being optionally substituted by one or more fluorines, O-(C₁-C₆) alkyl, in particular OCH₃, the alkyl being optionally substituted by one or more fluorines, NH-(C₁-C₆) alkyl, S(C₃-C₆)cycloalkyl,
4) R1 is:
OPO₃H₂, OPO-(O-(C₁-C₂)alkyl)₂, the alkyl being optionally substituted by one or more fluorines,
CF₃, CH₂OR', R' being H or (C₁-C₆) alkyl, the alkyl being optionally substituted by one or more fluorines, CHO,
CONR_{c}R_{d}, R_{c} and R_{d} being independently of each other: H, a C₁-C₆ alkyl, and NR_{c}R_{d} is an amino acid bound by its amine function, in particular a serine or a threonine, a C₃-C₆ cycloalkyl, or R_{c} and R_{d} are together a C₂-C₆ alkyl,
NO₂, N₃, ≡, C(=N)NH₂, SO₃H, SO₂NH₂, SO₂NHCH₃, NHSO₂CH₃, CH₂SO₂NR_{c}R_{d}, CH₂NHSO₂R_{c}, SO₂-NRₐR_{b}, SO₂-imidazole, NRₐR_{b}, where:
Rₐ and R_{b} are independently of each other: H, CHO, a C₁-C₆ alkyl, a C₃-C₆ cycloalkyl, an amino acid bound by its acid function, in particular a serine, or
Rₐ is H, a C₁-C₆ alkyl, a C₃-C₆ cycloalkyl, and Rb = COR_{f}, COOR_{f} or CONR_{f}R_{f'}, R_{f} and R_{f} being H, a C₃-C₆ cycloalkyl or an amino acid chain (such as CH(CH₂OH)NH₂ for serine), or
Rₐ and R_{b} are together a C₂-C₆ alkyl,
Rₐ and R_{b} can form a C₅ to C₇ ring, in particular a pyrrolidine, a piperazine, a morpholine, a thiomorpholine,
a heteroaryl, substituted or not, in particular a pyridine, an imidazole, an oxazole, a triazole, a pyrrole, a tetrazole,
5) R2 is:
a phenyl substituted or not by one to three substituents chosen from:
a halogen, an OH, NHRa,
ORe, Re being a benzyl, a methylene triazole, substituted or not, in particular by a C₁-C₆ alkyl,
OPO₃H₂, OPO-(O-(C₁-C₂) alkyl)₂, the alkyl being optionally substituted by one or more fluorines, an NH₂,
an NH-CORc group in which Rc is H, a C₁-C₆ alkyl, a C₃-C₆ cycloalkyl or an amino acid chain (such as CH(CH₂OH)NH₂ for serine),
an O-(C₁-C₆) alkyl, in particular OCH₃, the alkyl being optionally substituted by one or more fluorines,
an NH-Rc group in which Rc is an amino acid bound by its acid function, in particular a serine,
an O-COR₁ where R₁ is O-(C₁-C₆)alkyl, or NRiRii, Ri and Rii being able to be C₁-C₆ alkyl,
a C₂-C₄ alkyl group, the alkyl group being optionally substituted by one or more fluorines, a C₂-C₄ alkenyl group, substituted or not,
a C₂-C₄ alkynyl group, substituted or not, in particular by a trimethylsilyl, a tert-butyl, a hydroxy-2-propyl or an isopropyl,
a heteroaryl, substituted or not, in particular a pyridine, an imidazole, an oxazole, a triazole, a pyrrole, a benzofuran, a thiophene, a benzothiophene, an indole, a cyclohexyl, a piperazine, a morpholine, a thiomorpholine, a piperidine,
a 4-(NH₂-(CH₂-CH₂O)p)Ph group in which p is an integer from 1 to 6
6) R3 is:
H, (C₁-C₆)alkyl, the alkyl being optionally substituted by one or more fluorines, (C₃-C₆) cycloalkyl, O-(C₁-C₆) alkyl, NH₂, a propargyl group, CH₂CN,
7) R4 and R5 are independently of each other:
H, OH, OPO₃H₂, OPO-(O-(C₁-C₂) alkyl)₂, the alkyl being optionally substituted by one or more fluorines, an O-(C₁-C₆)alkyl, in particular OCH₃, the alkyl being optionally substituted by one or more fluorines, a C₁-C₆ alkyl, the alkyl being optionally substituted by one or more fluorines, a C₃-C₆ cycloalkyl, a halogen,
R4 and R5 are together a (C₁-C₂) alkenyl dioxy optionally substituted by one or more fluorines,
with the exclusion of the following compounds:
when X=O, R3=H, and the bond between carbon 1 and the X group is double:
a. R1=NH₂, R2=4-methoxy-phenyl, R4=OCH₃ and R5=H,
b. R1=NH₂, R2=phenyl, R4=H and R5=H,
c. R1=NO₂, R2=4-methoxy-phenyl, R4=OCH₃ and R5=H,
d. R1=NO₂, R2=4-methoxy-phenyl, R4=H and R5=H,
e. R1=NH₂, R2=4-methoxy-phenyl, R4=H and R5=H,
f. R1=NHCHO, R2=phenyl, R4=OCH₃ and R5=H,
g. R1=NH₂, R2=phenyl, R4=OCH₃ and R5=H,
h. R1=NH₂, R2=phenyl or 4-OH-phenyl, substituted or not, R4=H, OH, an O-(C₁-C₆)alkyl, a C₁-C₆ alkyl, a C₃-C₆ cycloalkyl, and R5=H, OH, an 0-(C₁-C₆) alkyl, a C₁-C₆ alkyl, a C₃-C₆ cycloalkyl,
i. the compounds in which one of R4 and R5 is a halogen.

28. The compound according to claim 27, of general formula (I): 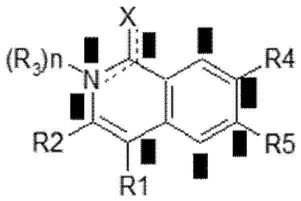 in which:
- n=0, the bond between carbon 1 and the X group is single and X is OCH₃, and R1 is NO₂, or
- n=1, the bond between carbon 1 and the X group is double, X = 0 or S and R2 is a phenyl substituted in position 4 by a group chosen from OH, OCH₃, O-benzyl, Br, a C₂-C₄ alkynyl group substituted or not in particular by a trimethylsilyl, a tert-butyl, a hydroxy-2-propyl or an isopropyl.

29. The compound according to claim 27, chosen from the following:

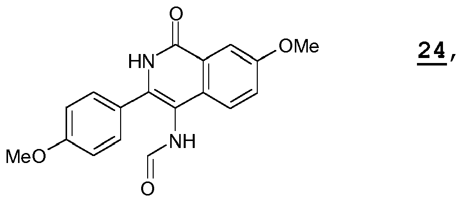

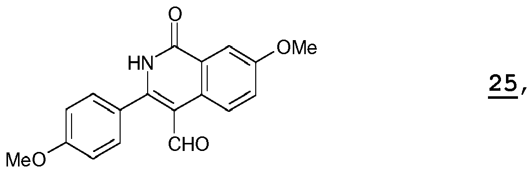

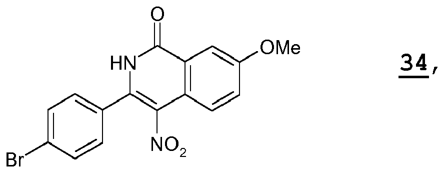

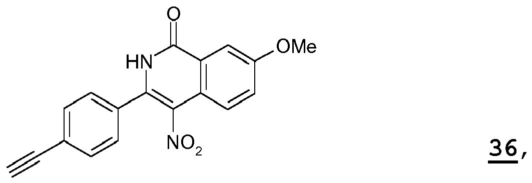

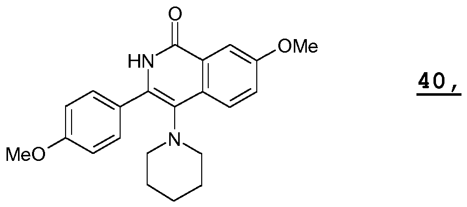

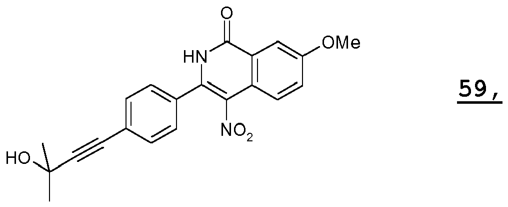

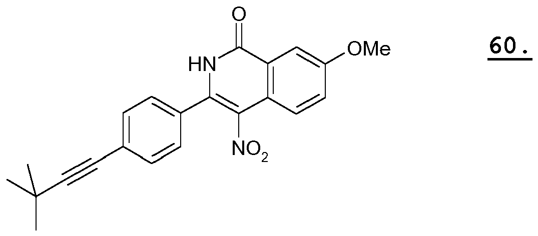

30. A product comprising a first pharmaceutical preparation of formula (I) according to claim 1, and a second pharmaceutical preparation comprising at least one antitumor as combined preparation for a simultaneous, separate or sequential use in the treatment of mammals, in particular humans, suffering from benign or malignant (cancerous) tumors.

31. The product according to claim 30 in which at least an antitumor is selected from:
abraxane, abarelix, aldesleukin, alemtuzumab, alitretinoin, allopurinol, altretamine, anastrozole, arsenic trioxide, asparaginase, azacitidine, bevacizumab, bexarotene, bicalutamide, bleomycin, bortezomib, intravenous busulphan, oral busulphan, calusterone, capecitabine, carboplatin, carmustine, cetuximab, chlorambucil, cisplatin, cladribine, clofarabine, cyclophosphamide, cytarabine, dacarbazine, dactinomycin, dalteparin sodium, dasatinib, daunorubicin, decitabine, denileukin, denileukin diftitox, dexrazoxane, docetaxel, doxorubicin, dromostanolone propionate, eculizumab, epirubicin, erlotinib, estramustine, etoposide phosphate, etoposide, exemestane, fentanyl citrate, filgrastim, floxuridine, fludarabine, 5-fluorouracil, fulvestrant, gefitinib, gemcitabine, gemtuzumab ozogamicin, goserelin acetate, histrelin acetate, ibritumomab tiuxetan, idarubicin, ifosfamide, imatinib mesylate, interferon alfa 2a, irinotecan, lapatinib ditosylate, lenalidomide, letrozole, leucovorin, leuprolide acetate, levamisole, lomustine, mechlorethamine, megestrol acetate, melphalan, mercaptopurine, methotrexate, methoxsalen, mitomycin C, mitotane, mitoxantrone, nandrolone phenpropionate, nelarabine, nofetumomab, oxaliplatin, paclitaxel, pamidronate, panitumumab, pegaspargase, pegfilgrastim, pemetrexed disodium, pentostatin, pipobroman, plicamycin, procarbazine, quinacrine, rasburicase, rituximab, sorafenib, streptozocin, sunitinib, sunitinib maleate, tamoxifen, taxol, taxotere, temozolomide, teniposide, testolactone, thalidomide, thioguanine, thiotepa, topotecan, toremifene, tositumomab, trastuzumab, tretinoin, uracil mustard, valrubicin, vinblastine, vincristine, vinorelbine, vorinostat, and zoledronate.
